# EUROPEAN PATENT APPLICATION

(11) **EP 4 349 818 A1**
(43) Date of publication of application: **10.04.2024**
(21) Application number: 22816423.2
(22) Date of filing: 31.05.2022
(51) Int. Cl.: C07D 263/32, A61K 31/421, A61P 37/08, A61P 11/06, A61P 17/00, C07D 413/12

(54) **NOVEL OXAZOLE DERIVATIVE AND PHARMACEUTICAL COMPOSITION CONTAINING SAME FOR PREVENTION OR TREATMENT OF ALLERGIC DISEASE**

(30) Priority: 01.06.2021 KR 20210071013
(71) Applicant: Azcuris Co., Ltd., Sejong 30019 (KR)
(72) Inventor: BYUN, Young Joo, Daejeon 34049 (KR); JEON, Young Ho, Sejong 30098 (KR); JANG, Geon Hee, Siheung-si, Gyeonggi-do 14923 (KR); LIM, Tae Hyeong, Sejong 30019 (KR); SON, Sang Hyun, Daejeon 34020 (KR); KIM, Eun Ji, Hwasun-gun, Jeollanam-do 58144 (KR); KIM, Ho Soon, Daejeon 34959 (KR)
(74) Representative: Graf von Stosch Patentanwaltsgesellschaft mbH
(86) International application number: PCT/KR2022/007712
(87) International publication number: WO 2022/255764

(57) **Abstract**

The present invention relates to a novel oxazole derivative, a preparation method therefor, and a pharmaceutical composition comprising same as an active ingredient for preventing or treating an allergic disease such as asthma or atopy. The novel oxazole derivative of the present invention exhibits an excellent inhibitory effect on the intracellular signaling of IL-33 and thus can be advantageously used as a pharmaceutical composition for prevention or treatment of an allergic disease such as asthma or atopy.

## Description

### FIELD

The present invention relates to novel oxazole derivatives, and more specifically, to novel oxazole derivatives exhibiting preventive or therapeutic effects in allergic diseases.

### BACKGROUND

Bronchodilators or anti-inflammatory drugs used to treat allergic inflammatory diseases are mainly used as a symptomatic therapy, which can be temporarily effective in relieving symptoms, but have the disadvantage of not being able to control the underlying stage of the allergic disease and thus not treating the disease fundamentally. Environmental diseases such as bronchial asthma, atopic dermatitis, and allergic rhinitis are known as immune diseases, and it is well known that Th2 cells play a pivotal role in triggering allergic reactions. When CD4 T cells are stimulated by antigens in lymphocytes, they can differentiate into various types of Th cells depending on the cytokines they recognize at the same time, and if the cytokines they recognize are type 2 cytokines such as thymic stromal lymphoprotein (TSLP) or IL-4, these cells differentiate into Th2 and cause an allergic reaction.

Interleukin-33 (IL-33) is an innate cytokine produced primarily by mucosal epithelial cells in response to a variety of external stimuli and is known to play an important role in regulating immune responses, primarily Th2 cell-mediated allergic responses such as asthma. The IL-33 receptor complex for IL-33-mediated signal transduction consists of the ligand IL-33, the ligand binding receptor ST2 (IL-1 R4), and the signal transducer IL-1 receptor accessory protein (IL-1RAcP; IL-1R3). Stimulation of IL-33 results in the production of Th2 inflammatory cytokines and chemokines, including IL-4, IL-5, IL-6, IL-13 and IL-8. Upon IL-33 binding, the IL-33 receptor complex activates molecules in the downstream signaling system such as NF-kB and AP-1 via IRAK (IL-1 receptor-associated kinase), TRAF6 (TNF receptor associated factor 6), and/or MAPKs. Taken together, TSLP and IL-33 are cytokines that play an important role in the differentiation of Th2 cells, and controlling them is expected to provide a fundamental treatment for allergic diseases.

### OBJECT OF THE INVENTION

The problem to be addressed by the present invention is to provide a novel oxazole derivative that can be used as a treatment for allergic diseases such as asthma or atopy.

Further, the problem to be addressed by the present invention is to provide methods for the preparation of the novel oxazole derivatives.

Further, the problem to be addressed by the present invention is to provide a pharmaceutical composition for preventing or treating a disease associated with IL-33, comprising the novel oxazole derivative as an active ingredient.

Further, the problem to be addressed by the present invention is to provide a pharmaceutical composition for preventing or treating an allergic disease, comprising the novel oxazole derivative as an active ingredient.

Further, the problem to be addressed by the present invention is to provide a pharmaceutical composition for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease, comprising the novel oxazole derivative as an active ingredient.

Further, the problem to be addressed by the present invention is to provide a method for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease, comprising: administering the novel oxazole derivative to a subject in need thereof.

Further, the problem to be addressed by the present invention is to provide a use of the novel oxazole derivative in manufacture of a medicament for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease.

Further, the problem to be addressed by the present invention is to provide a food composition for improving symptoms of allergic diseases, comprising the novel oxazole derivative.

The problem to be addressed by the present invention is not limited to the problems mentioned above, and other technical problems not mentioned can be clearly understood by those skilled in the art from the description below.

### SUMMARY

To address the problem above, according to one aspect of the present invention, there is provided an oxazole derivative compound represented by the following Formula I, a hydrate thereof, a solvate thereof or a pharmaceutically acceptable salt thereof, wherein: 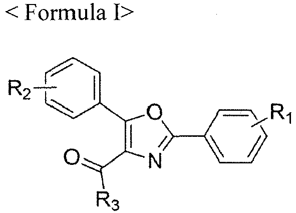
R₁ is hydrogen, C₁-C₆ straight or branched alkyl, C₃-C₆ cycloalkyl, C₁-C₄ alkoxy, halogen, C₁-C₄ haloalkyl, C₁-C₄ haloalkoxy, C₁-C₄ alkylthio, hydroxy, cyano, nitro, NRₐR_{b}, or C₅-C₁₂ aryl of 1-2 ring(s),
R₁ is singular or plural substituent, each of which is independent when existing plurally,
R₂ is hydrogen, C₁-C₄ alkoxy, C₁-C₄ haloalkyl, C₁-C₄ haloalkoxy, C₁-C₄ alkylthio, C₁-C₄ alkylsulfinyl, C₁-C₄ alkylsulfonyl, acetyl, hydroxy, cyano, nitro, NRₐR_{b}, or 4- to 7-membered heteroaryl having 1 to 3 of N,
R₂ is singular or plural substituent, each of which is independent when existing plurally,
R₃ is X substituted with Y or not,
X is C₁-C₄ alkoxy, amino(-NH-), C₅-C₇ aryl, or 5- to 7-membered non-aromatic heterocycle having 1 to 2 heteroatom(s) selected from the group consisting of N and O,
Y is hydrogen, C₁-C₆ straight or branched alkyl, C₃-C₆ cycloalkyl, hydroxy, COO, COO-alkyl, C₅-C₇ aryl, alkylaryl, or 5- to 7-membered aromatic or non-aromatic heterocycle having 1 to 2 of N,
Y is substituted with Z or not,
Z is C₁-C₆ straight or branched alkyl, C₁-C₄ alkoxy, hydroxy, C₁-C₄ haloalkyl, acetyl, guanidino, NRₐR_{b}, C₅-C₇ aryl, alkoxyaryl, 5- to 7-membered aromatic or non-aromatic heterocycle having 1 to 2 heteroatom(s) selected from the group consisting of N and O,
wherein Rₐ and R_{b} 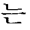 independently hydrogen, C₁-C₄ straight or branched alkyl, acetyl, C₁-C₄ alkylsulfonyl or C₁-C₆ alkoxycarbonyl,
Z is substituted with Q or not,
Q is C₁-C₆ straight or branched alkyl, hydroxy, halogen, acetyl, nitro, C₁-C₄ alkylsulfonyl, or C₁-C₆ alkoxycarbonyl.

According to another aspect of the present invention, there is provided a pharmaceutical composition for preventing or treating a disease associated with IL-33, comprising the oxazole derivative compound represented by the above Formula I, a hydrate thereof, a solvate thereof or a pharmaceutically acceptable salt thereof as an active ingredient.

According to another aspect of the present invention, there is provided a pharmaceutical composition for preventing or treating an allergic disease, comprising the oxazole derivative compound represented by the above Formula I, a hydrate thereof, a solvate thereof or a pharmaceutically acceptable salt thereof as an active ingredient.

According to another aspect of the present invention, there is provided a pharmaceutical composition for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease, comprising the oxazole derivative compound represented by the above Formula I, a hydrate thereof, a solvate thereof or a pharmaceutically acceptable salt thereof as an active ingredient.

According to another aspect of the present invention, there is provided a method for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease, comprising: administering the oxazole derivative compound represented by the above Formula I, a hydrate thereof, a solvate thereof or a pharmaceutically acceptable salt thereof, to a subject in need thereof.

According to another aspect of the present invention, there is provided a use of the oxazole derivative compound represented by the above Formula I, a hydrate thereof, a solvate thereof or a pharmaceutically acceptable salt thereof in manufacture of a medicament for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease.

According to another aspect of the present invention, there is provided a food composition for improving symptoms of allergic diseases, comprising the oxazole derivative compound represented by the above Formula I, a hydrate thereof, a solvate thereof or a pharmaceutically acceptable salt thereof.

According to the present invention, it was found that the novel oxazole derivatives provided in one aspect of the present invention exhibit excellent inhibitory effects on the intracellular signaling of IL-33, and thus can be advantageously used as a pharmaceutical composition for preventing or treating an allergic disease such as asthma or atopy.

The effect of the present invention is not limited to those described above, but should be understood to include all effects that can be inferred from the composition of the invention described in the detailed description or claims of the present invention.

### BRIEF DESCRIPTION OF THE DRAWINGS

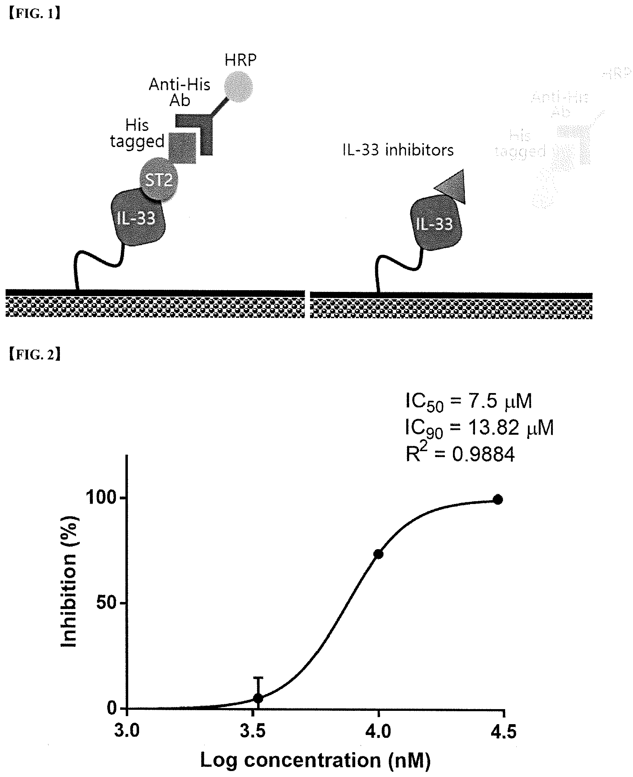

FIG. 1 shows an overview of an ELISA assay determining inhibition of IL-33 and IL-33 receptor (ST-2) binding.
FIG. 2 is a dose-response curve determining the IC₅₀ value of Compound 294.

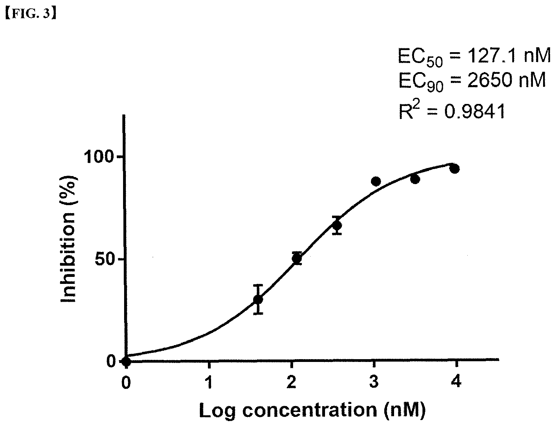

FIG. 3 is a dose-response curve of the EC₅₀ value of Compound 294 in a mast cell line.

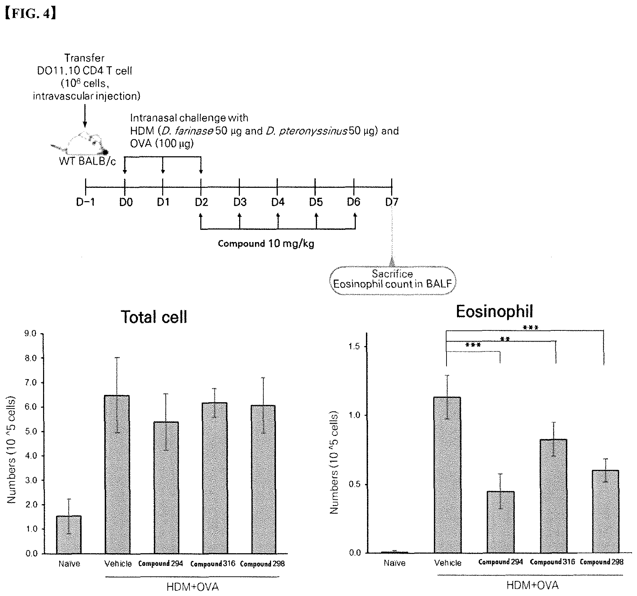

FIG. 4 shows the anti-asthmatic activity (eosinophil reducing effect) of the Compounds of the invention in HDM- and ovalbumin-induced asthma animal models.

### DETAILED DESCRIPTION OF THE INVENTION

The present invention provides an oxazole derivative compound represented by the following Formula I, a hydrate thereof, a solvate thereof or a pharmaceutically acceptable salt thereof, wherein: 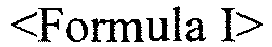 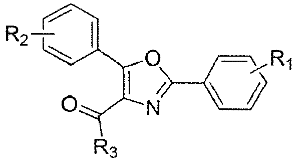
R₁ is hydrogen, C₁-C₆ straight or branched alkyl, C₃-C₆ cycloalkyl, C₁-C₄ alkoxy, halogen, C₁-C₄ haloalkyl, C₁-C₄ haloalkoxy, C₁-C₄ alkylthio, hydroxy, cyano, nitro, NRₐR_{b}, or C₅-C₁₂ aryl of 1-2 ring(s),
R₁ is singular or plural substituent, each of which is independent when existing plurally,
R₂ is hydrogen, C₁-C₄ alkoxy, C₁-C₄ haloalkyl, C₁-C₄ haloalkoxy, C₁-C₄ alkylthio, C₁-C₄ alkylsulfinyl, C₁-C₄ alkylsulfonyl, acetyl, hydroxy, cyano, nitro, NRₐR_{b}, or 4- to 7-membered heteroaryl having 1 to 3 of N,
R₂ is singular or plural substituent, each of which is independent when existing plurally,
R₃ is X substituted with Y or not,
X is C₁-C₄ alkoxy, amino(-NH-), C₅-C₇ aryl, or 5- to 7-membered non-aromatic heterocycle having 1 to 2 heteroatom(s) selected from the group consisting of N and O,
Y is hydrogen, C₁-C₆ straight or branched alkyl, C₃-C₆ cycloalkyl, hydroxy, COO, COO-alkyl, C₅-C₇ aryl, alkylaryl, or 5- to 7-membered aromatic or non-aromatic heterocycle having 1 to 2 of N,
Y is substituted with Z or not,
Z is C₁-C₆ straight or branched alkyl, C₁-C₄ alkoxy, hydroxy, C₁-C₄ haloalkyl, acetyl, guanidino, NRₐR_{b}, C₅-C₇ aryl, alkoxyaryl, 5- to 7-membered aromatic or non-aromatic heterocycle having 1 to 2 heteroatom(s) selected from the group consisting of N and O,
wherein Rₐ and R_{b} 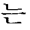 independently hydrogen, C₁-C₄ straight or branched alkyl, acetyl, C₁-C₄ alkylsulfonyl or C₁-C₆ alkoxycarbonyl,
Z is substituted with Q or not,
Q is C₁-C₆ straight or branched alkyl, hydroxy, halogen, acetyl, nitro, C₁-C₄ alkylsulfonyl, or C₁-C₆ alkoxycarbonyl.

This specification uses the following definitions when defining the compound of Formula I unless specifically defined.

The term "alkyl" refers to a straight or branched chain hydrocarbonyl group and may contain a single bond, double bond, or triple bond, preferably C₁-C₁₀ alkyl. Examples of alkyl include, but are not limited to, methyl, ethyl, *n*-propyl, iso-propyl, n-butyl, iso-butyl, tert-butyl, acetylene, vinyl, trifluoromethyl.

The term "cycloalkyl" refers to a partially or fully saturated single or fused ring hydrocarbon, preferably C₃-C₁₀-cycloalkyl. Examples of cycloalkyl include, but are not limited to, cyclopropyl, cyclobutyl, cyclopentyl, cyclohexyl, and cyclohexenyl.

The term "alkoxy" means an alkyloxy with 1 to 10 carbon atoms, unless otherwise defined.

The term "halogen" or "halo" refers to fluorine (F), chlorine (Cl), bromine (Br) or iodine (I).

The term "haloalkyl" and "haloalkoxy" means alkyl or alkoxy substituted with one or more halogen atoms.

The term "heteroatom" means N, O or S.

The term "aryl" means aromatic hydrocarbon, includes a polycycle aromatic ring system in which a carbocycle aromatic ring or heteroaryl ring is fused with one or more other rings, preferably C₅-C₁₂ aryl, more preferably C₅-C₁₀ aryl. For example, aryl includes, but are not limited to, phenyl, naphthyl, tetrahydronaphthyl, etc.

The term "heteroaryl" or "aromatic heterocycle" means a 3- to 12-membered, more preferably 5- to 10-membered aromatic hydrocarbon forming a single or fused cyclic ring that contains one or more heteroatoms selected from N, O and S as ring atoms, and that can be fused with a benzo or C₃-C₈ cycloalkyl. For example, heteroaryl includes, but are not limited to, pyrrolyl, imidazolyl, pyrazolyl, triazolyl, pyridyl, pyrazinyl, pyrimidyl, pyridazinyl, triazinyl, oxadiazolyl, isoxadiazolyl, tetrazolyl, indolyl, indazolyl, isoxazolyl, oxazolyl, thiazolyl, isothiazolyl, furanyl, benzofuranyl, thiophenyl, benzothiazolyl, benzooxazolyl, benzimidazolyl, quinolinyl, isoquinolinyl, etc.

The term "non-aromatic heterocycle" means a non-aromatic carbocyclic ring comprising one or more heteroatom selected from N, O and S as ring atoms. The ring may be 5, 6, 7 or 8-membered and/or fused to another ring such as a cycloalkyl or aromatic ring.

Arylalkyl, alkylaryl and heteroarylalkyl refer to a group formed by combining aryl and alkyl or heteroaryl and alkyl as defined above, and include, for example, benzyl, thiophene methyl, pyrimidine methyl etc, but are not limited thereto.

In one embodiment, R₁ may be hydrogen, butyl, cyclopropyl, methoxy, F, Cl, trifluoromethyl, trifluoromethoxy, methylthio, hydroxy, cyano, nitro, NRₐR_{b}, phenyl, or naphthyl, wherein Rₐ and R_{b} may be independently hydrogen, methyl, acetyl, or butoxycarbonyl.

In one embodiment, R₂ may be hydrogen, methoxy, trifluoromethyl, trifluoromethoxy, methylthio, methylsulfinyl, methylsulfonyl, acetyl, hydroxy, cyano, nitro, NRₐR_{b}, or pyridinyl, wherein Rₐ and R_{b} may be independently hydrogen, acetyl, or methylsulfonyl.

In one embodiment, X may be ethoxy, amino(-NH-), phenyl, pyrrolidinyl, piperidinyl, piperazinyl, or morpholino.

In one embodiment, Y may be hydrogen, methyl, ethyl, propyl, cyclopropyl, hydroxy, COO, COO-ethyl, phenyl, benzyl, piperidinyl, or pyridinyl.

In one embodiment, Z may be ethyl, methoxy, hydroxy, trifluoromethyl, acetyl, guanidino, NRₐR_{b}, phenyl, benzyloxy, pyrrolidinyl, tetrahydrofuranyl, piperidinyl, piperazinyl, morpholino, imidazolyl, or pyridinyl, wherein Rₐ and R_{b} may be independently hydrogen, methyl, ethyl, propyl, acetyl, methylsulfonyl or butoxycarbonyl.

Representative examples of oxazole derivative compounds according to the present invention are as follows:
ethyl 5-phenyl-2-(2-(trifluoromethyl)phenyl)oxazole-4-carboxylate (27),
ethyl 5-(2-nitrophenyl)-2-(2-(trifluoromethyl)phenyl)oxazole-4-carboxylate (28),
ethyl 5-phenyl-2-(3-(trifluoromethyl)phenyl)oxazole-4-carboxylate (29),
ethyl 5-(2-nitrophenyl)-2-(3-(trifluoromethyl)phenyl)oxazole-4-carboxylate (30),
ethyl 5-phenyl-2-(4-(trifluoromethyl)phenyl)oxazole-4-carboxylate (31),
ethyl 5-(2-nitrophenyl)-2-(4-(trifluoromethyl)phenyl)oxazole-4-carboxylate (32),
ethyl 5-(3-nitrophenyl)-2-(4-(trifluoromethyl)phenyl)oxazole-4-carboxylate (33),
ethyl 5-(4-nitrophenyl)-2-(4-(trifluoromethyl)phenyl)oxazole-4-carboxylate (34),
ethyl 5-(2-acetamidophenyl)-2-(4-(trifluoromethyl)phenyl)oxazole-4-carboxylate (35),
ethyl 5-(3-(methylsulfonyl)phenyl)-2-(4-(trifluoromethyl)phenyl)oxazole-4-carboxylate (36),
ethyl 5-(4-(methylsulfonyl)phenyl)-2-(4-(trifluoromethyl)phenyl)oxazole-4-carboxylate (37),
ethyl 5-(3-acetylphenyl)-2-(4-(trifluoromethyl)phenyl)oxazole-4-carboxylate (38),
ethyl 5-(4-acetylphenyl)-2-(4-(trifluoromethyl)phenyl)oxazole-4-carboxylate (39),
ethyl 5-(3-(methylthio)phenyl)-2-(4-(trifluoromethyl)phenyl)oxazole-4-carboxylate (40),
ethyl 5-(4-(methylthio)phenyl)-2-(4-(trifluoromethyl)phenyl)oxazole-4-carboxylate (41),
ethyl 5-(pyridin-3-yl)-2-(4-(trifluoromethyl)phenyl)oxazole-4-carboxylate (42),
ethyl 5-(pyridin-4-yl)-2-(4-(trifluoromethyl)phenyl)oxazole-4-carboxylate (43),
ethyl 5-(2-methoxyphenyl)-2-(4-(trifluoromethyl)phenyl)oxazole-4-carboxylate (44),
ethyl 5-(3-methoxyphenyl)-2-(4-(trifluoromethyl)phenyl)oxazole-4-carboxylate (45),
ethyl 5-(4-methoxyphenyl)-2-(4-(trifluoromethyl)phenyl)oxazole-4-carboxylate (46),
ethyl 5-(3,4-dimethoxyphenyl)-2-(4-trifluoromethyl)phenyl)oxazole-4-carboxylate (47),
ethyl 5-(3,5-dimethoxyphenyl)-2-(4-trifluoromethyl)phenyl)oxazole-4-carboxylate (48),
ethyl 2-(4-trifluoromethyl)phenyl)-5-(3,4,5-trimethoxyphenyl)oxazole-4-carboxylate (49),
ethyl 5-(2-(trifluoromethyl)phenyl)-2-(4-(trifluoromethyl)phenyl)oxazole-4-carboxylate (50),
ethyl 5-(3-(trifluoromethyl)phenyl)-2-(4-(trifluoromethyl)phenyl)oxazole-4-carboxylate (51),
ethyl 5-(2-(trifluoromethoxy)phenyl)-2-(4-(trifluoromethyl)phenyl)oxazole-4-carboxylate (52),
ethyl 5-(3-(trifluoromethoxy)phenyl)-2-(4-(trifluoromethyl)phenyl)oxazole-4-carboxylate (53),
ethyl 5-(2-cyanophenyl)-2-(4-(trifluoromethyl)phenyl)oxazole-4-carboxylate (54),
ethyl 5-(3-cyanophenyl)-2-(4-(trifluoromethyl)phenyl)oxazole-4-carboxylate (55),
ethyl 5-phenyl-2-(3-(trifluoromethoxy)phenyl)oxazole-4-carboxylate (56),
ethyl 5-(2-nitrophenyl)-2-(3-(trifluoromethoxy)phenyl)oxazole-4-carboxylate (57),
ethyl 5-phenyl-2-(4-(trifluoromethoxy)phenyl)oxazole-4-carboxylate (58),
ethyl 5-(2-nitrophenyl)-2-(4-(trifluoromethoxy)phenyl)oxazole-4-carboxylate (59),
ethyl 2-(3-methoxyphenyl)-5-phenyloxazole-4-carboxylate (60),
ethyl 2-(3-methoxyphenyl)-5-(2-nitrophenyl)oxazole-4-carboxylate (61),
ethyl 2-(4-methoxyphenyl)-5-phenyloxazole-4-carboxylate (62),
ethyl 2-(4-methoxyphenyl)-5-(2-nitrophenyl)oxazole-4-carboxylate (63),
ethyl 2-(4-methoxyphenyl)-5-(4-nitrophenyl)oxazole-4-carboxylate (64),
ethyl 2-(3-fluorophenyl)-5-phenyloxazole-4-carboxylate (65),
ethyl 2-(3-fluorophenyl)-5-(2-nitrophenyl)oxazole-4-carboxylate (66),
ethyl 2-(4-fluorophenyl)-5-phenyloxazole-4-carboxylate (67),
ethyl 2-(4-fluorophenyl)-5-(2-nitrophenyl)oxazole-4-carboxylate (68),
ethyl 2-(4-fluorophenyl)-5-(4-nitrophenyl)oxazole-4-carboxylate (69),
ethyl 2-(3,4-difluorophenyl)-5-phenyloxazole-4-carboxylate (70),
ethyl 2-(3,4-difluorophenyl)-5-(2-nitrophenyl)oxazole-4-carboxylate (71),
ethyl 2-(3,4-difluorophenyl)-5-(4-(methylthio)phenyl)oxazole-4-carboxylate (72),
ethyl 2-(3,5-difluorophenyl)-5-phenyloxazole-4-carboxylate (73),
ethyl 2-(3,5-difluorophenyl)-5-(2-nitrophenyl)oxazole-4-carboxylate (74),
ethyl 2-(3-chlorophenyl)-5-phenyloxazole-4-carboxylate (75),
ethyl 2-(3-chlorophenyl)-5-(2-nitrophenyl)oxazole-4-carboxylate (76),
ethyl 2-(4-chlorophenyl)-5-phenyloxazole-4-carboxylate (77),
ethyl 2-(4-chlorophenyl)-5-(2-nitrophenyl)oxazole-4-carboxylate (78),
ethyl 2-(4-cyanophenyl)-5-phenyloxazole-4-carboxylate (79),
ethyl 2-(4-cyanophenyl)-5-(2-nitrophenyl)oxazole-4-carboxylate (80),
ethyl 2-([1,1 '-biphenyl]-4-yl)-5-phenyloxazole-4-carboxylate (81),
ethyl 2-([1,1'-biphenyl]-4-yl)-5-(2-nitrophenyl)oxazole-4-carboxylate (82),
ethyl 2-(naphthalen-2-yl)-5-phenyloxazole-4-carboxylate (83),
ethyl 2-(naphthalen-2-yl)-5-(2-nitrophenyl)oxazole-4-carboxylate (84),
ethyl 2-(4-(dimethylamino)phenyl)-5-phenyloxazole-4-carboxylate (85),
ethyl 2-(4-(dimethylamino)phenyl)-5-(2-nitrophenyl)oxazole-4-carboxylate (86),
ethyl 2-(4-(*tert*-butyl)phenyl)-5-phenyloxazole-4-carboxylate (87),
ethyl 2-(4-(*tert*-butyl)phenyl)-5-(2-nitrophenyl)oxazole-4-carboxylate (88),
ethyl 2-(4-(*tert*-butyl)phenyl)-5-(4-nitrophenyl)oxazole-4-carboxylate (89),
ethyl 2-(4-(methylthio)phenyl)-5-phenyloxazole-4-carboxylate (90),
ethyl 2-(4-(methylthio)phenyl)-5-(2-nitrophenyl)oxazole-4-carboxylate (91),
ethyl 5-(3-methoxyphenyl)-2-(4-(methylthio)phenyl)oxazole-4-carboxylate (92),
ethyl 5-(4-methoxyphenyl)-2-(4-(methylthio)phenyl)oxazole-4-carboxylate (93),
ethyl 2-(4-nitrophenyl)-5-phenyloxazole-4-carboxylate (94),
ethyl 5-(2-nitrophenyl)-2-(4-nitrophenyl)oxazole-4-carboxylate (95),
ethyl 2-(4-((*tert*-butoxycarbonyl)amino)phenyl)-5-(3-(methylthio)phenyl)oxazole-4-carboxylate (96),
ethyl 2-(4-cyclopropylphenyl)-5-phenyloxazole-4-carboxylate (97),
ethyl 2-(4-chloro-3-(trifluoromethyl)phenyl)-5-phenyloxazole-4-carboxylate (98),
ethyl 5-(2-aminophenyl)-2-(4-(trifluoromethyl)phenyl)oxazole-4-carboxylate (99),
ethyl 5-(3-aminophenyl)-2-(4-(trifluoromethyl)phenyl)oxazole-4-carboxylate (100),
ethyl 5-(4-aminophenyl)-2-(4-(trifluoromethyl)phenyl)oxazole-4-carboxylate (101),
ethyl 5-(4-aminophenyl)-2-(4-(tert-butyl)phenyl)oxazole-4-carboxylate (102),
ethyl 5-(3-(methylsulfinyl)phenyl)-2-(4-(trifluoromethyl)phenyl)oxazole-4-carboxylate (103),
ethyl 5-(4-(methylsulfinyl)phenyl)-2-(4-(trifluoromethyl)phenyl)oxazole-4-carboxylate (104),
ethyl 2-(4-aminophenyl)-5-(3-(methylthio)phenyl)oxazole-4-carboxylate (105),
ethyl 5-(3-acetamidophenyl)-2-(4-(trifluoromethyl)phenyl)oxazole-4-carboxylate (106),
ethyl 5-(4-acetamidophenyl)-2-(4-(trifluoromethyl)phenyl)oxazole-4-carboxylate (107),
ethyl 5-(3-(N-acetylacetamido)phenyl)-2-(4-(trifluoromethyl)phenyl)oxazole-4-carboxylate (108),
ethyl 5-(4-(N-acetylacetamido)phenyl)-2-(4-(trifluoromethyl)phenyl)oxazole-4-carboxylate (109),
ethyl 5-(4-(N-acetylacetamido)phenyl)-2-(4-(tert-butyl)phenyl)oxazole-4-carboxylate (110),
ethyl 5-(4-acetamidophenyl)-2-(4-(tert-butyl)phenyl)oxazole-4-carboxylate (111),
ethyl 2-(4-acetamidophenyl)-5-(3-(methylthio)phenyl)oxazole-4-carboxylate(112),
ethyl 5-(4-(methylsulfonamido)phenyl)-2-(4-(trifluoromethyl)phenyl)oxazole-4-carboxylate (113),
N-(2-dimethylamino)ethyl-2-(4-(trifluoromethyl)phenyl)oxazole-4-carboxamide (114),
N-(2-(4-methylpiperazin-1-yl)ethyl)-2-(4-(trifluoromethyl)phenyl)oxazole-4-carboxamide (115),
(4-(2-(dimethylamino)ethyl)piperazin-1-yl)(2-(4- (trifluoromethyl)phenyl)oxazol-4-yl)methanone (116),
N-(2-(dimethylamino)ethyl)-5-phenyl-2-(2-(trifluoromethyl)phenyl)oxazole-4-carboxamide (117),
N-(2-(4-methylpiperazin-1-yl)ethyl)-5-phenyl-2-(2-(trifluoromethyl)phenyl)oxazole-4-carboxamide (118),
N-(2-(dimethylamino)ethyl)-5-(2-nitrophenyl)-2-(2-(trifluoromethyl)phenyl)oxazole-4-carboxamide (119),
N-(2-(4-methylpiperazin-1-yl)ethyl)-5-(2-nitrophenyl)-2-(2-(trifluoromethyl)phenyl)oxazole-4-carboxamide (120),
N-(2-(dimethylamino)ethyl)-5-phenyl-2-(3-(trifluoromethyl)phenyl)oxazole-4-carboxamide (121),
N-(2-(4-methylpiperazin-1-yl)ethyl)-5-phenyl-2-(3-(trifluoromethyl)phenyl)oxazole-4-carboxamide (122),
N-(2-(dimethylamino)ethyl)-5-(2-nitrophenyl)-2-(3-(trifluoromethyl)phenyl)oxazole-4-carboxamide (123),
N-(2-(4-methylpiperazin-1-yl)ethyl)-5-(2-nitrophenyl)-2-(3-(trifluoromethyl)phenyl)oxazole-4-carboxamide (124),
(4-(2-(dimethylamino)ethyl)piperazin-1-yl)(5-(2-nitrophenyl)-2-(3-(trifluoromethyl)phenyl)oxazol-4-yl)methanone (125),
N-(2-(dimethylamino)ethyl)-5-phenyl-2-(4-(trifluoromethyl)phenyl)oxazole-4-carboxamide (126),
N-(2-(4-methylpiperazin-1-yl)ethyl)-5-phenyl-2-(4-(trifluoromethyl)phenyl)oxazole-4-carboxamide (127),
N-(2-(4-acetylpiperazin-1-yl)ethyl)-5-phenyl-2-(2-(trifluoromethyl)phenyl)oxazole-4-carboxamide (128),
(4-(2-(dimethylamino)ethyl)piperazin-1-yl)-5-phenyl-2-(4-(trifluoromethyl)phenyl)oxazol-4-yl)methanone (129),
N-(4-Hydroxyphenethyl)-5-phenyl-2-(4-(trifluoromethyl)phenyl)oxazole-4-carboxamide (130),
N-(2-(dimethylamino)ethyl)-5-(2-nitrophenyl)-2-(4-(trifluoromethyl)phenyl)oxazole-4-carboxamide (131),
(5-(2-nitrophenyl)-2-(4-(trifluoromethyl)phenyl)oxazol-4-yl)(piperazin-1-yl)methanone (132),
(4-(2-(dimethylamino)ethyl)piperazin-1-yl)(5-(2-nitrophenyl)-2-(4-(trifluoromethyl)phenyl)oxazol-4-yl)methanone (133),
(4-(2-methoxyphenyl)piperazin-1-yl)(5-(2-nitrophenyl)-2-(4-(trifluoromethyl)phenyl)oxazol-4-yl)methanone (134),
ethyl 4-(5-(2-nitrophenyl)-2-(4-(trifluoromethyl)phenyl)oxazole-4-carbonyl)piperazin-1-carboxylate (135),
4-nitrophenyl4-(5-(2-nitrophenyl)-2-(4-(trifluoromethyl)phenyl)oxazole-4-carbonyl)piperazin-1-carboxylate (136),
2-(dimethylamino)ethyl 4-(5-(2-nitrophenyl)-2-(4-(trifluoromethyl)phenyl)oxazole-4-carbonyl) piperazin-1-carboxylate (137),
(5-(2-nitrophenyl)-2-(4-(trifluoromethyl)phenyl)oxazol-4-yl)(pyrrolidin-1-yl)methanone (138),
5-(2-nitrophenyl)-N-(2-(pyrrolidin-1-yl)ethyl)-2-(4-(trifluoromethyl)phenyl)oxazole-4-carboxamide (139),
5-(2-nitrophenyl)-N-(3-(pyrrolidin-1-yl)propyl)-2-(4-(trifluoromethyl)phenyl)oxazole-4-carboxamide (140),
5-(2-nitrophenyl)-N-(pyridin-4-ylmethyl)-2-(4-(trifluoromethyl)phenyl)oxazole-4-carboxamide (141),
5-(2-nitrophenyl)-N-(pyridin-3-ylmethyl)-2-(4-(trifluoromethyl)phenyl)oxazole-4-carboxamide (142),
5-(2-nitrophenyl)-N-(pyridin-2-yl)-2-(4-(trifluoromethyl)phenyl)oxazole-4-carboxamide (143),
(5-(2-nitrophenyl)-2-(4-(trifluoromethyl)phenyl)oxazol-4-yl)(piperidin-1-yl)methanone (144),
(5-(2-nitrophenyl)-2-(4-(trifluoromethyl)phenyl)oxazol-4-yl)(4-phenylpiperidin-1-yl)methanone (145),
(4-cyclopropylpiperazin-1-yl)(5-(2-nitrophenyl)-2-(4-(trifluoromethyl)phenyl)oxazol-4-yl)methanone (146),
N-(4-acetylphenyl)-5-(2-nitrophenyl)-2-(4-(trifluoromethyl)phenyl)oxazole-4-carboxamide (147),
5-(2-nitrophenyl)-N-(4-(trifluoromethyl)benzyl)-2-(4-(trifluoromethyl)phenyl)oxazole-4-carboxamide (148),
N-(4-fluorophenethyl)-5-(2-nitrophenyl)-2-(4-(trifluoromethyl)phenyl)oxazole-4-carboxamide (149),
(4-(3-(dimethylamino)propyl)piperazin-1-yl)(5-(2-nitrophenyl)-2-(4-(trifluoromethyl)phenyl)oxazol-4-yl)methanone (150),
(5-(2-nitrophenyl)-2-(4-(trifluoromethyl)phenyl)oxazol-4-yl)(4-(2-(piperidin-1-yl)ethyl)piperazin-1-yl)methanone (151),
(4-(2-morpholinoethyl)piperazin-1-yl)(5-(2-nitrophenyl)-2-(4-(trifluoromethyl)phenyl)oxazol-4-yl)methanone (152),
N-(2-(4-methylpiperazin-1-yl)ethyl)-5-(2-nitrophenyl)-2-(4-(trifluoromethyl)phenyl)oxazole-4-carboxamide (153),
(4-(2-(diethylamino)ethyl)piperazin-1-yl)(5-(2-nitrophenyl)-2-(4-(trifluoromethyl)phenyl)oxazol-4-yl)methanone (154),
(4-methylpiperazin-1-yl)(5-(2-nitrophenyl)-2-(4-(trifluoromethyl)phenyl)oxazol-4-yl)methanone (155),
morpholino(5-(2-nitrophenyl)-2-(4-(trifluoromethyl)phenyl)oxazol-4-yl)methanone (156),
N-(3-morpholinopropyl)-5-(2-nitrophenyl)-2-(4-(trifluoromethyl)phenyl)oxazole-4-carboxamide (157),
N-(3-(1H-imidazol-1-yl)propyl)-5-(2-nitrophenyl)-2-(4-(trifluoromethyl)phenyl)oxazole-4-carboxamide (158),
5-(2-nitrophenyl)-N-((tetrahydrofuran-2-yl)methyl)-2-(4-(trifluoromethyl)phenyl)oxazole-4-carboxamide (159),
N-(2-methoxyethyl)-5-(2-nitrophenyl)-2-(4-(trifluoromethyl)phenyl)oxazole-4-carboxamide (160),
(4-hydroxypiperidin-1-yl)(5-(2-nitrophenyl)-2-(4-(trifluoromethyl)phenyl)oxazol-4-yl)methanone (161),
(5-(2-nitrophenyl)-2-(4-(trifluoromethyl)phenyl)oxazol-4-yl)(4-(piperidin-1-yl)phenyl)methanone (162),
ethyl 4-(5-(2-nitrophenyl)-2-(4-(trifluoromethyl)phenyl)oxazole-4-carbonyl)piperazin-1-carboxylate (163),
N-benzyl-5-(2-nitrophenyl)-2-(4-(trifluoromethyl)phenyl)oxazole-4-carboxamide (164),
N-(4-(benzyloxy)phenyl)-5-(2-nitrophenyl)-2-(4-(trifluoromethyl)phenyl)oxazole-4-carboxamide (165),
N-(4-methoxybenzyl)-5-(2-nitrophenyl)-2-(4-(trifluoromethyl)phenyl)oxazole-4-carboxamide (166),
(4-(2-(diisopropylamino)ethyl)piperazin-1-yl)(5-(2-nitrophenyl)-2-(4-(trifluoromethyl)phenyl)oxazol-4-yl)methanone (167),
(4-isopropylpiperazin-1-yl)(5-(2-nitrophenyl)-2-(4-(trifluoromethyl)phenyl)oxazol-4-yl)methanone (168),
(4-(2-hydroxyethyl)piperazin-1-yl)(5-(2-nitrophenyl)-2-(4-(trifluoromethyl)phenyl)oxazol-4-yl)methanone (169),
(5-(2-nitrophenyl)-2-(4-(trifluoromethyl)phenyl)oxazol-4-yl)(4-(2-(pyrrolidin-1-yl)ethyl)piperazin-1-yl)methanone (170),
N-(3-(4-methylpiperazin-1-yl)propyl)-5-(2-nitrophenyl)-2-(4-(trifluoromethyl)phenyl)oxazole-4-carboxamide (171),
*tert*-butyl-4-(2-(5-(2-nitrophenyl)-2-(4-(trifluoromethyl)phenyl)oxazole-4-carboxamido)ethyl)piperazin-1-carboxylate (172),
5-(2-nitrophenyl)-N-(2-(piperidin-1-yl)ethyl)-2-(4-(trifluoromethyl)phenyl)oxazole-4-carboxamide (173),
*tert-*butyl (2-(5-(2-nitrophenyl)-2-(4-(trifluoromethyl)phenyl)oxazole-4-carboxamido)ethyl)carbamate (174),
N-(4-acetamidophenyl)-5-(2-nitrophenyl)-2-(4-(trifluoromethyl)phenyl)oxazole-4-carboxamide (175),
N-(4-Hydroxyphenethyl)-5-(2-nitrophenyl)-2-(4-(trifluoromethyl)phenyl)oxazole-4-carboxamide (176),
N-(2-(dimethylamino)ethyl)-5-(3-nitrophenyl)-2-(4-(trifluoromethyl)phenyl)oxazole-4-carboxamide (177),
(5-(3-nitrophenyl)-2-(4-(trifluoromethyl)phenyl)oxazol-4-yl)(piperazin-1-yl)methanone (178),
(4-(2-(dimethylamino)ethyl)piperazin-1-yl)(5-(3-nitrophenyl)-2-(4-(trifluoromethyl)phenyl)oxazol-4-yl)methanone (179),
N-(2-(dimethylamino)ethyl)-5-(4-nitrophenyl)-2-(4-(trifluoromethyl)phenyl)oxazole-4-carboxamide (180),
(5-(4-nitrophenyl)-2-(4-(trifluoromethyl)phenyl)oxazol-4-yl)(piperazin-1-yl)methanone (181),
(4-(2-(dimethylamino)ethyl)piperazin-1-yl)(5-(4-nitrophenyl)-2-(4-(trifluoromethyl)phenyl)oxazol-4-yl)methanone (182),
5-(2-aminophenyl)-N-(2-(dimethylamino)ethyl)-2-(4-(trifluoromethyl)phenyl)oxazole-4-carboxamide (183),
(5-(2-aminophenyl)-2-(4-(trifluoromethyl)phenyl)oxazol-4-yl)(4-(2-(dimethylamino)ethyl)piperazin-1-yl)methanone (184),
5-(3-aminophenyl)-N-(2-(dimethylamino)ethyl)-2-(4-(trifluoromethyl)phenyl)oxazole-4-carboxamide (185),
5-(4-aminophenyl)-N-(2-(dimethylamino)ethyl)-2-(4-(trifluoromethyl)phenyl)oxazole-4-carboxamide (186),
5-(2-acetamidophenyl)-N-(2-(dimethylamino)ethyl)-2-(4-(trifluoromethyl)phenyl)oxazole-4-carboxamide (187),
N-(2-(4-(4-(2-(dimethylamino)ethyl)piperazin-1-carbonyl)-2-(4-(trifluoromethyl)phenyl)oxazol-5-yl)phenyl)acetamide (188),
5-(3-acetamidophenyl)-N-(2-(dimethylamino)ethyl)-2-(4-(trifluoromethyl)phenyl)oxazole-4-carboxamide (189),
N-(3-(4-(4-(2-(dimethylamino)ethyl)piperazin-1-carbonyl)-2-(4-(trifluoromethyl)phenyl)oxazol-5-yl)phenyl)acetamide (190),
5-(4-acetamidophenyl)-N-(2-(dimethylamino)ethyl)-2-(4-(trifluoromethyl)phenyl)oxazole-4-carboxamide (191),
N-(4-(4-(4-(2-(dimethylamino)ethyl)piperazin-1-carbonyl)-2-(4-(trifluoromethyl)phenyl)oxazol-5-yl)phenyl)acetamide (192),
5-(4-(methylsulfonyl)phenyl)-N-(pyridin-3-ylmethyl)-2-(4-(trifluoromethyl)phenyl)oxazole-4-carboxamide (193),
N-(2-(4-acetylpiperazin-1-yl)ethyl)-5-(4-(methylthio)phenyl)-2-(4-trifluoromethyl)phenyl)oxazole-4-carboxamide (194),
5-(4-(methylthio)phenyl)-N-(pyridin-3-ylmethyl)-2-(4-(trifluoromethyl)phenyl)oxazole-4-carboxamide (195),
N-(2-(dimethylamino)ethyl)-5-(pyridin-3-yl)-2-(4-(trifluoromethyl)phenyl)oxazole-4-carboxamide (196),
N-(2-(4-acetylpiperazin-1-yl)ethyl)-5-(pyridin-3-yl)-2-(4-(trifluoromethyl)phenyl)oxazole-4-carboxamide (197),
N-(2-(4-acetylpiperazin-1-yl)ethyl)-5-(3-methoxyphenyl)-2-(4-(trifluoromethyl)phenyl)oxazole-4-carboxamide (198),
N-(2-(dimethylamino)ethyl)-5-phenyl-2-(3-(trifluoromethoxy)phenyl)oxazole-4-carboxamide (199),
N-(2-(4-methylpiperazin-1-yl)ethyl)-5-phenyl-2-(3-(trifluoromethoxy)phenyl)oxazole-4-carboxamide (200),
N-(2-(dimethylamino)ethyl)-5-(2-nitrophenyl)-2-(3-(trifluoromethoxy)phenyl)oxazole-4-carboxamide (201),
N-(2-(4-methylpiperazin-1-yl)ethyl)-5-(2-nitrophenyl)-2-(3-(trifluoromethoxy)phenyl)oxazole-4-carboxamide (202),
N-(2-(dimethylamino)ethyl)-5-phenyl-2-(4-(trifluoromethoxy)phenyl)oxazole-4-carboxamide (203),
N-(2-(4-methylpiperazin-1-yl)ethyl)-5-phenyl-2-(4-(trifluoromethoxy)phenyl)oxazole-4-carboxamide (204),
N-(2-(dimethylamino)ethyl)-5-(2-nitrophenyl)-2-(4-(trifluoromethoxy)phenyl)oxazole-4-carboxamide (205),
N-(2-(4-methylpiperazin-1-yl)ethyl)-5-(2-nitrophenyl)-2-(4-(trifluoromethoxy)phenyl)oxazole-4-carboxamide (206),
(4-(2-(dimethylamino)ethyl)piperazin-1-yl)(5-(2-nitrophenyl)-2-(4-(trifluoromethoxy)phenyl)oxazol-4-yl)methanone (207),
N-(2-(dimethylamino)ethyl)-2-(3-methoxyphenyl)-5-phenyloxazole-4-carboxamide (208),
2-(3-methoxyphenyl)-N-(2-(4-methylpiperazin-1-yl)ethyl)-5-phenyloxazole-4-carboxamide (209),
N-(2-(dimethylamino)ethyl)-2-(3-methoxyphenyl)-5-(2-nitrophenyl)oxazole-4-carboxamide (210),
2-(3-methoxyphenyl)-N-(2-(4-methylpiperazin-1-yl)ethyl)-5-(2-nitrophenyl)oxazole-4-carboxamide (211),
N-(2-(dimethylamino)ethyl)-2-(4-methoxyphenyl)-5-phenyloxazole-4-carboxamide (212),
2-(4-methoxyphenyl)-N-(2-(4-methylpiperazin-1-yl)ethyl)-5-phenyloxazole-4-carboxamide (213),
N-(2-(dimethylamino)ethyl)-2-(4-methoxyphenyl)-5-(2-nitrophenyl)oxazole-4-carboxamide (214),
2-(4-methoxyphenyl)-N-(2-(4-methylpiperazin-1-yl)ethyl)-5-(2-nitrophenyl)oxazole-4-carboxamide (215),
(4-(2-(dimethylamino)ethyl)piperazin-1-yl)(2-(4-methoxyphenyl)-5-(2-nitrophenyl)oxazol-4-yl)methanone (216),
N-(2-(dimethylamino)ethyl)-2-(4-methoxyphenyl)-5-(4-nitrophenyl)oxazole-4-carboxamide (217),
(4-(2-(dimethylamino)ethyl)piperazin-1-yl)(2-(4-methoxyphenyl)-5-(4-nitrophenyl)oxazol-4-yl)methanone (218),
2-(4-methoxyphenyl)-N-(2-(4-methylpiperazin-1-yl)ethyl)-5-(4-nitrophenyl)oxazole-4-carboxamide (219),
N-(2-(dimethylamino)ethyl)-2-(3-fluorophenyl)-5-phenyloxazole-4-carboxamide (220),
2-(3-fluorophenyl)N-(2-(4-methylpiperazin-1-yl)ethyl)-5-phenyloxazole-4-carboxamide (221),
N-(2-(dimethylamino)ethyl)-2-(3-fluorophenyl)-5-(2-nitrophenyl)oxazole-4-carboxamide (222),
2-(3-fluorophenyl)-N-(2-(4-methylpiperazin-1-yl)ethyl)-5-(2-nitrophenyl)oxazole-4-carboxamide (223),
N-(2-(dimethylamino)ethyl)-2-(4-fluorophenyl)-5-phenyloxazole-4-carboxamide (224),
2-(4-fluorophenyl)-N-(2-(4-methylpiperazin-1-yl)ethyl)-5-phenyloxazole-4-carboxamide (225),
N-(2-(dimethylamino)ethyl)-2-(4-fluorophenyl)-5-(2-nitrophenyl)oxazole-4-carboxamide (226),
2-(4-fluorophenyl)-N-(2-(4-methylpiperazin-1-yl)ethyl)-5-(2-nitrophenyl)oxazole-4-carboxamide (227),
(4-(2-(dimethylamino)ethyl)piperazin-1-yl)(2-(4-fluorophenyl)-5-(2-nitrophenyl)oxazol-4-yl)methanone (228),
N-(2-(dimethylamino)ethyl)-2-(4-fluorophenyl)-5-(4-nitrophenyl)oxazole-4-carboxamide (229),
2-(4-fluorophenyl)-N-(2-(4-methylpiperazin-1-yl)ethyl)-5-(4-nitrophenyl)oxazole-4-carboxamide (230),
2-(3,4-difluorophenyl)-N-(2-(dimethylamino)ethyl)-5-phenyloxazole-4-carboxamide (231),
2-(3,4-difluorophenyl)-N-(2-(4-methylpiperazin-1-yl)ethyl)-5-phenyloxazole-4-carboxamide (232),
2-(3,4-difluorophenyl)-N-(2-(dimethylamino)ethyl)-5-(2-nitrophenyl)oxazole-4-carboxamide (233),
2-(3,4-difluorophenyl)-N-(2-(4-methylpiperazin-1-yl)ethyl)-5-(2-nitrophenyl)oxazole-4-carboxamide (234),
2-(3,5-difluorophenyl)-N-(2-(dimethylamino)ethyl)-5-phenyloxazole-4-carboxamide (235),
2-(3,5-difluorophenyl)-N-(2-(4-methylpiperazin-1-yl)ethyl)-5-phenyloxazole-4-carboxamide (236),
2-(3,5-difluorophenyl)-N-(2-(dimethylamino)ethyl)-5-(2-nitrophenyl)oxazole-4-carboxamide (237),
2-(3,5-difluorophenyl)-N-(2-(4-methylpiperazin-1-yl)ethyl)-5-(2-nitrophenyl)oxazole-4-carboxamide (238),
tert-butyl 4-(2-(2-(3,5-difluorophenyl)-5-(2-nitrophenyl)oxazole-4-carboxamido)ethyl)piperazin-1-carboxylate (239),
2-(3-chlorophenyl)-N-(2-(dimethylamino)ethyl)-5-phenyloxazole-4-carboxamide (240),
2-(3-chlorophenyl)-N-(2-(4-methylpiperazin-1-yl)ethyl)-5-phenyloxazole-4-carboxamide (241),
2-(3-chlorophenyl)-N-(2-(dimethylamino)ethyl)-5-(2-nitrophenyl)oxazole-4-carboxamide (242),
2-(3-chlorophenyl)-N-(2-(4-methylpiperazin-1-yl)ethyl)-5-(2-nitrophenyl)oxazole-4-carboxamide (243),
2-(4-chlorophenyl)-N-(2-(dimethylamino)ethyl)-5-phenyloxazole-4-carboxamide (244),
2-(4-chlorophenyl)-N-(2-(4-methylpiperazin-1-yl)ethyl)-5-phenyloxazole-4-carboxamide (245),
2-(4-chlorophenyl)-N-(2-(dimethylamino)ethyl)-5-(2-nitrophenyl)oxazole-4-carboxamide (246),
2-(4-(chlorophenyl)-N-(2-(4-methylpiperazin-1-yl)ethyl)-5-(2-nitrophenyl)oxazole-4-carboxamide (247),
(2-(4-chlorophenyl)-5-(2-nitrophenyl)oxazol-4-yl)(4-(2-(dimethylamino)ethyl)piperazin-1-yl)methanone (248),
2-(4-cyanophenyl)-N-(2-(dimethylamino)ethyl)-5-phenyloxazole-4-carboxamide (249),
2-(4-cyanophenyl)-N-(2-(4-methylpiperazin-1-yl)ethyl)-5-phenyloxazole-4-carboxamide (250),
2-(4-cyanophenyl)-N-(2-(dimethylamino)ethyl)-5-(2-nitrophenyl)oxazole-4-carboxamide (251),
2-(4-cyanophenyl)-N-(2-(4-methylpiperazin-1-yl)ethyl)-5-(2-nitrophenyl)oxazole-4-carboxamide (252),
2-([1,1'-biphenyl]-4-yl)-N-(2-(dimethylamino)ethyl)-5-phenyloxazole-4-carboxamide (253),
2-([1,1'-biphenyl]-4-yl)-N-2-(4-methylpiperazin-1 -yl)ethyl)-5-phenyloxazole-4-carboxamide (254),
2-([1,1'-biphenyl]-4-yl)-N-(2-(dimethylamino)ethyl)-5-(2-nitrophenyl)oxazole-4-carboxamide (255),
2-([1,1'-biphenyl]-4-yl)-N-(2-(4-methylpiperazin-1-yl)ethyl)-5-(2-nitrophenyl)oxazole-4-carboxamide (256),
N-(2-(dimethylamino)ethyl)-2-(naphthalen-2-yl)-5-phenyloxazole-4-carboxamide (257),
N-(2-(4-methylpiperazin-1-yl)ethyl)-2-(naphthalen-2-yl)-5-phenyloxazole-4-carboxamide (258),
N-(2-(dimethylamino)ethyl)-2-(naphthalen-2-yl)-5-(2-nitrophenyl)oxazole-4-carboxamide (259),
N-(2-(4-methylpiperazin-1-yl)ethyl)-2-(naphthalen-2-yl)-5-(2-nitrophenyl)oxazole-4-carboxamide (260),
N-(2-(dimethylamino)ethyl)-2-(4-(dimethylamino)phenyl)-5-phenyloxazole-4-carboxamide (261),
2-(4-(dimethylamino)phenyl)-N-(2-(4-methylpiperazin-1-yl)ethyl)-5-phenyloxazole-4-carboxamide (262),
N-(2-(dimethylamino)ethyl)-2-(4-(dimethylamino)phenyl)-5-(2-nitrophenyl)oxazole-4-carboxamide (263),
2-(4-(dimethylamino)phenyl)-N-(2-(4-methylpiperazin-1-yl)ethyl)-5-(2-nitrophenyl)oxazole-4-carboxamide (264),
2-(4-(*tert*-butyl)phenyl)-N-(2-(dimethylamino)ethyl)-5-phenyloxazole-4-carboxamide (265),
2-(4-(*tert*-butyl)phenyl)-N-(2-(4-methylpiperazin-1-yl)ethyl)-5-phenyloxazole-4-carboxamide (266),
2-(4-(*tert*-butyl)phenyl)-N-(2-(diethylamino)ethyl)-5-phenyloxazole-4-carboxamide (267),
2-(4-(*tert*-butyl)phenyl)-N-(2-(dimethylamino)ethyl)-5-(2-nitrophenyl)oxazole-4-carboxamide (268),
2-(4-(*tert*-butyl)phenyl)-N-(2-(4-methylpiperazin-1-yl)ethyl)-5-(2-nitrophenyl)oxazole-4-carboxamide (269),
2-(4-(*tert*-butyl)phenyl)-N-(2-(diethylamino)ethyl)-5-(2-nitrophenyl)oxazole-4-carboxamide (270),
N-(2-(dimethylamino)ethyl)-2-(4-(methylthio)phenyl)-5-phenyloxazole-4-carboxamide (271),
N-(2-(4-methylpiperazin-1-yl)ethyl)-2-(4-(methylthio)phenyl)-5-phenyloxazole-4-carboxamide (272),
N-(2-(dimethylamino)ethyl)-2-(4-(methylthio)phenyl)-5-(2-nitrophenyl)oxazole-4-carboxamide (273),
N-(2-(4-methylpiperazin-1-yl)ethyl)-2-(4-(methylthio)phenyl)-5-(2-nitrophenyl)oxazole-4-carboxamide (274),
N-(2-(dimethylamino)ethyl)-2-(4-nitrophenyl)-5-phenyloxazole-4-carboxamide (275),
N-(2-(4-methylpiperazin-1-yl)ethyl)-2-(4-nitrophenyl)-5-phenyloxazole-4-carboxamide (276),
N-(2-(dimethylamino)ethyl)-5-(2-nitrophenyl)-2-(4-nitrophenyl)oxazole-4-carboxamide (277),
N-(2-(4-methylpiperazin-1-yl)ethyl)-5-(2-nitrophenyl)-2-(4-nitrophenyl)oxazole-4-carboxamide (278),
2-(4-cyclopropylphenyl)-N-(2-(dimethylamino)ethyl)-5-phenyloxazole-4-carboxamide (279),
2-(4-cyclopropylphenyl)-N-(2-(4-methylpiperazin-1-yl)ethyl)-5-phenyloxazole-4-carboxamide (280),
5-(2-nitrophenyl)-N-(2-(piperazin-1-yl)ethyl)-2-(4-(trifluoromethyl)phenyl)oxazole-4-carboxamide (281),
N-(2-aminoethyl)-5-(2-nitrophenyl)-2-(4-(trifluoromethyl)phenyl)oxazole-4-carboxamide (282),
2-(3,5-difluorophenyl)-5-(2-nitrophenyl)-N-(2-(piperazin-1-yl)ethyl)oxazole-4-carboxamide (283),
N-(2-(4-acetylpiperazin-1-yl)ethyl)-5-(2-nitrophenyl)-2-(4-(trifluoromethyl)phenyl)oxazole-4-carboxamide (284),
N-(2-acetamidoethyl)-5-(2-nitrophenyl)-2-(4-(trifluoromethyl)phenyl)oxazole-4-carboxamide (285),
N-(2-(4-acetylpiperazin-1-yl)ethyl)-2-(3,5-difluorophenyl)-5-(2-nitrophenyl)oxazole-4-carboxamide (286),
N-(2-(4-(methylsulfonyl)piperazin-1-yl)ethyl)-5-(2-nitrophenyl)-2-(4-(trifluoromethyl)phenyl)oxazole-4-carboxamide (287),
N-(2-(methylsulfonamido)ethyl)-5-(2-nitrophenyl)-2-(4-(trifluoromethyl)phenyl)oxazole-4-carboxamide (288),
N-(2-guanidinoethyl)-5-(2-nitrophenyl)-2-(4-(trifluoromethyl)phenyl)oxazole-4-carboxamide (289),
N-methyl-5-(3-(methylthio)phenyl)-2-(4-(trifluoromethyl)phenyl)oxazole-4-carboxamide (290),
N-ethyl-5-(3-(methylthio)phenyl)-2-(4-(trifluoromethyl)phenyl)oxazole-4-carboxamide (291),
5-(3-(methylthio)phenyl)-N-propyl-2-(4-(trifluoromethyl)phenyl)oxazole-4-carboxamide (292),
N-(2-methoxyethyl)-5-(3-(methylthio)phenyl)-2-(4-(trifluoromethyl)phenyl)oxazole-4-carboxamide (293),
N-ethyl-5-phenyl-2-(4-(trifluoromethyl)phenyl)oxazole-4-carboxamide (294),
5-phenyl-N-propyl-2-(4-(trifluoromethyl)phenyl)oxazole-4-carboxamide (295),
N-ethyl-5-(2-methoxyphenyl)-2-(4-(trifluoromethyl)phenyl)oxazole-4-carboxamide (296),
5-(2-methoxyphenyl)-N-propyl-2-(4-(trifluoromethyl)phenyl)oxazole-4-carboxamide (297),
N-ethyl-5-(3-methoxyphenyl)-2-(4-(trifluoromethyl)phenyl)oxazole-4-carboxamide (298),
5-(3-methoxyphenyl)-N-propyl-2-(4-(trifluoromethyl)phenyl)oxazole-4-carboxamide (299),
N-ethyl-5-(4-methoxyphenyl)-2-(4-(trifluoromethyl)phenyl)oxazole-4-carboxamide (300),
5-(4-methoxyphenyl)-N-propyl-2-(4-(trifluoromethyl)phenyl)oxazole-4-carboxamide (301),
5-(3,4-dimethoxyphenyl)-N-ethyl-2-(4-(trifluoromethyl)phenyl)oxazole-4-carboxamide (302),
5-(3,5-dimethoxyphenyl)-N-ethyl-2-(4-(trifluoromethyl)phenyl)oxazole-4-carboxamide (303),
N-ethyl-2-(4-(trifluoromethyl)phenyl)-5-(3,4,5-trimethoxyphenyl)oxazole-4-carboxamide (304),
N-ethyl-5-(2-(trifluoromethoxy)phenyl)-2-(4-(trifluoromethyl)phenyl)oxazole-4-carboxamide (305),
N-propyl-5-(2-(trifluoromethoxy)phenyl)-2-(4-(trifluoromethyl)phenyl)oxazole-4-carboxamide (306),
N-ethyl-5-(pyridin-3-yl)-2-(4-(trifluoromethyl)phenyl)oxazole-4-carboxamide (307),
N-propyl-5-(pyridin-3-yl)-2-(4-(trifluoromethyl)phenyl)oxazole-4-carboxamide (308),
N-ethyl-5-(pyridin-4-yl)-2-(4-(trifluoromethyl)phenyl)oxazole-4-carboxamide (309),
N-propyl-5-(pyridin-4-yl)-2-(4-(trifluoromethyl)phenyl)oxazole-4-carboxamide (310),
5-(3-cyanophenyl)-N-ethyl-2-(4-(trifluoromethyl)phenyl)oxazole-4-carboxamide (311),
N-ethyl-2-(4-methoxyphenyl)-5-phenyloxazole-4-carboxamide (312),
2-(3,5-difluorophenyl)-N-ethyl-5-phenyloxazole-4-carboxamide (313),
2-(3-chlorophenyl)-N-ethyl-5-phenyloxazole-4-carboxamide (314),
2-(4-cyanophenyl)-N-ethyl-5-phenyloxazole-4-carboxamide (315),
N-ethyl-2-(4-(methylthio)phenyl)-5-phenyloxazole-4-carboxamide (316),
2-(4-(methylthio)phenyl)-5-phenyl-N-propyloxazole-4-carboxamide (317),
N-ethyl-5-(3-hydroxyphenyl)-2-(4-(trifluoromethyl)phenyl)oxazole-4-carboxamide (318),
5-(3-hydroxyphenyl)-N-propyl-2-(4-(trifluoromethyl)phenyl)oxazole-4-carboxamide (319),
N-ethyl-5-(4-hydroxyphenyl)-2-(4-(trifluoromethyl)phenyl)oxazole-4-carboxamide (320),
5-(3,4-dihydroxyphenyl)-N-ethyl-2-(4-(trifluoromethyl)phenyl)oxazole-4-carboxamide (321),
5-(3,5-dihydroxyphenyl)-N-ethyl-2-(4-(trifluoromethyl)phenyl)oxazole-4-carboxamide (322),
N-ethyl-2-(4-(trifluoromethyl)phenyl)-5-(3,4,5-trihydroxyphenyl)oxazole-4-carboxamide (323), and
N-ethyl-2-(4-hydroxyphenyl)-5-phenyloxazole-4-carboxamide (324).

The compound represented by Formula I according to the present invention can be prepared and used in the form of prodrugs, hydrates, solvates and pharmaceutically acceptable salts to enhance *in vivo* absorption or increase solubility, so the prodrugs, hydrates, solvates and pharmaceutically acceptable salts are also within the scope of the present invention.

The term "prodrug" refers to a substance that is transformed into a parent drug *in vivo.* Prodrugs are often used because, in some cases, they are easier to administer than the parent drug. For example, they may be bioavailable by oral administration, whereas the parent drug may not be. Prodrugs may also have improved solubility in pharmaceutical compositions than the parent drug. For example, a prodrug may be an *in vivo* hydrolysable ester of the compound according to the present invention and a pharmaceutically acceptable salt thereof. Another example of a prodrug may be a short peptide (polyamino acid) in which the peptide is coupled to an acid group that is metabolically converted to reveal the active site.

The term "hydrate" refers to a compound of the present invention or a salt thereof containing a stoichiometric or non-stoichiometric amount of water bound by non-covalent intermolecular forces.

The term "solvate" refers to a compound of the present invention or a salt thereof containing a stoichiometric or non-stoichiometric amount of solvent bound by non-covalent intermolecular forces. Preferred solvents therefor include solvents that are volatile, non-toxic, and/or suitable for administration to humans.

The term "isomer" refers to a compound of the present invention or a salt thereof that has the same chemical formula or molecular formula but is structurally or sterically different. Such isomers include both structural isomer such as tautomer, and stereoisomers such as R or S isomers with asymmetric carbon center and geometric isomers (trans, cis). All of these isomers and their mixtures thereof are also included within the scope of the present invention.

The term "pharmaceutically acceptable salt" refers to a salt form of a compound that does not cause serious irritation to the organism to which the compound is administered and does not impair the biological activity and physical properties of the compound. The pharmaceutical salts include an acid addition salt formed by an acid containing a pharmaceutically acceptable anion and forming a non-toxic acid addition salt, for example, inorganic acids such as hydrochloric acid, sulfuric acid, nitric acid, phosphoric acid, hydrobromic acid, hydrogen iodide, etc., organic carbon acids such as tartaric acid, formic acid, citric acid, acetic acid, trichloroacetic acid, trifluoroacetic acid, gluconic acid, benzoic acid, lactic acid, fumaric acid, malic acid, salicylic acid, etc., sulfonic acids such as methanesulfonic acid, ethanesulfonic acid, benzenesulfonic acid, p-toluenesulfonic acid, etc. For example, pharmaceutically acceptable carboxylic acid salts include metal salts or alkaline earth metal salts formed by lithium, sodium, potassium, calcium, magnesium, etc., amino acid salts such as lysine, arginine, guanidine, etc., organic salts such as dicyclohexylamine, N-methyl-D-glucamine, tris(hydroxymethyl)methylamine, diethanolamine, choline and triethylamine etc. The compound of Formula I according to the present invention can also be converted into its salt by conventional methods.

The oxazole derivative of Formula I of the present invention may be synthesized according to Scheme 1 to Scheme 5 below. After synthesizing compound **(2)** in which the amine group at C2 of the oxazole ring is replaced by a chloride group using the Sandmeyer reaction, compounds **(3-26)** are synthesized in which phenyl rings with various functional groups substituted at the *ortho, meta* and *para* positions are introduced to C2 of oxazole using the Suzuki-Miyaura cross-coupling reaction (see Scheme 1). Subsequently, compounds **(27-113)** are synthesized by introducing phenyl rings substituted with various functional groups via Heck reaction on C5 of oxazole (see Scheme 2). The compounds **(114-280)** are synthesized via amide coupling after hydrolysis of ethyl carboxylate at C4 of oxazole under basic conditions (see Scheme 3).

Schemes 1 to 5 are exemplified as a process for synthesizing the compounds of Formula I of the present invention, and the process for synthesizing of these Schemes 1 toh 5 are not intended to be limiting the methods for preparing the compounds of Formula I according to the present invention. It is obvious that the process for synthesis in Scheme 1 to Scheme 5 are exemplary only, and can be readily modified by those skilled in the art depending on the specific substituents.

The present invention also provides a pharmaceutical composition for preventing or treating a disease associated with IL-33 comprising an oxazole derivative compound represented by the above Formula I, a hydrate thereof, a solvate thereof or a pharmaceutically acceptable salt thereof as an active ingredient.

The present invention also provides a pharmaceutical composition for preventing or treating an allergic disease comprising an oxazole derivative compound represented by the above Formula I, a hydrate thereof, a solvate thereof or a pharmaceutically acceptable salt thereof as an active ingredient.

The present invention also provides a pharmaceutical composition for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease, comprising an oxazole derivative compound represented by the above Formula I, a hydrate thereof, a solvate thereof or a pharmaceutically acceptable salt thereof.

The present invention also provides a method for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease, comprising: administering the an oxazole derivative compound represented by the above Formula I, a hydrate thereof, a solvate thereof or a pharmaceutically acceptable salt thereof, to a subject in need thereof.

The present invention also provides a use of an oxazole derivative compound represented by the above Formula I, a hydrate thereof, a solvate thereof or a pharmaceutically acceptable salt thereof in manufacture of a medicament for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease.

The present invention also provides a dietary supplement composition for improving symptoms of an allergic disease, comprising an oxazole derivative compound represented by the above Formula I, a hydrate thereof, a solvate thereof or a pharmaceutically acceptable salt thereof.

The present invention also provides a pharmaceutical composition comprising an oxazole derivative compound represented by the above Formula I, a hydrate thereof, a solvate thereof or a pharmaceutically acceptable salt thereof and a pharmaceutically acceptable additive.

The additive may include a pharmaceutically acceptable carrier or diluent, each of which may be formulated according to conventional methods in the form of oral formulations such as powders, granules, tablets, capsules, suspensions, emulsions, syrups, aerosols; topicals; suppositories; and sterile injectable solutions.

The pharmaceutically acceptable carriers include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate and mineral oil, and the like. They also include diluents or excipients such as fillers, bulking agents, binders, wetting agents, disintegrating agents, and surfactants. Oral solid dosage forms include tablets, pills, powders, granules, capsules, and the like, which may include at least one excipient, such as starch, calcium carbonate, sucrose, lactose, gelatin, and the like, and may include lubricants such as magnesium stearate and talc. Oral liquid preparations may include suspensions, oral solutions, emulsions, syrups, and the like, and may include diluents such as water and liquid paraffin, wetting agents, sweeteners, flavourings, preservatives, and the like. Parenteral preparations include sterilized aqueous solutions, non-aqueous solvents, suspensions, emulsions, creams, lyophilised preparations, and suppositories; non-aqueous solvents and suspensions include propylene glycol, polyethylene glycol, vegetable oils such as olive oil, and injectable esters such as ethylolate. Substrates for suppositories may be witepsol, macrogol, tween 61, cacao gum, laurin gum, glycerogelatin, etc.

The dosage of the active ingredient in the pharmaceutical composition of the present invention depends on the condition and weight of the patient, the extent of the disease, the formulation of the active ingredient, the route and duration of administration, and may be appropriately adjusted depending on the patient. For example, the active ingredient can be administered at a dose of 0.0001 to 1000 mg/kg per day, preferably 0.01 to 100 mg/kg, and the dose may be administered once or in several divided doses per day. Furthermore, the pharmaceutical composition of the present invention may comprise the active ingredient from 0.001 to 90% by weight, based on the total weight of the composition.

The pharmaceutical composition of the present invention may be administered to mammals such as rats, mice, livestock, and humans by various routes, for example, orally, by dermal, intraperitoneally, rectally or intravenously, intramuscular, subcutaneous, intrauterine dura, or intracerebroventricular injection.

Hereinafter, the present disclosure is described in more detail with Synthesis Examples, Examples and Experimental examples. However, the following Synthesis Examples, Examples and Experimental examples are intended to illustrate the present invention, and the scope of the present invention is not limited thereto.

### Synthesis Example 1. Synthesis of oxazole compound

### (1) Summary of synthesis reactions

The oxazole derivative of the present invention was synthesized according to Schemes 1 to 5 below. Compound **2** was synthesized by replacing the amine group at C2 of the oxazole ring with a chloride group using the Sandmeyer reaction. Using the Suzuki-Miyaura cross-coupling reaction, compound **3-26** was synthesized by introducing a phenyl ring with various functional groups substituted at the *ortho, meta,* and *para* positions to C2 of oxazole (see Scheme 1).

Compound **27-113** was synthesized by introducing a phenyl ring substituted with various functional groups at C5 of oxazole through a Heck reaction (see Scheme 2). After hydrolysis of ethyl carboxylate at C4 of oxazole under basic conditions, the final compound **114-280** was synthesized through amide coupling (see Scheme 3).

### (2) Material and methods

All chemicals and solvents used in the reaction were purchased from Sigma-Aldrich, TCI and Acros and were used without further purification. The reaction progress was monitored by thin-layer chromatography (TLC) on precoated silica gel plates with silica gel 60F₂₅₄ (Merck; Darmstadt, Germany) and visualized by UV254 light and/or KMnO₄ staining for detection purpose Column chromatography was performed on a silica gel (silica gel 60; 230-400 mesh ASTM, Merck, Darmstadt, Germany). Nuclear magnetic resonance (NMR) spectra were recorded at room temperature on a Bruker UltraShield 600 MHz Plus (¹H, 600 MHz; ¹³C, 150 MHz) spectrometer. All chemical shifts are reported in parts per million (ppm) from tetramethylsilane (*δ* = 0) and were measured relative to the solvent in which the sample was analyzed (CDCl₃: *δ* 7.26 for ¹H NMR, *δ* 77.0 for ¹³C NMR; MeOD: *δ* 3.31 for ¹H NMR, *δ* 49.0 for ¹³C NMR; DMSO-*d*₆: 2.50 for ¹H NMR, *δ* 39.5 for ¹³C NMR). The ¹ H NMR shift values are reported as chemical shift (*δ*), the corresponding integral, multiplicity (s = singlet, br = broad, d = doublet, t = triplet, q = quartet, m = multiplet, dd = doublet of doublets, td = triplet of doublets, qd = quartet of doublets), coupling constant (*J* in Hz) and assignments. High resolution mass spectra (HRMS) were recorded on an Agilent 6530 Accurate Mass Q-TOF LC/MS spectrometer.

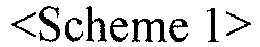

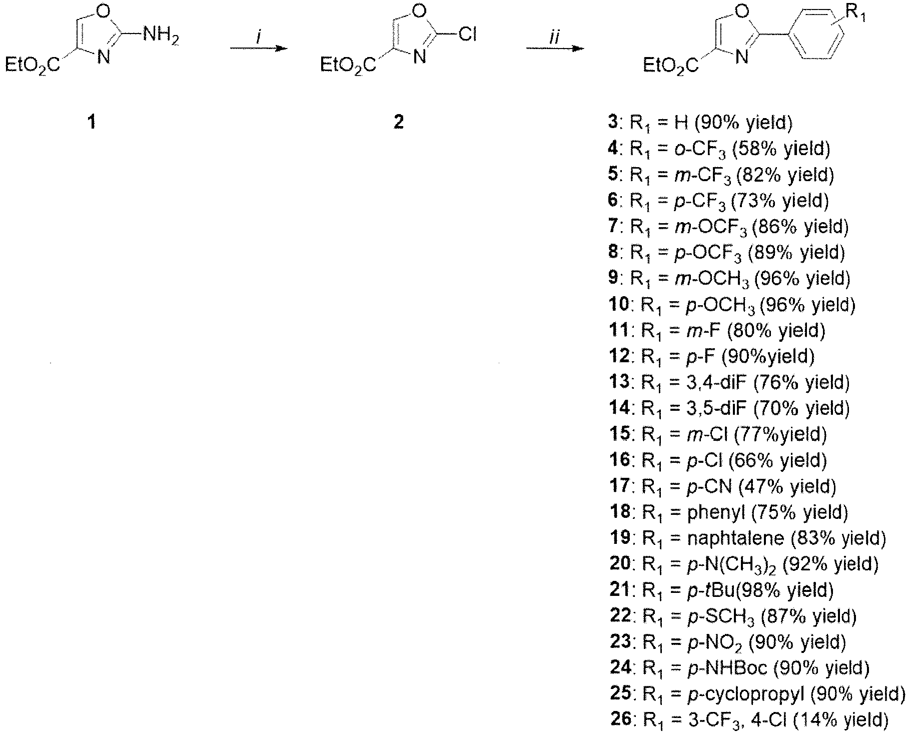

### [Reagents and condition : i) t-BuONO, CuCl₂, acetonitrile, 80 °C, 6 h; ii) appropriate boronic acid, 2 M K₂CO₃, toluene, 90 °C, 12 h.]

### (3) Synthesis of Ethyl 2-chlorooxazole-4-carboxylate (2)

Ethyl 2-aminooxazole-4-carboxylate (5.0 g, 32 mmol) was added in portions to a solution of tert-butyl nitrite (5.7 mL, 48 mmol) and copper (II) chloride (6.5 g, 48 mmol) in acetonitrile (150 mL) at 60 °C. The reaction mixture was then stirred at 80 °C for 6 h (until the disappearance of the starting material by TLC). The reaction mixture was poured into a mixture of ice and coned HCl and extracted with CH₂Cl₂. The combined organics washed with brine, dried over MgSO₄, filtered and evaporated under reduced pressure. The crude products were purified by column chromatography on silica gel (eluting with hexane : Et₂O, 7:1 to 4:1, v/v) to afford compound **2** as a white solid (3.69 g, 66%). ¹H NMR (300 MHz, CDCl₃) *δ* 8.20 (s, 1H), 4.40 (q, *J=* 7.2 Hz, 2H), 1.39 (t, *J=* 6.9 Hz, 3H). LRMS (ESI) *m*/*z* 176.1 [M + H]⁺. All spectroscopic data were in complete agreement with those reported previously.

### Synthesis Example 2. Typical procedure Suzuki coupling for the synthesis of ethyl 2-phenyloxazole-4-carboxylate (3)

The ethyl 2-chlorooxazole-4-carboxylate (1.0 g, 5.69 mmol) (compound **2),** phenylboronic acid (1.04 g, 8.54 mmol, 1.5 eq) and tetrakis(triphenylphosphine) palladium (0) (806 mg, 0.28 mmol, 0.05 eq) were dissolved in toluene (40 mL) and then added 2.0 M potassium carbonate solution (4.0 mL, 8.0 mmol) at room temperature. The reaction mixture was heated at 80° C for 12 h under an argon atmosphere. The reaction mixture was cooled to room temperature, water and 3 N HCl were added, and extracted with EtOAc. The organic layer was washed with brine, dried over MgSO₄, filtered, and concentrated *in vacuo.* The crude products were purified by column chromatography on silica gel (eluting with hexane : Et₂O, 5:1 to 2:1, v/v) to afford compound **3** as a white solid (1.11 g, 90%). ¹H NMR (600 MHz, CDCl₃) *δ* 8.28 (s, 1H), 8.12 (dd, *J=* 7.8 Hz and *J =* 1.2 Hz, 2H), 7.51-7.46 (m, 3H), 4.43 (q, *J=* 7.2 Hz, 2H), 1.41 (t, *J* = 7.2 Hz, 3H).

Compound **4-26** were prepared using a similar method as described for compound **3.**

### Synthesis Example 3. Ethyl 2-(2-(trifluoromethyl)phenyl)oxazole-4-carboxylate (4)

This compound was obtained in 58% yield as a white solid, following the same procedure described for the synthesis of compound **3** with 3-(trifluoromethyl)phenylboronic acid instead of phenylboronic acid. ¹H NMR (600 MHz, CDCl₃) *δ* 8.37 (s, 1H), 8.09 (d, *J* = 7.2 Hz, 1H), 7.83 (d, *J=* 7.2 Hz, 1H), 7.69-7.64 (m, 2H), 4.45 (q, *J* = 7.2 Hz, 2H), 1.42 (t, *J* = 7.2 Hz, 3H).

### Synthesis Example 4. Ethyl 2-(3-(trifluoromethyl)phenyl)oxazole-4-carboxylate (5)

This compound was obtained in 82% yield as a white solid, following the same procedure described for the synthesis of compound **3** with 3-(trifluoromethyl)phenylboronic acid instead of phenylboronic acid. ¹H NMR (600 MHz, CDCl₃) *δ* 8.41 (s, 1H), 8.34 (s, 1H), 8.32 (s, 1H), 7.77 (d, *J=* 7.8 Hz, 1H), 7.63 (t, *J* = 7.8 Hz, 1H), 4.46 (q, *J=* 7.2 Hz, 2H), 1.43 (t, *J=* 7.2 Hz, 3H).

### Synthesis Example 5. Ethyl 2-(4-(trifluoromethyl)phenyl)oxazole-4-carboxylate (6)

This compound was obtained in 73% yield as a white solid, following the same procedure described for the synthesis of compound **3** with 4-(trifluoromethyl)phenylboronic acid instead of phenylboronic acid. ¹H NMR (600 MHz, CDCl₃) *δ* 8.33 (s, 1H), 8.26 (d, *J=* 8.4 Hz, 2H), 7.76 (d, *J=* 8.4 Hz, 2H), 4.46 (q, *J=* 7.2 Hz, 2H), 1.43 (t, *J=* 7.2 Hz, 3H).

### Synthesis Example 6. Ethyl 2-(3-(trifluoromethoxy)phenyl)oxazole-4-carboxylate (7)

This compound was obtained in 86% yield as a white solid, following the same procedure described for the synthesis of compound **3** with 3-(trifluoromethoxy)phenylboronic acid instead of phenylboronic acid. ¹H NMR (600 MHz, CDCl₃) *δ* 8.30 (s, 1H), 8.08 (d, *J=* 7.8 Hz, 1H), 7.99 (s, 1H), 7.53 (t, *J=* 7.8 Hz, 1H), 7.38-7.37 (m, 1H), 4.45 (q, *J* = 7.2 Hz, 2H), 1.43 (t, *J* = 7.2 Hz, 3H).

### Synthesis Example 7. Ethyl 2-(4-(trifluoromethoxy)phenyl)oxazole-4-carboxylate (8)

This compound was obtained in 89% yield as a white solid, following the same procedure described for the synthesis of compound **3** with 4-(trifluoromethoxy)phenylboronic acid instead of phenylboronic acid. ¹H NMR (600 MHz, CDCl₃) *δ* 8.29 (s, 1H), 8.17 (d, *J=* 9.0 Hz, 2H), 7.33 (d, *J=* 9.0 Hz, 2H), 4.45 (q, *J=* 7.2 Hz, 2H), 1.42 (t, *J=* 7.2 Hz, 3H).

### Synthesis Example 8. Ethyl 2-(3-methoxyphenyl)oxazole-4-carboxylate (9)

This compound was obtained in 96% yield as a white needlelike crystal, following the same procedure described for the synthesis of compound **3** with 3-methoxyphenylboronic acid instead of phenylboronic acid. ¹H NMR (600 MHz, CDCl₃) *δ* 8.23 (s, 1H), 7.63 (d, *J=* 7.8 Hz, 1H), 7.60 (s, 1H), 7.32 (t, *J* = 7.8 Hz, 1H), 6.99 (dd, *J=* 1.8 Hz and *J =* 7.8 Hz, 1H), 4.38 (q, *J* = 7.2 Hz, 2H), 3.81 (s, 3H), 1.36 (t, *J=* 7.2 Hz, 3H).

### Synthesis Example 9. Ethyl 2-(4-methoxyphenyl)oxazole-4-carboxylate (10)

This compound was obtained in 96% yield as a white solid, following the same procedure described for the synthesis of compound **3** with 4-methoxyphenylboronic acid instead of phenylboronic acid. ¹H NMR (600 MHz, CDCl₃) *δ* 8.23 (s, 1H), 8.06 (d, *J=* 9.0 Hz, 2H), 6.99 (d, *J=* 9.0 Hz, 2H), 4.44 (q, *J=* 7.2 Hz, 2H), 3.88 (s, 3H), 1.42 (t, *J=* 7.2 Hz, 3H).

### Synthesis Example 10. Ethyl 2-(3-fluorophenyl)oxazole-4-carboxylate (11)

This compound was obtained in 80% yield as a white solid, following the same procedure described for the synthesis of compound **3** with 3-fluorophenylboronic acid instead of phenylboronic acid. ¹H NMR (600 MHz, CDCl₃) *δ* 8.29 (s, 1H), 7.92 (d, *J=* 7.8 Hz, 1H), 7.83 (d, *J* = 9.6 Hz, 1H), 7.50-7.46 (m, 1H), 7.20 (td, *J=* 8.4 Hz and *J =* 2.4 Hz, 1H), 4.45 (q, *J=* 7.2 Hz, 2H), 1.42 (t, *J=* 7.2 Hz, 3H).

### Synthesis Example 11. Ethyl 2-(4-fluorophenyl)oxazole-4-carboxylate (12)

This compound was obtained in 90% yield as a white solid, following the same procedure described for the synthesis of compound 3 with 4-fluorophenylboronic acid instead of phenylboronic acid. ¹H NMR (600 MHz, CDCl₃) *δ* 8.27 (s, 1H), 8.12 (dd, *J=* 5.4 Hz and *J* = 8.4 Hz, 2H), 7.17 (t, *J* = 8.4 Hz, 2H), 4.44 (q, *J* = 7.2 Hz, 2H), 1.42 (t, *J* = 7.2 Hz, 3H).

### Synthesis Example 12. Ethyl 2-(3,4-difluorophenyl)oxazole-4-carboxylate (13)

This compound was obtained in 76% yield as a white solid, following the same procedure described for the synthesis of compound **3** with 3,4-difluorophenylboronic acid instead of phenylboronic acid. ¹H NMR (600 MHz, CDCl₃) *δ* 8.28 (s, 1H), 7.99-7.95 (m, 1H), 7.92-7.89 (m, 1H), 7.32-7.29 (m, 1H), 4.45 (q, *J=* 7.2 Hz, 2H), 1.42 (t, *J=* 7.2 Hz, 3H).

### Synthesis Example 13. Ethyl 2-(3,5-difluorophenyl)oxazole-4-carboxylate (14)

This compound was obtained in 70% yield as a white solid, following the same procedure described for the synthesis of compound **3** with 3,5-difluorophenylboronic acid instead of phenylboronic acid. ¹H NMR (600 MHz, CDCl₃) *δ* 8.30 (s, 1H), 7.67-7.64 (m, 2H), 6.99-6.95 (m, 1H), 4.45 (q, *J=* 7.2 Hz, 2H), 1.42 (t, *J=* 7.2 Hz, 3H).

### Synthesis Example 14. Ethyl 2-(3-chlorophenyl)oxazole-4-carboxylate (15)

This compound was obtained in 77% yield as a white solid, following the same procedure described for the synthesis of **3** with 3-chlorophenylboronic acid instead of phenylboronic acid. ¹H NMR (600 MHz, CDCl₃) *δ* 8.29 (s, 1H), 8.16-8.15 (m, 1H), 8.01 (d, *J* = 7.8 Hz, 1H), 7.50-7.48 (m, 1H), 7.43 (t, *J* = 7.8 Hz, 1H), 4.45 (q*, J =* 7.2 Hz, 2H), 1.42 (t*, J =* 7.2 Hz, 3H).

### Synthesis Example 15. Ethyl 2-(4-chlorophenyl)oxazole-4-carboxylate (16)

This compound was obtained in 66% yield as a white solid, following the same procedure described for the synthesis of compound 3 with 4-chlorophenylboronic acid instead of phenylboronic acid. ¹H NMR (600 MHz, CDCl₃) *δ* 8.28 (s, 1H), 8.06 (d, *J=* 8.4 Hz, 2H), 7.47 (d, *J=* 8.4 Hz, 2H), 4.45 (q, *J=* 7.2 Hz, 2H), 1.42 (t, *J=* 7.2 Hz, 3H).

### Synthesis Example 16. Ethyl 2-(4-cyanophenyl)oxazole-4-carboxylate (17)

This compound was obtained in 47% yield as a white solid, following the same procedure described for the synthesis of compound **3** with 4-cyanophenylboronic acid instead of phenylboronic acid. ¹H NMR (600 MHz, CDCl₃) *δ* 8.34 (s, 1H), 8.24 (d, *J* = 8.4 Hz, 2H), 7.79 (d, *J=* 8.4 Hz, 2H), 4.46 (q, *J=* 7.2 Hz, 2H), 1.43 (t, *J=* 7.2 Hz, 3H).

### Synthesis Example 17. Ethyl 2-([1,1'-biphenyl]-4-yl)oxazole-4-carboxylate (18)

This compound was obtained in 75% yield as a white solid, following the same procedure described for the synthesis of compound **3** with 4-biphenylboronic acid instead of phenylboronic acid. ¹H NMR (600 MHz, CDCl₃) *δ* 8.31 (s, 1H), 8.20 (d, *J=* 8.4 Hz, 2H), 7.72 (d, *J=* 8.4 Hz, 2H), 7.65 (d, *J=* 8.4 Hz, 2H), 7.48 (t, *J=* 7.2 Hz, 2H), 7.43-7.40 (m, 1H), 4.46 (q, *J=* 7.2 Hz, 2H), 1.43 (t, *J=* 7.2 Hz, 3H).

### Synthesis Example 18. Ethyl 2-(naphthalen-2-yl)oxazole-4-carboxylate (19)

This compound was obtained in 83% yield as a white solid, following the same procedure described for the synthesis of compound **3** with 2-naphthaleneboronic acid instead of phenylboronic acid. ¹H NMR (600 MHz, CDCl₃) *δ* 8.66 (s, 1H), 8.34 (s, 1H), 8.20 (dd, *J* = 1.8 Hz and *J* = 7.8 Hz, 1H), 7.97-7.95 (m, 2H), 7.89-7.88 (m, 1H), 7.59-7.54 (m, 2H), 4.47 (q, *J=* 7.2 Hz, 2H), 1.44 (t, *J=* 7.2 Hz, 3H).

### Synthesis Example 19. Ethyl 2-(4-(dimethylamino)phenyl)oxazole-4-carboxylate (20)

This compound was obtained in 92% yield as a fluffy white solid, following the same procedure described for the synthesis of compound **3** with 4-(dimethylamino)phenylboronic acid instead of phenylboronic acid.¹H NMR (600 MHz, CDCl₃) *δ* 8.19 (s, 1H), 7.98 (d, *J* = 9.0 Hz, 2H), 6.73 (d, *J=* 9.0 Hz, 2H), 4.43 (q, *J=* 7.2 Hz, 2H), 1.41 (t, *J=* 7.2 Hz, 3H).

### Synthesis Example 20. Ethyl 2-(4-(tert-butyl)phenyl)oxazole-4-carboxylate (21)

This compound was obtained in 98% yield as a fluffy white solid, following the same procedure described for the synthesis of compound **3** with 4-tert-butylphenylboronic acid instead of phenylboronic acid. ¹H NMR (600 MHz, CDCl₃) *δ* 8.26 (s, 1H), 8.05 (d, *J* = 9.0 Hz, 2H), 7.50 (d, *J* = 9.0 Hz, 2H), 4.44 (q, *J=* 7.2 Hz, 2H), 1.41 (t, *J* = 7.2 Hz, 3H), 1.36 (s, 9H).

### Synthesis Example 21. Ethyl 2-(4-(methylthio)phenyl)oxazole-4-carboxylate (22)

This compound was obtained in 87% yield as a fluffy white solid, following the same procedure described for the synthesis of compound **3** with 4-(methylthio)phenylboronic acid instead of phenylboronic acid. ¹H NMR (600 MHz, CDCl₃) *δ* 8.25 (s, 1H), 8.02 (d, *J* = 8.4 Hz, 2H), 7.31 (d, *J=* 8.4 Hz, 2H), 4.44 (q, *J=* 7.2 Hz, 2H), 2.54 (s, 3H), 1.42 (t, *J=* 7.2 Hz, 3H).

### Synthesis Example 22. Ethyl 2-(4-nitrophenyl)oxazole-4-carboxylate (23)

This compound was obtained in 90% yield as a fluffy white solid, following the same procedure described for the synthesis of compound **3** with 4-nitrophenylboronic acid instead of phenylboronic acid. ¹H NMR (600 MHz, CDCl₃) *δ* 8.37-8.35 (m, 3H), 8.32 (d, *J=* 9.0 Hz, 2H), 4.46 (q, *J=* 7.2 Hz, 2H), 1.43 (t, *J=* 7.2 Hz, 3H).

### Synthesis Example 23. Ethyl 2-(4-((tert-butoxycarbonyl)amino)phenyl)oxazole-4-carboxylate (24)

This compound was obtained in 90% yield as a fluffy white solid, following the same procedure described for the synthesis of compound **3** with (4*-((tert-*butoxycarbonyl)amino)phenyl)boronic acid instead of phenylboronic acid. ¹H NMR (600 MHz, CDCl₃) *δ* 8.24 (s, 1H), 8.05 (d, *J=* 9.0 Hz, 2H), 7.49 (d, *J* = 8.4 Hz, 2H), 4.43 (q, *J* = 7.1 Hz, 2H), 1.54 (s, 9H), 1.41 (t, *J=* 7.2 Hz, 3H).

### Synthesis Example 24. Ethyl 2-(4-cyclopropylphenyl)oxazole-4-carboxylate (25)

This compound was obtained in 90% yield as a fluffy white solid, following the same procedure described for the synthesis of compound **3** with 4-cyclopropyl-benzeneboronic acid instead of phenylboronic acid. ¹H NMR (600 MHz, CDCl₃) *δ* 8.25 (s, 1H), 8.00 (d, *J* = 8.4 Hz, 2H), 7.16 (d, *J =* 8.4 Hz, 2H), 4.44 (q, *J=* 7.2 Hz, 2H), 1.99-1.94 (m, 1H), 1.42 (t, *J =* 7.2 Hz, 3H), 1.07-1.04 (m, 2H), 0.80-0.77 (m, 2H).

### Synthesis Example 25. Ethyl 2-(4-chloro-3-(trifluoromethyl)phenyl)oxazole-4-carboxylate (26)

This compound was obtained in 14% yield as a fluffy white solid, following the same procedure described for the synthesis of compound **3** with (4-chloro-3-(trifluoromethyl)phenyl)boronic acid instead of phenylboronic acid. ¹H NMR (600 MHz, CDCl₃) *δ* 8.45 (s, 1H), 8.33 (s, 1H), 8.22 (d, *J=* 8.4 Hz, 1H), 7.64 (d, *J* = 8.4 Hz, 1H), 4.46 (q, *J=* 7.2 Hz, 2H), 1.43 (t, *J=* 7.2 Hz, 3H).

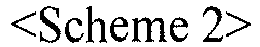

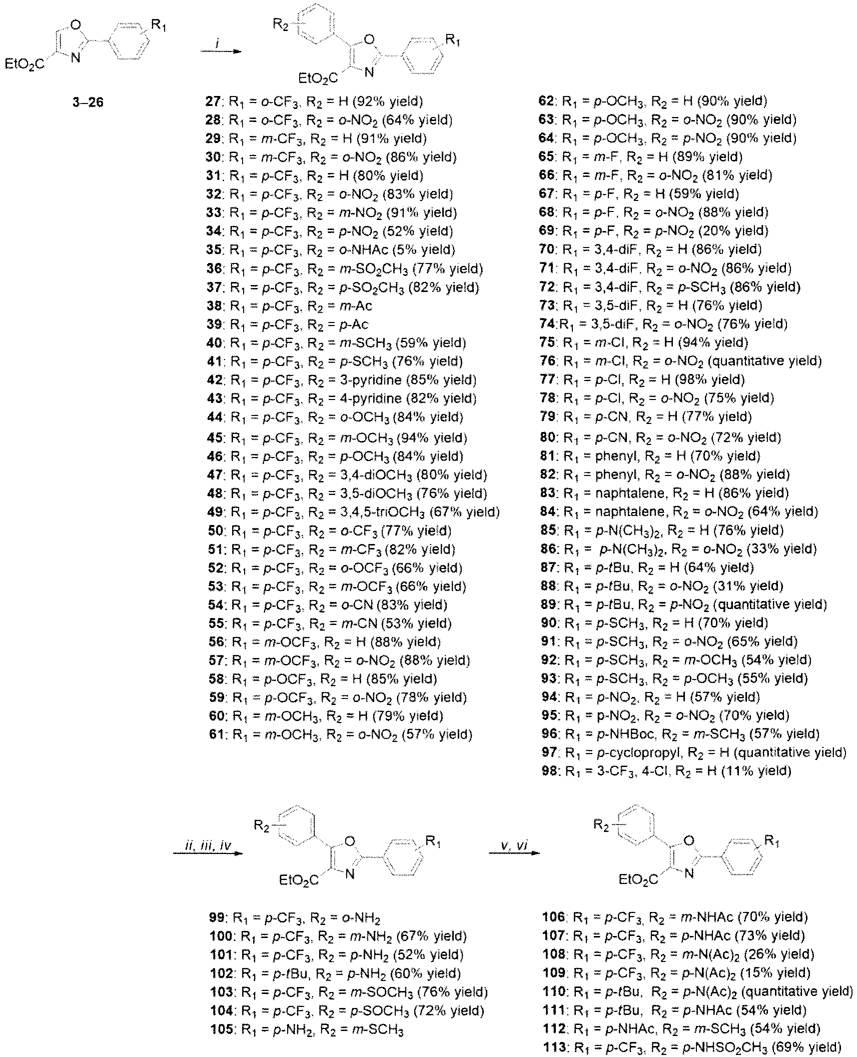

[*Reagents and condition* : *i*) appropriate iodobenzene, Cs₂CO₃, Pd(OAc)₂, tri(o-tolyl)phosphine, toluene, 90 °C, 12 h; *ii*) 10% Pd/C, H₂ gas, MeOH, rt, 12 h; *iii*) *m*-CPBA, anhydrous CH₂Cl₂, 0 °C, 4 h; iv) 25% TFA in anhydrous CH₂Cl₂, 0 °C to rt, 2 h; v) acetyl chloride, DIPEA, anhydrous CH₂Cl₂, 0 °C to rt, 6 h; vi) methanesulfonyl chloride, DIPEA, anhydrous CH₂Cl₂, 0 °C to rt, 6 **h.]**

### Example 1. Typical procedure Heck reaction for the synthesis of Ethyl 5-phenyl-2-(2-(trifluoromethyl)phenyl)oxazole-4-carboxylate (27)

A mixture of compound **4** (295 mg, 1.03 mmol), iodobenzene (138.24 mL, 1.83 mmol, 1.5 eq), palladium acetate (47 mg, 0.21 mmol, 0.2 eq), tri(o-tolyl)phosphine (64 mg, 0.21 mmol, 0.2 eq), cesium carbonate (505 mg, 1.55 mmol, 1.5 eq) in toluene (15 mL) was flushed with argon and stirred at 90 °C for 12 h.

The reaction mixture was cooled, diluted with water, and extracted three times with EtOAc. The combined organics washed with brine, dried over MgSO₄, filtered and evaporated under reduced pressure. The crude products were purified by column chromatography on silica gel (eluting with hexane : Et₂O = 3:1 to 1:1, v/v) to afford compound **27** as a white solid (342 mg, 92%). ¹H NMR (600 MHz, CDCl₃) *δ* 8.20 (d, *J=* 7.2 Hz, 1H), 8.17 (dd, *J=* 1.2 Hz and *J* = 8.4 Hz, 2H), 7.86 (d, *J=* 7.2 Hz, 1H), 7.70 (t, *J=* 7.2 Hz, 1H), 7.65 (t, *J=* 7.2 Hz, 1H), 7.53-7.48 (m, 3H), 4.48 (q, *J=* 7.2 Hz, 2H), 1.44 (t, 3H).

Compound **28-89** were prepared using a similar method as described for compound 27.

### Example 2. Ethyl 5-(2-nitrophenyl)-2-(2-(trifluoromethyl)phenyl)oxazole-4-carboxylate (28)

This compound was obtained in 64% yield as a white solid, following the same procedure described for the synthesis of compound **27** with 1-iodo-2-nitrobenzene instead of iodobenzene. ¹H NMR (600 MHz, CDCl₃) *δ* 8.19 (t, *J=* 7.8 Hz, 2H), 7.85 (d, *J=* 7.8 Hz, 1H), 7.78 (t, *J* = 7.2 Hz, 1H), 7.75-7.69 (m, 3H), 7.66 (t, *J* = 7.8 Hz, 1H), 4.33 (q, *J* = 7.2 Hz, 2H), 1.28 (t, *J* = 7.2 Hz, 3H).

### Example 3. Ethyl 5-phenyl-2-(3-(trifluoromethyl)phenyl)oxazole-4-carboxylate (29)

This compound was obtained in 91% yield as a white solid, following the same procedure described for the synthesis of compound **27.** ¹H NMR (600 MHz, CDCl₃) *δ* 8.43 (s, 1H), 8.36 (d, *J* = 7.8 Hz, 1H), 8.12 (dd, *J* = 1.8 Hz and *J* = 8.4 Hz, 2H), 7.77 (d, *J =* 8.4 Hz, 1H), 7.65 (t, *J* = 7.8 Hz, 1H), 7.55-7.51 (m, 3H), 4.48 (q, *J* = 7.2 Hz, 2H), 1.44 (t, *J* = 7.2 Hz, 3H).

### Example 4. Ethyl 5-(2-nitrophenyl)-2-(3-(trifluoromethyl)phenyl)oxazole-4-carboxylate (30)

This compound was obtained in 86% yield as a fluffy white solid, following the same procedure described for the synthesis of compound **27** with 1-iodo-2-nitrobenzene instead of iodobenzene. ¹H NMR (600 MHz, CDCl₃) *δ* 8.40 (s, 1H), 8.31 (d, *J* = 8.4 Hz, 1H), 8.21 (d*, J =* 8.4 Hz, 1H), 7.80-7.71 (m, 4H), 7.63 (t, *J* = 7.8 Hz, 1H), 4.33 (q, *J* = 7.2 Hz, 2H), 1.27 (t, *J* = 7.2 Hz, 3H).

### Example 5. Ethyl 5-phenyl-2-(4-(trifluoromethyl)phenyl)oxazole-4-carboxylate (31)

This compound was obtained in 80% yield as a white solid, following the same procedure described for the synthesis of compound **27.¹H** NMR (600 MHz, CDCl₃) *δ* 8.30 (d, *J* = 7.8 Hz, 2H), 8.12 (dd, *J* = 1.8 Hz and *J* = 7.8 Hz, 2H), 7.77 (d, *J=* 7.8 Hz, 2H), 7.54-7.50 (m, 3H), 4.49 (q, *J=* 7.2 Hz, 2H), 1.44 (t, *J=* 7.2 Hz, 3H).

### Example 6. Ethyl 5-(2-nitrophenyl)-2-(4-(trifluoromethyl)phenyl)oxazole-4-carboxylate (32)

This compound was obtained in 83% yield as a white solid, following the same procedure described for the synthesis of compound **27** with 1-iodo-2-nitrobenzene instead of iodobenzene. ¹H NMR (600 MHz, CDCl₃) *δ* 8.24 (d, *J =* 8.1 Hz, 2H), 8.21 (d, *J* = 10.5 Hz, 1H), 7.86-7.68 (m, 5H), 4.32 (q, *J* = 7.2 Hz, 2H), 1.27 (t, *J* = 7.2 Hz, 3H); ¹³C NMR (75 MHz, CDCl₃) *δ* 166.1, 159.9, 151.7, 148.5, 133.0, 132.6, 131.5, 130.5, 129.3, 127.3, 126.0, 125.9, 124.9, 122.4, 61.8, 14.0. LRMS (ESI) *m*/*z* 407.0 [M + H]⁺, 428.7 [M + Na]⁺ and 445.3 [M + K]⁺. HRMS (ESI) m/z calculated for C₁₉H₁₄F₃N₂O₅⁺ [M + H]⁺: 407.0849; found: 407.0809.

### Example 7. Ethyl 5-(3-nitrophenyl)-2-(4-(trifluoromethyl)phenyl)oxazole-4-carboxylate (33)

This compound was obtained in 91% yield as a fluffy white solid, following the same procedure described for the synthesis of compound **27** with 1-iodo-3-nitrobenzene with instead of iodobenzene. ¹H NMR (600 MHz, CDCl₃) *δ* 9.07 (s, 1H), 8.55 (d, *J=* 7.8 Hz, 1H), 8.39-8.37 (m, 1H), 8.34 (d, *J=* 8.4 Hz, 2H), 7.82 (d, *J* = 8.4 Hz, 2H), 7.75 (t, *J* = 8.4 Hz, 1H), 4.54 (q, *J* = 7.2 Hz, 2H), 1.48 (t, *J=* 7.2 Hz, 3H).

### Example 8. Ethyl 5-(4-nitrophenyl)-2-(4-(trifluoromethyl)phenyl)oxazole-4-carboxylate (34)

This compound was obtained in 52% yield, following the same procedure described for the synthesis of compound **27** with 1-iodo-4-nitrobenzene instead of iodobenzene. ¹H NMR (600 MHz, CDCl₃) *δ* 8.68 (td*, J =* 8.8 Hz and *J* = 1.9 Hz, 4H), 8.31 (d, *J* = 8.1 Hz, 2H), 7.79 (d, *J* = 8.1 Hz, 2H), 4.51 (q, *J* = 7.1 Hz, 2H), 1.46 (t, *J* = 7.1 Hz, 3H).

### Example 9. Ethyl 5-(2-acetamidophenyl)-2-(4-(trifluoromethyl)phenyl)oxazole-4-carboxylate (35)

This compound was obtained in 5% yield, following the same procedure described for the synthesis of compound 27 with 1-iodo-4-nitrobenzene instead of iodobenzene. ¹H NMR (600 MHz, CDCl₃) *δ* 8.61 (brs, 1H), 8.26 (d, *J=* 8.4 Hz, 2H), 7.96(d, *J* = 8.4 Hz, 1H), 7.78 (d, *J=* 8.4 Hz, 2H), 7.56 *(d, J=* 8.4 Hz, 1H), 7.33 *(t, J=* 7.2 Hz, 1H), 4.48 (q, *J=* 6.6 Hz, 2H), 2.09 (s, 3H), 1.43 (t, *J=* 7.2 Hz, 3H).

### Example 10. Ethyl 5-(3-(methylsulfonyl)phenyl)-2-(4-(trifluoromethyl)phenyl)oxazole-4-carboxylate (36)

This compound was obtained in 77% yield as a fluffy white solid, following the same procedure described for the synthesis of compound **27** with 3-bromophenyl methyl sulfone instead of iodobenzene. ¹H NMR (600 MHz, CDCl₃) *δ* 8.73 (s, 1H), 8.47 (d, *J* = 7.9 Hz, 1H), 8.31 (d, *J =* 8.1 Hz, 2H), 8.07 (d, *J* = 7.9 Hz, 1H), 7.78 (d, *J=* 8.3 Hz, 2H), 7.75 (t, *J=* 7.8 Hz, 1H), 4.50 (q, *J =* 7.1 Hz, 2H), 3.15 (s, 3H), 1.46 (t, *J* = 7.1 Hz, 3H). HRMS m/z: calcd for C₂₀H₁₆F₃NO₅S [M + H]⁺: 440.0735; found: 440.0809.

### Example 11. Ethyl 5-(4-(methylsulfonyl)phenyl)-2-(4-(trifluoromethyl)phenyl)oxazole-4-carboxylate (37)

This compound was obtained in 82% yield as a fluffy white solid, following the same procedure described for the synthesis of compound **27** with 4-bromophenyl methyl sulfone instead of iodobenzene. ¹H NMR (600 MHz, CDCl₃) *δ* 8.37 (d, *J=* 8.3 Hz, 2H), 8.30 (d, *J* = 7.8 Hz, 2H), 8.09 (d, *J* = 8.4 Hz, 2H), 7.79 (d, *J=* 7.9 Hz, 2H), 4.50 (q, *J* = 7.1 Hz, 2H), 3.11 (s, 3H), 1.46 (t, *J=* 7.1 Hz, 3H). HRMS m/z: cacld for C₂₀H₁₆F₃NO₅S [M + H]⁺: 440.0735; found: 440.0784.

### Example 12. Ethyl 5-(3-acetylphenyl)-2-(4-(trifluoromethyl)phenyl)oxazole-4-carboxylate (38)

This compound was obtained in 73% yield as a white solid, following the same procedure described for the synthesis of compound **27** with 3'-iodoacetophenone instead of iodobenzene. ¹H NMR (600 MHz, CDCl₃) *δ* 8.76 (s, 1H), 8.34-8.31 (m, 3H), 8.09 (d, *J=* 7.8 Hz, 1H), 7.65 (t, *J=* 7.8 Hz, 1H), 4.50 (q, *J=* 7.2 Hz, 2H), 2.71 (s, 3H), 1.45 (t, *J=* 7.2 Hz, 3H). HRMS m/z: calcd for C₂₁H₁₆F₃NO₄ [M + H]⁺: 404.1065; found: 404.1123.

### Example 13. Ethyl 5-(4-acetylphenyl)-2-(4-(trifluoromethyl)phenyl)oxazole-4-carboxylate (39)

This compound was obtained in 68% yield as a white solid, following the same procedure described for the synthesis of compound **27** with 4'-iodoacetophenone instead of iodobenzene. ¹H NMR (600 MHz, CDCl₃) *δ* 8.31 (d, *J=* 8.4 Hz, 2H), 8.26 (d, *J* = 8.4 Hz, 2H), 8.11 (d, *J* = 8.4 Hz, 2H), 7.78 (d, *J=* 8.4 Hz, 2H), 4.50 (q, *J* = 7.2 Hz, 2H), 2.68 (s, 3H), 1.46 (t, *J* = 7.2 Hz, 3H). HRMS m/z : calcd for C₂₁H₁₆F₃NO₄ [M + H]⁺: 404.1065; found: 404.1122.

### Example 14. Ethyl 5-(3-(methylthio)phenyl)-2-(4-(trifluoromethyl)phenyl)oxazole-4-carboxylate (40)

This compound was obtained in 59% yield as a fluffy white solid, following the same procedure described for the synthesis of compound **27** with 3-bromothioanisole instead of iodobenzene. ¹H NMR (600 MHz, CDCl₃) *δ* 8.31 (d, *J* = 8.1 Hz, 2H), 8.07 (s, 1H), 7.87 (d, *J=* 7.7 Hz, 1H), 7.79 (d, *J=* 8.2 Hz, 2H), 7.46 (t, *J=* 7.8 Hz, 1H), 7.40 (d, *J=* 8.0 Hz, 1H), 4.69 (q, *J=* 7.1 Hz, 2H), 2.60 (s, 3H), 1.46 (t, *J* = 7.1 Hz, 3H).

### Example 15. Ethyl 5-(4-(methylthio)phenyl)-2-(4-(trifluoromethyl)phenyl)oxazole-4-carboxylate (41)

This compound was obtained in 76% yield as a fluffy white solid, following the same procedure described for the synthesis of compound **27** with 4-bromothioanisole instead of iodobenzene. ¹H NMR (600 MHz, CDCl₃) *δ* 8.27 (d, *J=* 8.2 Hz, 2H), 8.07 (dd, *J=* 1.7 Hz and *J* = 6.8 Hz, 2H), 7.75 (d, *J* = 8.3 Hz, 2H), 7.35 (d, *J* = 8.6 Hz, 2H), 4.47 (q, *J =* 7.1 Hz, 2H), 2.55 (s, 3H), 1.44 (t, *J=* 7.1 Hz, 3H). HRMS m/z: calcd for C₂₀H₁₆F₃NO₃S [M + H]⁺: 408.0837; found: 408.0888.

### Example 16. Ethyl 5-(pyridin-3-yl)-2-(4-(trifluoromethyl)phenyl)oxazole-4-carboxylate (42)

This compound was obtained in 85% yield as a white solid, following the same procedure described for the synthesis of compound **27** with 3-iodopyridine instead of iodobenzene. ¹H NMR (600 MHz, CDCl₃) *δ* 9.26 (d, *J* = 1.8 Hz, 1H), 8.73 (dd, *J=* 1.8 Hz and *J* = 4.8 Hz, 1H), 8.53 (td, *J=* 8.1 Hz and *J* = 1.8 Hz, 1H), 8.30 (d, *J* = 7.8 Hz, 2H), 7.79 (d, *J* = 8.4 Hz, 2H), 7.47 (dd, *J=* 4.8 Hz and *J* = 7.8 Hz, 1H), 4.49 (q, *J=* 7.2 Hz, 2H), 1.45 (t, *J* = 7.2 Hz, 3H).

### Example 17. Ethyl 5-(pyridin-4-yl)-2-(4-(trifluoromethyl)phenyl)oxazole-4-carboxylate (43)

This compound was obtained in 82% yield as a white solid, following the same procedure described for the synthesis of compound 27 4-iodopyridine instead of iodobenzene. ¹H NMR (600 MHz, CDCl₃) *δ* 8.81-8.80 (m, 2H), 8.31 *(d, J=* 8.4 Hz, 2H), 8.08-8.07 (m, 2H), 7.79 (d, *J=* 8.4 Hz, 2H), 4.52 (q, *J=* 7.2 Hz, 2H), 1.47 (t, *J=* 7.2 Hz, 3H).

### Example 18. Ethyl 5-(2-methoxyphenyl)-2-(4-(trifluoromethyl)phenyl)oxazole-4-carboxylate (44)

This compound was obtained in 84% yield as a pale yellow solid, following the same procedure described for the synthesis of compound **27** with 1-iodo-2-methoxybenzene instead of iodobenzene. ¹H NMR (600 MHz, CDCl₃) *δ* 8.28 (d, *J=* 7.8 Hz, 2H), 7.76 (d, *J=* 7.8 Hz, 2H), 7.58 (dd, *J =* 1.8 Hz and *J =* 8.2 Hz, 1H), 7.53-7.50 (m, 1H), 7.11 (td, *J=* 7.5 Hz and *J* = 1.2 Hz, 1H), 7.05 (d, *J=* 8.4 Hz, 1H), 4.37 (q, *J=* 7.2 Hz, 2H), 1.30 (t, *J=* 6.9 Hz, 3H).

### Example 19. Ethyl 5-(3-methoxyphenyl)-2-(4-(trifluoromethyl)phenyl)oxazole-4-carboxylate (45)

This compound was obtained in 94% yield as a pale yellow solid, following the same procedure described for the synthesis of compound **27** with 1-iodo-3-methoxybenzene instead of iodobenzene. ¹H NMR (600 MHz, CDCl₃) *δ* 8.29 (d, *J=* 8.4 Hz, 2H), 7.77 (d, *J=* 8.4 Hz, 3H), 7.69 (d, *J* = 7.8 Hz, 1H), 7.44 (t, *J=* 7.8 Hz, 1H), 7.06 (dd, *J=* 2.4 Hz and *J* = 8.4 Hz, 1H), 4.48 (q, *J=* 7.2 Hz, 2H), 3.91 (s, 3H), 1.44 (t, *J=* 7.2 Hz, 3H).

### Example 20. Ethyl 5-(4-methoxyphenyl)-2-(4-(trifluoromethyl)phenyl)oxazole-4-carboxylate (46)

This compound was obtained in 84% yield as a white solid, following the same procedure described for the synthesis of compound **27** with 1-iodo-4-methoxybenzene instead of iodobenzene. ¹H NMR (600 MHz, CDCl₃) *δ* 8.27 (d, *J* = 8.4 Hz, 2H), 8.12 (d, *J* = 8.4 Hz, 2H), 7.76 (d, *J* = 8.4 Hz, 2H), 7.04 (d, *J* = 8.4 Hz, 2H), 4.48 (q, *J* = 6.6 Hz, 2H), 3.91 (s, 3H), 1.45 (t, *J* = 7.2 Hz, 3H).

### Example 21. Ethyl 5-(3,4-dimethoxyphenyl)-2-(4-(trifluoromethyl)phenyl)oxazole-4-carboxylate (47)

This compound was obtained in 80% yield as a white solid, following the same procedure described for the synthesis of compound **27** with 4-bromo-1,2-dimethoxybenzene instead of iodobenzene. ¹H NMR (600 MHz, CDCl₃) *δ* 8.28 (d, *J=* 7.8 Hz, 2H), 7.90 (d, *J* = 1.8 Hz, 1H), 7.77-7.75 (m, 3H), 7.00 (d, *J=* 8.4 Hz, 1H), 4.49 (q, *J* = 7.2 Hz, 2H), 4.01 (s, 3H), 3.98 (s, 3H), 1.46 (t, *J =* 7.2 Hz, 3H).

### Example 22. Ethyl 5-(3,5-dimethoxyphenyl)-2-(4-(trifluoromethyl)phenyl)oxazole-4-carboxylate (48)

This compound was obtained in 76% yield as a white solid, following the same procedure described for the synthesis of compound **27** with 1-bromo-3,5-dimethoxybenzene instead of iodobenzene. ¹H NMR (600 MHz, CDCl₃) *δ* 8.28 (d, *J=* 8.4 Hz, 2H), 7.77 (d, *J=* 9.0 Hz, 2H), 7.36 (d, *J* = 1.8 Hz, 2H), 4.48 (q, *J* = 7.2 Hz, 2H), 3.89 (s, 6H), 1.45 (t, *J* = 7.2 Hz, 3H).

### Example 23. Ethyl 2-(4-(trifluoromethyl)phenyl)-5-(3,4,5-trimethoxyphenyl)oxazole-4-carboxylate (49)

This compound was obtained in 67% yield as a white solid, following the same procedure described for the synthesis of compound **27** with 5-bromo-1,2,3-trimethoxybenzene instead of iodobenzene. ¹H NMR (600 MHz, CDCl₃) *δ* 8.28 (d, *J=* 7.8 Hz, 2H), 7.78 (d, *J* = 8.4 Hz, 2H), 7.52 (s, 2H), 4.49 (q, *J=* 7.2 Hz, 2H), 3.99 (s, 6H), 3.95 (s, 3H), 1.46 (t, *J=* 7.2 Hz, 3H).

### Example 24. Ethyl 5-(2-(trifluoromethyl)phenyl)-2-(4-(trifluoromethyl)phenyl)oxazole-4-carboxylate (50)

This compound was obtained in 77% yield as a pink solid, following the same procedure described for the synthesis of compound **27** with 2-iodobenzotrifluoride instead of iodobenzene. ¹H NMR (600 MHz, CDCl₃) *δ* 8.28 (d, *J=* 7.8 Hz, 2H), 7.78 (d, *J=* 8.4 Hz, 2H), 7.52 (s, 2H), 4.49 (q, *J* = 7.2 Hz, 2H), 3.99 (s, 6H), 3.95 (s, 3H), 1.46 (t, *J =* 7.2 Hz, 3H).

### Example 25. Ethyl 5-(3-(trifluoromethyl)phenyl)-2-(4-(trifluoromethyl)phenyl)oxazole-4-carboxylate (51)

This compound was obtained in 82% yield as a white solid, following the same procedure described for the synthesis of compound 27 with 3-iodobenzotrifluoride instead of iodobenzene. ¹H NMR (600 MHz, CDCl₃) *δ* 8.41 (s, 1H), 8.35 (d, *J* = 7.8 Hz, 1H), 8.31 (d, *J=* 7.8 Hz, 2H), 7.79 (d, *J=* 7.8 Hz, 2H), 7.77 (d, *J=* 7.8 Hz, 1H), 7.67 (t, *J=* 7.8 Hz, 1H), 4.50 (q, J= 7.2 Hz, 2H), 1.44 (t, *J=* 7.2 Hz, 2H).

### Example 26. Ethyl 5-(2-(trifluoromethoxy)phenyl)-2-(4-(trifluoromethyl)phenyl)oxazole-4-carboxylate (52)

This compound was obtained in 66% yield as a white solid, following the same procedure described for the synthesis of compound **27** with 1-iodo-2-(trifluoromethoxy)benzene instead of iodobenzene. ¹H NMR (600 MHz, CDCl₃) *δ* 8.29 (d, *J* = 7.8 Hz, 2H), 7.80-7.78 (m, 3H), 7.63-7.60 (m, 1H), 7.49-7.45 (m, 2H), 4.39 (q, *J=* 7.2 Hz, 2H), 1.32 (t, *J* = 6.6 Hz, 2H).

### Example 27. Ethyl 5-(3-(trifluoromethoxy)phenyl)-2-(4-(trifluoromethyl)phenyl)oxazole-4-carboxylate (53)

This compound was obtained in 66% yield as a white solid, following the same procedure described for the synthesis of compound **27** with 1-iodo-3-(trifluoromethoxy)benzene instead of iodobenzene.

¹H NMR (600 MHz, CDCl₃) *δ* 8.31 (d, *J=* 7.8 Hz, 2H), 8.12 (d, *J =* 7.8 Hz, 1H), 8.08 (s, 1H), 7.80 (d, *J=* 8.4 Hz, 2H), 7.58 (t, *J=* 8.4 Hz, 1H), 7.39 (d, *J=* 8.4 Hz, 1H), 4.52 (q, *J* = 7.8 Hz, 2H), 1.47 (t, *J=* 7.2 Hz, 3H).

### Example 28. Ethyl 5-(2-cyanophenyl)-2-(4-(trifluoromethyl)phenyl)oxazole-4-carboxylate (54)

This compound was obtained in 83% yield as a yellow solid, following the same procedure described for the synthesis of compound **27** with 2-iodobenzonitrile instead of iodobenzene. ¹H NMR (600 MHz, CDCl₃) *δ* 8.35 (d, *J=* 8.4 Hz, 2H), 8.06 (d, *J=* 8.4 Hz, 1H), 7.89 (d, *J* = 7.8 Hz, 1H), 7.80 (d, *J=* 8.4 Hz, 2H), 7.79 (d, *J =* 7.8 Hz, 1H), 7.65 (t, *J=* 7.8 Hz, 1H), 4.46 (q, *J=* 4.8 Hz, 2H), 1.40 (t, *J=* 5.4 Hz, 3H).

### Example 29. Ethyl 5-(3-cyanophenyl)-2-(4-(trifluoromethyl)phenyl)oxazole-4-carboxylate (55)

This compound was obtained in 53% yield as a white solid, following the same procedure described for the synthesis of compound **27** with 3-iodobenzonitrile instead of iodobenzene. ¹H NMR (600 MHz, CDCl₃) *δ* 8.50 (s, 1H), 8.45 (d, *J* = 7.8 Hz, 1H), 8.32 (d, *J* = 7.8 Hz, 2H), 7.80 (t, *J* = 7.8 Hz, 3H), 7.68 (t, *J* = 8.4 Hz, 1H), 4.52 (q, *J* = 4.8 Hz, 2H), 1.40 (t, *J* = 5.4 Hz, 3H).

### Example 30. Ethyl 5-phenyl-2-(3-(trifluoromethoxy)phenyl)oxazole-4-carboxylate (56)

This compound was obtained in 88% yield as a white solid, following the same procedure described for the synthesis of compound **27.** ¹H NMR (600 MHz, CDCl₃) *δ* 8.13-8.10 (m, 3H), 8.02 (s, 1H), 7.56-7.51 (m, 4H), 7.37 (d, *J=* 8.4 Hz, 1H), 4.48 (q, *J=* 7.2 Hz, 2H), 1.44 (t, *J = 7.2* Hz, 3H).

### Example 31. Ethyl 5-(2-nitrophenyl)-2-(3-(trifluoromethoxy)phenyl)oxazole-4-carboxylate (57)

This compound was obtained in 88% yield as a white solid, following the same procedure described for the synthesis of compound **27** with 1-iodo-2-nitrobenzene instead of iodobenzene. ¹H NMR (600 MHz, CDCl₃) *δ* 8.21 (dd, *J=* 1.2 Hz and *J* = 7.8 Hz, 1H), 8.08 (d, *J* = 7.8 Hz, 1H), 7.99 (s, 1H), 7.80-7.71 (m, 3H), 7.54 (t, *J* = 7.8 Hz, 1H), 7.40-7.38 (m, 1H), 4.33 (q, *J=* 7.2 Hz, 2H), 1.27 (t, *J=* 7.2 Hz, 3H).

### Example 32. Ethyl 5-phenyl-2-(4-(trifluoromethoxy)phenyl)oxazole-4-carboxylate (58)

This compound was obtained in 85% yield as a white solid, following the same procedure described for the synthesis of compound **27.** ¹H NMR (600 MHz, CDCl₃) *δ* 8.22 (d, *J* = 8.4 Hz, 2H), 8.11 (dd, *J* = 1.8 Hz and *J* = 7.8 Hz, 2H), 7.54-7.50 (m, 3H), 7.35 (d,J= 8.4 Hz, 2H), 4.48 (q, *J* = 7.2 Hz, 2H), 1.43 (t, *J* = 7.2 Hz, 3H).

### Example 33. Ethyl 5-(2-nitrophenyl)-2-(4-(trifluoromethoxy)phenyl)oxazole-4-carboxylate (59)

This compound was obtained in 78% yield as a white solid, following the same procedure described for the synthesis of compound **27** with 1-iodo-2-nitrobenzene instead of iodobenzene. ¹H NMR (600 MHz, CDCl₃) *δ* 8.20 (d, *J=* 8.4 Hz, 1H), 8.17 (d, *J=* 8.4 Hz, 2H), 7.80-7.71 (m, 3H), 7.34 (d, *J=* 8.4 Hz, 2H), 4.33 (q, *J=* 7.2 Hz, 2H), 1.27 (t, *J=* 7.2 Hz, 3H).

### Example 34. Ethyl 2-(3-methoxyphenyl)-5-phenyloxazole-4-carboxylate (60)

This compound was obtained in 79% yield as a fluffy white solid, following the same procedure described for the synthesis of compound 27. ¹H NMR (600 MHz, CDCl₃) *δ* 8.12 (d, *J=* 6.6 Hz, 2H), 7.76 (d, *J=* 6.6 Hz, 1H), 7.69 (s, 1H), 7.53-7.50 (m, 3H), 7.44-7.41 (m, 1H), 7.06 (d, *J=* 7.8 Hz, 1H), 4.47 (q, *J=* 7.2 Hz, 2H), 3.91 (s, 3H), 1.44 (t, *J=* 7.2 Hz, 3H).

### Example 35. Ethyl 2-(3-methoxyphenyl)-5-(2-nitrophenyl)oxazole-4-carboxylate (61)

This compound was obtained in 57% yield as a fluffy white solid, following the same procedure described for the synthesis of compound **27** with 1-iodo-2-nitrobenzene instead of iodobenzene. ¹H NMR (600 MHz, CDCl₃) *δ* 8.19 (d, *J* = 8.4 Hz, 1H), 7.79-7.77 (m, 2H), 7.72-7.69 (m, 2H), 7.66 (s, 1H), 7.39 (t, *J* = 7.8 Hz, 1H), 7.07 (ddd, *J* = 0.6 Hz, *J* = 2.4 Hz and *J* = 8.4 Hz, 1H), 4.33 (q, *J* = 7.2 Hz, 2H), 3.90 (s, 3H), 1.27 (t, *J* = 7.2 Hz, 3H).

### Example 36. Ethyl 2-(4-methoxyphenyl)-5-phenyloxazole-4-carboxylate (62)

This compound was obtained in 90% yield as a fluffy white solid, following the same procedure described for the synthesis of compound **27.** ¹H NMR (600 MHz, CDCl₃) *δ* 8.12-8.10 (m, 4H), 7.52-7.47 (m, 3H), 7.01 (d, *J* = 8.4 Hz, 2H), 4.47 (q, *J* = 7.2 Hz, 2H), 3.89 (s, 3H), 1.43 (t, *J* = 7.2 Hz, 3H).

### Example 37. Ethyl 2-(4-methoxyphenyl)-5-(2-nitrophenyl)oxazole-4-carboxylate (63)

This compound was obtained in 90% yield as a fluffy white solid, following the same procedure described for the synthesis of compound **27** with 1-iodo-2-nitrobenzene instead of iodobenzene. ¹H NMR (600 MHz, CDCl₃) *δ* 8.17 (d, *J=* 8.4 Hz, 1H), 8.06 (d, *J=* 8.4 Hz, 2H), 7.78-7.74 (m, 2H), 7.69 (s, 1H), 6.99 (d, *J=* 8.4 Hz, 2H), 4.33 (q, *J=* 7.2 Hz, 2H), 3.88 (s, 3H), 1.28 (t, *J* = 7.2 Hz, 3H).

### Example 38. Ethyl 2-(4-methoxyphenyl)-5-(4-nitrophenyl)oxazole-4-carboxylate (64)

This compound was obtained in 90% yield as a fluffy white solid, following the same procedure described for the synthesis of compound **27** with 1-iodo-4-nitrobenzene instead of iodobenzene. ¹H NMR (600 MHz, CDCl₃) *δ* 8.38 (d, *J=* 9.0 Hz, 2H), 8.35 (d, *J=* 9.0 Hz, 2H), 8.13 (d, *J* = 9.0 Hz, 2H), 7.03 (d, *J* = 9.0 Hz, 2H), 4.50 (q, *J=* 7.2 Hz, 2H), 3.91 (s, 3H), 1.47 (t, *J* = 7.2 Hz, 3H).

### Example 39. Ethyl 2-(3-fluorophenyl)-5-phenyloxazole-4-carboxylate (65)

This compound was obtained in 89% yield as a white solid, following the same procedure described for the synthesis of compound **27.** ¹H NMR (600 MHz, CDCl₃) *δ* 8.12-8.11 (m, 2H), 7.97 (d, *J* = 7.8 Hz, 1H), 7.87 (d, *J* = 9.0 Hz, 1H), 7.54-7.46 (m, 4H), 7.21 (td, *J* = 8.4 Hz and *J* = 2.4 Hz, 1H), 4.47 (q, *J* = 7.2 Hz, 2H), 1.44 (t, *J=* 7.2 Hz, 3H).

### Example 40. Ethyl 2-(3-fluorophenyl)-5-(2-nitrophenyl)oxazole-4-carboxylate (66)

This compound was obtained in 81% yield as a yellow solid, following the same procedure described for the synthesis of compound **27** with 1-iodo-2-nitrobenzene instead of iodobenzene. ¹H NMR (600 MHz, CDCl₃) *δ* 8.21 (dd, *J=* 0.6 Hz and *J* = 9.6 Hz, 1H), 7.97 (d, *J=* 7.8 Hz, 1H), 7.83 (td, *J=* 9.6 Hz and *J* = 1.8 Hz, 1H), 7.80-7.75 (m, 2H), 7.75-7.72 (m, 1H), 7.50-7.47 (m, 1H), 7.25-7.22 (m, 1H), 4.33 (q, *J=* 7.2 Hz, 2H), 1.28 (t, *J=* 7.2 Hz, 3H).

### Example 41. Ethyl 2-(4-fluorophenyl)-5-phenyloxazole-4-carboxylate (67)

This compound was obtained in 59% yield as a white solid, following the same procedure described for the synthesis of compound **27.** ¹H NMR (600 MHz, CDCl₃) *δ* 8.17 (dd, *J* = 5.4 Hz and *J* = 8.4 Hz, 2H), 8.10 (d, *J=* 6.6 Hz, 2H), 7.53-7.48 (m, 3H), 7.19 (t, *J* = 8.4 Hz, 2H), 4.47 (q, *J=* 7.2 Hz, 2H), 1.43 (t, *J=* 7.2 Hz, 3H).

### Example 42. Ethyl 2-(4-fluorophenyl)-5-(2-nitrophenyl)oxazole-4-carboxylate (68)

This compound was obtained in 88% yield as a white solid, following the same procedure described for the synthesis of compound **27** with 1-iodo-2-nitrobenzene instead of iodobenzene. ¹H NMR (600 MHz, CDCl₃) *δ* 8.19 (d, *J=* 8.4 Hz, 1H), 8.13 (dd, *J=* 5.4 Hz and *J* = 9.0 Hz, 2H), 7.78-7.76 (m, 2H), 7.73-7.69 (m, 1H), 7.18 *(t, J=* 9.0 Hz, 2H), 4.33 (q, *J* = 7.2 Hz, 2H), 1.28 (t, *J=* 7.2 Hz, 3H).

### Example 43. Ethyl 2-(4-fluorophenyl)-5-(4-nitrophenyl)oxazole-4-carboxylate (69)

This compound was obtained in 20% yield as a fluffy white solid, following the same procedure described for the synthesis of compound **27** with 1-iodo-4-nitrobenzene instead of iodobenzene. ¹H NMR (600 MHz, CDCl₃) *δ* 8.39-8.35 (m, 4H), 8.20 (dd, *J =* 5.4 Hz and *J* = 9.0 Hz, 2H), 7.22 (t, *J=* 9.0 Hz, 2H), 4.51 (q, *J=* 7.2 Hz, 2H), 1.46 (t, *J=* 7.2 Hz, 3H).

### Example 44. Ethyl 2-(3,4-difluorophenyl)-5-phenyloxazole-4-carboxylate (70)

This compound was obtained in 86% yield as a fluffy white solid, following the same procedure described for the synthesis of compound **27.** ¹H NMR (600 MHz, CDCl₃) *δ* 8.10 (dd, *J=* 1.2 Hz and *J* = 7.8 Hz, 2H), 8.01-7.98 (m, 1H), 7.94-7.92 (m, 1H), 7.54-7.50 (m, 3H), 7.32-7.28 (m, 1H), 4.47 (q, *J=* 7.2 Hz, 2H), 1.43 (t, *J=* 7.2 Hz, 3H).

### Example 45. Ethyl 2-(3,4-difluorophenyl)-5-(2-nitrophenyl)oxazole-4-carboxylate (71)

This compound was obtained in 86% yield as a yellow solid, following the same procedure described for the synthesis of compound **27** with 1-iodo-2-nitrobenzene instead of iodobenzene. ¹H NMR (600 MHz, CDCl₃) *δ* 8.21 (d, *J* = 8.4 Hz, 1H), 7.98-7.96 (m, 1H), 7.90-7.88 (m, 1H), 7.80-7.71 (m, 3H), 7.31-7.28 (m, 1H), 4.33 (q, *J=* 7.2 Hz, 2H), 1.27 (t, *J=* 7.2 Hz, 3H).

### Example 46. Ethyl 2-(3,4-difluorophenyl)-5-(4-(methylthio)phenyl)oxazole-4-carboxylate (72)

This compound was obtained in 86% yield as a yellow solid, following the same procedure described for the synthesis of compound **27** with 4-bromothioanisole instead of iodobenzene. ¹H NMR (600 MHz, CDCl₃) *δ* 8.04 (d, *J=* 8.5 Hz, 2H), 7.97 (t, *J=* 8.3 Hz, 1H), 7.96-7.85 (m, 1H), 7.34 (d, *J=* 8.5 Hz, 2H), 7.29 (q, *J=* 8.5 Hz, 1H), 4.49 (q, *J* = 7.1 Hz, 2H), 2.54 (s, 3H), 1.43 (t, *J=* 7.1 Hz, 3H).

### Example 47. Ethyl 2-(3,5-difluorophenyl)-5-phenyloxazole-4-carboxylate (73)

This compound was obtained in 76% yield as a white solid, following the same procedure described for the synthesis of compound **27.** ¹H NMR (600 MHz, CDCl₃) *δ* 8.10 (dd, *J=* 1.8 Hz and *J* = 7.8 Hz, 2H), 7.71-7.68 (m, 2H), 7.53-7.50 (m, 3H), 6.98-6.94 (m, 1H), 4.48 (q, *J=* 7.2 Hz, 2H), 1.43 (t, *J=* 7.2 Hz, 3H).

### Example 48. Ethyl 2-(3,5-difluorophenyl)-5-(2-nitrophenyl)oxazole-4-carboxylate (74)

This compound was obtained in 76% yield as a white solid, following the same procedure described for the synthesis of compound **27** with 1-iodo-2-nitrobenzene instead of iodobenzene. ¹H NMR (600 MHz, CDCl₃) *δ* 8.22-8.20 (m, 1H), 7.81-7.78 (m, 1H), 7.74-7.71 (m, 2H), 7.64-7.62 (m, 2H), 6.98-6.95 (m, 1H), 4.30 (q, *J =* 7.2 Hz, 2H), 1.25 (t, *J =* 7.2 Hz, 3H).

### Example 49. Ethyl 2-(3-chlorophenyl)-5-phenyloxazole-4-carboxylate (75)

This compound was obtained in 94% yield as a white solid, following the same procedure described for the synthesis of compound **27.** ¹H NMR (600 MHz, CDCl₃) *δ* 8.17 (t, *J=* 1.8 Hz, 1H), 8.12 (dd, *J=* 1.8 Hz and *J* = 8.4 Hz, 2H), 8.06 (td, *J=* 7.2 Hz and *J =* 1.2 Hz, 1H), 7.54-7.47 (m, 4H), 7.44 (t, *J =* 7.8 Hz, 1H), 4.48 (q, *J =* 7.2 Hz, 2H), 1.44 (t, *J =* 7.2 Hz, 3H).

### Example 50. Ethyl 2-(3-chlorophenyl)-5-(2-nitrophenyl)oxazole-4-carboxylate (76)

This compound was obtained in quantitative yield as a white solid, following the same procedure described for the synthesis of compound **27** with 1-iodo-2-nitrobenzene instead of iodobenzene. ¹H NMR (600 MHz, CDCl₃) *δ* 8.19 (dd, *J=* 2.4 Hz and *J* = 8.4 Hz, 1H), 8.12 (t, *J=* 1.8 Hz, 1H), 8.00 (td, *J=* 7.8 Hz and *J* = 1.2 Hz, 1H), 7.78-7.74 (m, 2H), 7.73-7.70 (m, 1H), 7.48-7.46 (m, 1H), 7.42 (t, *J=* 7.8 Hz, 1H), 4.31 (q, *J =* 7.2 Hz, 2H), 1.26 (t, *J=* 7.2 Hz, 3H).

### Example 51. Ethyl 2-(4-chlorophenyl)-5-phenyloxazole-4-carboxylate (77)

This compound was obtained in 98% yield as a white solid, following the same procedure described for the synthesis of compound **27.** ¹H NMR (600 MHz, CDCl₃) *δ* 8.12-8.10 (m, 4H), 7.53-7.47 (m, 5H), 4.47 (q, *J* = 7.2 Hz, 2H), 1.43 (t, *J* = 7.2 Hz, 3H).

### Example 52. Ethyl 2-(4-chlorophenyl)-5-(2-nitrophenyl)oxazole-4-carboxylate (78)

This compound was obtained in 75% yield as a white solid, following the same procedure described for the synthesis of compound **27** with 1-iodo-2-nitrobenzene instead of iodobenzene. ¹H NMR (600 MHz, CDCl₃) *δ* 8.20 (d, *J=* 7.8 Hz, 1H), 8.07 (d, *J* = 8.4 Hz, 2H), 7.79-7.76 (m, 2H), 7.73-7.71 (m, 1H), 7.48 (d, *J* = 8.4 Hz, 2H), 4.33 (q, *J =* 7.2 Hz, 2H), 1.27 (t, *J* = 7.2 Hz, 3H).

### Example 53. Ethyl 2-(4-cyanophenyl)-5-phenyloxazole-4-carboxylate (79)

This compound was obtained in 77% yield as a white solid, following the same procedure described for the synthesis of compound **27.** ¹H NMR (600 MHz, CDCl₃) *δ* 8.29 (d, *J =* 8.4 Hz, 2H), 8.12-8.10 (m, 2H), 7.81 (d, *J=* 8.4 Hz, 2H), 7.54-7.52 (m, 3H), 4.48 (q, *J* = 7.2 Hz, 2H), 1.44 (t, *J=* 7.2 Hz, 3H).

### Example 54. Ethyl 2-(4-cyanophenyl)-5-(2-nitrophenyl)oxazole-4-carboxylate (80)

This compound was obtained in 72% yield as a white solid, following the same procedure described for the synthesis of compound **27** with 1-iodo-2-nitrobenzene instead of iodobenzene. ¹H NMR (600 MHz, CDCl₃) *δ* 8.24-8.21 (m, 3H), 7.81-7.73 (m, 5H), 4.33 (q, *J* = 7.2 Hz, 2H), 1.27 (t, *J=* 7.2 Hz, 3H).

### Example 55. Ethyl 2-([1,1'-biphenyl]-4-yl)-5-phenyloxazole-4-carboxylate (81)

This compound was obtained in 70% yield as a white solid, following the same procedure described for the synthesis of compound **27.** ¹H NMR (600 MHz, CDCl₃) *δ* 8.25 (d, *J* = 8.4 Hz, 2H), 8.15 (d, *J =* 8.4 Hz, 2H), 7.74 (d, *J=* 8.4 Hz, 2H), 7.67 (d, *J=* 8.4 Hz, 2H), 7.54-7.48 (m, 5H), 7.42-7.40 (m, 1H), 4.49 (q*, J=* 7.2 Hz, 2H), 1.45 (t, *J=* 7.2 Hz, 3H).

### Example 56. Ethyl 2-([1,1'-biphenyl]-4-yl)-5-(2-nitrophenyl)oxazole-4-carboxylate (82)

This compound was obtained in 88% yield as a white solid, following the same procedure described for the synthesis of compound **27** with 1-iodo-2-nitrobenzene instead of iodobenzene. ¹H NMR (600 MHz, CDCl₃) *δ* 8.21 (d, *J* = 8.4 Hz, 3H), 7.80-7.77 (m, 2H), 7.73-7.71 (m, 3H), 7.66 (d, *J =* 7.2 Hz, 2H), 7.49 (t, *J=* 8.4 Hz, 2H), 7.41 (t, *J =* 7.2 Hz, 1H), 4.35 (q, *J=* 7.2 Hz, 2H), 1.29 (t, *J=* 7.2 Hz, 3H).

### Example 57. Ethyl 2-(naphthalen-2-yl)-5-phenyloxazole-4-carboxylate (83)

This compound was obtained in 86% yield as a white solid, following the same procedure described for the synthesis of compound **27.¹H** NMR (600 MHz, CDCl₃) *δ* 8.69 (s, 1H), 8.25 (dd, *J* = 1.2 Hz and *J* = 8.4 Hz, 1H), 8.20-8.18 (m, 2H), 7.99-7.96 (m, 2H), 7.92-7.90 (m, 1H), 7.59-7.50 (m, 5H), 4.50 (q, *J=* 7.2 Hz, 2H), 1.46 (t, *J =* 7.2 Hz, 3H).

### Example 58. Ethyl 2-(naphthalen-2-yl)-5-(2-nitrophenyl)oxazole-4-carboxylate (84)

This compound was obtained in 64% yield as a white solid, following the same procedure described for the synthesis of compound **27** with 1-iodo-2-nitrobenzene instead of iodobenzene. ¹H NMR (600 MHz, CDCl₃) *δ* 8.85 (s, 1H), 8.21 (t, *J* = 8.4 Hz, 2H), 7.96 (d, *J* = 9.0 Hz, 2H), 7.90 (d, *J* = 7.8 Hz, 1H), 7.83-7.76 (m, 2H), 7.74-7.71(m, 1H), 7.60-7.55 (m, 2H), 4.36 (q, *J=* 7.2 Hz, 2H), 1.30 (t, *J=* 7.2 Hz, 3H).

### Example 59. Ethyl 2-(4-(dimethylamino)phenyl)-5-phenyloxazole-4-carboxylate (85)

This compound was obtained in 76% yield as a white solid, following the same procedure described for the synthesis of compound **27.** ¹H NMR (600 MHz, CDCl₃) *δ* 8.12 (d, *J=* 7.2 Hz, 2H), 8.03 (d, *J=* 9.0 Hz, 2H), 7.49 (t, *J=* 7.2 Hz, 2H), 7.46-7.44 (m, 1H), 6.75 (d, *J=* 9.0 Hz, 2H), 4.46 (q, *J=* 7.2 Hz, 2H), 3.06 (s, 6H), 1.43 (t, *J=* 7.2 Hz, 3H).

### Example 60. Ethyl 2-(4-(dimethylamino)phenyl)-5-(2-nitrophenyl)oxazole-4-carboxylate (86)

This compound was obtained in 33% yield as a white solid, following the same procedure described for the synthesis of compound **27** with 1-iodo-2-nitrobenzene instead of iodobenzene. ¹H NMR (600 MHz, CDCl₃) *δ* 8.14 (d, *J* = 8.4 Hz, 1H), 7.97 (d, *J=* 9.0 Hz, 2H), 7.79 (d, *J=* 7.8 Hz, 1H), 7.74 (t, *J* = 7.8 Hz, 1H), 7.66 (td, *J=* 7.8 Hz and *J* = 1.2 Hz, 1H), 6.73 (d, *J=* 9.0 Hz, 2H), 4.33 (q, *J=* 7.2 Hz, 2H), 3.06 (s, 6H), 1.28 (t, *J=* 7.2 Hz, 3H).

### Example 61. Ethyl 2-(4-(tert-butyl)phenyl)-5-phenyloxazole-4-carboxylate (87)

This compound was obtained in 64% yield as a white solid, following the same procedure described for the synthesis of compound **27.** ¹H NMR (600 MHz, CDCl₃) *δ* 8.12 (d, *J=* 7.8 Hz, 2H), 8.10 (d, *J=* 7.8 Hz, 2H), 7.52-7.48 (m, 5H), 4.47 (q, *J=* 7.2 Hz, 2H), 1.43 (t, *J* = 7.2 Hz, 3H), 1.37 (s, 9H).

### Example 62. Ethyl 2-(4-(tert-butyl)phenyl)-5-(2-nitrophenyl)oxazole-4-carboxylate (88)

This compound was obtained in 31% yield as a white solid, following the same procedure described for the synthesis of compound **27** with 1-iodo-2-nitrobenzene instead of iodobenzene. ¹H NMR (600 MHz, CDCl₃) *δ* 8.19 (d, *J* = 8.4 Hz, 1H), 8.07 (d, *J=* 8.4 Hz, 2H), 7.78-7.77 (m, 2H), 7.73-7.68 (m, 1H), 7.52 (d, *J =* 8.4 Hz, 2H), 4.34 (q, *J=* 7.2 Hz, 2H), 1.38 (s, 9H), 1.29 (t, *J=* 7.2 Hz, 3H).

### Example 63. Ethyl 2-(4-(tert-butyl)phenyl)-5-(4-nitrophenyl)oxazole-4-carboxylate (89)

This compound was obtained in quantitative as a white solid, following the same procedure described for the synthesis of compound **27** with 1-iodo-4-nitrobenzene instead of iodobenzene. ¹H NMR (600 MHz, CDCl₃) *δ* 8.38-8.37 (m, 2H), 8.34-8.33 (m, 2H), 8.10 (d, *J=* 7.2 Hz, 2H), 7.54 (d, *J* = 7.0 Hz, 2H), 4.49 (q, *J=* 7.2 Hz, 2H), 1.46 (t, *J=* 7.2 Hz, 3H), 1.38 (s, 9H).

### Example 64. Ethyl 2-(4-(methylthio)phenyl)-5-phenyloxazole-4-carboxylate (90)

This compound was obtained in 70% yield as a white solid, following the same procedure described for the synthesis of compound **27.** ¹H NMR (600 MHz, CDCl₃) *δ* 8.11 (dd, *J=* 1.2 Hz and *J* = 8.4 Hz, 2H), 8.06 (d, *J=* 8.4 Hz, 2H), 7.51-7.47 (m, 3H), 7.31 (d, *J* = 8.4 Hz, 2H), 4.46 (q, *J* =7.2 Hz, 2H), 2.53 (s, 3H), 1.43 (t, *J* = 7.2 Hz, 3H).

### Example 65. Ethyl 2-(4-(methylthio)phenyl)-5-(2-nitrophenyl)oxazole-4-carboxylate (91)

This compound was obtained in 65% yield as a white solid, following the same procedure described for the synthesis of compound **27** with 1-iodo-2-nitrobenzene instead of iodobenzene. ¹H NMR (600 MHz, CDCl₃) *δ* 8.17 (d, *J=* 7.8 Hz, 1H), 8.02 (d, *J* = 8.4 Hz, 2H), 7.77-7.76 (m, 2H), 7.71-7.68 (m, 1H), 7.31 (d, *J=* 8.4 Hz, 2H), 4.32 (q, *J=* 7.2 Hz, 2H), 2.54 (s, 3H), 1.27 (t, *J=* 7.2 Hz, 3H).

### Example 66. Ethyl 5-(3-methoxyphenyl)-2-(4-(methylthio)phenyl)oxazole-4-carboxylate (92)

This compound was obtained in 54% yield as a fluffy white solid, following the same procedure described for the synthesis of compound **27** with 1-iodo-3-methoxybenzene instead of iodobenzene. ¹H NMR (600 MHz, CDCl₃) *δ* 8.08 (d, *J* = 9.0 Hz, 2H), 7.77 (s, 1H), 7.72-7.70 (m, 1H), 7.43 (t, *J=* 8.4 Hz, 2H), 7.05-7.04 (m, 1H), 4.48 (q, *J=* 7.2 Hz, 2H), 3.92 (s, 3H), 2.57 (s, 3H), 1.45 (t, *J=* 7.2 Hz, 3H).

### Example 67. Ethyl 5-(4-methoxyphenyl)-2-(4-(methylthio)phenyl)oxazole-4-carboxylate (93)

This compound was obtained in 54% yield as a fluffy white solid, following the same procedure described for the synthesis of compound **27** with 1-iodo-4-methoxybenzene instead of iodobenzene. ¹H NMR (600 MHz, CDCl₃) *δ* 8.12 (d, *J=* 9.0 Hz, 2H), 8.07 (d, *J=* 8.4 Hz, 2H), 7.34 (d, *J* = 9.0 Hz, 2H), 7.04 (d, *J=* 9.0 Hz, 2H), 4.48 (q, *J=* 7.2 Hz, 2H), 3.91 (s, 3H), 2.56 (s, 3H), 1.46 (t, *J=* 6.6 Hz, 3H).

### Example 68. Ethyl 2-(4-nitrophenyl)-5-phenyloxazole-4-carboxylate (94)

This compound was obtained in 57% yield as a white solid, following the same procedure described for the synthesis of compound **27.** ¹H NMR (600 MHz, MeOD) *δ* 8.41 (d, *J* = 9.0 Hz, 1H), 8.37 (d, *J* = 9.0 Hz, 2H), 8.13-8.11 (m, 2H), 7.55-7.53 (m, 3H), 4.41 (q, *J* = 7.2 Hz, 2H), 1.38 (t, *J=* 7.2 Hz, 3H).

### Example 69. Ethyl 5-(2-nitrophenyl)-2-(4-nitrophenyl)oxazole-4-carboxylate (95)

This compound was obtained in 70% yield as a white solid, following the same procedure described for the synthesis of compound **27** with 1-iodo-2-nitrobenzene instead of iodobenzene. ¹H NMR (600 MHz, MeOD) *δ* 8.43 (d, *J=* 8.4 Hz, 2H), 8.35 (d, *J=* 8.4 Hz, 2H), 8.27 (d, *J* = 7.8 Hz, 1H), 7.92-7.89 (m, 2H), 7.89-7.86 (m, 1H), 4.24 (q, *J* = 7.2 Hz, 2H), 1.18 (t, *J* = 7.2 Hz, 3H).

### Example 70. Ethyl 2-(4-((tert-butoxycarbonyl)amino)phenyl)-5-(3-(methylthio)phenyl)oxazole-4-carboxylate (96)

This compound was obtained in 57% yield as a white solid, following the same procedure described for the synthesis of compound **27** with 3-bromothioanisole instead of iodobenzene. ¹H NMR (600 MHz, CDCl₃) *δ* 8.09 (d, *J=* 9.0 Hz, 2H), 8.06-8.05 (m, 1H), 7.87 (d, *J* = 7.8 Hz, 1H), 7.51 (d, *J=* 9.0 Hz, 2H), 7.42 (t, *J =* 7.8 Hz, 1H), 7.36 (d, *J =* 8.4 Hz, 1H), 6.66 (s, 1H), 4.46 (q, *J =* 7.2 Hz, 2H), 2.57 (s, 3H), 1.54 (s, 9H), 1.43 (t, *J* = 7.2 Hz, 3H).

### Example 71. Ethyl 2-(4-cyclopropylphenyl)-5-phenyloxazole-4-carboxylate (97)

This compound was obtained in quantitative yield as a white solid, following the same procedure described for the synthesis of compound **27.** ¹H NMR (600 MHz, CDCl₃) *δ* 8.14-8.12 (m, 2H), 8.05 (d, *J =* 8.4 Hz, 2H), 7.52-7.48 (m, 3H), 7.17 (d, *J =* 8.4 Hz, 2H), 4.47 (q, *J* = 7.2 Hz, 2H), 1.98-1.95 (m, 1H), 1.43 (t, *J =* 7.2 Hz, 3H), 1.08-1.05 (m, 2H), 0.81-0.78 (m, 2H).

### Example 72. Ethyl 2-(4-chloro-3-(trifluoromethyl)phenyl)-5-phenyloxazole-4-carboxylate (98)

This compound was obtained in 11% yield as a fluffy white solid, following the same procedure described for the synthesis of compound **27.** ¹H NMR (600 MHz, CDCl₃) *δ* 8.49 (s, 1H), 8.28 (d, *J=* 8.4 Hz, 1H), 8.12-8.11 (m, 2H), 7.67 (d, *J=* 6.0 Hz, 1H), 7.56-7.53 (m, 3H), 4.49 (q, *J=* 7.2 Hz, 2H), 1.45 (t, *J=* 6.6 Hz, 3H).

### Example 73. Ethyl 5-(2-aminophenyl)-2-(4-(trifluoromethyl)phenyl)oxazole-4-carboxylate (99)

To a solution of compound **32** (100 mg, 0.25 mmol) in MeOH (7.0 mL) was added 10% Pd/C (52.3 mg, 0.49 mmol). The reaction mixture was purged with Hz (hydrogen filled balloon) and stirred for 18 h. After adding further MeOH (10 mL) the reaction mixture was briefly heated with a heat gun and filtered through a Celite pad. The volatiles were removed by evaporation and gave compound **99** as a grey powder (91.6 mg, 99%). ¹H NMR (600 MHz, CDCl₃) *δ* 8.26 (d, *J=* 7.8 Hz, 2H), 7.75 (d, *J=* 8.4 Hz, 2H), 7.52-7.31 (m, 4H), 4.47 (q, *J* = 7.2 Hz, 2H), 3.89 (brs, 2H), 1.45 (t, *J=* 7.2 Hz, 3H).

### Example 74. Ethyl 5-(3-aminophenyl)-2-(4-(trifluoromethyl)phenyl)oxazole-4-carboxylate (100)

To a solution of compound **33** (200 mg, 0.49 mmol) in MeOH (14 mL) was added 10% Pd/C (105 mg, 0.098 mmol). The reaction mixture was purged with H₂ (hydrogen filled balloon) and stirred for 12 h. After adding further MeOH (10 mL) the reaction mixture was briefly heated with a heat gun and filtered through a Celite pad. The volatiles were removed by evaporation and the residue was purified by column chromatography on silica gel (eluting with hexane : EtzOAc = 4:1 to 2:1, v/v) to afford compound **100** as a grey powder (125 mg, 67%). ¹H NMR (600 MHz, CDCl₃) *δ* 8.27 (d, *J=* 7.8 Hz, 2H), 7.76 (d, *J=* 8.4 Hz, 2H), 7.50-7.48 (m, 2H), 7.30 (t, *J=* 7.2 Hz, 1H), 6.83-6.82 (m, 1H), 4.48 (q, *J=* 7.2 Hz, 2H), 3.89 (brs, 2H), 1.45 (t, *J=* 7.2 Hz, 3H).

### Example 75. Ethyl 5-(4-aminophenyl)-2-(4-(trifluoromethyl)phenyl)oxazole-4-carboxylate (101)

To a solution of compound **34** (200 mg, 0.49 mmol) in MeOH (14 mL) was added 10% Pd/C (105 mg, 0.098 mmol). The reaction mixture was purged with Hz (hydrogen filled balloon) and stirred for 12 h. After adding further MeOH (10 mL) the reaction mixture was briefly heated with a heat gun and filtered through a Celite pad. The volatiles were removed by evaporation and the residue was purified by column chromatography on silica gel (eluting with hexane : Et₂OAc = 4:1 to 2:1, v/v) to afford pure compound **101** as a grey powder (132 mg, 52%). ¹H NMR (600 MHz, CDCl₃) *δ* 8.25 (d, *J=* 7.9 Hz, 2H), 8.01 (d, *J=* 8.3 Hz, 2H), 7.74 (d, *J =* 8.0 Hz, 2H), 6.77 (d, *J =* 8.3 Hz, 2H), 4.46 (q, *J =* 7.0 Hz, 2H), 4.10-3.94 (m, 2H), 1.44 (t, *J* = 7.0 Hz, 3H).

### Example 76. Ethyl 5-(4-aminophenyl)-2-(4-(tert-butyl)phenyl)oxazole-4-carboxylate (102)

To a solution of compound **89** (270 mg, 0.68 mmol) in MeOH (20 mL) was added 10% Pd/C (146 mg, 0.069 mmol). The reaction mixture was purged with H₂ (hydrogen filled balloon) and stirred for 12 h. After adding further MeOH (10 mL) the reaction mixture was briefly heated with a heat gun and filtered through a Celite pad. The volatiles were removed by evaporation and the residue was purified by column chromatography on silica gel (eluting with hexane : ether = 3:1 to 1:1, v/v) to afford pure compound **102** as a grey powder (149 mg, 60%). ¹H NMR (600 MHz, CDCl₃) *δ* 8.07 (d, *J=* 8.4 Hz, 2H), 8.00 (d, *J=* 8.4 Hz, 2H), 7.49 (d, *J=* 9.0 Hz, 2H), 6.77 (d, *J=* 8.4 Hz, 2H), 4.46 (q, *J=* 7.2 Hz, 2H), 4.00 (brs, 2H), 1.44 (t, *J=* 7.2 Hz, 3H), 1.36 (s, 9H).

### Example 77. Ethyl 5-(3-(methylsulfinyl)phenyl)-2-(4-(trifluoromethyl)phenyl)oxazole-4-carboxylate (103)

To a stirred solution of m-CPBA (51 mg, 0.29 mmol, 1.0 eq) in anhydrous CH₂Cl₂ (2.0 mL) was added dropwise to a solution of **36** (120 mg, .29 mmol) in anhydrous CH₂Cl₂ (8.0 mL) at 0 °C. The reaction mixture was slowly warmed up to room temperature and stirred for 4 h under argon atmosphere. The reaction was quenched with water and extracted with CH₂Cl₂ (× 3). The combined organic layer was dried over MgSO₄, filtered and evaporated under reduced pressure. The residue was purified by column chromatography on a silica gel (eluting with hexane : EtOAc = 2:1 to 1:3, v/v) to afford compound **103** (93 mg, 76%). ¹H NMR (600 MHz, CDCl₃) *δ* 8.47 (s, 1H), 8.33 (d, *J* = 8.1 Hz, 3H), 7.80 (d, *J=* 8.3 Hz, 3H), 7.72 (t, *J=* 7.8 Hz, 1H), 4.51 (q, *J =* 7.1 Hz, 2H), 2.85 (s, 3H), 1.48 (t, *J =* 7.1 Hz, 3H). HRMS m/z: calcd for C₂₀H₁₆F₃NO₄S [M + H]⁺: 424.0786; found: 424.0830.

### Example 78. Ethyl 5-(4-(methylsulfinyl)phenyl)-2-(4-(trifluoromethyl)phenyl)oxazole-4-carboxylate (104)

To a stirred solution of m-CPBA (65 mg, 0.38 mmol, 1.0 eq) in anhydrous CH₂Cl₂ (2.0 mL) was added dropwise to a solution of **37** (154 mg, 0.38 mmol) in anhydrous CH₂Cl₂ (8.0 mL) at 0 °C. The reaction mixture was slowly warmed up to room temperature and stirred for 4 h under argon atmosphere. The reaction was quenched with water and extracted with CH₂Cl₂ (× 3). The combined organic layer was dried over MgSO₄, filtered and evaporated under reduced pressure. The residue was purified by column chromatography on a silica gel (eluting with hexane : EtOAc = 2:1 to 1:3, v/v) to afford compoud **104** (116 mg, 72%). ¹H NMR (600 MHz, CDCl₃) *δ* 8.30-8.37 (m, 4H), 7.79-7.85 (m, 4H), 4.52 (q, *J* = 7.1 Hz, 2H), 2.82 (s, 3H), 1.48 (t, *J* = 7.08 Hz, 3H). HRMS *m*/*z*: calcd for C₂₀H₁₆F₃NO₄S [M + H]⁺: 424.0786; found: 424.0835.

### Example 79. Ethyl 2-(4-aminophenyl)-5-(3-(methylthio)phenyl)oxazole-4-carboxylate (105)

The compound **96** (240 mg, 0.53 mmol) was dissolved in anhydrous CH₂Cl₂ (10 mL). Trifluoroacetic acid (16 eq; per amine function) was added and stirred at room temperature for 2 h. The volatile components were evaporated and replaced by anhydrous toluene which was then evaporated to azeotrope excess trifluoroacetic acid. This operation was repeated three times to yield an oil which was dried *in vacuo.* to afford crude product for the next step without further purification. ¹H NMR (600 MHz, DMSO-d₆) *δ* 7.98 (m, 1H), 7.79 (d, *J=* 7.8 Hz, 1H), 7.76 (d, *J =* 8.4 Hz, 2H), 7.47 (t, *J=* 7.8 Hz, 2H), 7.41-7.37 (m, 1H), 6.67 (d, *J =* 8.4 Hz, 2H), 5.88 (s, 2H), 4.31 (q, *J=* 7.2 Hz, 2H), 2.54 (s, 3H), 1.30 (t, *J=* 7.2 Hz, 3H).

### Example 80. Ethyl 5-(3-acetamidophenyl)-2-(4-(trifluoromethyl)phenyl)oxazole-4-carboxylate (106)

To a solution of compound **100** (70 mg, 0.19 mmol) in anhydrous CH₂Cl₂ (4.0 mL) was added DIPEA (97 µL, 0.59 mmol) under argon atmosphere. After the mixture was cooled to 0 °C, acetyl chloride (27 µL, 0.37 mmol) was added slowly dropwise to the mixture. The reaction mixture was slowly warmed up to room temperature and stirred for 6 h. The volatile components were evaporated and the residue was purified by column chromatography in a silica gel (eluting with hexane : EtOAc = 4:1 v/v) to afford compound **106** (55 mg, 70%). ¹H NMR (600 MHz, CDCl₃) *δ* 8.36 (s, 1H), 8.25 (d, *J=* 7.8 Hz, 2H), 7.83 (d, *J=* 7.2 Hz, 2H), 7.74 (d, *J* = 8.4 Hz, 3H), 7.45 (t, *J=* 7.8 Hz, 1H), 4.47 (q, *J=* 7.2 Hz, 2H), 2.23 (s, 3H), 1.44 (t, *J =* 7.2 Hz, 3H).

Compound **107-112** were prepared using a similar method as described for compound **106.**

### Example 81. Ethyl 5-(4-acetamidophenyl)-2-(4-(trifluoromethyl)phenyl)oxazole-4-carboxylate (107)

This compound was obtained in 73% yield as a fluffy white solid, following the same procedure described for the synthesis of compound **106.** ¹H NMR (600 MHz, CDCl₃) *δ* 8.28 (d, *J* = 8.4 Hz, 2H), 8.14 (d, *J =* 9.0 Hz, 2H), 7.77 (d, *J=* 8.4 Hz, 2H), 7.71 (d, *J=* 8.4 Hz, 2H), 7.62 (brs, 1H), 4.48 (q, *J* = 7.2 Hz, 2H), 2.25 (s, 3H), 1.45 (t, *J=* 7.2 Hz, 3H).

### Example 82. Ethyl 5-(3-(N-acetylacetamido)phenyl)-2-(4-(trifluoromethyl)phenyl)oxazole-4-carboxylate (108)

This compound was obtained in 26% yield as a fluffy white solid, following the same procedure described for the synthesis of compound **106.** ¹H NMR (600 MHz, CDCl₃) *δ* 8.30 (d, *J=* 8.4 Hz, 2H), 8.22 (d, *J=* 7.8 Hz, 1H), 8.03 (s, 1H), 7.79 (d, *J=* 7.8 Hz, 2H), 7.65 (t, *J=* 8.4 Hz, 1H), 7.31 (d, *J=* 0.6 Hz, 1H), 4.50 (q, *J=* 7.2 Hz, 2H), 2.39 (s, 6H), 1.47 (t, *J =* 7.8 Hz, 3H).

### Example 83. Ethyl 5-(4-(N-acetylacetamido)phenyl)-2-(4-(trifluoromethyl)phenyl)oxazole-4-carboxylate (109)

This compound was obtained in 15% yield as a fluffy white solid, following the same procedure described for the synthesis of compound **106.** ¹H NMR (600 MHz, CDCl₃) *δ* 8.31 (d, *J =* 8.4 Hz, 4H), 7.80 (d, *J =* 8.4 Hz, 2H), 7.33 (d, *J* = 8.4 Hz, 2H), 4.51 (q, *J =* 7.2 Hz, 2H), 2.37 (s, 6H), 1.47 (t, *J=* 7.8 Hz, 3H).

### Example 84. Ethyl 5-(4-(N-acetylacetamido)phenyl)-2-(4-(tert-butyl)phenyl)oxazole-4-carboxylate (110)

This compound was obtained in quantitative yield as a fluffy white solid, following the same procedure described for the synthesis of compound **106.** ¹H NMR (600 MHz, CDCl₃) *δ* 8.31 (d, *J* = 8.4 Hz, 2H), 8.10 (d, *J* = 8.4 Hz, 2H), 7.54 (d, *J* = 8.4 Hz, 2H), 7.31 (d, *J =* 9.0 Hz, 2H), 4.50 (q, *J* = 7.2 Hz, 2H), 2.37 (s, 6H), 1.47 (t, *J=* 7.2 Hz, 3H), 1.39 (s, 9H).

### Example 85. Ethyl 5-(4-acetamidophenyl)-2-(4-(tert-butyl)phenyl)oxazole-4-carboxylate (111)

This compound was obtained in 54% yield as a fluffy white solid, following the same procedure described for the synthesis of compound **106.** ¹H NMR (600 MHz, CDCl₃) *δ* 8.15 (d, *J =* 9.0 Hz, 2H), 8.09 (d, *J=* 7.8 Hz, 2H), 7.69 (d, *J =* 8.4 Hz, 2H), 7.52 (d, *J =* 8.4 Hz, 2H), 7.47 (s, 1H), 4.48 (q, *J=* 7.2 Hz, 2H), 2.45 (s, 3H), 1.45 (t, *J=* 7.2 Hz, 3H), 1.38 (s, 9H).

### Example 86. Ethyl 2-(4-acetamidophenyl)-5-(3-(methylthio)phenyl)oxazole-4-carboxylate (112)

This compound was obtained in 74% yield as a white solid, following the same procedure described for the synthesis of compound **106.** ¹H NMR (600 MHz, CDCl₃) *δ* 8.11 (d, *J =* 8.4 Hz, 2H), 8.04 (s, 1H), 7.86 (d, *J =* 7.2 Hz, 1H), 7.68 (d, *J* = 8.4 Hz, 2H), 7.52 (s, 1H), 7.42 (t, *J=* 7.2 Hz, 1H), 7.37-7.35 (m, 1H), 4.45 (q, *J=* 6.6 Hz, 2H), 2.57 (s, 3H), 2.22 (s, 3H), 1.43 *(t, J=* 7.2 Hz, 3H).

### Example 87. Ethyl 5-(4-(methylsulfonamido)phenyl)-2-(4-(trifluoromethyl)phenyl)oxazole-4-carboxylate (113)

To a solution of compound **101** (87 mg, 0.23 mmol) in anhydrous CH₂Cl₂ (5.0 mL) was added pyridine (90 µL, 0.46 mmol) under argon atmosphere. After the mixture was cooled to 0 °C, methanesulfonyl chloride (20 µL, 0.28 mmol) was added slowly dropwise to the mixture. The reaction mixture was slowly warmed up to room temperature and stirred for 16 h. The volatile components were evaporated under reduced pressure and the residue was purified by column chromatography in a silica gel (eluting with hexane : EtOAc = 5:1 to 1:1, v/v) to afford compound **113** (72 mg, 69%). ¹H NMR (600 MHz, CDCl₃) *δ* 8.27 (d, *J=* 8.2 Hz, 2H), 8.18 (d, *J* = 8.6 Hz, 2H), 7.76 (d, *J=* 8.7 Hz, 2H), 6.58 (s, 1H), 4.48 (q, *J =* 7.1 Hz, 2H), 3.11 (s, 3H), 1.45 (t, *J=* 7.14 Hz, 3H). HRMS m/z: calcd for C₂₀H₁₇F₃N₂O₅S [M + H]⁺: 455.0844; found: 455.0908.

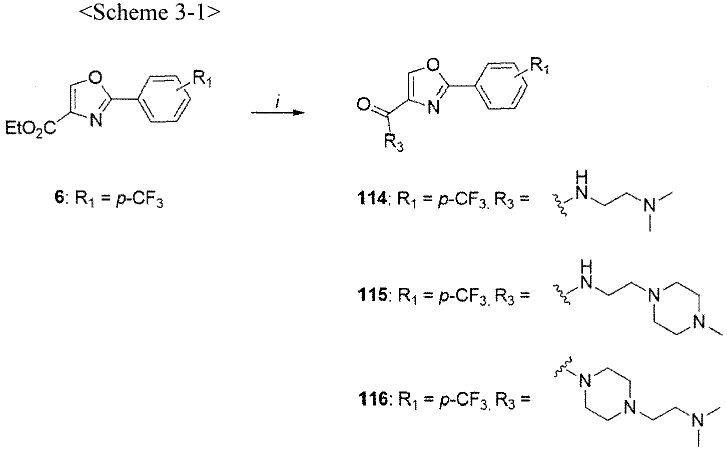

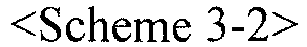

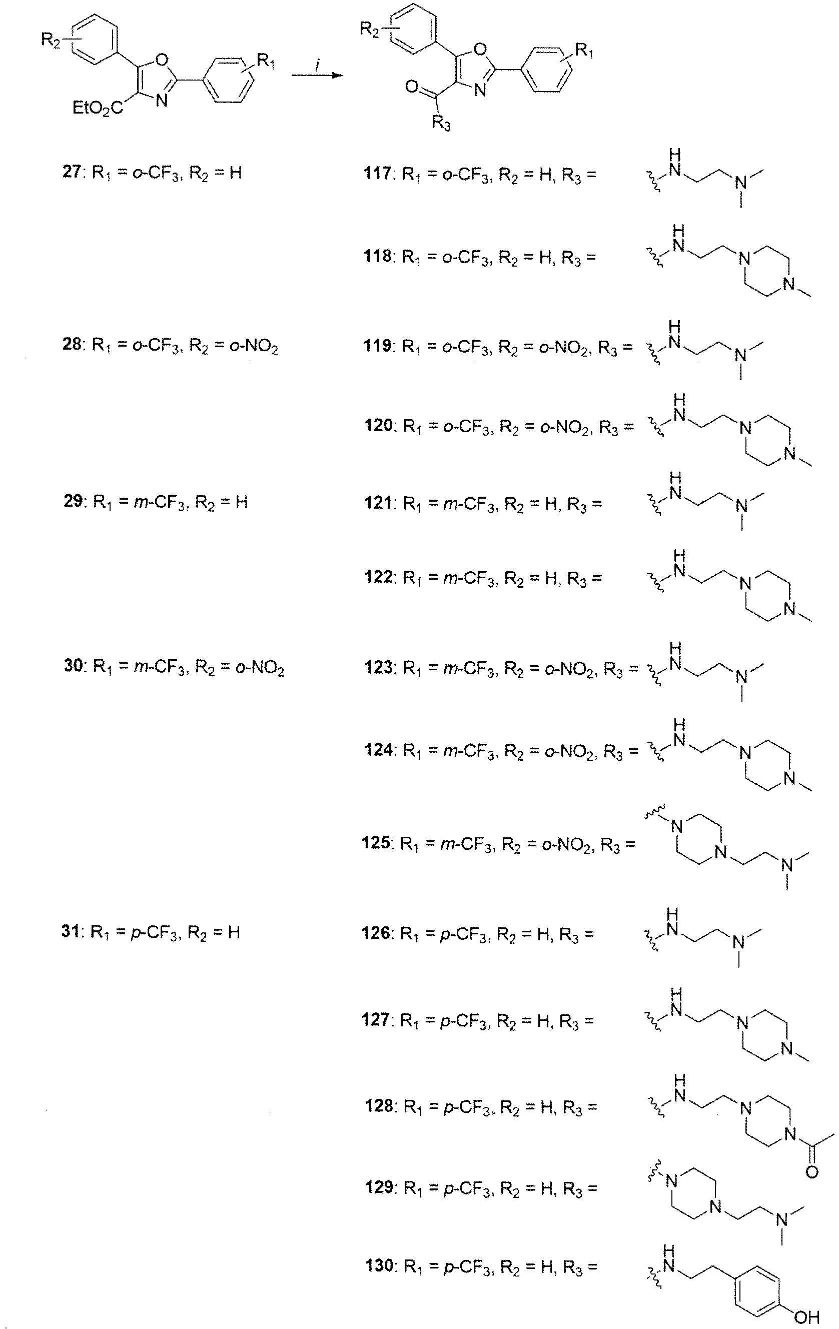

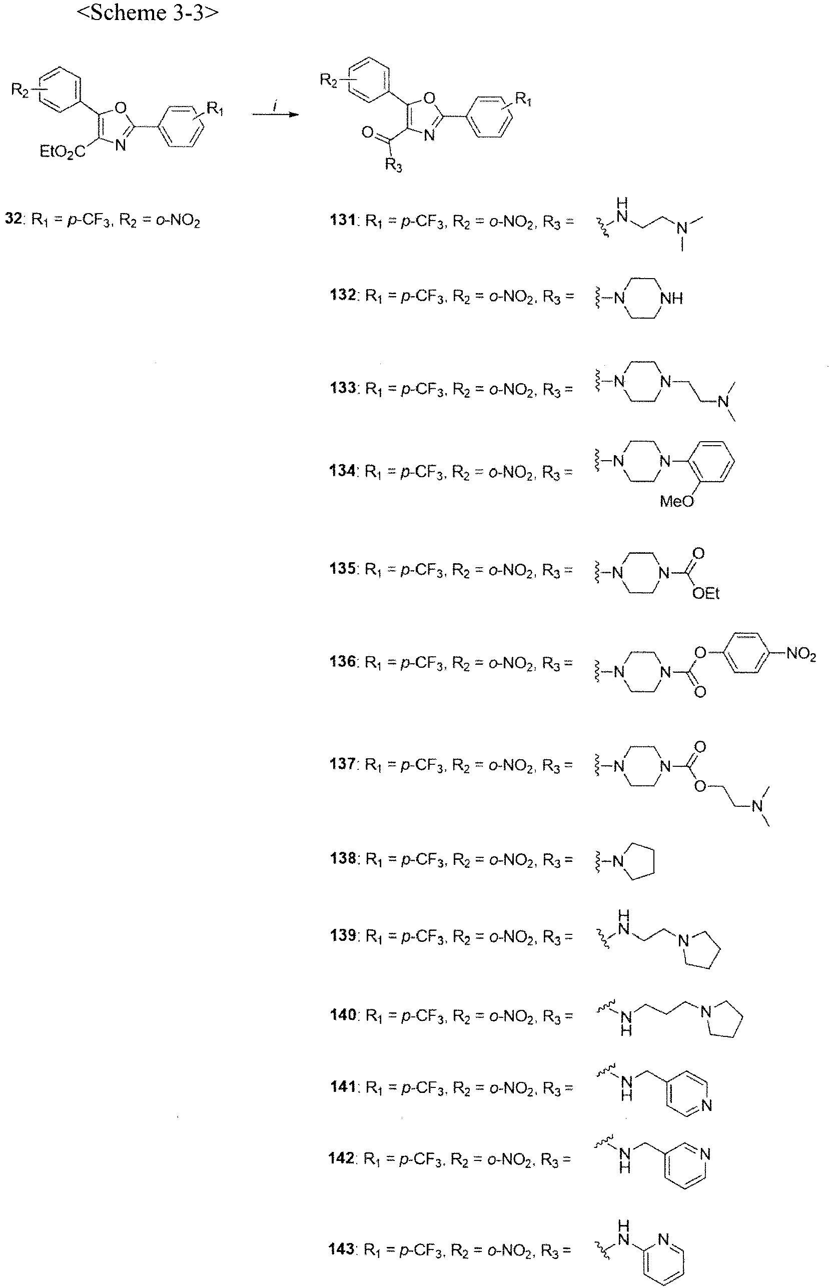

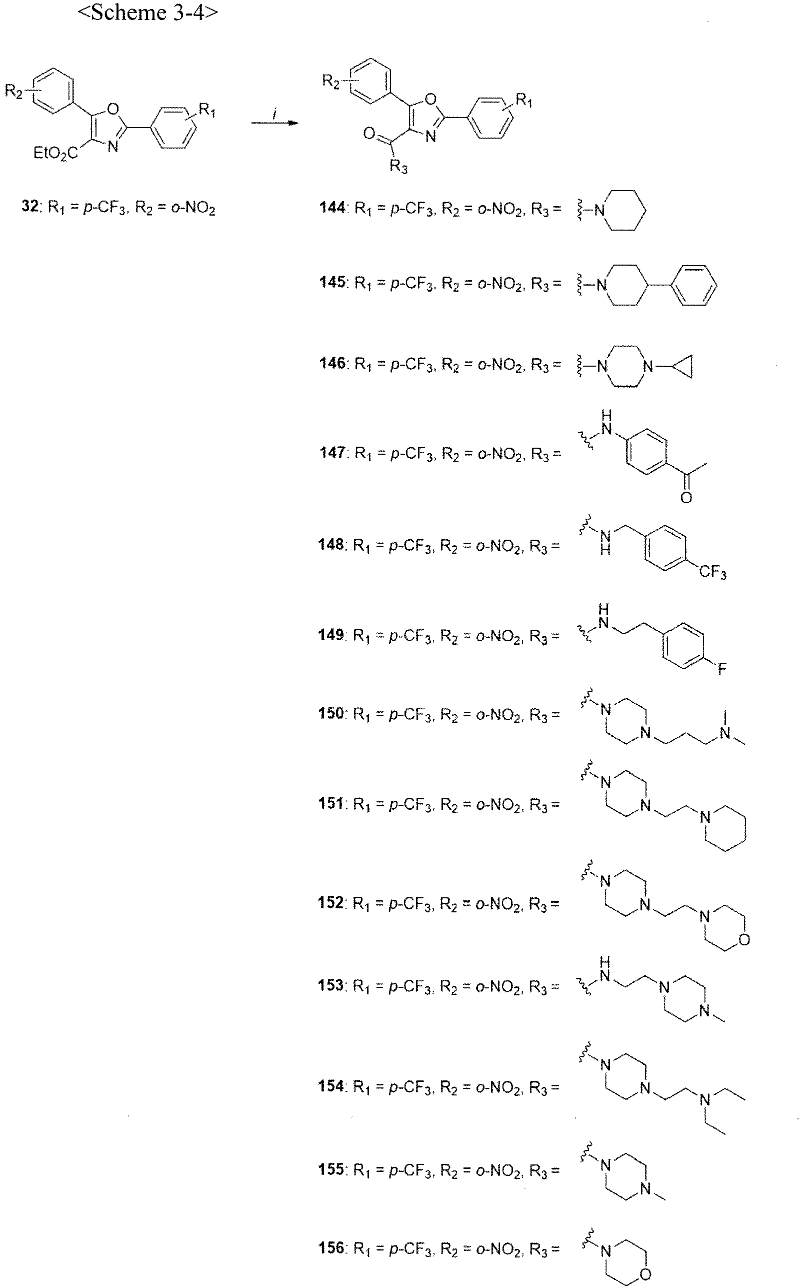

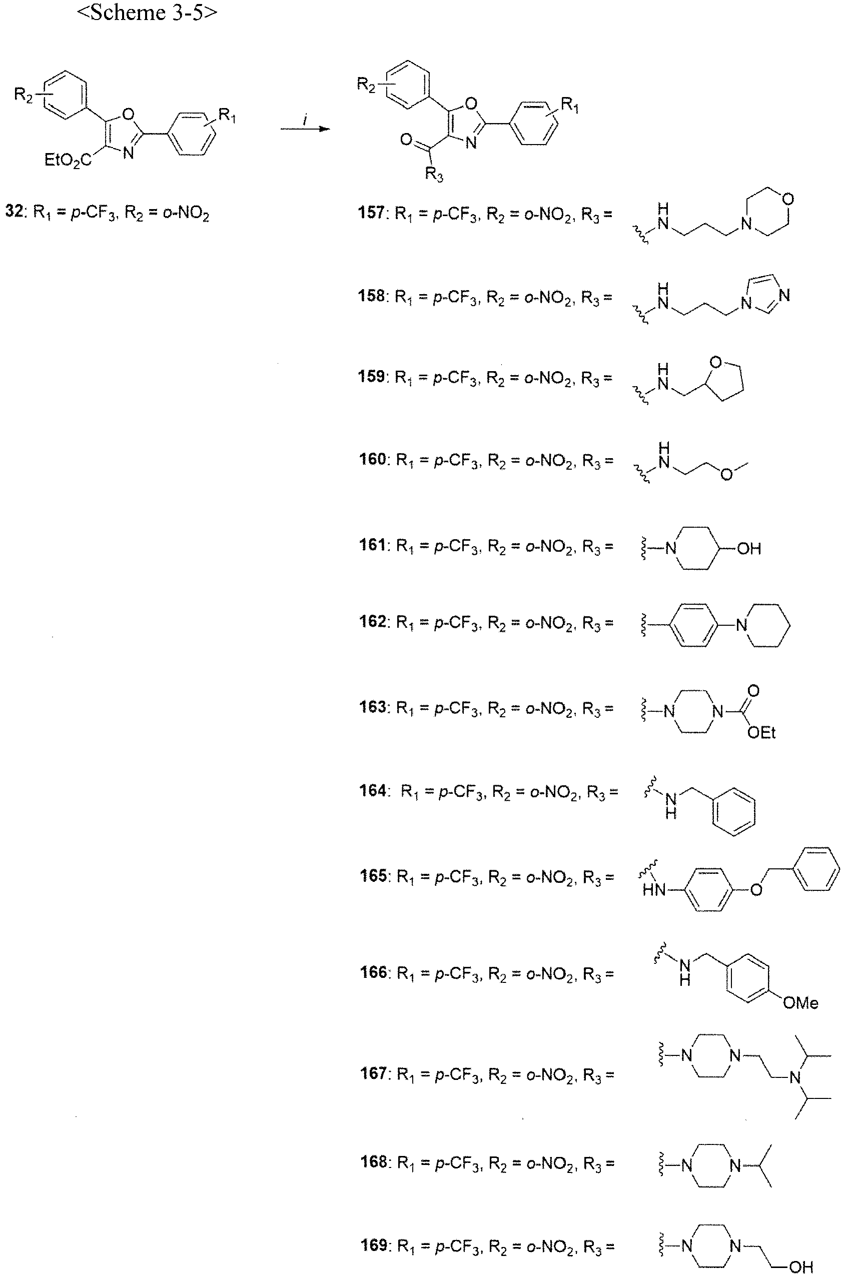

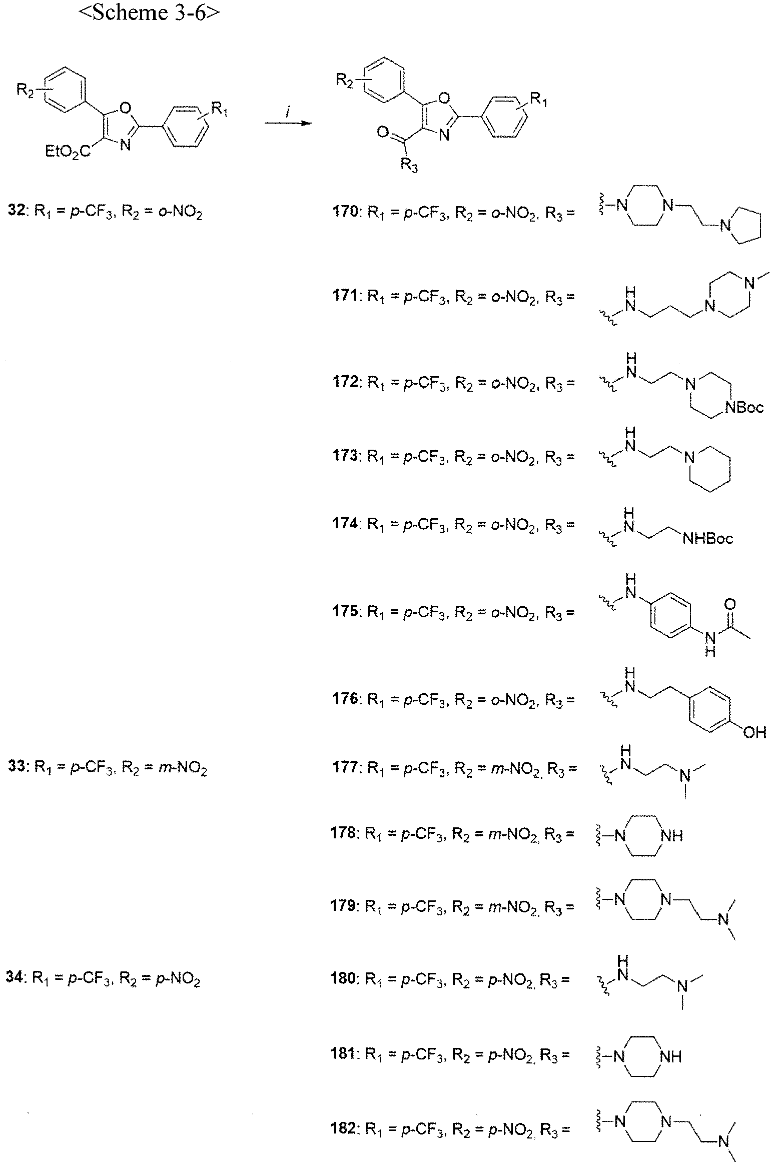

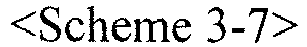

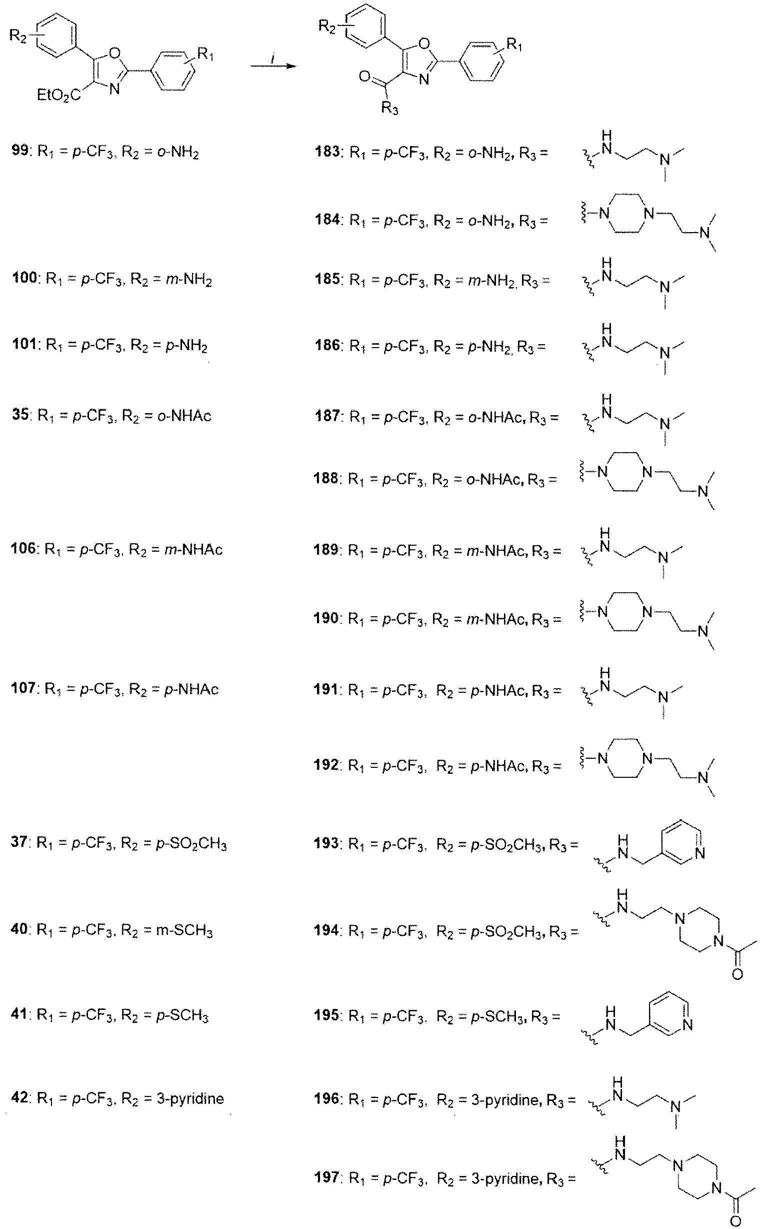

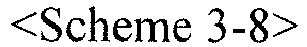

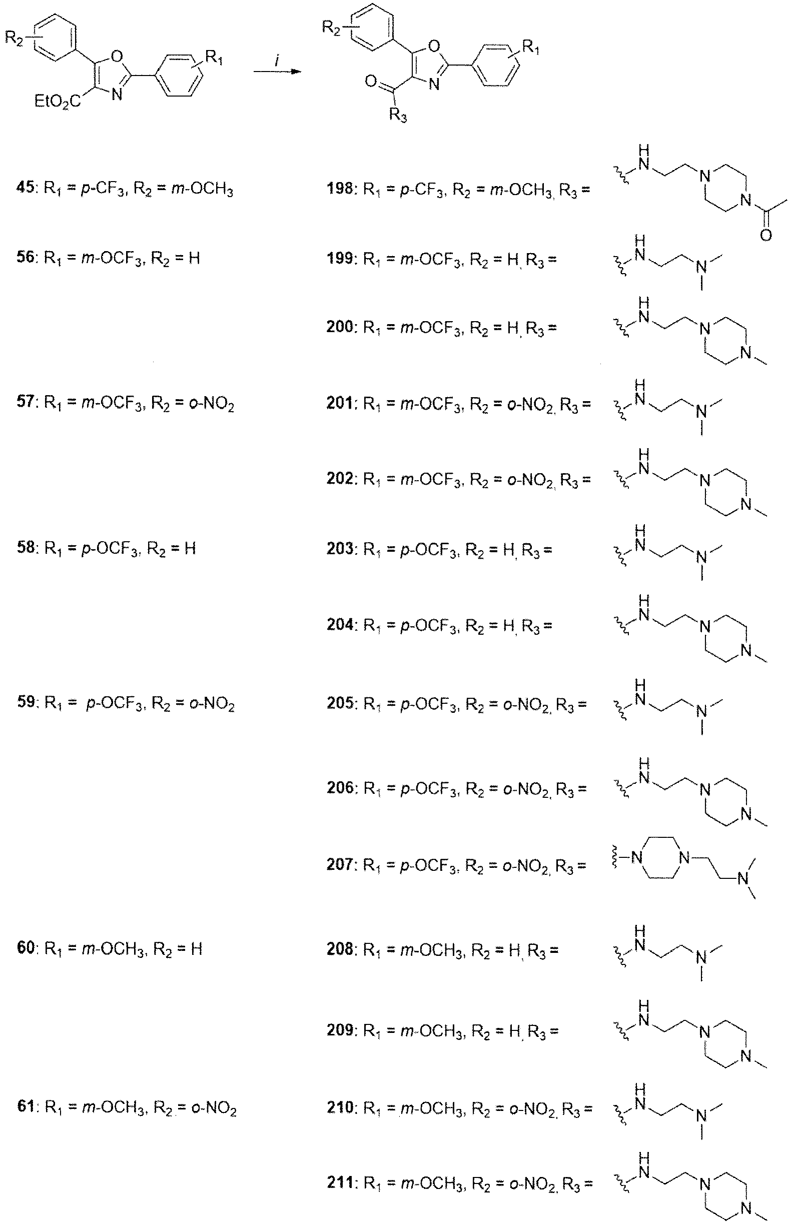

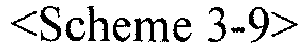

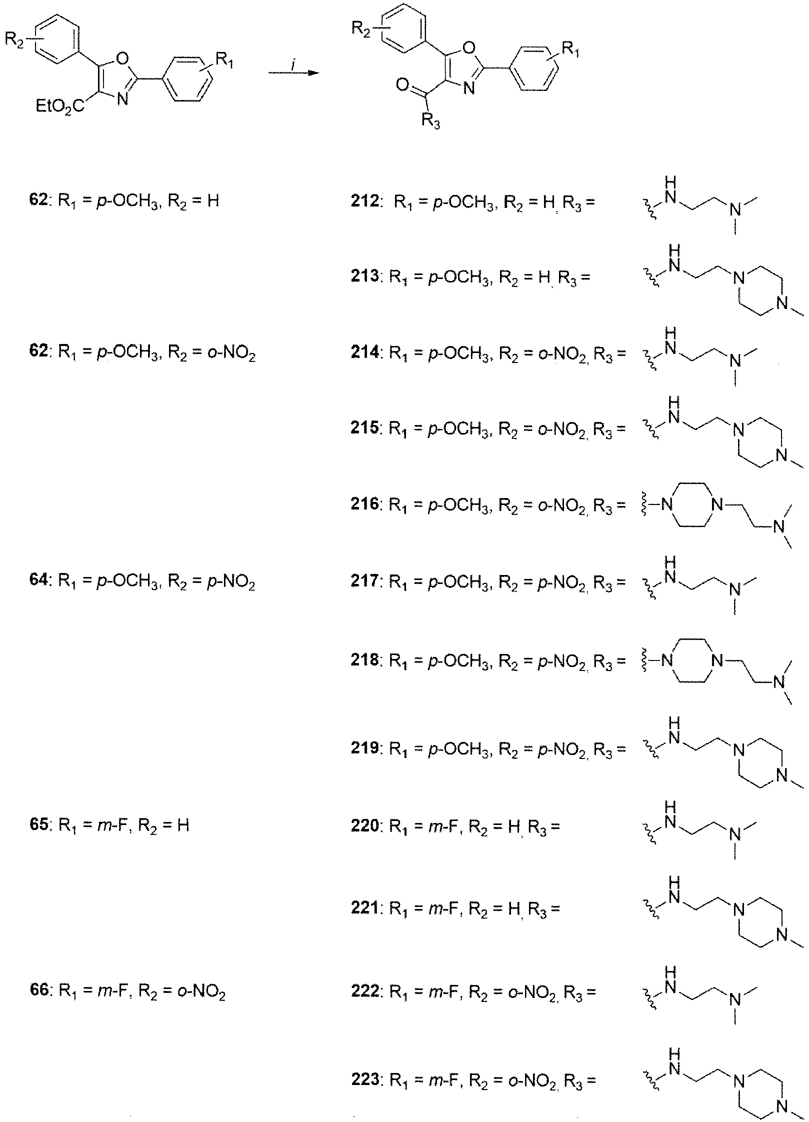

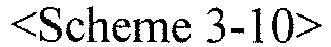

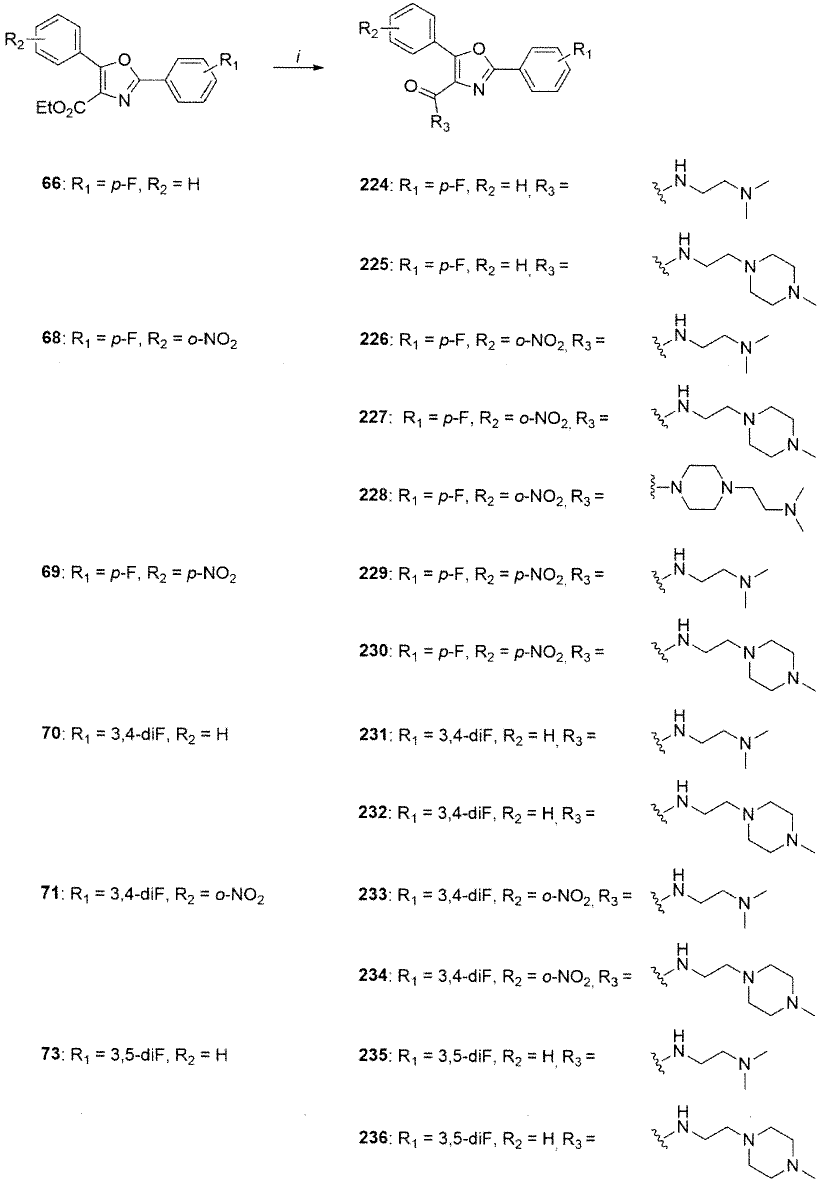

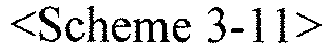

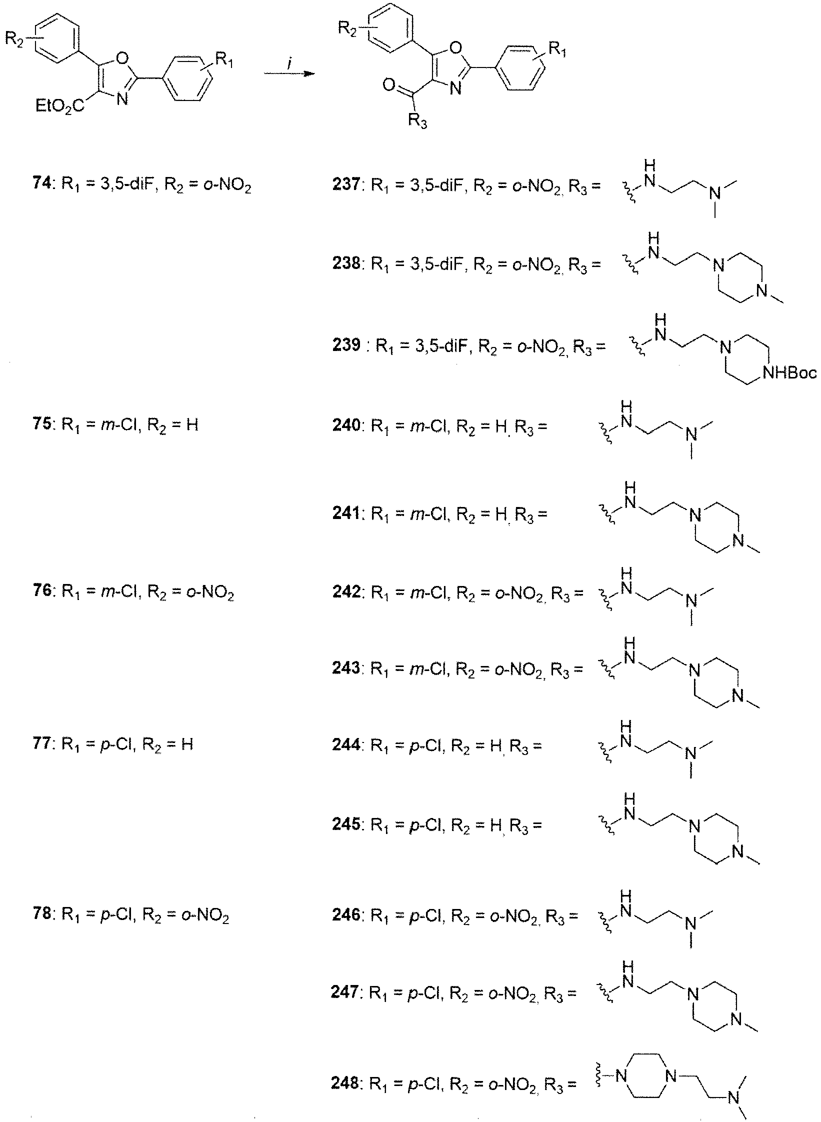

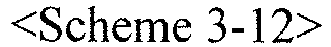

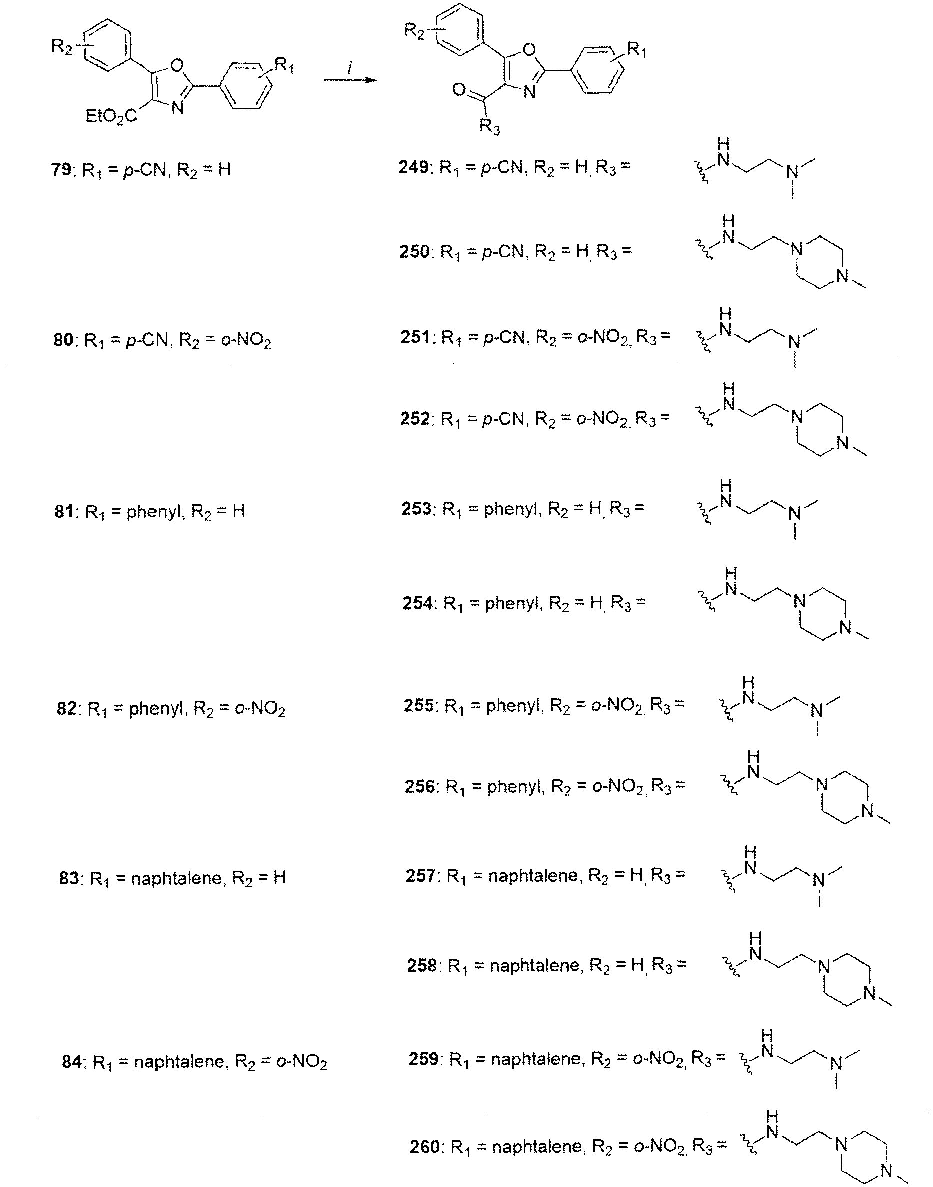

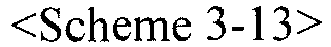

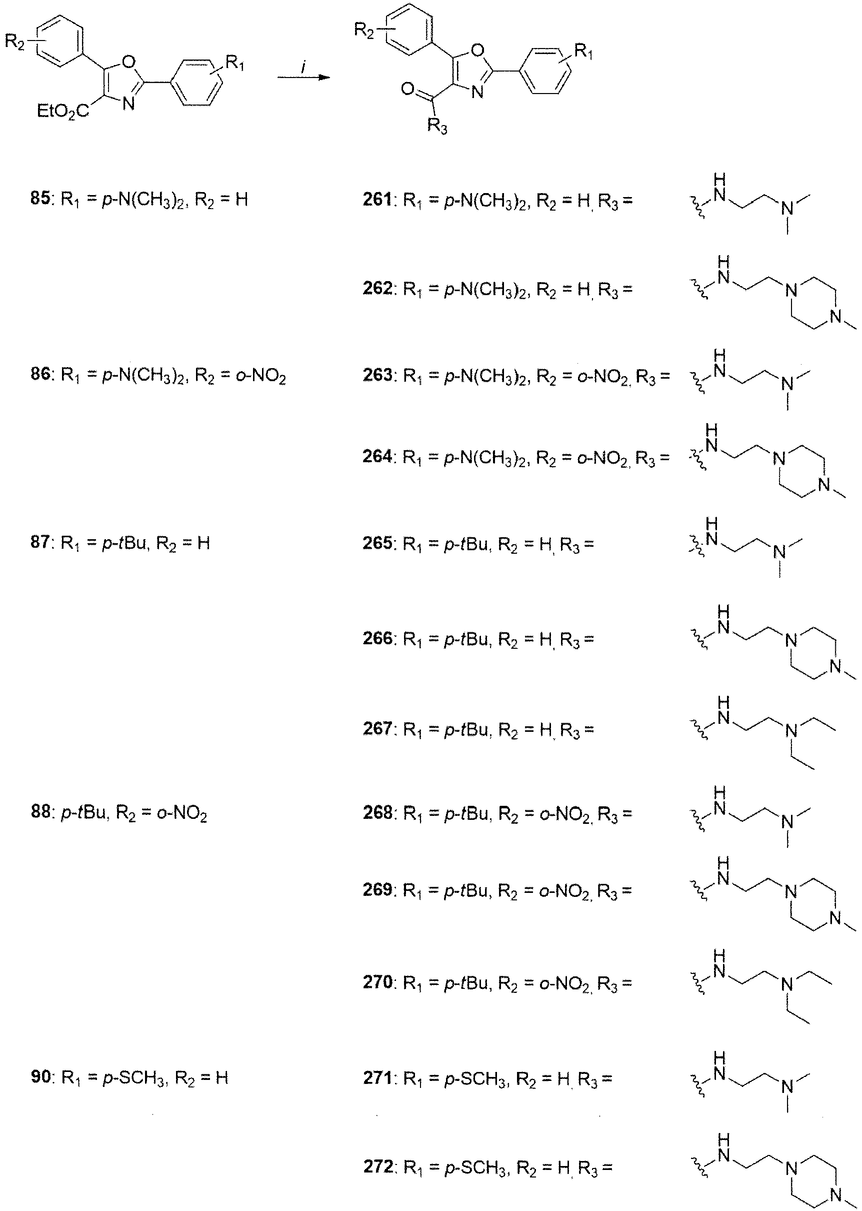

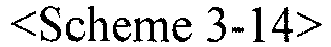

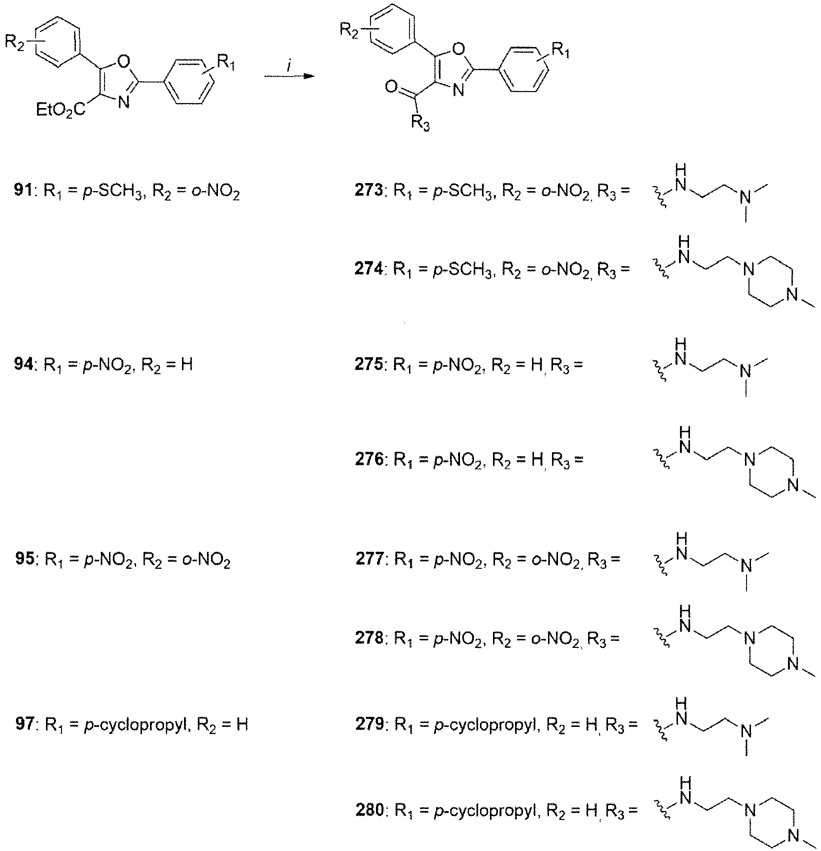

### Example 88. Typical procedure of hydrolysis and amide coupling reaction for the synthesis: N-(2-(dimethylamino)ethyl)-2-(4-(trifluoromethyl)phenyl)oxazole-4-carboxamide (114)

The ethyl 5-phenyl-2-(2-(trifluoromethyl)phenyl)oxazole-4-carboxylate (150 mg, 0.53 mmol) was dissolved in EtOH (10 mL). 3 N NaOH (1.5 mL) was added and the reaction mixture was stirred at rt for 1 h. Upon completion, the reaction mixture was evaporated under reduced pressure and acidified by 3 N HCl, extracted with EtOAc. The organic layer was washed with brine, dried over MgSO₄ and concentrated *in vacuo* to afford carboxylic acid compound as a white solid for the next step without further purification. To a solution of the carboxylic acid compound (132 mg, 0.51 mmol) in DMF (5 mL) were added *N,N*-dimethylethylenediamine (67 µL, 0.61 mmol), 1-ethyl-3-(3-(dimethylamino)propyl) carbodiimide hydrochloride (117 mg, 0.61 mmol), 1- hydroxybenzotriazole (82 mg, 0.61 mmol) and DIPEA (178 µL, 1.0 mmol). After stirring at room temperature for 15h, the reaction was completed as indication by TLC. The reaction mixture was evaporated, then subjected to co-evaporation with toluene three times to completely remove DMF. The mixture was diluted with water and extracted with EtOAc three times. The combined organics washed with brine, dried over MgSO₄, filtered and evaporated under reduced pressure. The crude products were purified by column chromatography on silica gel (eluting with CH₂Cl₂: MeOH = 20:1 to 10:1, v/v) to afford compound **114** as a pale yellow solid (66 mg, 40%). ¹H NMR (600 MHz, CDCl₃) *δ* 8.29 (s, 1H), 8.19 (d, *J=* 8.4 Hz, 2H), 7.76 (d, *J=* 8.4 Hz, 2H), 7.44 (br s, 1H), 3.57 (t, *J=* 6.0 Hz, 2H), 2.57 (t, *J=* 6.0 Hz, 2H), 2.34 (s, 6H).

Compound **115-280** were prepared using a similar method as described for compound **114.**

### Example 89. N-(2-(4-methylpiperazin-1-yl)ethyl)-2-(4-(trifluoromethyl)phenyl)oxazole-4-carboxamide (115)

This compound was obtained in 91% yield as a white solid with a typical procedure of hydrolysis and amide coupling reaction, following the same procedure described for the synthesis of compound **114.** ¹H NMR (600 MHz, MeOD) *δ* 8.48 (s, 1H), 8.24 (d, J = 8,4 Hz, 2H), 7.83 (d, J = 8,4 Hz, 2H), 3.55 (t, J = 6.6 Hz, 2H), 2.63 (t, J = 6.6 Hz, 2H), 2.53 (br s, 8H), 2.29 (s, 3H).

### Example 90. (4-(2-(dimethylamino)ethyl)piperazin-1-yl)(2-(4-(trifluoromethyl)phenyl)oxazol-4-yl)methanone (116)]

This compound was obtained in 59% yield with a typical procedure of hydrolysis and amide coupling reaction, following the same procedure described for the synthesis of compound **114.** ¹H NMR (600 MHz, MeOD) *δ* 8.47 (s, 1H), 8.25 (d, *J=* 7.8 Hz, 2H), 7.85 (d, *J=* 7.8 Hz, 2H), 4.14 (br s, 2H), 3.79 (br s, 2H), 2.61 (br s, 8H), 2.36 (s, 6H).

### Example 91. Typical procedure of hydrolysis and amide coupling reaction for the synthesis: N-(2-(Dimethylamino)ethyl)-5-phenyl-2-(2-(trifluoromethyl)phenyl)oxazole-4-carboxamide (117)

This compound was obtained in 59% yield with a typical procedure of hydrolysis and amide coupling reaction, following the same procedure described for the synthesis of compound **114** (75 mg, 25%). ¹H NMR (600 MHz, CDCl₃) *δ* 8.42 (d, *J=* 7.2 Hz, 2H), 8.17 (d, *J=* 7.8 Hz, 1H), 7.89 (d, *J* = 7.8 Hz, 1H), 7.73-7.70 (m, 2H), 7.65 (t, *J* = 7.8 Hz, 1H), 7.49 (t, *J* = 7.2 Hz, 2H), 7.45-7.42 (m, 1H), 3.58 (q, *J=* 6.0 Hz, 2H), 2.57 (t, *J* = 6.0 Hz, 2H), 2.32 (s, 6H).

### Example 92. N-(2-(4-Methylpiperazin-1-yl)ethyl)-5-phenyl-2-(2-(trifluoromethyl)phenyl)oxazole-4-carboxamide (118)

This compound was obtained in 41% yield as a white solid with a typical procedure of hydrolysis and amide coupling reaction, following the same procedure described for the synthesis of compound **114.** ¹H NMR (600 MHz, CDCl₃) *δ* 8.42 (d, *J=* 7.2 Hz, 2H), 8.17 (d, *J* = 7.2 Hz, 1H), 7.90 (d, *J=* 7.8 Hz, 1H), 7.83 (t, *J =* 4.8 Hz, 1H), 7.72 (t, *J=* 7.8 Hz, 1H), 7.65 (t, *J=* 7.8 Hz, 1H), 7.49 (t, *J=* 7.8 Hz, 2H), 7.47-7.44 (m, 1H), 3.59 (q, *J=* 6.0 Hz, 2H), 2.65 (t, *J=* 6.0 Hz, 2H), 2.64-2.45 (brs, 8H), 2.33 (s, 3H).

### Example 93. N-(2-(Dimethylamino)ethyl)-5-(2-nitrophenyl)-2-(2-(trifluoromethyl)phenyl)oxazole-4-carboxamide (119)

This compound was obtained in 51% yield as a white solid with a typical procedure of hydrolysis and amide coupling reaction, following the same procedure described for the synthesis of compound **114.** ¹H NMR (600 MHz, CDCl₃) *δ* 8.13 (d, *J* = 8.4 Hz, 1H), 8.10 (d, *J* = 7.8 Hz, 1H), 7.90 (d, *J=* 7.8 Hz, 1H), 7.86 (d, *J=* 7.2 Hz, 1H), 7.75-7.70 (m, 2H), 7.65 (q, *J* = 8.4 Hz, 2H), 7.55 (brs, 1H), 3.49 (q, *J=* 6.0 Hz, 2H), 2.51 (t, *J* = 6.0 Hz, 2H), 2.29 (s, 6H).

### Example 94. N-(2-(4-Methylpiperazin-1-yl)ethyl)-5-(2-nitrophenyl)-2-(2-(trifluoromethyl)phenyl)oxazole-4-carboxamide (120)

This compound was obtained in 49% yield as a white solid with a typical procedure of hydrolysis and amide coupling reaction, following the same procedure described for the synthesis of compound **114.** ¹H NMR (600 MHz, CDCl₃) *δ* 8.13 (d, *J=* 8.4 Hz, 1H), 8.08 (d, *J* = 7.8 Hz, 1H), 7.93 (d, *J=* 7.8 Hz, 1H), 7.88 (d, *J=* 7.8 Hz, 1H), 7.75-7.63 (m, 5H), 3.50 (q, *J* = 6.0 Hz, 2H), 2.60 (t, *J=* 6.0 Hz, 2H), 2.52 (brs, 8H), 2.32 (s, 3H).

### Example 95. N-(2-(Dimethylamino)ethyl)-5-phenyl-2-(3-(trifluoromethyl)phenyl)oxazole-4-carboxamide (121)

This compound was obtained in 42% yield as a white solid with a typical procedure of hydrolysis and amide coupling reaction, following the same procedure described for the synthesis of compound **114.** ¹H NMR (600 MHz, CDCl₃) *δ* 8.39-8.37 (m, 3H), 8.32 *(d, J=* 8.4 Hz, 1H), 7.77 (d, *J=* 7.8 Hz, 1H), 7.69 (brs, 1H), 7.66 (t, *J =* 7.8 Hz, 1H), 7.53-7.45 (m, 3H), 3.60 (q, *J=* 6.0 Hz, 2H), 2.59 (t, *J=* 6.0 Hz, 2H), 2.33 (s, 6H).

### Example 96. N-(2-(4-Methylpiperazin-1-yl)ethyl)-5-phenyl-2-(3-(trifluoromethyl)phenyl)oxazole-4-carboxamide (122)

This compound was obtained in 74% yield as a white solid with a typical procedure of hydrolysis and amide coupling reaction, following the same procedure described for the synthesis of compound **114.** ¹H NMR (600 MHz, CDCl₃) *δ* 8.42-8.40 (m, 3H), 8.30 *(d, J=* 8.4 Hz, 1H), 7.88 (t, *J* = 4.8 Hz, 1H), 7.77 (d, *J* = 7.8 Hz, 1H), 7.67 (t, *J* = 7.8 Hz, 1H), 7.51 (t, *J* = 7.8 Hz, 2H), 7.48-7.46 (m, 1H), 3.60 (q, *J* = 6.0 Hz, 2H), 2.67 (t, *J* = 6.0 Hz, 2H), 2.65-2.45 (brs, 8H), 2.33 (s, 3H).

### Example 97. N-(2-(Dimethylamino)ethyl)-5-(2-nitrophenyl)-2-(3-(trifluoromethyl)phenyl)oxazole-4-carboxamide (123)

This compound was obtained in 56% yield as a white solid with a typical procedure of hydrolysis and amide coupling reaction, following the same procedure described for the synthesis of compound **114.** ¹H NMR (600 MHz, CDCl₃) *δ* 8.33 (s, 1H), 8.24 (d, *J* = 7.8 Hz, 1H), 8.15 (dd, *J* = 1.2 Hz and *J =* 7.8 Hz, 1H), 7.93 (dd, *J =* 1.2 Hz and *J =* 7.8 Hz, 1H), 7.77-7.74 (m, 2H), 7.68-7.63 (m, 2H), 7.52 (t, *J* = 4.8 Hz, 1H), 3.51 (q, *J=* 6.0 Hz, 2H), 2.54 (t, *J* = 6.0 Hz, 2H), 2.31 (s, 6H).

### Example 98. N-(2-(4-Methylpiperazin-1-yl)ethyl)-5-(2-nitrophenyl)-2-(3-(trifluoromethyl)phenyl)oxazole-4-carboxamide (124)

This compound was obtained in 28% yield as a white solid with a typical procedure of hydrolysis and amide coupling reaction, following the same procedure described for the synthesis of compound **114.** ¹H NMR (600 MHz, CDCl₃) *δ* 8.35 (s, 1H), 8.21 (d, *J =* 7.8 Hz, 1H), 8.14 (dd, *J* = 1.2 Hz and *J* = 7.8 Hz, 1H), 7.94 (dd, *J=* 1.2 and *J =* 7.8 Hz, 1H), 7.78 (d, *J* = 7.8 Hz, 1H), 7.75 (td, *J=* 7.8 Hz and *J* = 1.2 Hz, 1H), 7.70-7.64 (m, 3H), 3.51 (q, *J* = 6.0 Hz, 2H), 2.63 (t, *J=* 6.0 Hz, 2H), 2.60 (brs, 8H), 2.34 (s, 3H).

### Example 99. (4-(2-(Dimethylamino)ethyl)piperazin-1-yl)(5-(2-nitrophenyl)-2-(3-(trifluoromethyl)phenyl)oxazol-4-yl)methanone (125)

This compound was obtained in 56% yield as a white solid with a typical procedure of hydrolysis and amide coupling reaction, following the same procedure described for the synthesis of compound **114.** ¹H NMR (600 MHz, CDCl₃) *δ* 8.31 (s, 1H), 8.23 (d, *J* = 7.8 Hz, 1H), 8.10 (d, *J=* 7.8 Hz, 1H), 7.87 (d, *J=* 7.2 Hz, 1H), 7.77-7.73 (m, 2H), 7.67-7.62 (m, 2H), 3.87 (brs, 2H), 3.73 (brs, 2H), 2.58-2.51 (m, 8H), 2.37 (s, 6H).

### Example 100. N-(2-(Dimethylamino)ethyl)-5-phenyl-2-(4-(trifluoromethyl)phenyl)oxazole-4-carboxamide (126)

This compound was obtained in 50% yield as a white solid with a typical procedure of hydrolysis and amide coupling reaction, following the same procedure described for the synthesis of compound **114.** ¹H NMR (600 MHz, CDCl₃) *δ* 8.38 (d, *J=* 7.2 Hz, 2H), 8.23 (d, *J* = 7.8 Hz, 2H), 7.76-7.73 (m, 3H), 7.51 (t, *J=* 7.2 Hz, 2H), 7.49-7.46 (m, 1H), 3.61 (q, *J* = 6.6 Hz, 2H), 2.62 (t, *J =* 6.6 Hz, 2H), 2.36 (s, 6H).

### Example 101. N-(2-(4-Methylpiperazin-1-yl)ethyl)-5-phenyl-2-(4-(trifluoromethyl)phenyl)oxazole-4-carboxamide (127)

This compound was obtained in 81% yield as a white solid with a typical procedure of hydrolysis and amide coupling reaction, following the same procedure described for the synthesis of compound **114.** ¹H NMR (600 MHz, MeOD) *δ* 8.28-8.24 (m, 4H), 7.82 (d, *J=* 8.4 Hz, 2H), 7.49-7.43 (m, 3H), 3.59 (t, *J=* 6.6 Hz, 2H), 2.63 (t, *J* = 6.6 Hz, 2H), 2.57 (brs, 8H), 2.29 (s, 3H).

### Example 102. N-(2-(4-acetylpiperazin-1-yl)ethyl)-5-phenyl-2-(2-(trifluoromethyl)phenyl)oxazole-4-carboxamide (128)

This compound was obtained in 60% yield as a white solid with a typical procedure of hydrolysis and amide coupling reaction, following the same procedure described for the synthesis of compound **114.** ¹H NMR (600 MHz, CDCl₃) *δ* 8.13 (d, *J* = 8.4 Hz, 1H), 8.08 (d, *J* = 7.8 Hz, 1H), 7.93 (d, *J=* 7.8 Hz, 1H), 7.88 (d, *J=* 7.8 Hz, 1H), 7.75-7.63 (m, 5H), 3.50 (q, *J* = 6.0 Hz, 2H), 2.60 (t, *J=* 6.0 Hz, 2H), 2.52 (brs, 8H), 2.32 (s, 3H).

### Example 103. (4-(2-(Dimethylamino)ethyl)piperazin-1-yl)-5-phenyl-2-(4-(trifluoromethyl)phenyl)oxazol-4-yl)methanone (129)

This compound was obtained in 34% yield as a white solid with a typical procedure of hydrolysis and amide coupling reaction, following the same procedure described for the synthesis of compound **114.** ¹H NMR (600 MHz, CDCl₃) *δ* 8.24 (d, *J=* 7.8 Hz, 2H), 7.86 (d, *J* = 7.8 Hz, 2H), 7.76 (d, *J=* 7.8 Hz, 2H), 7.48 (t, *J=* 7.2 Hz, 2H), 7.43-7.41 (m, 1H), 3.89 (brs, 2H), 3.57 (brs, 2H), 2.61 (brs, 2H), 2.52-2.51 (m, 2H), 2.47-2.46 (m, 2H), 2.43 (brs, 2H), 2.28 (s, 6H).

### Example 104. N-(4-Hydroxyphenethyl)-5-phenyl-2-(4-(trifluoromethyl)phenyl)oxazole-4-carboxamide (130)

This compound was obtained in 34% yield with a typical procedure of hydrolysis and amide coupling reaction, following the same procedure described for the synthesis of compound **114.** ¹H NMR (600 MHz, CDCl₃) *δ* 8.38 (d, *J=* 7.8 Hz, 2H), 8.21 (d, *J=* 8.4 Hz, 2H), 7.78 (d, *J=* 7.8 Hz, 2H), 7.52 (t, *J=* 7.2 Hz, 3H), 7.15 (d, *J=* 8.4 Hz, 2H), 6.84 (d, *J* = 8.4 Hz, 2H), 3.71 (q, *J=* 7.2 Hz, 2H), 2.92 (t, *J* = 7.2 Hz, 2H).

### Example 105. N-(2-(Dimethylamino)ethyl)-5-(2-nitrophenyl)-2-(4-(trifluoromethyl)phenyl)oxazole-4-carboxamide (131)

This compound was obtained in 92% yield with a typical procedure of hydrolysis and amide coupling reaction, following the same procedure described for the synthesis of compound **114.** ¹H NMR (600 MHz, CDCl₃) *δ* 8.19 (t, *J* = 7.8 Hz, 2H), 7.85 (d, *J=* 7.8 Hz, 1H), 7.78 (t, *J* = 7.2 Hz, 1H), 7.75-7.69 (m, 3H), 7.66 (t, *J* = 7.8 Hz, 1H), 3.59 (q, *J* = 6.6 Hz, 2H), 2.61 (t, *J=* 6.6 Hz, 2H), 2.53 (s, 6H).

### Example 106. (5-(2-Nitrophenyl)-2-(4-(trifluoromethyl)phenyl)oxazol-4-yl)(piperazin-1-yl)methanone (132)

This compound was obtained in 100% yield with a typical procedure of hydrolysis and amide coupling reaction, following the same procedure described for the synthesis of compound **114.** ¹H NMR (600 MHz, MeOD) *δ* 8.31 (d, *J* = 8.4 Hz, 2H), 8.19 (d, *J =* 8.1 Hz, 1H), 7.96 (d, *J* = 7.8 Hz, 1H), 7.90 (d, *J=* 8.4 Hz, 3H), 7.91-7.88 (m, 1H), 7.80 (t, *J=* 8.4 Hz, 1H), 4.30 (s, 2H), 3.92 (s, 2H), 3.34 (s, 4H).

### Example 107. (4-(2-(Dimethylamino)ethyl)piperazin-1-yl)(5-(2-nitrophenyl)-2-(4-(trifluoromethyl)phenyl)oxazol-4-yl)methanone (133)

This compound was obtained in 91% yield with a typical procedure of hydrolysis and amide coupling reaction, following the same procedure described for the synthesis of compound **114.** ¹H NMR (600 MHz, CDCl₃) *δ* 8.25 (d, *J=* 8.1 Hz, 2H), 8.15 (d, *J=* 6 Hz, 1H), 7.91-7.81 (m, 4H), 7.80-7.72 (m, 1H), 3.88 (brs, 2H), 3.71 (brs, 2H), 2.61-2.45 (m, 8H), 2.29 (s, 6H).

### Example 108. (4-(2-Methoxyphenyl)piperazin-1-yl)(5-(2-nitrophenyl)-2-(4-(trifluoromethyl)phenyl)oxazol-4-yl)methanone (134)

This compound was obtained in 27% yield with a typical procedure of hydrolysis and amide coupling reaction, following the same procedure described for the synthesis of compound **114.** ¹H NMR (600 MHz, CDCl₃) *δ* 8.19 (d, *J=* 8.4 Hz, 2H), 8.09 (d, *J=* 7.8 Hz, 1H), 7.91 (d, *J*=7.8 Hz, 1H), 7.79-7.71 (m, 3H), 7.64 (t, *J=* 7.8 Hz, 1H), 7.05 (t, *J =* 7.8 Hz, 1H), 6.97-6.89 (m, 2H), 6.88 (d, *J=* 8.4 Hz, 1H), 4.03 (s, 2H), 3.92 (s, 2H), 3.88 (s, 3H), 3.11 (s, 2H), 3.07 (s, 2H).

### Example 109. Ethyl 4-(5-(2-nitrophenyl)-2-(4-(trifluoromethyl)phenyl)oxazole-4-carbonyl)piperazine-1-carboxylate (135)

This compound was obtained in 91% yield as a yellow solid with a typical procedure of hydrolysis and amide coupling reaction, following the same procedure described for the synthesis of compound **114.** ¹H NMR (600 MHz, CDCl₃) *δ* 8.17 *(d, J=* 8.4 Hz, 2H), 8.10 (d, *J* = 7.8 Hz, 1H), 7.88 (d, *J=* 7.2 Hz, 1H), 7.76-7.74 (m, 3H), 7.65 (t, *J=* 7.8 Hz, 1H), 4.17 (q, *J* = 7.2 Hz, 2H), 3.90 (brs, 2H), 3.70 (brs, 2H), 3.56 (brs, 4H), 1.28 (t, *J=* 7.2 Hz, 3H).

### Example 110. 4-Nitrophenyl4-(5-(2-nitrophenyl)-2-(4-(trifluoromethyl)phenyl)oxazole-4-carbonyl)piperazine-1-carboxylate (136)

This compound was obtained in 73% yield as a yellow solid with a typical procedure of hydrolysis and amide coupling reaction, following the same procedure described for the synthesis of compound **114.** ¹H NMR (600 MHz, CDCl₃) *δ* 8.28 (d, *J=* 9.0 Hz, 2H), 8.20 (d, *J* = 8.4 Hz, 2H), 8.13 (dd, *J =* 1.2 Hz and *J =* 8.4 Hz, 1H), 7.90 (brs, 1H), 7.80-7.77 (m, 3H), 7.70-7.68 (m, 1H), 7.34 (d, *J=* 8.4 Hz, 2H), 4.09 (brs, 2H), 3.82 (brs, 4H), 3.72 (brs, 2H).

### Example 111. 2-(Dimethylamino)ethyl 4-(5-(2-nitrophenyl)-2-(4-(trifluoromethyl)phenyl)oxazole-4-carbonyl) piperazine-1-carboxylate (137)

This compound was obtained in 34% yield as a yellow solid with a typical procedure of hydrolysis and amide coupling reaction, following the same procedure described for the synthesis of compound **114.** ¹H NMR (600 MHz, CDCl₃) *δ* 8.17 (d, *J=* 7.8 Hz, 2H), 8.11 (d, *J* = 7.8 Hz, 1H), 7.89 *(d, J=* 7.2 Hz, 1H), 7.77-7.74 (m, 3H), 7.66 (t, *J* = 7.8 Hz, 1H), 4.23 (t, *J* = 6.0 Hz, 2H), 3.91 (brs, 2H), 3.70 (brs, 2H), 3.57 (brs, 4H), 2.61 (brs, 2H), 2.31 (s, 6H).

### Example 112. (5-(2-Nitrophenyl)-2-(4-(trifluoromethyl)phenyl)oxazol-4-yl)(pyrrolidin-1-yl)methanone (138)

This compound was obtained in 96% yield with a typical procedure of hydrolysis and amide coupling reaction, following the same procedure described for the synthesis of compound **114.** ¹H NMR (600 MHz, CDCl₃) *δ* 8.16 (d, *J=* 7.8 Hz, 2H), 8.10 (dd, *J=* 0.6 Hz and *J* = 8.1 Hz, 1H), 7.95 (dd, *J=* 1.2 Hz and *J* = 7.8 Hz, 1H), 7.74-7.71 (m, 3H), 7.63 (t, *J =* 8.4 Hz, 1H), 3.95 (t, *J=* 6.9 Hz, 2H), 3.60 (t, *J=* 6.9 Hz, 2H), 2.00-1.96 (m, 2H), 1.93-1.88 (m, 2H).

### Example 113. 5-(2-Nitrophenyl)-N-(2-(pyrrolidin-1-yl)ethyl)-2-(4-(trifluoromethyl)phenyl)oxazole-4-carboxamide (139)

This compound was obtained in 90% yield with a typical procedure of hydrolysis and amide coupling reaction, following the same procedure described for the synthesis of compound **114.** ¹H NMR (600 MHz, CDCl₃) *δ* 8.17 (d, *J* = 8.4 Hz, 2H), 8.12 (dd, *J* = 1.2 Hz and *J* = 7.8 Hz, 1H), 7.94 (dd, *J* = 1.8 Hz and *J =* 7.5 Hz, 1H), 7.75 (d, *J* = 8.4 Hz, 2H), 7.73 (dd, *J* = 1.2 Hz and *J* = 7.8 Hz, 1H), 7.66 (t, *J* = 7.8 Hz, 1H), 7.60 (brs, 1H), 3.56 (dd, *J=* 6.0 Hz and *J* = 12.0 Hz, 2H), 2.75 (t, *J=* 6.0 Hz, 2H), 2.63 (brs, 4H), 1.84 (brs, 6H).

### Example 114. 5-(2-Nitrophenyl)-N-(3-(pyrrolidin-1-yl)propyl)-2-(4-(trifluoromethyl)phenyl)oxazole-4-carboxamide (140)

This compound was obtained in 90% yield with a typical procedure of hydrolysis and amide coupling reaction, following the same procedure described for the synthesis of compound **114.** ¹H NMR (600 MHz, CDCl₃) *δ* 8.33 (brs, 1H), 8.17 (d, *J* = 8.4 Hz, 2H), 8.14 (dd, *J=* 0.6 Hz and *J* = 8.4 Hz, 1H), 7.96 (dd, *J* = 1.2 Hz and *J* = 7.5 Hz, 1H), 7.78-7.74 (m, 3H), 7.68 (t, *J* = 8.7 Hz, 1H), 3.55 (q, *J* = 9.0 Hz, 2H), 2.72-2.68 (m, 6H), 1.91-1.87 (m, 6H).

### Example 115. 5-(2-Nitrophenyl)-N-(pyridin-4-ylmethyl)-2-(4-(trifluoromethyl)phenyl)oxazole-4-carboxamide (141)

This compound was obtained in 90% yield with a typical procedure of hydrolysis and amide coupling reaction, following the same procedure described for the synthesis of compound **114.** ¹H NMR (600 MHz, CDCl₃) *δ* 8.58 (dd, *J=* 1.2 Hz and *J* = 4.8 Hz, 2H), 8.16-8.14 (m, 3H), 7.94 (dd, *J=* 1.2 Hz and *J* = 7.8 Hz, 2H), 7.78-7.75 (m, 3H), 7.71-7.67 (m, 2H), 7.29 (d, *J* = 6.0 Hz, 2H), 4.63 (d, *J* = 6.0 Hz, 2H).

### Example 116. 5-(2-Nitrophenyl)-N-(pyridin-3-ylmethyl)-2-(4-(trifluoromethyl)phenyl)oxazole-4-carboxamide (142)

This compound was obtained in quantitative yield with a typical procedure of hydrolysis and amide coupling reaction, following the same procedure described for the synthesis of compound **114.** ¹H NMR (600 MHz, CDCl₃) *δ* 8.64 (d, *J=* 1.2 Hz, 1H), 8.57 (dd, *J =* 1.2 Hz and *J =* 4.8 Hz, 1H), 8.16 (t, *J =* 6.9 Hz, 3H), 7.96 (dd, *J =* 1.2 Hz and *J =* 7.8 Hz, 1H), 7.79-7.68 (m, 5H), 7.61 (t, *J=* 6.0 Hz, 1H), 7.31-7.28 (m, 1H), 4.65 (d, *J=* 6.0 Hz, 2H).

### Example 117. 5-(2-Nitrophenyl)-N-(pyridin-2-yl)-2-(4-(trifluoromethyl)phenyl)oxazole-4-carboxamide (143)

This compound was obtained in 47% yield with a typical procedure of hydrolysis and amide coupling reaction, following the same procedure described for the synthesis of compound **114.** ¹H NMR (600 MHz, CDCl₃) *δ* 9.58 (s, 1H), 8.37 (s, 1H), 8.26 (d, *J* = 8.2 Hz, 1H), 8.22 (d, *J=* 8.2 Hz, 2H), 8.19 (dd, *J=* 1.2 Hz and *J* = 8.2 Hz, 1H), 7.94 (dd, *J =* 1.3 Hz and *J* = 7.8 Hz, 1H), 7.81-7.78 (m, 3H), 7.73-7.69 (m, 2H), 7.09 (ddd, *J =* 1.0 Hz, *J =* 4.9 Hz and *J =* 7.3 Hz, 1H).

### Example 118. (5-(2-Nitrophenyl)-2-(4-(trifluoromethyl)phenyl)oxazol-4-yl)(piperidin-1-yl)methanone (144)

This compound was obtained in 93% yield with a typical procedure of hydrolysis and amide coupling reaction, following the same procedure described for the synthesis of compound **114.** ¹H NMR (600 MHz, CDCl₃) *δ* 8.16 (d, *J=* 8.2 Hz, 2H), 8.07 (dd, *J* = 0.9 Hz and *J* = 8.2 Hz, 1H), 7.91 (dd, *J=* 1.2 Hz and *J* = 7.6 Hz, 1H), 7.75-7.22 (m, 3H), 7.63 (t, *J* = 6.0 Hz, 1H), 3.69-3.65 (m, 4H), 1.67-1.60 (m, 4H), 1.55 (brs, 2H).

### Example 119. (5-(2-Nitrophenyl)-2-(4-(trifluoromethyl)phenyl)oxazol-4-yl)(4-phenylpiperidin-1-yl)methanone (145)

This compound was obtained in 85% yield with a typical procedure of hydrolysis and amide coupling reaction, following the same procedure described for the synthesis of compound **114.** ¹H NMR (600 MHz, CDCl₃) *δ* 8.18 (d, *J =* 8.4 Hz, 2H), 8.09 (dd, *J=* 1.2 Hz and *J* = 8.4 Hz, 1H), 7.94 (dd, *J=* 1.8 Hz and *J* = 7.8 Hz, 1H), 7.78-7.75 (m, 3H), 7.68-7.65 (m, 1H), 7.32 (t, *J=* 7.2 Hz, 2H), 7.24-7.21 (m, 3H), 4.80 (d, *J=* 12.6 Hz, 1H), 4.57 (d, *J=* 13.2 Hz, 1H), 3.23 (t, *J =* 12.6 Hz, 1H), 2.86-2.77 (m, 2H), 1.96 (d, *J=* 12.6 Hz, 1H), 1.88 (d, *J =* 12.6 Hz, 1H), 1.80-1.73 (m, 2H).

### Example 120. (4-Cyclopropylpiperazin-1-yl)(5-(2-nitrophenyl)-2-(4-(trifluoromethyl)phenyl)oxazol-4-yl)methanone (146)

This compound was obtained in 98% yield with a typical procedure of hydrolysis and amide coupling reaction, following the same procedure described for the synthesis of compound **114.** ¹H NMR (600 MHz, CDCl₃) *δ* 8.16 (d, *J=* 8.4 Hz, 2H), 8.08 (d, *J=* 7.8 Hz, 1H), 7.88 (d, *J* = 7.2 Hz, 1H), 7.74 (t, *J* = 8.4 Hz, 3H), 7.65 (t, *J* = 7.8 Hz, 1H), 3.79 (s, 2H), 3.69 (s, 2H), 2.66 (s, 2H), 2.60 (s, 2H), 1.65-1.60 (m, 1H), 1.31-1.22 (m, 2H), 1.30-1.23 (m, 1H), 0.91-0.41 (m, 4H).

### Example 121: N-(4-Acetylphenyl)-5-(2-nitrophenyl)-2-(4-(trifluoromethyl)phenyl)oxazole-4-carboxamide (147)

This compound was obtained in 78% yield with a typical procedure of hydrolysis and amide coupling reaction, following the same procedure described for the synthesis of compound **114.** ¹H NMR (600 MHz, CDCl₃) *δ* 9.12 (s, 1H), 8.23-8.29 (m, 3H), 7.98-7.94 (m, 3H), 7.82-7.79 (m, 5H), 7.73 *(t, J=* 12.0 Hz, 1H), 2.59 (s, 3H).

### Example 122. 5-(2-Nitrophenyl)-N-(4-(trifluoromethyl)benzyl)-2-(4-(trifluoromethyl)phenyl)oxazole-4-carboxamide (148)

This compound was obtained in quantitative yield with a typical procedure of hydrolysis and amide coupling reaction, following the same procedure described for the synthesis of compound **114.** ¹H NMR (600 MHz, CDCl₃) *δ* 8.15 (d, *J=* 7.8 Hz, 3H), 7.95 (d, *J* = 7.8 Hz, 1H), 7.78-7.74 (m, 3H), 7.68 (t, *J=* 7.8 Hz, 1H), 7.61 (d, *J=* 7.8 Hz, 3H), 7.48 (d, *J* = 7.8 Hz, 2H), 4.67(d, *J* = 6.6 Hz, 2H).

### Example 123. N-(4-Fluorophenethyl)-5-(2-nitrophenyl)-2-(4-(trifluoromethyl)phenyl)oxazole-4-carboxamide (149)

This compound was obtained in quantitative yield with a typical procedure of hydrolysis and amide coupling reaction, following the same procedure described for the synthesis of compound **114.** ¹H NMR (600 MHz, CDCl₃) *δ* 8.12 (t, *J=* 8.4 Hz, 3H), 7.92 (dd, *J* = 1.2 Hz and *J* = 7.8 Hz, 1H), 7.76-7.73 (m, 3H), 7.66 (td, *J* = 8.1 Hz and *J* = 1.8 Hz, 1H), 7.28-7.26 (m, 1H), 7.21-7.18 (m, 2H), 7.00 (t, *J =* 8.7 Hz, 2H), 3.63 (q, *J=* 6.6 Hz, 2H), 2.89 (t, *J* = 7.2 Hz, 2H).

### Example 124. (4-(3-(Dimethylamino)propyl)piperazin-1-yl)(5-(2-nitrophenyl)-2-(4-(trifluoromethyl)phenyl)oxazol-4-yl)methanone (150)

This compound was obtained in quantitative yield with a typical procedure of hydrolysis and amide coupling reaction, following the same procedure described for the synthesis of compound **114.** ¹H NMR (600 MHz, MeOD) *δ* 8.23 (d, *J=* 8.4 Hz, 2H), 8.13 (d, *J* = 7.8 Hz, 1H), 7.87-7.84 (m, 3H), 7.70-7.74 (m, 1H), 7.68 (dd, *J* = 7.8 Hz and *J* = 19.8 Hz, 1H), 7.29-7.24 (m, 1H), 3.86 (brs, 2H), 3.69 (brs, 2H), 3.19 (t, *J=* 7.2 Hz, 2H), 2.88 (s, 6H), 2.52-2.48 (m, 6H), 1.93-1.88 (m, 2H).

### Example 125. (5-(2-Nitrophenyl)-2-(4-(trifluoromethyl)phenyl)oxazol-4-yl)(4-(2-(piperidin-1-yl)ethyl)piperazin-1-yl)methanone (151)

This compound was obtained in 92% yield with a typical procedure of hydrolysis and amide coupling reaction, following the same procedure described for the synthesis of compound **114.** ¹H NMR (600 MHz, CDCl₃) *δ* 8.16 (d, *J=* 8.3 Hz, 2H), 8.08 (d, *J=* 8.3 Hz, 1H), 7.87 (d, *J* = 7.7 Hz, 1H), 7.75-7.73 (m, 3H), 7.65 (t, *J=* 8.4 Hz, 1H), 3.85 (brs, 2H), 3.73 (brs, 2H), 2.49-2.60 (m, 12H), 1.62 (t, *J=* 6.0 Hz, 4H), 1.45 (brs, 2H).

### Example 126. (4-(2-Morpholinoethyl)piperazin-1-yl)(5-(2-nitrophenyl)-2-(4-(trifluoromethyl)phenyl)oxazol-4-yl)methanone (152)

This compound was obtained in 31% yield with a typical procedure of hydrolysis and amide coupling reaction, following the same procedure described for the synthesis of compound **114.** ¹H NMR (600 MHz, CDCl₃) *δ* 8.16 (d, *J=* 7.8 Hz, 2H), 8.09 (dd, *J* = 1.2 Hz and *J* = 8.4 Hz, 1H), 7.88 (dd, *J =* 1.2 Hz and *J =* 7.8 Hz, 1H), 7.76-7.73 (m, 3H), 7.67-7.64 (m, 1H), 3.86 (brs, 2H), 3.73-3.70 (m, 6H), 2.55-2.49 (m, 12H).

### Example 127. N-(2-(4-Methylpiperazin-1-yl)ethyl)-5-(2-nitrophenyl)-2-(4-(trifluoromethyl)phenyl)oxazole-4-carboxamide (153)

This compound was obtained in 90% yield with a typical procedure of hydrolysis and amide coupling reaction, following the same procedure described for the synthesis of compound **114.** ¹H NMR (600 MHz, CDCl₃) *δ* 8.17 (d, *J=* 8.3 Hz, 2H), 8.12 (d, *J* = 8.3 Hz, 1H), 7.94 (d, *J* = 7.7 Hz, 1H), 7.77-7.73 (m, 3H), 7.66 (t, *J* = 7.7 Hz, 1H), 7.62 (t, *J* = 5.0 Hz, 1H), 3.51 (q, *J* = 6.0 Hz, 2H), 2.63-2.46 (m, 10H), 2.35 (s, 3H).

### Example 128. (4-(2-(Diethylamino)ethyl)piperazin-1-yl)(5-(2-nitrophenyl)-2-(4-(trifluoromethyl)phenyl)oxazol-4-yl)methanone (154)

This compound was obtained in 82% yield with a typical procedure of hydrolysis and amide coupling reaction, following the same procedure described for the synthesis of compound **114.** ¹H NMR (600 MHz, CDCl₃) *δ* 8.16 (d, *J=* 8.4 Hz, 2H), 8.09 (d, *J=* 7.8 Hz, 1H), 7.86 (dd, *J=* 1.2 Hz andJ= 7.8 Hz, 1H), 7.74 (t, *J=* 7.8 Hz, 3H), 7.65 (t, *J=* 8.4 Hz, 1H), 3.92 (brs, 2H), 3.75 (brs, 2H), 3.05 (brs, 4H), 2.97 (brs, 2H), 2.82 (brs, 2H), 2.57 (d, *J=* 4.2 Hz, 4H), 1.32 (t, *J* = 6.6 Hz, 6H).

### Example 129. (4-Methylpiperazin-1-yl)(5-(2-nitrophenyl)-2-(4-(trifluoromethyl)phenyl)oxazol-4-yl)methanone (155)

This compound was obtained in 42% yield with a typical procedure of hydrolysis and amide coupling reaction, following the same procedure described for the synthesis of compound **114.** ¹H NMR (600 MHz, CDCl₃) *δ* 8.17 (s, 1H), 8.16 (s, 1H), 8.09 (d, *J=* 8.3 Hz, 1H), 7.89 (dd, *J=* 1.1 Hz and *J* = 7.7 Hz, 1H), 7.75-7.73 (m, 3H), 7.65 (t, *J=* 8.4 Hz, 1H), 3.87 (brs, 2H), 3.74 (brs, 2H), 2.46 (brs, 2H), 2.41 (brs, 2H), 2.31 (s, 3H).

### Example 130. Morpholino(5-(2-nitrophenyl)-2-(4-(trifluoromethyl)phenyl)oxazol-4-yl)methanone (156)

This compound was obtained in quantitative yield with a typical procedure of hydrolysis and amide coupling reaction, following the same procedure described for the synthesis of compound **114.** ¹H NMR (600 MHz, CDCl₃) *δ* 8.16 (d, *J=* 7.8 Hz, 2H), 8.10 (dd, *J =* 0.6 Hz and *J =* 8.1 Hz, 1H), 7.88 (dd, *J* = 1.2 Hz and *J* = 7.8 Hz, 1H), 7.79-7.72 (m, 3H), 7.66 (t, *J=* 9.0 Hz, 1H), 3.93 (s, 2H), 3.83-3.62 (m, 6H).

### Example 131. N-(3-Morpholinopropyl)-5-(2-nitrophenyl)-2-(4-(trifluoromethyl)phenyl)oxazole-4-carboxamide (157)

This compound was obtained in 27% yield with a typical procedure of hydrolysis and amide coupling reaction, following the same procedure described for the synthesis of compound **114.** ¹H NMR (600 MHz, CDCl₃) *δ* 8.38 (t, *J=* 5.4 Hz, 1H), 8.15 (d, *J* = 8.4 Hz, 2H), 8.12 (d, *J* = 8.4 Hz, 1H), 7.92 (dd, *J =* 1.2 Hz and *J* = 7.8 Hz, 1H), 7.77 (d, *J =* 8.4, 2H), 7.74 (t, *J=* 7.2 Hz, 1H), 7.65 (t, *J* = 7.8 Hz, 1H), 3.87 (t, *J* = 4.8 Hz, 4H), 3.52 (dd, *J=* 6.0 Hz and *J* = 12.0 Hz, 2H), 2.54 (t, *J=* 6.6 Hz, 6H), 1.91-1.76 (m, 2H).

### Example 132. N-(3-(1H-Imidazol-1-yl)propyl)-5-(2-nitrophenyl)-2-(4-(trifluoromethyl)phenyl)oxazole-4-carboxamide (158)

This compound was obtained in 46% yield with a typical procedure of hydrolysis and amide coupling reaction, following the same procedure described for the synthesis of compound **114.** ¹H NMR (600 MHz, CDCl₃) *δ* 8.17-8.14 (m, 3H), 7.91 (dd, *J=* 1.2 Hz and *J* = 7.8 Hz, 1H), 7.78-7.75 (m, 3H), 7.70-7.66 (m, 2H), 7.29 (t, *J* = 6.0 Hz, 1H), 7.07 (s, 1H), 6.99 (s, 1H), 4.06 (t, *J=* 7.2 Hz, 2H), 3.47-3.43 (m, 2H), 2.14-2.10 (m, 2H).

### Example 133. 5-(2-Nitrophenyl)-N-((tetrahydrofuran-2-yl)methyl)-2-(4-(trifluoromethyl)phenyl)oxazole-4-carboxamide (159)

This compound was obtained in 96% yield with a typical procedure of hydrolysis and amide coupling reaction, following the same procedure described for the synthesis of compound **114.** ¹H NMR (600 MHz, CDCl₃) *δ* 8.18 (d, *J=* 8.4 Hz, 2H), 8.13 (dd, *J=* 1.2 Hz and *J* = 8.4 Hz, 1H), 7.94 (dd, *J =* 1.2 Hz and *J =* 7.8 Hz, 1H), 7.77-7.73 (m, 3H), 7.67-7.65 (m, 1H), 7.51 *(t, J=* 6.0 Hz, 1H), 4.10-4.06 (m, 1H), 3.96-3.93 (m, 1H), 3.84 -3.80 (m, 1H), 3.73-3.69 (m, 1H), 3.37-3.33 (m, 1H), 2.05-1.99 (m, 1H), 1.98-1.90 (m, 2H), 1.64-1.60 (m, 1H).

### Example 134. N-(2-Methoxyethyl)-5-(2-nitrophenyl)-2-(4-(trifluoromethyl)phenyl)oxazole-4-carboxamide (160)

This compound was obtained in 53% yield with a typical procedure of hydrolysis and amide coupling reaction, following the same procedure described for the synthesis of compound **114.** ¹H NMR (600 MHz, CDCl₃) *δ* 8.15 *(d, J=* 8.4 Hz, 2H), 8.10 *(d, J=* 8.4 Hz, 1H), 7.91 (dd, *J* = 1.2 Hz and *J* = 7.2 Hz, 1H), 7.74-7.71 (m, 3H), 7.65-7.62 (m, 1H), 7.50 *(t, J=* 5.4 Hz, 1H), 3.59 (q, *J=* 4.8 Hz, 2H), 3.54 (t, *J=* 4.8 Hz, 2H), 3.40 (s, 3H).

### Example 135. (4-Hydroxypiperidin-1-yl)(5-(2-nitrophenyl)-2-(4-(trifluoromethyl)phenyl)oxazol-4-yl)methanone (161)

This compound was obtained in 95% yield with a typical procedure of hydrolysis and amide coupling reaction, following the same procedure described for the synthesis of compound **114.** ¹H NMR (600 MHz, CDCl₃) *δ* 8.17 (d, *J=* 7.8 Hz, 2H), 8.08 (dd, *J=* 1.2 Hz and *J =* 8.4 Hz, 1H), 7.90 (dd, *J=* 1.2 Hz and *J* = 7.8 Hz, 1H), 7.76-7.73 (m, 3H), 7.66-7.63 (m, 1H), 4.13 (brs, 2H), 3.99-3.98 (m, 1H), 3.52 (t, *J=* 9.0 Hz, 1H), 3.37 (t, *J=* 9.6 Hz, 1H), 1.94 (brs, 2H), 1.61-1.59 (m, 2H).

### Example 136. (5-(2-Nitrophenyl)-2-(4-(trifluoromethyl)phenyl)oxazol-4-yl)(4-(piperidin-1-yl)phenyl)methanone (162)

This compound was obtained in 32% yield with a typical procedure of hydrolysis and amide coupling reaction, following the same procedure described for the synthesis of compound **114.** ¹H NMR (600 MHz, CDCl₃) *δ* 8.85 (s, 1H), 8.21 *(d, J=* 8.4 Hz, 2H), 8.15 (dd*, J* = 1.2 Hz and *J* = 8.4 Hz, 1H), 8.00 (dd, *J* = 1.2 Hz and *J* = 7.2 Hz, 1H), 7.79-7.75 (m, 3H), 7.70-7.67 (m, 1H), 7.55 (d, *J* = 9.0 Hz, 2H), 6.92 (d, *J* = 9.0 Hz, 2H), 3.14 (t, *J* = 6.0 Hz, 4H), 1.74-1.70 (m, 4H), 1.60-1.56 (m, 2H).

### Example 137. Ethyl 4-(5-(2-nitrophenyl)-2-(4-(trifluoromethyl)phenyl)oxazole-4-carbonyl)piperazine-1-carboxylate (163)

This compound was obtained in quantitative yield with a typical procedure of hydrolysis and amide coupling reaction, following the same procedure described for the synthesis of compound **114.** ¹H NMR (600 MHz, CDCl₃) *δ* 8.16 (d, *J=* 8.4 Hz, 2H), 8.10 (dd, *J =* 1.2 Hz and *J =* 8.4 Hz, 1H), 7.88 (d, *J* = 7.8 Hz, 1H), 7.75 (t, *J=* 8.4 Hz, 3H), 7.66 (t, *J* = 8.4 Hz, 1H), 4.17 (dd, *J=* 7.2 Hz and *J* = 13.8 Hz, 2H), 3.90 (s, 2H), 3.70 (s, 2H), 3.56 (s, 4H), 1.27 (dd, *J=* 6.6 Hz and *J* = 14.1 Hz, 3H).

### Example 138. N-Benzyl-5-(2-nitrophenyl)-2-(4-(trifluoromethyl)phenyl)oxazole-4-carboxamide (164)

This compound was obtained in 99% yield with a typical procedure of hydrolysis and amide coupling reaction, following the same procedure described for the synthesis of compound **114.** ¹H NMR (600 MHz, CDCl₃) *δ* 8.14 (dd, *J=* 1.2 Hz and *J* = 8.4 Hz, 3H), 7.97 (dd, *J=* 1.2 Hz and *J =* 7.5 Hz, 1H), 7.76-7.72 (m, 3H), 7.67 (t, *J=* 6.0 Hz, 1H), 7.49 (t, *J=* 5.4 Hz, 5H), 7.36 (d, *J=* 7.2 Hz, 4H), 7.31-7.28 (m, 1H), 4.61 (d, *J=* 6.0 Hz, 2H).

### Example 139. N-(4-(Benzyloxy)phenyl)-5-(2-nitrophenyl)-2-(4-(trifluoromethyl)phenyl)oxazole-4-carboxamide (165)

This compound was obtained in 82% yield with a typical procedure of hydrolysis and amide coupling reaction, following the same procedure described for the synthesis of compound **114.** ¹H NMR (600 MHz, CDCl₃) *δ* 8.89 (s, 1H), 8.20 (d, *J* = 8.4 Hz, 2H), 8.16 (dd, *J* = 1.2 Hz and *J* = 8.4 Hz, 1H), 7.98 (dd, *J* = 1.2 Hz and *J* = 7.8 Hz, 1H), 7.80-7.75 (m, 3H), 7.70-7.67 (m, 1H), 7.59 (d, *J=* 9.0 Hz, 2H), 7.44 (d, *J=* 7.2 Hz, 2H), 7.40 (t, *J=* 7.2 Hz, 2H), 7.35-7.33 (m, 1H), 6.98 (d, *J=* 9.0 Hz, 2H), 5.07 (s, 2H).

### Example 140. N-(4-Methoxybenzyl)-5-(2-nitrophenyl)-2-(4-(trifluoromethyl)phenyl)oxazole-4-carboxamide (166)

This compound was obtained in quantitative yield with a typical procedure of hydrolysis and amide coupling reaction, following the same procedure described for the synthesis of compound **114.** ¹H NMR (600 MHz, CDCl₃) *δ* 8.16-8.13 (m, 3H), 7.98 (dd, *J* = 1.2 Hz and *J* = 7.8 Hz, 1H), 7.78-7.74 (m, 3H), 7.70-7.67 (m, 1H), 7.42 *(t, J=* 5.4 Hz, 1H), 7.31 (d, *J=* 9.0 Hz, 2H), 4.55 (d, *J=* 6.0 Hz, 2H), 3.82 (s, 3H).

### Example 141. (4-(2-(Diisopropylamino)ethyl)piperazin-1-yl)(5-(2-nitrophenyl)-2-(4-(trifluoromethyl)phenyl)oxazol-4-yl)methanone (167)

This compound was obtained in 43% yield with a typical procedure of hydrolysis and amide coupling reaction, following the same procedure described for the synthesis of compound **114.** ¹H NMR (600 MHz, CDCl₃) *δ* 8.16 (d, *J=* 8.4 Hz, 2H), 8.09 (d, *J=* 7.8 Hz, 1H), 7.87 (dd, *J* = 1.2 Hz and J=7.8 Hz, 1H), 7.74 (t, *J =* 7.8 Hz, 3H), 7.65 (t, *J =* 8.4 Hz, 1H), 3.68 (brs, 2H), 3.73 (brs, 2H), 3.56 (brs, 3H), 2.53 (brs, 2H), 1.17 (brs, 12H).

### Example 142. (4-Isopropylpiperazin-1-yl)(S-(2-nitrophenyl)-2-(4-(trifluoromethyl)phenyl)oxazol-4-yl)methanone (168)

This compound was obtained in quantitative yield with a typical procedure of hydrolysis and amide coupling reaction, following the same procedure described for the synthesis of compound **114.** ¹H NMR (600 MHz, CDCl₃) *δ* 8.18 (d, *J* = 8.4 Hz, 2H), 8.07 (d, *J* = 7.8 Hz, 1H), 7.84 (t, *J=* 7.8 Hz, 1H), 7.75 (d, *J =* 8.4 Hz, 2H), 7.74 (t, *J=* 7.8 Hz, 1H), 7.64 (t, *J* = 7.8 Hz, 1H), 4.17 (s, 2H), 3.91 (s, 2H), 3.21-3.11 (m, 1H), 2.92 (s, 4H), 1.19 (s, 3H), 1.18 (s, 3H).

### Example 143. (4-(2-Hydroxyethyl)piperazin-1-yl)(5-(2-nitrophenyl)-2-(4-(trifluoromethyl)phenyl)oxazol-4-yl)methanone (169)

This compound was obtained in 64% yield with a typical procedure of hydrolysis and amide coupling reaction, following the same procedure described for the synthesis of compound **114.** ¹H NMR (600 MHz, CDCl₃) *δ* 8.16 (d, *J=* 8.1 Hz, 2H), 8.09 (dd, *J=* 0.8 Hz and *J* = 8.2 Hz, 1H), 7.88 (dd, *J =* 1.3 Hz and *J* = 7.7 Hz, 1H), 7.76-7.74 (m, 3H), 7.65 (t, *J* = 8.4 Hz, 1H), 3.9 (brs, 2H), 3.75 (brs, 2H), 3.65 (t, *J* = 6.0 Hz, 2H), 1.19-1.18 (m, 6H).

### Example 144. (5-(2-Nitrophenyl)-2-(4-(trifluoromethyl)phenyl)oxazol-4-yl)(4-(2-(pyrrolidin-1-yl)ethyl)piperazin-1-yl)methanone (170)

This compound was obtained in 90% yield with a typical procedure of hydrolysis and amide coupling reaction, following the same procedure described for the synthesis of compound **114.** ¹H NMR (600 MHz, CDCl₃) *δ* 8.14 (d, *J* = 8.3 Hz, 2H), 8.06 (dd, *J =* 1.1 Hz and *J* = 8.1 Hz, 1H), 7.76-7.73 (m, 2H), 7.67-7.64 (m, 2H), 7.31 (t, *J* = 8.4 Hz, 1H), 3.82 (brs, 2H), 3.66 (brs, 2H), 3.37 (brs, 4H), 3.20 (t, *J=* 6.0 Hz, 2H), 2.83 (t, *J* = 6.0 Hz, 2H), 2.51 (t, *J=* 4.4 Hz, 4H), 2.09 (brs, 4H).

### Example 145. N-(3-(4-Methylpiperazin-1-yl)propyl)-5-(2-nitrophenyl)-2-(4-(trifluoromethyl)phenyl)oxazole-4-carboxamide (171)

This compound was obtained in 51% yield with a typical procedure of hydrolysis and amide coupling reaction, following the same procedure described for the synthesis of compound **114.** ¹H NMR (600 MHz, MeOD) *δ* 8.32 (d, *J* = 8.2 Hz, 2H), 8.22-8.20 (m, 1H), 7.92-7.86(m, 4H), 7.81-7.78 (m, 1H), 7.74-7.69 (m, 1H), 7.33-7.29 (m, 1H), 3.44 (t, *J=* 6.6 Hz, 2H), 3.04 (s, 4H), 2.83 (s, 2H), 2.78 (s, 1H), 2.67-2.65 (m, 6H), 1.87-1.83 (m, 4H).

### Example 146. tert-Butyl-4-(2-(5-(2-nitrophenyl)-2-(4-(trifluoromethyl)phenyl)oxazole-4-carboxamido)ethyl)piperazine-1-carboxylate (172)

This compound was obtained in 87% yield with a typical procedure of hydrolysis and amide coupling reaction, following the same procedure described for the synthesis of compound **114.** ¹H NMR (600 MHz, CDCl₃) *δ* 8.18 (d, *J* = 8.2 Hz, 2H), 8.14 (dd, *J=* 1.2 Hz and *J* = 8.2 Hz, 1H), 7.95 (dd, *J=* 1.3 Hz and *J* = 7.8 Hz, 1H), 7.79 (d, *J=* 8.5 Hz, 2H), 7.76-7.74 (m, 1H), 7.69-7.66 (m, 1H), 7.61 (t, *J=* 5.3 Hz, 1H), 3.55-3.50 (m, 7H), 2.64 (t, *J=* 6.2 Hz, 2H), 2.64 (t, *J* = 6.2 Hz, 2H), 1.49 (s, 9H).

### Example 147. 5-(2-Nitrophenyl)-N-(2-(piperidin-1-yl)ethyl)-2-(4-(trifluoromethyl)phenyl)oxazole-4-carboxamide (173)

This compound was obtained in quantitative yield with a typical procedure of hydrolysis and amide coupling reaction, following the same procedure described for the synthesis of compound **114.** ¹H NMR (600 MHz, CDCl₃) *δ* 8.11 (d, *J=* 7.9 Hz, 1H), 7.93-7.92 (m, 1H), 7.84 (d, *J =* 8.2 Hz, 2H), 7.74 (d, *J=* 7.3 Hz, 2H), 7.66-7.64 (m, 1H), 7.58 (d, *J=* 7.9 Hz, 3H), 3.90 (q, *J* = 5.9 Hz, 2H), 3.25 (t, *J=* 5.6 Hz, 3H), 2.17 (s, 6H), 1.88 (s, 3H).

### Example 148. tert-Butyl (2-(5-(2-nitrophenyl)-2-(4-(trifluoromethyl)phenyl)oxazole-4-carboxamido)ethyl)carbamate (174)

This compound was obtained in 93% yield with a typical procedure of hydrolysis and amide coupling reaction, following the same procedure described for the synthesis of compound **114.** ¹H NMR (600 MHz, CDCl₃) *δ* 8.19 (d, *J=* 2.4 Hz, 2H), 8.15 (dd, *J=* 1.2 Hz andJ= 8.4 Hz, 1H), 7.95 (dd, *J=* 1.2 Hz and *J* = 7.8 Hz, 1H), 7.78-7.77 (m, 3H), 7.70-7.67 (m, 1H), 7.62 (brs, 1H), 3.55 (q, *J* = 5.4 Hz, 2H), 3.41 (d, *J=* 5.4 Hz, 2H), 1.48 (s, 9H).

### Example 149. N-(4-Acetamidophenyl)-5-(2-nitrophenyl)-2-(4-(trifluoromethyl)phenyl)oxazole-4-carboxamide (175)

This compound was obtained in quantitative yield as a fluffy white solid with a typical procedure of hydrolysis and amide coupling reaction, following the same procedure described for the synthesis of compound **114.** ¹ H NMR (600 MHz, CDCl₃) *δ* 8.23 (d, *J=* 8.4 Hz, 2H), 8.19 (d, *J =* 7.8 Hz, 1H), 7.98 (d, *J=* 7.2 Hz, 1H), 7.81=7.79 (m, 3H), 7.72 (t, *J =* 7.8 Hz, 1H), 7.67-7.65 (m, 2H), 7.53-7.51 (m, 2H), 2.19 (s, 3H).

### Example 150. N-(4-Hydroxyphenethyl)-5-(2-nitrophenyl)-2-(4-(trifluoromethyl)phenyl)oxazole-4-carboxamide (176)

This compound was obtained in quantitative yield as a fluffy white solid with a typical procedure of hydrolysis and amide coupling reaction, following the same procedure described for the synthesis of compound **114.** ¹H NMR (600 MHz, CDCl₃) *δ* 8.13 (dd, *J=* 1.8 Hz and *J* = 8.4 Hz, 3H), 7.91 (dd, *J =* 1.2 Hz and *J =* 7.8 Hz, 1H), 7.75-7.72 (m, 3H), 7.66-7.63(m, 1H), 7.38 (t, *J =* 5.4 Hz, 1H), 7.04 (d, *J=* 8.4 Hz, 2H), 6.74 (d, *J* = 8.4 Hz, 2H), 6.43 (brs, 1H), 3.62 (q, *J=* 6.6 Hz, 2H), 2.84 (t, *J=* 7.2 Hz, 2H).

### Example 151. N-(2-(Dimethylamino)ethyl)-5-(3-nitrophenyl)-2-(4-(trifluoromethyl)phenyl)oxazole-4-carboxamide (177)

This compound was obtained in 65% yield with a typical procedure of hydrolysis and amide coupling reaction, following the same procedure described for the synthesis of compound **114.** ¹H NMR (600 MHz, CDCl₃) *δ* 9.24 (s, 1H), 8.92 (d, *J=* 8.4 Hz, 1H), 8.28 (t, *J=* 9.0 Hz, 3H), 7.80 (d, *J=* 7.8 Hz, 2H), 7.78 (s, 1H), 7.69 (t, *J=* 7.8 Hz, 1H), 3.60 (dd, *J=* 5.4 Hz and *J* = 11.7 Hz, 2H), 2.58 (t, *J=* 6.0 Hz, 2H), 2.33 (s, 6H).

### Example 152. (5-(3-Nitrophenyl)-2-(4-(trifluoromethyl)phenyl)oxazol-4-yl)(piperazin-1-yl)methanone (178)

This compound was obtained in 97% yield with a typical procedure of hydrolysis and amide coupling reaction, following the same procedure described for the synthesis of compound **114.** ¹H NMR (600 MHz, MeOD) *δ* 8.92 (s, 1H), 8.49-8.31 (m, 4H), 7.90 (d, *J* =8.4 Hz, 2H), 7.80 (t, *J=* 8.4 Hz, 1H), 4.09 (s, 4H), 3.41 (s, 2H), 3.37 (s, 2H).

### Example 153. (4-(2-(Dimethylamino)ethyl)piperazin-1-yl)(5-(3-nitrophenyl)-2-(4-(trifluoromethyl)phenyl)oxazol-4-yl)methanone (179)

This compound was obtained in 36% yield with a typical procedure of hydrolysis and amide coupling reaction, following the same procedure described for the synthesis of compound **114.** ¹H NMR (600 MHz, MeOD) *δ* 8.76 (s, 1H), 8.38 (d, *J =* 7.8 Hz, 2H), 8.35 (dd, *J =* 1.2 Hz and *J* = 8.4 Hz, 1H), 8.31 (d, *J* = 7.8 Hz, 1H), 7.92 (d, *J =* 8.4 Hz, 2H), 7.83 (t, *J* = 7.8Hz, 1H), 3.91 (t, *J =* 9.6 Hz, 2H), 3.70 (t, *J =* 9.6 Hz, 2H), 2.69 (t, *J =* 10.2 Hz, 2H), 2.58-2.51 (m, 6H), 2.30 (s, 6H).

### Example 154. N-(2-(Dimethylamino)ethyl)-5-(4-nitrophenyl)-2-(4-(trifluoromethyl)phenyl)oxazole-4-carboxamide (180)

This compound was obtained in 52% yield with a typical procedure of hydrolysis and amide coupling reaction, following the same procedure described for the synthesis of compound **114.** ¹H NMR (600 MHz, CDCL₃) *δ* 8.53 (d, *J =* 8.4 Hz, 2H), 8.33 (d, *J* = 8.4 Hz, 2H), 8.26 (d, *J =* 8.4 Hz, 2H), 7.80 (d, *J* = 8.4 Hz, 2H), 3.59 (dd, *J =* 6.0 Hz and *J =* 12.0 Hz, 2H), 2.59 (t, *J* = 6.6 Hz, 2H), 2.34 (s, 6H).

### Example 155. (5-(4-Nitrophenyl)-2-(4-(trifluoromethyl)phenyl)oxazol-4-yl)(piperazin-1-yl)methanone (181)

This compound was obtained in 99% yield with a typical procedure of hydrolysis and amide coupling reaction, following the same procedure described for the synthesis of compound **114.** ¹H NMR (600 MHz, MeOD) *δ* 8.40 (d, *J =* 6.6 Hz, 2H), 8.39 (d, *J =* 5.4 Hz, 2H), 8.26 (d, *J* = 9.0 Hz, 2H), 7.92 (d, *J =* 8.4 Hz, 2H), 4.11 (s, 2H), 4.04 (s, 2H), 3.44 (s, 2H), 3.38 (s, 2H), 3.37 (s, 6H).

### Example 156. (4-(2-(Dimethylamino)ethyl)piperazin-1-yl)(5-(4-nitrophenyl)-2-(4-(trifluoromethyl)phenyl)oxazol-4-yl)methanone (182)

This compound was obtained in 30% yield with a typical procedure of hydrolysis and amide coupling reaction, following the same procedure described for the synthesis of compound **114.** ¹H NMR (600 MHz, MeOD) *δ* 8.39 (t, *J =* 4.8 Hz, 4H), 8.15 (d, *J =* 9.0 Hz, 2H), 7.92 (d, *J =* 8.4 Hz, 2H), 3.90 (t, *J =* 4.2 Hz, 2H), 3.66 (t, *J =* 5.4 Hz, 2H), 2.67 (t, *J* = 4.8 Hz, 2H), 2.63-2.41 (m, 6H), 2.31 (s, 6H).

### Example 157. 5-(2-Aminophenyl)-N-(2-(dimethylamino)ethyl)-2-(4-(trifluoromethyl)phenyl)oxazole-4-carboxamide (183)

This compound was obtained in 71% yield with a typical procedure of hydrolysis and amide coupling reaction, following the same procedure described for the synthesis of compound **114.** ¹H NMR (600 MHz, CDCl₃) *δ* 8.23 (d, *J =* 7.2 Hz, 2H), 7.98 (s, 1H), 7.74 (d, *J =* 7.2 Hz, 2H), 7.50 (d, *J =* 7.8 Hz, 1H), 7.29-7.26 (m, 1H), 6.88-6.83 (m, 3H), 3.64 (t, *J =* 4.2 Hz, 2H), 2.77 (t, *J* = 4.2 Hz, 2H), 2.43 (s, 6H).

### Example 158. (5-(2-Aminophenyl)-2-(4-(trifluoromethyl)phenyl)oxazol-4-yl)(4-(2-(dimethylamino)ethyl)piperazin-1-yl)methanone (184)

This compound was obtained in quantitative yield with a typical procedure of hydrolysis and amide coupling reaction, following the same procedure described for the synthesis of compound **114.** ¹H NMR (600 MHz, CDCl₃) δ 8.19 (d, *J=* 8.4 Hz, 2H), 7.74 (d, *J* = 8.4 Hz, 2H), 7.36 (d, *J* = 9.0 Hz, 1H), 7.29-7.21 (m, 1H), 6.83 (t, *J=* 7.2 Hz, 1H), 6.78 (d, *J* = 8.4 Hz, 1H), 3.81 (s, 2H), 3.53 (s, 2H), 2.59-2.32 (m, 6H), 2.31-2.25 (m, 2H), 2.23 (s, 6H).

### Example 159. 5-(3-Aminophenyl)-N-(2-(dimethylamino)ethyl)-2-(4-(trifluoromethyl)phenyl)oxazole-4-carboxamide (185)

This compound was obtained in 43% yield with a typical procedure of hydrolysis and amide coupling reaction, following the same procedure described for the synthesis of compound **114.** ¹H NMR (600 MHz, CDCl₃) *δ* 8.61 (brs, 1H), 8.26 *(d, J=* 8.4 Hz, 2H), 7.96(d, *J* = 8.4 Hz, 1H), 7.78 (d, *J =* 8.4 Hz, 2H), 7.56 (d, J = 8.4 Hz, 1H), 7.33 (t, J = 7.2 Hz, 1H), 3.59 (q*, J* = 6.6 Hz, 2H), 2.61 (t, *J =* 6.6 Hz, 2H), 2.53 (s, 6H).

### Example 160. 5-(4-Aminophenyl)-N-(2-(dimethylamino)ethyl)-2-(4-(trifluoromethyl)phenyl)oxazole-4-carboxamide (186)

This compound was obtained in 59% yield with a typical procedure of hydrolysis and amide coupling reaction, following the same procedure described for the synthesis of compound **114.** ¹H NMR (600 MHz, CDCl₃) *δ* 8.25 (d, *J* = 8.4 Hz, 2H), 8.21 (d, *J=* 8.4 Hz, 2H), 7.75 (d, *J* = 8.4 Hz, 2H), 7.69 (brs, 1H), 6.76 (d, *J=* 8.4 Hz, 2H), 3.59 (q, *J* = 6.6 Hz, 2H), 2.61 (t, *J* = 6.6 Hz, 2H), 2.53 (s, 6H).

### Example 161. 5-(2-Acetamidophenyl)-N-(2-(dimethylamino)ethyl)-2-(4-(trifluoromethyl)phenyl)oxazole-4-carboxamide (187)

This compound was obtained in 69% yield with a typical procedure of hydrolysis and amide coupling reaction, following the same procedure described for the synthesis of compound **114.** ¹H NMR (600 MHz, MeOD) *δ* 8.31 (d, *J=* 8.4 Hz, 2H), 7.88 (d, *J=* 8.4 Hz, 2H), 7.76 (d, *J=* 7.8 Hz, 1H), 7.70 (d, *J=* 7.8 Hz, 1H), 7.57 (t, *J* = 7.8 Hz, 1H), 7.39 (t, *J* = 7.8 Hz, 1H), 3.61 (t, *J=* 6.0 Hz, 2H), 2.72 (t, *J=* 6.6 Hz, 2H), 2.43 (s, 6H), 2.05 (s, 3H).

### Example 162. N-(2-(4-(4-(2-(Dimethylamino)ethyl)piperazine-1-carbonyl)-2-(4-(trifluoromethyl)phenyl)oxazol-5-yl)phenyl)acetamide (188)

This compound was obtained in 78% yield with a typical procedure of hydrolysis and amide coupling reaction, following the same procedure described for the synthesis of compound **114.** ¹H NMR (600 MHz, MeOD) *δ* 8.29 (d, *J=* 7.8 Hz, 2H), 7.89 (d, *J* = 7.8 Hz, 2H), 7.67 (d, *J= 8.4 Hz,* 1H), 7.64 (d, *J* = 7.2 Hz, 1H), 7.56 (t, *J* = 7.2 Hz, 1H), 7.40 *(t, J=* 7.8 Hz, 1H), 3.72 (s, 2H), 3.53 (s, 2H), 2.53 (s, 2H), 2.48 (s, 4H), 2.28 (s, 6H), 2.17 (s, 2H), 2.07 (s, 3H).

### Example 163. 5-(3-Acetamidophenyl)-N-(2-(dimethylamino)ethyl)-2-(4-(trifluoromethyl)phenyl)oxazole-4-carboxamide (189)

This compound was obtained in 91% yield with a typical procedure of hydrolysis and amide coupling reaction, following the same procedure described for the synthesis of compound **114.** ¹H NMR (600 MHz, MeOD) *δ* 8.45 (s, 1H), 8.29 (d, *J* = 7.8 Hz, 2H), 8.07 (d, *J* = 7.8 Hz, 1H), 7.85 (d, *J* = 8.4 Hz, 2H), 7.68 (dd, *J* = 1.2 Hz and *J* = 8.4 Hz, 1H), 7.44 (t, *J=* 7.8 Hz, 1H), 3.58 (t, *J=* 6.6 Hz, 2H), 2.69 (t, *J* = 6.6 Hz, 2H), 2.41 (s, 6H), 2.18 (s, 3H).

### Example 164. N-(3-(4-(4-(2-(Dimethylamino)ethyl)piperazine-1-carbonyl)-2-(4-(trifluoromethyl)phenyl)oxazol-5-yl)phenyl)acetamide (190)

This compound was obtained in 83% yield with a typical procedure of hydrolysis and amide coupling reaction, following the same procedure described for the synthesis of compound **114.** ¹H NMR (600 MHz, MeOD) *δ* 8.33 *(d, J=* 6.0 Hz, 2H), 8.24 (s, 1H), 7.89 *(d, J=* 8.4 Hz, 2H), 7.58 (d, *J=* 7.8 Hz, 1H), 7.53 (d, *J=* 9.0 Hz, 1H), 7.47 (t, *J=* 7.8 Hz, 1H), 3.90 (t, *J* = 4.8 Hz, 2H), 3.57 (t, *J* = 4.8 Hz, 2H), 2.66 (t, *J* = 5.4 Hz, 2H), 2.61-2.51 (m, 4H), 2.45 (t, *J* = 4.8 Hz, 2H), 2.29 (s, 6H), 2.17 (s, 3H).

### Example 165. 5-(4-Acetamidophenyl)-N-(2-(dimethylamino)ethyl)-2-(4-(trifluoromethyl)phenyl)oxazole-4-carboxamide (191)

This compound was obtained in 71% yield with a typical procedure of hydrolysis and amide coupling reaction, following the same procedure described for the synthesis of compound **114.** ¹H NMR (600 MHz, MeOD) *δ* 8.27 (d, *J=* 8.4 Hz, 2H), 8.26 (d, *J=* 8.4 Hz, 2H), 7.83 (d, *J=* 8.4 Hz, 2H), 7.69 (d, *J=* 9.0 Hz, 2H), 3.61 (t, *J=* 6.6 Hz, 2H), 2.81 (t, *J=* 6.6 Hz, 2H), 2.51 (s, 6H), 2.17 (s, 3H).

### Example 166. N-(4-(4-(4-(2-(Dimethylamino)ethyl)piperazine-1-carbonyl)-2-(4-(trifluoromethyl)phenyl)oxazol-5-yl)phenyl)acetamide (192)

This compound was obtained in 81% yield with a typical procedure of hydrolysis and amide coupling reaction, following the same procedure described for the synthesis of compound **114.** ¹H NMR (600 MHz, CDCl₃) *δ* 8.20 (d, *J=* 8.4 Hz, 2H), 7.76 (d, *J=* 7.8 Hz, 2H), 7.68 (d, *J* = 8.4 Hz, 2H), 7.62 (d, *J=* 8.4 Hz, 2H), 3.61-3.43 (m, 12H), 2.61 (s, 6H), 2.05 (s, 3H).

### Example 167. 5-(4-(Methylsulfonyl)phenyl)-N-(pyridin-3-ylmethyl)-2-(4-(trifluoromethyl)phenyl)oxazole-4-carboxamide (193)

This compound was obtained in 87% yield with a typical procedure of hydrolysis and amide coupling reaction, following the same procedure described for the synthesis of compound **114.** ¹H NMR (600 MHz, CDCl₃) *δ* 8.68 (s, 1H), 8.63 (d, *J=* 8.5 Hz, 2H), 8.58 (d, *J=* 4.3 Hz, 1H), 8.24 (d, *J=* 8.2 Hz, 2H), 8.08 (d, *J=* 8.5 Hz, 2H), 7.82 (t, *J=* 5.7 Hz, 1H), 7.79 *(d, J=* 8.2 Hz, 2H), 7.75 (d, *J* = 7.9 Hz, 1H), 7.31 (q, *J=* 4.9 Hz, 1H), 4.72 (d, *J=* 6.1 Hz, 2H), 3.10 (s, 3H). HRMS m/z: calcd for C₂₄H₁₈F₃N₃O₄S [M + H]⁺: 502.1004; found: 502.1059.

### Example 168. N-(2-(4-acetylpiperazin-1-yl)ethyl)-5-(4-(methylthio)phenyl)-2-(4-(trifluoromethyl)phenyl)oxazole-4-carboxamide (194)

This compound was obtained in 48% yield as a white solid with a typical procedure of hydrolysis and amide coupling reaction, following the same procedure described for the synthesis of compound **114.** ¹H NMR (600 MHz, CDCl₃) *δ* 8.36 (t, *J=* 1.5 Hz, 1H), 8.22 (d, *J*= 8.4 Hz, 2H), 8.17 (d, *J* = 7.8 Hz, 1H), 7.79 (d, *J* = 7.8 Hz, 2H), 7.75 (d, *J* = 5.1 Hz, 1H), 7.42 (t, *J* = 7.8 Hz, 1H), 7.35 (d, *J=* 8.4 Hz, 1H), 3.69 (t, *J* = 4.8 Hz, 2H), 3.61 (q, *J=* 6.2 Hz, 2H), 3.52 (t, *J=* 5.1 Hz, 2H), 2.67 (t, *J=* 6.0 Hz, 2H), 2.59 (s, 3H), 2.56 (t, *J=* 4.8 Hz, 2H), 2.53 (t, *J* = 5.1 Hz, 2H), 2.12 (s, 3H).

### Example 169. 5-(4-(Methylthio)phenyl)-N-(pyridin-3-ylmethyl)-2-(4-(trifluoromethyl)phenyl)oxazole-4-carboxamide (195)

This compound was obtained in 89% yield with a typical procedure of hydrolysis and amide coupling reaction, following the same procedure described for the synthesis of compound **114.** ¹H NMR (600 MHz, CDCL₃) *δ* 8.67 (s, 1H), 8.56 (d, *J* = 4.4 Hz, 1H), 8.33 (d, *J=* 8.3 Hz, 2H), 8.20 (d, *J=* 8.2 Hz, 2H), 7.75 (d, *J* = 8.1 Hz, 4 H), 7.35 (d, *J=* 8.3 Hz, 2H), 7.30 (q, *J=* 4.9 Hz, 1H), 4.70 (d, *J=* 6.1 Hz, 2H), 2.54 (s, 3H).

### Example 170. N-(2-(dimethylamino)ethyl)-5-(pyridin-3-yl)-2-(4-(trifluoromethyl)phenyl)oxazole-4-carboxamide (196)

This compound was obtained in 19% yield as a white solid with a typical procedure of hydrolysis and amide coupling reaction, following the same procedure described for the synthesis of compound **114.** ¹H NMR (600 MHz, CDCl₃) *δ* 9.35 (d, *J=* 1.8 Hz, 1H), 8.97 (td, *J* = 1.8 Hz and *J* = 7.8 Hz, 1H), 8.67 (dd, *J* = 1.8 Hz and *J* = 4.8 Hz, 1H), 8.26 (d, *J* = 8.4 Hz, 2H), 7.80 (d, J = 8.4 Hz, 2H), 7.80 (brs, 1H), 7.46-7.42 (m, 1H), 3.59 (q, *J* = 6.0 Hz, 2H), 2.60 (t, *J* = 6.0 Hz, 2H), 2.34 (s, 6H).

### Example 171. N-(2-(4-acetylpiperazin-1-yl)ethyl)-5-(pyridin-3-yl)-2-(4-(trifluoromethyl)phenyl)oxazole-4-carboxamide (197)

This compound was obtained in 49% yield as a white solid with a typical procedure of hydrolysis and amide coupling reaction, following the same procedure described for the synthesis of compound **114.** ¹H NMR (600 MHz, CDCL₃) *δ* 9.36 (d, *J=* 1.2, 1H), 8.96 (d, *J*= 7.8 Hz, 1H), 8.68 (d, *J* = 3.6 Hz, 1H), 8.24 (d, *J=* 7.8 Hz, 2H), 7.81 (d, *J=* 8.4 Hz, 2H), 7.76 (t, *J=* 5.1 Hz, 1H), 7.45 (dd, *J* = 4.8 Hz and *J* = 7.8 Hz, 1H), 3.69 (t, *J=* 4.8 Hz, 2H), 3.62 (q, *J=* 6.0 Hz, 2H), 3.53 (t, *J* = 4.8 Hz, 2H), 2.68 (t, *J=* 6.3 Hz, 2H), 2.57 (t, *J=* 5.1 Hz, 2H), 2.54 (t, *J* = 5.1 Hz, 2H), 2.12 (s, 3H).

### Example 172. N-(2-(4-acetylpiperazin-1-yl)ethyl)-5-(3-methoxyphenyl)-2-(4-(trifluoromethyl)phenyl)oxazole-4-carboxamide (198)

This compound was obtained in 72% yield as a white solid with a typical procedure of hydrolysis and amide coupling reaction, following the same procedure described for the synthesis of compound **114.** ¹H NMR (600 MHz, CDCl₃) *δ* 8.22 (d, *J=* 7.8 Hz, 2H), 8.14 (d, *J* = 3.6 Hz, 1H), 7.94 (d, *J* = 7.8 Hz, 1H), 7.79 (d, *J* = 8.4 Hz, 2H), 7.76 (t, *J* = 5.1 Hz, 1H), 7.42 (t, *J* = 7.8 Hz, 1H), 7.02 (dd, *J* = 2.4 Hz and *J* = 7.8 Hz, 1H), 3.92 (s, 3H), 3.69 (t, *J* = 5.1 Hz, 2H), 3.62 (q, *J=* 6.0 Hz, 2H), 3.52 (t, *J=* 4.2 Hz, 2H), 2.68 (t, *J=* 6.3 Hz, 2H), 2.56 (t, *J=* 4.8 Hz, 2H), 2.53 (t, *J* = 4.8 Hz, 2H), 2.12 (s, 3H).

### Example 173. N-(2-(Dimethylamino)ethyl)-5-phenyl-2-(3-(trifluoromethoxy)phenyl)oxazole-4-carboxamide (199)

This compound was obtained in 42% yield as a white solid with a typical procedure of hydrolysis and amide coupling reaction, following the same procedure described for the synthesis of compound **114.** ¹H NMR (600 MHz, CDCl₃) *δ* 8.37 (d, *J=* 7.3 Hz, 2H), 8.07 (d, *J* = 7.6 Hz, 1H), 7.97 (s, 1H), 7.71 (brs, 1H), 7.56 (t, *J=* 7.9 Hz, 1H), 7.50 (t, *J* = 7.5 Hz, 2H), 7.45 (t, *J=* 7.2 Hz, 1H), 7.37 (d, *J=* 8.2 Hz, 1H), 3.59 (q, *J* = 6.1 Hz, 2H), 2.60 (t, *J=* 6.2 Hz, 2H), 2.35 (s, 6H).

### Example 174. N-(2-(4-Methylpiperazin-1-yl)ethyl)-5-phenyl-2-(3-(trifluoromethoxy)phenyl)oxazole-4-carboxamide (200)

This compound was obtained in 60% yield as a white solid with a typical procedure of hydrolysis and amide coupling reaction, following the same procedure described for the synthesis of compound **114.** ¹H NMR (600 MHz, MeOD) *δ* 8.25 *(dd, J=* 1.2 Hz and *J* = 7.8 Hz, 2H), 8.03 (d, *J* = 7.8 Hz, 1H), 7.93 (s, 1H), 7.60 (t, *J=* 7.8 Hz, 1H), 7.47-7.41 (m, 4H), 3.51 (t, *J=* 6.6 Hz, 2H), 2.62 (t, *J=* 6.6 Hz, 2H), 2.57 (brs, 8H), 2.28 (s, 3H).

### Example 175. N-(2-(Dimethylamino)ethyl)-5-(2-nitrophenyl)-2-(3-(trifluoromethoxy)phenyl)oxazole-4-carboxamide (201)

This compound was obtained in 82% yield as a white solid with a typical procedure of hydrolysis and amide coupling reaction, following the same procedure described for the synthesis of compound **114.** ¹H NMR (600 MHz, MeOD) *δ* 8.17 *(dd, J=* 1.2 Hz and *J* = 8.4 Hz, 1H), 8.05 (d, *J* = 7.8 Hz, 1H), 7.98 (s, 1H), 7.91 (dd, *J* = 1.8 Hz and *J* = 7.8 Hz, 1H), 7.82 (td, *J* = 7.8 Hz and *J* = 1.2 Hz, 1H), 7.75 (td, *J* = 7.8 Hz and *J* =1.2 Hz, 1H), 7.65 (t, *J* = 7.8 Hz, 1H), 7.48-7.47 (m, 1H), 3.46 (t, *J=* 7.2 Hz, 2H), 2.55 (t, *J=* 7.2 Hz, 2H), 2.30 (s, 6H).

### Example 176. N-(2-(4-Methylpiperazin-1-yl)ethyl)-5-(2-nitrophenyl)-2-(3-(trifluoromethoxy)phenyl)oxazole-4-carboxamide (202)

This compound was obtained in 80% yield as a white solid with a typical procedure of hydrolysis and amide coupling reaction, following the same procedure described for the synthesis of compound **114.** ¹H NMR (600 MHz, MeOD) *δ* 8.16 (dd, *J=* 1.2 Hz and *J* = 8.4 Hz, 1H), 8.04 (td, *J* = 7.8 Hz and *J =* 1.2 Hz, 1H), 7.97 (s, 1H), 7.90 (dd, *J=* 1.8 Hz and *J* = 7.8 Hz, 1H), 7.82 (td, *J=* 7.2 Hz and *J =* 1.2 Hz, 1H), 7.75 (td, *J=* 7.8 Hz and *J* = 1.8 Hz, 1H), 7.65 (t, *J=* 7.8 Hz, 1H), 7.51-7.49 (m, 1H), 3.46 (t, *J* = 6.6 Hz, 2H), 2.59 (t, *J=* 6.6 Hz, 2H), 2.54 (brs, 8H), 2.28 (s, 3H).

### Example 177. N-(2-(Dimethylamino)ethyl)-5-phenyl-2-(4-(trifluoromethoxy)phenyl)oxazole-4-carboxamide (203)

This compound was obtained in 83% yield as a white solid with a typical procedure of hydrolysis and amide coupling reaction, following the same procedure described for the synthesis of compound **114.** ¹H NMR (600 MHz, CDCl₃) *δ* 8.37 (d, *J=* 7.2 Hz, 2H), 8.17 (d, *J* = 8.4 Hz, 2H), 7.70 (brs, 1H), 7.51-7.43 (m, 3H), 7.36 (d, *J=* 8.4 Hz, 2H), 3.58 (q, *J=* 6.0 Hz, 2H), 2.58 (t, *J* = 6.0 Hz, 2H), 2.33 (s, 6H).

### Example 178. N-(2-(4-Methylpiperazin-1-yl)ethyl)-S-phenyl-2-(4-(trifluoromethoxy)phenyl)oxazole-4-carboxamide (204)

This compound was obtained in 39% yield as a white solid with a typical procedure of hydrolysis and amide coupling reaction, following the same procedure described for the synthesis of compound **114.** ¹H NMR (600 MHz, CDCL₃) *δ* 8.38 (d, *J* = 7.2 Hz, 2H), 8.17 (d, *J* = 9.0 Hz, 2H), 7.78 (brt, *J* = 4.8 Hz, 1H), 7.50 (t, *J=* 7.2 Hz, 2H), 7.48-7.45 (m, 1H), 7.38 (d, *J=* 9.0 Hz, 2H), 3.60 (q, *J* = 6.0 Hz, 2H), 2.66 (t, *J* = 6.0 Hz, 2H), 2.61-2.54 (brs, 8H), 2.34 (s, 3H).

### Example 179. N-(2-(Dimethylamino)ethyl)-5-(2-nitrophenyl)-2-(4-(trifluoromethoxy)phenyl)oxazole-4-carboxamide (205)

This compound was obtained in 58% yield as a white solid with a typical procedure of hydrolysis and amide coupling reaction, following the same procedure described for the synthesis of compound **114.** ¹H NMR (600 MHz, CDCl₃) *δ* 8.12-8.09 (m, 3H), 7.96 *(dd, J=* 1.2 Hz and *J* = 7.2 Hz, 1H), 7.74 (td, *J* = 7.2 Hz and *J* = 1.2 Hz, 1H), 7.65 (td, *J* = 7.2 Hz and *J* = 1.2 Hz, 1H), 7.55 (brs, 1H), 7.34 (d, *J* = 8.4 Hz, 2H), 3.50 (q, *J* = 6.0 Hz, 2H), 2.54 (t, *J=* 6.0 Hz, 2H), 2.31 (s, 6H).

### Example 180. N-(2-(4-methylpiperazin-1-yl)ethyl)-5-(2-nitrophenyl)-2-(4-(trifluoromethoxy)phenyl)oxazole-4-carboxamide (206)

This compound was obtained in 39% yield as a white solid with a typical procedure of hydrolysis and amide coupling reaction, following the same procedure described for the synthesis of compound **114.** ¹H NMR (600 MHz, MeOD) *δ* 8.18-8.15 (m, 3H), 7.89 (dd, *J =* 1.2 Hz and *J* = 7.8 Hz, 1H), 7.81 (td, *J* = 7.8 Hz andJ= 1.2 Hz, 1H), 7.74 (td, *J* = 8.4 Hz and *J* = 1.8 Hz, 1H), 7.46 (d, *J* = 7.8 Hz, 2H), 3.47 (t, *J* = 6.6 Hz, 2H), 2.59 (t, *J* = 6.6 Hz, 2H), 2.56 (brs, 8H), 2.28 (s, 3H).

### Example 181. (4-(2-(Dimethylamino)ethyl)piperazin-1-yl)(5-(2-nitrophenyl)-2-(4-(trifluoromethoxy)phenyl)oxazol-4-yl)methanone (207)

This compound was obtained in 39% yield as a yellow solid with a typical procedure of hydrolysis and amide coupling reaction, following the same procedure described for the synthesis of compound **114.** ¹H NMR (600 MHz, CDCL₃) *δ* 8.09-8.07 (m, 3H), 7.87 (d, *J=* 7.8 Hz, 1H), 7.73 (t, *J* = 7.8 Hz, 1H), 7.64 (t, *J=* 7.8 Hz, 1H), 7.33 (d, *J* = 8.4 Hz, 2H), 3.88 (brs, 2H), 3.75 (brs, 2H), 2.64-2.51 (m, 8H), 2.46 (s, 6H).

### Example 182. N-(2-(Dimethylamino)ethyl)-2-(3-methoxyphenyl)-5-phenyloxazole-4-carboxamide (208)

This compound was obtained in 25% yield as a white solid with a typical procedure of hydrolysis and amide coupling reaction, following the same procedure described for the synthesis of compound **114.** ¹H NMR (600 MHz, CDCL₃) *δ* 8.38 (d, *J=* 7.8 Hz, 2H), 7.74 (brs, 1H), 7.73 (d, *J=* 7.8 Hz, 1H), 7.65 (s, 1H), 7.49 (t, *J=* 7.8 Hz, 2H), 7.45-7.41(m, 2H), 7.06 (dd, *J=* 1.8 Hz and *J =* 8.4 Hz, 1H), 3.93 (s, 3H), 3.61 (q, *J=* 6.0 Hz, 2H), 2.62 (t, *J=* 6.0 Hz, 2H), 2.35 (s, 6H).

### Example 183. 2-(3-Methoxyphenyl)-N-(2-(4-methylpiperazin-1-yl)ethyl)-5-phenyloxazole-4-carboxamide (209)

This compound was obtained in 46% yield as a white solid with a typical procedure of hydrolysis and amide coupling reaction, following the same procedure described for the synthesis of compound **114.** ¹H NMR (600 MHz, CDCL₃) *δ* 8.39 (d, *J=* 7.8 Hz, 2H), 7.82 (brs, 1H), 7.72 (d, *J* = 7.8 Hz, 1H), 7.65 (s, 1H), 7.49 (t, *J=* 7.8 Hz, 2H), 7.43 (t, *J* = 7.8 Hz, 2H), 7.08- 7.06 (m, 1H), 3.93 (s, 3H), 3.60 (q, *J* = 6.0 Hz, 2H), 2.66 (t, *J=* 6.0 Hz, 2H), 2.60-2.50 (m, 8H), 2.32 (s, 3H).

### Example 184. N-(2-(Dimethylamino)ethyl)-2-(3-methoxyphenyl)-5-(2-nitrophenyl)oxazole-4-carboxamide (210)

This compound was obtained in 61% yield as a white solid with a typical procedure of hydrolysis and amide coupling reaction, following the same procedure described for the synthesis of compound **114.** ¹H NMR (600 MHz, MeOD) *δ* 8.15 (dd, *J* = 1.2 Hz and *J* = 8.4 Hz, 1H), 7.90 (dd, *J* = 1.2 Hz and *J* = 7.8 Hz, 1H), 7.82 (td, *J=* 7.8 Hz and *J* = 1.2 Hz, 1H), 7.75 (td, *J=* 7.8 Hz and *J* = 1.2 Hz, 1H), 7.64 (d, *J=* 8.4 Hz, 1H), 7.61-7.60 (m, 1H), 7.42 (t, *J=* 7.8 Hz, 1H), 7.14 (ddd, *J=* 0.6 Hz, *J=* 2.4 Hz and *J* = 8.4 Hz, 1H), 3.86 (s, 3H), 3.46 (t, *J=* 6.6 Hz, 2H), 2.55 (t, *J=* 6.6 Hz, 2H), 2.29 (s, 6H).

### Example 185. 2-(3-methoxyphenyl)-N-(2-(4-methylpiperazin-1-yl)ethyl)-5-(2-nitrophenyl)oxazole-4-carboxamide (211)

This compound was obtained in 73% yield as a white solid with a typical procedure of hydrolysis and amide coupling reaction, following the same procedure described for the synthesis of compound **114.** ¹H NMR (600 MHz, MeOD) *δ* 8.12 (dd, *J* = 1.2 Hz and *J* = 8.4 Hz, 1H), 7.88 (dd, *J=* 1.2 Hz and *J* = 7.8 Hz, 1H), 7.79 (td, *J* = 7.8 Hz and *J* = 1.2 Hz, 1H), 7.71 *(td, J=* 7.8 Hz and *J* = 1.2 Hz, 1H), 7.61 *(td, J=* 7.8 Hz and *J* = 1.2 Hz, 1H), 7.57-7.56 (m, 1H), 7.41 (t, *J=* 7.8 Hz, 1H), 7.09 (ddd, *J* = 0.6 Hz, *J* = 2.4 and *J=* 8.4 Hz, 1H), 3.85 (s, 3H), 3.45 (t, *J=* 6.6 Hz, 2H), 2.57 (t, *J=* 6.6 Hz, 2H), 2.51 (brs, 8H), 2.27 (s, 6H).

### Example 186. N-(2-(Dimethylamino)ethyl)-2-(4-methoxyphenyl)-5-phenyloxazole-4-carboxamide (212)

This compound was obtained in 45% yield as a white solid with a typical procedure of hydrolysis and amide coupling reaction, following the same procedure described for the synthesis of compound **114.** ¹H NMR (600 MHz, CDCL₃) *δ* 8.37 (d, *J* = 8.4 Hz, 2H), 8.07 (d, *J* = 7.8 Hz, 2H), 7.70 (brs, 1H), 7.48 (t, *J* = 8.4 Hz, 2H), 7.43-7.40 (m, 1H), 7.02 (d, *J=* 8.4 Hz, 2H), 3.90 (s, 3H), 3.59 (q, *J=* 6.0 Hz, 2H), 2.58 (t, *J=* 6.0 Hz, 2H), 2.33 (s, 6H).

### Example 187. 2-(4-Methoxyphenyl)-N-(2-(4-methylpiperazin-1-yl)ethyl)-5-phenyloxazole-4-carboxamide (213)

This compound was obtained in 77% yield as a white solid with a typical procedure of hydrolysis and amide coupling reaction, following the same procedure described for the synthesis of compound **114.** ¹H NMR (600 MHz, MeOD) *δ* 8.21 (dd, *J* = 1.2 Hz and *J* = 8.4 Hz, 2H), 7.92 (d, *J=* 8.4 Hz, 2H), 7.44-7.38 (m, 3H), 6.96 (d, *J* = 8.4 Hz, 2H), 3.79 (s, 3H), 3.49 (t, *J* = 6.6 Hz, 2H), 2.59 (t, *J=* 6.6 Hz, 2H), 2.50 (brs, 8H), 2.26 (s, 3H).

### Example 188. N-(2-(Dimethylamino)ethyl)-2-(4-methoxyphenyl)-5-(2-nitrophenyl)oxazole-4-carboxamide (214)

This compound was obtained in 69% yield as a yellow solid with a typical procedure of hydrolysis and amide coupling reaction, following the same procedure described for the synthesis of compound **114.** ¹H NMR (600 MHz, MeOD) *δ* 8.14 (d, *J=* 7.8 Hz, 1H), 8.02 (d, *J* = 8.4 Hz, 2H), 7.90 (d, *J=* 7.8 Hz, 1H), 7.81 (t, *J=* 7.2 Hz, 1H), 7.73 (t, *J=* 7.2 Hz, 1H), 7.08 (d, *J=* 8.4 Hz, 2H), 3.88 (s, 3H), 3.47 (t, *J=* 6.6 Hz, 2H), 2.56 (t, *J=* 6.6 Hz, 2H), 2.31 (s, 6H).

### Example 189. 2-(4-Methoxyphenyl)-N-(2-(4-methylpiperazin-1-yl)ethyl)-5-(2-nitrophenyl)oxazole-4-carboxamide (215)

This compound was obtained in 82% yield as a white solid with a typical procedure of hydrolysis and amide coupling reaction, following the same procedure described for the synthesis of compound **114.** ¹H NMR (600 MHz, MeOD) *δ* 8.14 (dd, *J* = 1.2 Hz and *J* = 8.4 Hz, 1H), 8.01 (d, *J* = 9.0 Hz, 2H), 7.90 (dd, *J=* 1.2 Hz and *J* = 7.8 Hz, 1H), 7.81 (td, *J=* 7.8 Hz and *J=* 1.2 Hz, 1H), 7.73 (td, *J=* 7.8 Hz and *J =* 1.2 Hz, 1H), 7.07 (t, *J=* 9.0 Hz, 2H), 3.88 (s, 3H), 3.47 (t, *J=* 6.6 Hz, 2H), 2.60 (t, *J=* 6.6 Hz, 2H), 2.58 (brs, 1H), 2.30 (s, 3H).

### Example 190. (4-(2-(Dimethylamino)ethyl)piperazin-1-yl)(2-(4-methoxyphenyl)-5-(2-nitrophenyl)oxazol-4-yl)methanone (216)

This compound was obtained in 67% yield as a white solid with a typical procedure of hydrolysis and amide coupling reaction, following the same procedure described for the synthesis of compound **114.** ¹H NMR (600 MHz, MeOD) *δ* 8.11 (d, *J=* 8.4 Hz, 1H), 8.00 (d, *J* = 9.0 Hz, 2H), 7.85-7.82 (m, 2H), 7.75-7.72 (m, 1H), 7.09 (d, *J* = 9.0 Hz, 2H), 3.89 (s, 3H), 3.73 (brs, 2H), 3.20 (brs, 2H), 2.84 (s, 6H), 2.73-2.70 (m, 2H), 2.60-2.55 (m, 6H).

### Example 191. N-(2-(Dimethylamino)ethyl)-2-(4-methoxyphenyl)-5-(4-nitrophenyl)oxazole-4-carboxamide (217)

This compound was obtained in 54% yield as a yellow solid with a typical procedure of hydrolysis and amide coupling reaction, following the same procedure described for the synthesis of compound **114.** ¹H NMR (600 MHz, DMSO-d₆) *δ* 8.68 (d, *J=* 9.0 Hz, 2H), 8.44 (t, *J* = 6.6 Hz, 1H), 8.35 (d, *J* = 9.0 Hz, 2H), 8.12 (d, *J* = 9.0 Hz, 2H), 7.17 (d, *J* = 9.0 Hz, 2H), 3.87 (s, 3H), 3.42 (q, *J=* 6.6 Hz, 2H), 2.44 (t, *J=* 6.6 Hz, 2H), 2.20 (s, 6H).

### Example 192. (4-(2-(Dimethylamino)ethyl)piperazin-1-yl)(2-(4-methoxyphenyl)-5-(4-nitrophenyl)oxazol-4-yl)methanone (218)

This compound was obtained in 65% yield as a yellow solid with a typical procedure of hydrolysis and amide coupling reaction, following the same procedure described for the synthesis of compound **114.** ¹H NMR (600 MHz, DMSO-d₆) *δ* 8.38 (d, *J =* 9.0 Hz, 2H), 8.09 (d, *J* = 8.4 Hz, 2H), 8.05 (d, *J=* 9.0 Hz, 2H), 7.15 (d, *J=* 8.4 Hz, 2H), 3.87 (s, 3H), 3.71 (brs, 2H), 3.46 (brs, 2H), 2.53-2.52 (m, 2H), 2.42-2.35 (m, 6H), 2.16 (s, 6H).

### Example 193. 2-(4-methoxyphenyl)-N-(2-(4-methylpiperazin-1-yl)ethyl)-5-(4-nitrophenyl)oxazole-4-carboxamide (219)

This compound was obtained in 76% yield as a white solid with a typical procedure of hydrolysis and amide coupling reaction, following the same procedure described for the synthesis of compound **114.** ¹H NMR (600 MHz, MeOD) *δ* 8.52 *(dd, J=* 9.0 Hz and *J* = 1.8 Hz, 2H), 8.25 (dd, *J* = 9.0 Hz and *J* = 1.8 Hz, 2H), 8.02 (dd, *J* = 9.0 Hz and *J* = 1.8 Hz, 2H), 7.05 (dd, *J=* 9.0 Hz and *J =* 1.8 Hz, 2H), 3.86 (s, 3H), 3.53 (t, *J=* 6.6 Hz, 2H), 2.64 (t, *J=* 6.6 Hz, 2H), 2.61 (brs, 8H), 2.30 (s, 3H).

### Example 194. N-(2-(Dimethylamino)ethyl)-2-(3-fluorophenyl)-5-phenyloxazole-4-carboxamide (220)

This compound was obtained in 48% yield as a white solid with a typical procedure of hydrolysis and amide coupling reaction, following the same procedure described for the synthesis of compound **114.** ¹H NMR (600 MHz, CDCL₃) *δ* 8.38 *(d, J=* 7.2 Hz, 2H), 7.92 (d, *J* = 7.8 Hz, 1H), 7.82 (d, *J* = 9.0 Hz, 1H), 7.70 (brs, 1H), 7.51-7.43 (m, 4H), 7.22 (td, *J=* 8.4 Hz and *J* = 1.8 Hz, 1H), 3.59 (q, *J* = 6.0 Hz, 2H), 2.58 (t, *J* = 6.0 Hz, 2H), 2.33 (s, 6H).

### Example 195. 2-(3-Fluorophenyl)N-(2-(4-methylpiperazin-1-yl)ethyl)-5-phenyloxazole-4-carboxamide (221)

This compound was obtained in 48% yield as a white solid with a typical procedure of hydrolysis and amide coupling reaction, following the same procedure described for the synthesis of compound **114.** ¹H NMR (600 MHz, MeOD) *δ* 8.24 (d, *J=* 7.2 Hz, 2H), 7.86 (d, *J* = 7.8 Hz, 1H), 7.76-7.74 (m, 1H), 7.53-7.49 (m, 1H), 7.47-7.41 (m, 3H) 7.26-7.23 (m, 1H), 3.51 (t, *J=* 6.6 Hz, 2H), 2.61 (t, *J=* 6.6 Hz, 2H), 2.58 (brs, 8H), 2.28 (s, 3H).

### Example 196. N-(2-(Dimethylamino)ethyl)-2-(3-fluorophenyl)-5-(2-nitrophenyl)oxazole-4-carboxamide (222)

This compound was obtained in 51% yield as a pale yellow solid with a typical procedure of hydrolysis and amide coupling reaction, following the same procedure described for the synthesis of compound **114.** ¹H NMR (600 MHz, CDCL₃) *δ* 8.12 (dd, *J=* 1.2 Hz and *J =* 8.4 Hz, 1H), 7.94 (dd, *J* = 1.2 Hz and *J =* 7.8 Hz, 1H), 7.85 (d, *J* = 8.4 Hz, 1H), 7.77 (td, *J* = 9.0 Hz and *J* = 1.8 Hz, 1H), 7.74 (td, *J* = 7.8 Hz and *J* = 1.2 Hz, 1H), 7.65 (td, *J=* 7.8 Hz and *J* = 1.2 Hz, 1H), 7.54 (brs, 1H), 7.49-7.46 (m, 1H), 7.22 (td, *J*= 7.8 Hz and *J =* 2.4 Hz, 1H), 3.50 (q, *J=* 6.0 Hz, 2H), 2.53 (t, *J=* 6.0 Hz, 2H), 2.31 (s, 6H).

### Example 197. 2-(3-Fluorophenyl)-N-(2-(4-methylpiperazin-1-yl)ethyl)-5-(2-nitrophenyl)oxazole-4-carboxamide (223)

This compound was obtained in 87% yield as a white solid with a typical procedure of hydrolysis and amide coupling reaction, following the same procedure described for the synthesis of compound **114.** ¹H NMR (600 MHz, MeOD) *δ* 8.14 (dd, *J* = 1.2 Hz and *J* = 8.4 Hz, 1H), 7.88-7.85 (m, 2H), 7.80 (td, *J* = 7.2 Hz and *J* = 1.2 Hz, 1H), 7.77-7.75 (m, 1H), 7.74-7.71 (m, 1H), 7.55 (td, *J=* 8.0 Hz and *J* = 5.7 Hz, 1H), 7.30-7.27 (m, 1H), 3.46 (t, *J* = 6.6 Hz, 2H), 2.58 (t, *J=* 6.6 Hz, 2H), 2.55 (brs, 8H), 2.27 (s, 3H).

### Example 198. N-(2-(Dimethylamino)ethyl)-2-(4-fluorophenyl)-5-phenyloxazole-4-carboxamide (224)

This compound was obtained in 25% yield as a white solid with a typical procedure of hydrolysis and amide coupling reaction, following the same procedure described for the synthesis of compound **114.** ¹H NMR (600 MHz, CDCl₃) *δ* 8.37 (d, *J* = 7.2 Hz, 2H), 8.13 (dd, *J=* 5.4 Hz and *J* = 8.4 Hz, 2H), 7.68 (brs, 1H), 7.49 *(t, J=* 7.2 Hz, 2H), 7.45-7.42 (m, 1H), 7.21 (t, *J=* 8.4 Hz, 2H), 3.58 (q, *J=* 6.0 Hz, 2H), 2.57 (t, *J=* 6.0 Hz, 2H), 2.32 (s, 6H).

### Example 199. 2-(4-Fluorophenyl)-N-(2-(4-methylpiperazin-1-yl)ethyl)-5-phenyloxazole-4-carboxamide (225)

This compound was obtained in 15% yield as a white solid with a typical procedure of hydrolysis and amide coupling reaction, following the same procedure described for the synthesis of compound **114.** ¹H NMR (600 MHz, CDCL₃) *δ* 8.37 (d, *J=* 7.2 Hz, 2H), 8.13 (dd, *J* = 4.4 Hz and *J =* 8.8 Hz, 2H), 7.78 (brs, 1H), 7.51-7.42 (m, 3H), 7.22 (t, *J=* 8.4 Hz, 2H), 3.59 (q, *J=* 6.6 Hz, 2H), 2.66 (t, *J=* 6.6 Hz, 2H), 2.60 (brs, 8H), 2.33 (s, 3H).

### Example 200. N-(2-(Dimethylamino)ethyl)-2-(4-fluorophenyl)-5-(2-nitrophenyl)oxazole-4-carboxamide (226)

This compound was obtained in 82% yield as a pale yellow solid with a typical procedure of hydrolysis and amide coupling reaction, following the same procedure described for the synthesis of compound **114.** ¹H NMR (600 MHz, MeOD) *δ* 8.17 (dd, *J=* 1.2 Hz and *J* = 7.8 Hz, 1H), 8.15-8.12 (m, 2H), 7.91 *(dd, J=* 1.2 Hz and *J* = 7.8 Hz, 1H), 7.83 (td*, J =* 7.8 Hz and *J=* 1.2 Hz, 1H), 7.76 (td, *J* = 7.8 Hz and *J* = 1.2 Hz, 1H), 7.30 (t, *J* = 8.4 Hz, 2H), 3.47 (t, *J* = 6.6 Hz, 2H), 2.56 (t, *J=* 6.6 Hz, 2H), 2.30 (s, 6H).

### Example 201. 2-(4-Fluorophenyl)-N-(2-(4-methylpiperazin-1-yl)ethyl)-5-(2-nitrophenyl)oxazole-4-carboxamide (227)

This compound was obtained in 50% yield as a white solid with a typical procedure of hydrolysis and amide coupling reaction, following the same procedure described for the synthesis of compound **114.** ¹H NMR (600 MHz, MeOD) *δ* 8.00 (dd, *J* = 0.6 Hz and *J* = 8.4 Hz, 1H), 7.96-7.93 (m, 2H), 7.73 (dd, *J* = 1.2 Hz and *J* = 7.8 Hz, 1H), 7.65 (td, *J* = 7.8 Hz and *J* = 1.2 Hz, 1H), 7.58 (td, *J* = 7.8 Hz and *J* = 1.2 Hz, 1H), 7.13 (t, *J* = 9.0, 2H), 3.31 (t, *J* = 6.6 Hz, 2H), 2.43 (t, *J=* 6.6 Hz, 2H), 2.41 (brs, 1H), 2.13 (s, 3H).

### Example 202. (4-(2-(Dimethylamino)ethyl)piperazin-1-yl)(2-(4-fluorophenyl)-5-(2-nitrophenyl)oxazol-4-yl)methanone (228)

This compound was obtained in 47% yield as a white solid with a typical procedure of hydrolysis and amide coupling reaction, following the same procedure described for the synthesis of compound **114.** ¹H NMR (600 MHz, MeOD) *δ* 8.13 (d, *J=* 8.4 Hz, 1H), 8.11 (dd, *J=* 5.4 Hz and *J =* 9.0 Hz, 2H), 7.85-7.84 (m, 2H), 7.77-7.74 (m, 1H), 7.30 (t, *J=* 9.0 Hz, 2H), 3.86 (brs, 2H), 3.70 (brs, 2H), 2.79 (brs, 2H), 2.61-2.58 (m, 6H), 2.50 (s, 6H).

### Example 203. N-(2-(Dimethylamino)ethyl)-2-(4-fluorophenyl)-5-(4-nitrophenyl)oxazole-4-carboxamide (229)

This compound was obtained in 36% yield as a white solid with a typical procedure of hydrolysis and amide coupling reaction, following the same procedure described for the synthesis of compound **114.** ¹H NMR (600 MHz, CDCl₃) *δ* 8.66 (d, *J=* 9.0 Hz, 2H), 8.32 (d, *J* = 9.0 Hz, 2H), 8.16 (dd, *J=* 5.4 Hz and *J* = 8.4 Hz, 2H), 7.80 (brs, 1H), 7.24 (t, *J=* 8.4 Hz, 2H), 3.59 (q, *J=* 6.0 Hz, 2H), 2.59 (t, *J=* 6.0 Hz, 2H), 2.34 (s, 6H).

### Example 204. 2-(4-Fluorophenyl)-N-(2-(4-methylpiperazin-1-yl)ethyl)-5-(4-nitrophenyl)oxazole-4-carboxamide (230)

This compound was obtained in 90% yield as a white solid with a typical procedure of hydrolysis and amide coupling reaction, following the same procedure described for the synthesis of compound **114.** ¹H NMR (600 MHz, MeOD) *δ* 8.62 (d, *J* = 9.0 Hz, 2H), 8.35 (d, *J* = 9.0 Hz, 2H), 8.24 (dd, *J=* 5.4 Hz and *J* = 9.0 Hz, 2H), 7.33 (t, *J=* 9.0 Hz, 2H), 3.58 *(d, J=* 6.6 Hz, 2H), 2.66 (d, *J=* 6.6 Hz, 2H), 2.55 (brs, 8H), 2.30 (s, 3H).

### Example 205. 2-(3,4-Difluorophenyl)-N-(2-(dimethylamino)ethyl)-5-phenyloxazole-4-carboxamide (231)

This compound was obtained in 50% yield as a white solid with a typical procedure of hydrolysis and amide coupling reaction, following the same procedure described for the synthesis of compound **114.** ¹H NMR (600 MHz, CDCl₃) *δ* 8.36 (d, *J=* 7.2 Hz, 2H), 7.96-7.94 (m, 1H), 7.90-7.87 (m, 1H), 7.67 (brs, 1H), 7.50 (t, *J* = 7.2 Hz, 2H), 7.46 (d, *J* = 7.2 Hz, 1H), 7.33-7.29 (m, 1H), 3.58 (q, *J=* 6.0 Hz, 2H), 2.57 (t, *J=* 6.0 Hz, 2H), 2.33 (s, 6H).

### Example 206. 2-(3,4-Difluorophenyl)-N-(2-(4-methylpiperazin-1-yl)ethyl)-5-phenyloxazole-4-carboxamide (232)

This compound was obtained in 81% yield as a white solid with a typical procedure of hydrolysis and amide coupling reaction, following the same procedure described for the synthesis of compound **114.** ¹H NMR (600 MHz, MeOD) *δ* 8.27 (dd, *J=* 1.8 Hz and J=8.4 Hz, 2H), 8.04-8.02 (m, 1H), 7.96-7.94 (m, 1H), 7.51-7.44 (m, 4H), 3.55 (t, *J* = 6.6 Hz, 2H), 2.64 (t, *J=* 6.6 Hz, 2H), 2.60 (brs, 8H), 2.29 (s, 3H).

### Example 207. 2-(3,4-Difluorophenyl)-N-(2-(dimethylamino)ethyl)-5-(2-nitrophenyl)oxazole-4-carboxamide (233)

This compound was obtained in 52% yield as a white solid with a typical procedure of hydrolysis and amide coupling reaction, following the same procedure described for the synthesis of compound **114.** ¹H NMR (600 MHz, CDCl₃) *δ* 8.12 (d, *J=* 8.4 Hz, 1H), 7.94 (d, *J* = 7.8 Hz, 1H), 7.91-7.89 (m, 1H), 7.82-7.80 (m, 1H), 7.74 (td, *J=* 7.8 Hz and *J =* 0.6 Hz, 1H), 7.65 *(td, J=* 7.8 Hz and *J* = 0.6 Hz, 1H), 7.52 (brs, 1H), 7.32-7.29 (m, 1H), 3.50 (q, *J =* 6.0 Hz, 2H), 2.53 (t, *J=* 6.0 Hz, 2H), 2.31 (s, 6H).

### Example 208. 2-(3,4-Difluorophenyl)-N-(2-(4-methylpiperazin-1-yl)ethyl)-5-(2-nitrophenyl)oxazole-4-carboxamide (234)

This compound was obtained in 89% yield as a white solid with a typical procedure of hydrolysis and amide coupling reaction, following the same procedure described for the synthesis of compound **114.** ¹H NMR (600 MHz, MeOD) *δ* 8.16 (dd, *J* = 0.6 Hz and *J* = 7.8 Hz, 1H), 7.99-7.96 (m, 1H), 7.90-7.87 (m, 2H), 7.81 (td, *J* = 7.2 Hz and *J* = 1.2 Hz, 1H), 7.75 (td, *J=* 7.2 Hz and *J* = 1.2 Hz, 1H), 7.48-7.44 (m, 1H), 3.46 (t, *J=* 6.6 Hz, 2H), 2.59 (t, *J=* 6.6 Hz, 2H), 2.55 (brs, 8H), 2.28 (s, 3H).

### Example 209. 2-(3,5-Difluorophenyl)-N-(2-(dimethylamino)ethyl)-5-phenyloxazole-4-carboxamide (235)

This compound was obtained in 41% yield as a white solid with a typical procedure of hydrolysis and amide coupling reaction, following the same procedure described for the synthesis of compound **114.** ¹H NMR (600 MHz, CDCl₃) *δ* 8.37 *(d, J=* 7.8 Hz, 2H), 7.67-7.64 (m, 3H), 7.52-7.44 (m, 3H), 6.98-6.95 (m, 1H), 3.58 (q, *J* = 6.0 Hz, 2H), 2.58 (t, *J=* 6.0 Hz, 2H), 2.33 (s, 6H).

### Example 210. 2-(3,5-Difluorophenyl)-N-(2-(4-methylpiperazin-1-yl)ethyl)-5-phenyloxazole-4-carboxamide (236)

This compound was obtained in quantitative yield as a white solid with a typical procedure of hydrolysis and amide coupling reaction, following the same procedure described for the synthesis of compound **114.** ¹H NMR (600 MHz, MeOD) *δ* 8.29 (dd, *J=* 1.2 Hz and *J* = 8.4 Hz, 1H), 7.68-7.62 (m, 2H), 7.52-7.48 (m, 3H), 7.17-7.11 (m, 1H), 3.56 *(t, J=* 6.6 Hz, 2H), 2.65 (t, *J=* 6.6 Hz, 2H), 2.55 (s, 3H), 2.30 (s, 3H).

### Example 211. 2-(3,5-Difluorophenyl)-N-(2-(dimethylamino)ethyl)-5-(2-nitrophenyl)oxazole-4-carboxamide (237)

This compound was obtained in 60% yield as a white solid with a typical procedure of hydrolysis and amide coupling reaction, following the same procedure described for the synthesis of compound **114.** ¹H NMR (600 MHz, MeOD) *δ* 8.18 (dd, *J* = 1.2 Hz and *J* = 8.4 Hz, 1H), 7.90 (dd, *J* = 1.2 Hz and *J* = 7.2 Hz, 1H), 7.83 (td, *J* = 7.8 Hz and *J* = 1.2 Hz, 1H), 7.76 (td, *J=* 7.8 Hz and *J* = 1.2 Hz, 1H), 7.68-7.66 (m, 2H), 7.21-7.17 (m, 1H), 3.46 (t, *J=* 6.6 Hz, 2H), 2.55 (t, *J=* 6.6 Hz, 2H), 2.30 (s, 6H).

### Example 212. 2-(3,5-Difluorophenyl)-N-(2-(4-methylpiperazin-1-yl)ethyl)-5-(2-nitrophenyl)oxazole-4-carboxamide (238)

This compound was obtained in quantitative yield as a white solid with a typical procedure of hydrolysis and amide coupling reaction, following the same procedure described for the synthesis of compound **114.** ¹H NMR (600 MHz, MeOD) *δ* 8.17 (dd, *J* = 1.2 Hz and *J* = 8.4 Hz, 1H), 7.88 (dd, *J* = 1.8 Hz and *J* = 7.8 Hz, 1H), 7.82 (td, *J* = 7.2 Hz and *J* = 1.2 Hz, 1H), 7.75 (td, *J=* 7.8 Hz and *J* = 1.8 Hz, 1H), 7.66-7.64 (m, 2H), 7.23-7.19 (m, 1H), 3.46 (t, *J* = 6.6 Hz, 2H), 2.59 (t, *J* = 6.6 Hz, 2H), 2.54 (brs, 8H), 2.82 (s, 3H).

### Example 213. tert-butyl 4-(2-(2-(3,5-difluorophenyl)-5-(2-nitrophenyl)oxazole-4-carboxamido)ethyl)piperazine-1-carboxylate (239)

This compound was obtained in quantitative yield as a white solid with a typical procedure of hydrolysis and amide coupling reaction, following the same procedure described for the synthesis of compound **114.** ¹H NMR (600 MHz, CDCl₃) *δ* 8.12 (dd, *J* = 0.6 Hz and *J* = 7.8 Hz, 1H), 7.88 (dd, *J* = 1.2 Hz and *J* = 8.4 Hz, 1H), 7.76-7.70 (m, 1H), 7.68-7.63 (m, 1H), 7.58-7.50 (m, 3H), 6.99-6.93 (m, 1H), 3.53-3.45 (m, 1H), 2.59 (t, *J* = 6.6 Hz, 2H), 2.45 (brs, 4H), 1.46 (brs, 9H).

### Example 214. 2-(3-Chlorophenyl)-N-(2-(dimethylamino)ethyl)-5-phenyloxazole-4-carboxamide (240)

This compound was obtained in 26% yield as a white solid with a typical procedure of hydrolysis and amide coupling reaction, following the same procedure described for the synthesis of compound **114.** ¹H NMR (600 MHz, CDCl₃) *δ* 8.38 (d, *J* = 7.2 Hz, 2H), 8.11 (s, 1H), 8.02 (d, *J=* 7.2 Hz, 1H), 7.68 (brs, 1H), 7.51-7.43 (m, 5H), 3.59 (q, *J* = 6.0 Hz, 2H), 2.58 (t, *J* = 6.0 Hz, 2H), 2.33 (s, 6H).

### Example 215. 2-(3-Chlorophenyl)-N-(2-(4-methylpiperazin-1-yl)ethyl)-5-phenyloxazole-4-carboxamide (241)

This compound was obtained in quantitative yield as a white solid with a typical procedure of hydrolysis and amide coupling reaction, following the same procedure described for the synthesis of compound **114.** ¹H NMR (600 MHz, MeOD) *δ* 8.27-8.25 (m, 2H), 8.08-8.07 (m, 1H), 8.01-7.99 (m, 1H), 7.54-7.44 (m, 5H), 3.53 (t, *J* = 6.6 Hz, 2H), 2.64 (t, *J* = 6.6 Hz, 2H), 2.54 (brs, 8H), 2.29 (s, 3H).

### Example 216. 2-(3-Chlorophenyl)-N-(2-(dimethylamino)ethyl)-5-(2-nitrophenyl)oxazole-4-carboxamide (242)

This compound was obtained in 48% yield as a white solid with a typical procedure of hydrolysis and amide coupling reaction, following the same procedure described for the synthesis of compound **114.** ¹H NMR (600 MHz, MeOD) *δ* 8.15 (dd, *J* = 1.2 Hz and *J* = 8.4 Hz, 1H), 8.05 (t, *J* = 1.8 Hz, 1H), 7.95 (td, *J* = 7.8 Hz and *J* = 1.2 Hz, 1H), 7.88 (dd, *J* = 1.8 Hz and *J* = 7.8 Hz, 1H), 7.80 (td, *J=* 7.8 Hz and *J* = 1.8 Hz, 1H), 7.73 (td, *J* = 7.8 Hz and *J* =1.8 Hz, 1H), 7.54-7.53 (m, 1H), 7.50 (t, *J* = 7.8 Hz, 1H), 3.45 (t, *J* = 6.6 Hz, 2H), 2.54 (t, *J* = 6.6 Hz, 2H), 2.29 (s, 6H).

### Example 217. 2-(3-chlorophenyl)-N-(2-(4-methylpiperazin-1-yl)ethyl)-5-(2-nitrophenyl)oxazole-4-carboxamide (243)

This compound was obtained in 77% yield as a white solid with a typical procedure of hydrolysis and amide coupling reaction, following the same procedure described for the synthesis of compound **114.** ¹H NMR (600 MHz, MeOD) *δ* 8.16 (dd, *J* = 1.2 Hz and *J* = 8.4 Hz, 1H), 8.08 (t, *J=* 1.8 Hz, 1H), 7.98 (td, *J=* 8.4 Hz and *J =* 1.8 Hz, 1H), 7.89 (dd, *J* = 1.8 Hz and *J* = 7.8 Hz, 1H), 7.81 (td, *J* = 7.8 Hz and *J =* 1.2 Hz, 1H), 7.75 (td, *J =* 7.2 Hz and *J* = 1.2 Hz, 1H), 7.57-7.56 (m, 1H), 7.53 (t, *J* = 7.8 Hz, 1H), 3.46 (t, *J=* 6.6 Hz, 2H), 2.59 (t, *J=* 6.6 Hz, 2H), 2.56 (brs, 8H), 2.29 (s, 3H).

### Example 218. 2-(4-Chlorophenyl)-N-(2-(dimethylamino)ethyl)-5-phenyloxazole-4-carboxamide (244)

This compound was obtained in 38% yield as a white solid with a typical procedure of drolysis and amide coupling reaction, following the same procedure described for the synthesis of compound **114.** ¹H NMR (600 MHz, CDCl₃) *δ* 8.37 *(d, J=* 7.2 Hz, 2H), 8.07 (d, *J* = 8.4 Hz, 2H), 7.69 (brs, 1H), 7.51-7.43 (m, 5H), 3.59 (q, *J=* 6.0 Hz, 2H), 2.58 (t, *J* = 6.0 Hz, 2H), 2.33 (s, 6H).

### Example 219. 2-(4-Chlorophenyl)-N-(2-(4-methylpiperazin-1-yl)ethyl)-5-phenyloxazole-4-carboxamide (245)

This compound was obtained in 75% yield as a white solid with a typical procedure of hydrolysis and amide coupling reaction, following the same procedure described for the synthesis of compound **114.** ¹H NMR (600 MHz, MeOD) *δ* 8.30 (dd, *J* = 1.2 Hz and *J* = 8.4 Hz, 2H), 8.16 (d, *J* = 9.0 Hz, 2H), 7.60 (d, *J* = 9.0 Hz, 2H), 7.54-7.48 (m, 3H), 3.58 (t, *J=* 6.6 Hz, 2H), 2.67 (t, *J* = 6.6 Hz, 2H), 2.56 (brs, 8H), 2.32 (s, 3H).

### Example 220. 2-(4-Chlorophenyl)-N-(2-(dimethylamino)ethyl)-5-(2-nitrophenyl)oxazole-4-carboxamide (246)

This compound was obtained in quantitative yield as a white solid with a typical procedure of hydrolysis and amide coupling reaction, following the same procedure described for the synthesis of compound **114.** ¹H NMR (600 MHz, MeOD) *δ* 8.17 (dd, *J=* 1.2 Hz and *J* = 7.8 Hz, 1H), 8.06 (d, *J=* 9.0 Hz, 2H), 7.90 (dd, *J=* 1.2 Hz and *J* = 7.8 Hz, 1H), 7.82 (td, *J* = 7.8 Hz and *J =* 1.2 Hz, 1H), 7.75 (td, *J* = 7.8 Hz and *J* = 1.2 Hz, 1H), 7.56 (d, *J* = 9.0 Hz, 2H), 3.46 (t, *J=* 6.6 Hz, 2H), 2.55 (t, *J* = 6.6 Hz, 2H), 2.30 (s, 6H).

### Example 221. 2-(4-Chlorophenyl)-N-(2-(4-methylpiperazin-1-yl)ethyl)-5-(2-nitrophenyl)oxazole-4-carboxamide (247)

This compound was obtained in 86% yield as a white solid with a typical procedure of hydrolysis and amide coupling reaction, following the same procedure described for the synthesis of compound **114.** ¹H NMR (600 MHz, MeOD) *δ* 8.16 (d, *J=* 8.4 Hz, 1H), 8.04 (d, *J* = 8.4 Hz, 2H), 7.89 (d, *J* = 7.2 Hz, 1H), 7.82 (td, *J* = 7.2 Hz and *J* = 1.2 Hz, 1H), 7.75 (td, *J* = 7.2 Hz and *J =* 1.2 Hz, 1H), 7.56 (d, *J* = 8.4 Hz, 2H), 3.48 (t, *J* = 6.6 Hz, 2H), 2.60 (t, *J* = 6.6 Hz, 2H), 2.54 (brs, 8H), 2.29 (s, 3H).

### Example 222. (2-(4-Chlorophenyl)-5-(2-nitrophenyl)oxazol-4-yl)(4-(2-(dimethylamino)ethyl)piperazin-1-yl)methanone (248)

This compound was obtained in 38% yield as a white solid with a typical procedure of hydrolysis and amide coupling reaction, following the same procedure described for the synthesis of compound **114.** ¹H NMR (600 MHz, CDCl₃) *δ* 8.07 (d, *J=* 7.2 Hz, 1H), 7.98 (d, *J* = 8.4 Hz, 2H), 7.87 (d, *J=* 7.2 Hz, 1H), 7.72 (t, *J=* 7.2 Hz, 1H), 7.63 (t, *J=* 7.2 Hz, 1H), 7.47 (d, *J=* 8.4 Hz, 2H), 3.88 (brs, 2H), 3.75 (brs, 2H), 2.60-2.51 (m, 8H), 2.41 (s, 6H).

### Example 223. 2-(4-Cyanophenyl)-N-(2-(dimethylamino)ethyl)-5-phenyloxazole-4-carboxamide (249)

This compound was obtained in quantitative yield as a white solid with a typical procedure of hydrolysis and amide coupling reaction, following the same procedure described for the synthesis of compound **114.** ¹H NMR (600 MHz, CDCl₃) *δ* 8.37 (d, *J* = 7.2 Hz, 2H), 8.24 (d, *J=* 8.4 Hz, 2H), 7.82 (d, *J=* 8.4 Hz, 2H), 7.69 (brs, 1H), 7.52-7.49 (m, 3H), 3.59 (q, *J* = 6.0 Hz, 2H), 2.58 (t, *J* = 6.0 Hz, 2H), 2.33 (s, 6H).

### Example 224. 2-(4-Cyanophenyl)-N-(2-(4-methylpiperazin-1-yl)ethyl)-5-phenyloxazole-4-carboxamide (250)

This compound was obtained in 22% yield as a white solid with a typical procedure of hydrolysis and amide coupling reaction, following the same procedure described for the synthesis of compound **114.** ¹H NMR (600 MHz, CDCl₃) *δ* 8.38-8.36 (m, 2H), 8.23 *(d, J=* 9.0 Hz, 2H), 7.83 (d, *J=* 9.0 Hz, 2H), 7.77 (brt, *J* = 4.8 Hz, 1H), 7.52-7.45 (m, 3H), 3.60 (q, *J* = 6.0 Hz, 2H), 2.66 (t, *J=* 6.0 Hz, 2H), 2.64-2.45 (brs, 8H), 2.33 (s, 3H).

### Example 225. 2-(4-Cyanophenyl)-N-(2-(dimethylamino)ethyl)-5-(2-nitrophenyl)oxazole-4-carboxamide (251)

This compound was obtained in 50% yield as a white solid with a typical procedure of hydrolysis and amide coupling reaction, following the same procedure described for the synthesis of compound **114.** ¹H NMR (600 MHz, CDCl₃) *δ* 8.17 (d, *J=* 8.4 Hz, 2H), 8.15 (dd, *J* = 1.2 Hz and *J* = 7.8 Hz, 1H), 7.94 (dd, *J* = 1.2 Hz and *J* = 7.8 Hz, 1H), 7.80 (d, *J* = 8.4 Hz, 2H), 7.75 (td, *J* = 7.8 Hz and *J* = 1.2 Hz, 1H), 7.67 (td, *J* = 7.8 Hz and *J* = 1.2 Hz, 1H), 7.54 (brt, *J* = 4.8 Hz, 1H), 3.50 (q, *J=* 6.0 Hz, 2H), 2.53 (t, *J=* 6.0 Hz, 2H), 2.31 (s, 6H).

### Example 226. 2-(4-Cyanophenyl)-N-(2-(4-methylpiperazin-1-yl)ethyl)-5-(2-nitrophenyl)oxazole-4-carboxamide (252)

This compound was obtained in 68% yield as a white solid with a typical procedure of hydrolysis and amide coupling reaction, following the same procedure described for the synthesis of compound **114.** ¹H NMR (600 MHz, CDCl₃) *δ* 8.17 (d, *J=* 9.0 Hz, 2H), 8.14 (dd, *J* = 1.2 Hz and *J =* 7.8 Hz, 1H), 7.94 (dd, *J=* 1.2 Hz and *J =* 7.8 Hz, 1H), 7.81 (d, *J=* 9.0 Hz, 2H), 7.75 (td, *J* = 7.8 Hz and *J* = 1.2 Hz, 1H), 7.68 (td, *J* = 7.8 Hz and *J* = 1.2 Hz, 1H), 7.60 (brt, *J* = 4.8 Hz, 1H), 3.52 (q, *J* = 6.0 Hz, 2H), 2.62 (t, *J=* 6.0 Hz, 2H), 2.61-2.45 (brs, 8H), 2.34 (s, 3H).

### Example 227. 2-([1,1'-Biphenyl]-4-yl)-N-(2-(dimethylamino)ethyl)-5-phenyloxazole-4-carboxamide (253)

This compound was obtained in 12% yield as a white solid with a typical procedure of hydrolysis and amide coupling reaction, following the same procedure described for the synthesis of compound **114.** ¹H NMR (600 MHz, CDCl₃) *δ* 8.41 (d, *J=* 7.8 Hz, 2H), 8.20 (d, *J* = 8.4 Hz, 2H), 7.76 (d, *J=* 8.4 Hz, 2H), 7.67 (d, *J=* 7.2 Hz, 2H), 7.52-7.49 (m, 4H), 7.45-7.41 (m, 2H), 3.61 (q, *J=* 6.0 Hz, 2H), 2.60 (t, *J=* 6.0 Hz, 2H), 2.35 (s, 6H).

### Example 228. 2-((1,1'-Biphenyl]-4-yl)-N-(2-(4-methylpiperazin-1-yl)ethyl)-5-phenyloxazole-4-carboxamide (254)

This compound was obtained in 64% yield as a white solid with a typical procedure of hydrolysis and amide coupling reaction, following the same procedure described for the synthesis of compound **114.** ¹H NMR (600 MHz, MeOD) *δ* 8.24 (dd, *J* = 1.2 Hz and *J* = 8.4 Hz, 2H), 8.06 (d, *J* = 8.4 Hz, 2H), 7.69 (d, *J* = 8.4 Hz, 2H), 7.62 (dd, *J* = 1.2 Hz and *J* = 8.4 Hz, 2H), 7.46-7.40 (m, 5H), 7.37-7.34 (m, 1H), 3.49 (t, *J=* 6.6 Hz, 2H), 2.59 (t, *J=* 6.6 Hz, 2H), 2.51(brs, 8H), 2.27 (s, 3H).

### Example 229. 2-([1,1'-Biphenyl]-4-yl)-N-(2-(dimethylamino)ethyl)-5-(2-nitrophenyl)oxazole-4-carboxamide (255)

This compound was obtained in quantitative yield as a white solid with a typical procedure of hydrolysis and amide coupling reaction, following the same procedure described for the synthesis of compound **114.** ¹H NMR (600 MHz, MeOD) *δ* 8.15 (dd, *J=* 1.2 Hz and *J* = 8.4 Hz, 1H), 8.11 (d, *J* = 8.4 Hz, 2H), 7.89 (dd, *J=* 1.2 Hz and *J* = 7.8 Hz, 1H), 7.80 (td, *J* = 7.8 Hz and *J* = 1.2 Hz, 1H), 7.77 (d, *J* = 8.4 Hz, 2H), 7.73 (td, *J* = 7.8 Hz and *J =* 1.8 Hz, 1H), 7.67-7.66 (m, 2H), 7.46 (t, *J=* 7.8 Hz, 2H), 7.39-7.36 (m, 1H), 3.46 (t, *J=* 6.6 Hz, 2H), 2.55 (t, *J* = 6.6 Hz, 2H), 2.30 (s, 3H).

### Example 230. 2-([1,1'-Biphenyl]-4-yl)-N-(2-(4-methylpiperazin-1-yl)ethyl)-5-(2-nitrophenyl)oxazole-4-carboxamide (256)

This compound was obtained in 46% yield as a white solid with a typical procedure of hydrolysis and amide coupling reaction, following the same procedure described for the synthesis of compound **114.** ¹H NMR (600 MHz, MeOD) *δ* 8.13 (d, *J=* 7.8 Hz, 1H), 8.10-8.07 (m, 2H), 7.87-7.86 (m, 1H), 7.80-7.70 (m, 4H), 7.66-7.65 (m, 2H), 7.46-7.43 (m, 2H), 7.38-7.35 (m, 1H), 3.46-3.44 (m, 2H), 2.59-2.56 (m, 2H), 2.51-2.30 (brs, 8H), 2.27 (s, 3H).

### Example 231. N-(2-(Dimethylamino)ethyl)-2-(naphthalen-2-yl)-5-phenyloxazole-4-carboxamide (257)

This compound was obtained in 44% yield as a white solid with a typical procedure of hydrolysis and amide coupling reaction, following the same procedure described for the synthesis of compound **114.** ¹H NMR (600 MHz, CDCL₃) *δ* 8.62 (s, 1H), 8.45 (d, *J=* 7.2 Hz, 2H), 8.21 (dd, *J =* 1.8 Hz and *J =* 9.0 Hz, 1H), 8.00-7.99 (m, 1H), 7.98 (d, *J* = 9.0 Hz, 1H), 7.92-7.90 (m, 1H), 7.76 (brt, *J=* 4.8 H, 1H), 7.60-7.57 (m, 2H), 7.52 (t, *J=* 7.2 Hz, 2H), 7.47-7.44 (m, 1H), 3.61 (q, *J* = 6.0 Hz, 2H), 2.60 (t, *J=* 6.0 Hz, 2H), 2.35 (s, 6H).

### Example 232. N-(2-(4-Methylpiperazin-1-yl)ethyl)-2-(naphthalen-2-yl)-5-phenyloxazole-4-carboxamide (258)

This compound was obtained in quantitative yield with a typical procedure of hydrolysis and amide coupling reaction, following the same procedure described for the synthesis of compound **114.** ¹H NMR (600 MHz, MeOD) *δ* 8.61 (s, 1H), 8.32-8.31 (m, 2H), 8.17 (dd, *J* = 1.8 Hz and *J* = 8.4 Hz, 1H), 8.01-7.98 (m, 2H), 7.92-7.91 (m 1H), 7.59-7.56 (m, 2H), 7.52-7.50 (m, 2H), 7.50-7.46 (m, 1H), 3.56 (t, *J* = 6.6 Hz, 2H), 2.66 (t, *J=* 6.6 Hz, 2H), 2.56 (brs, 8H), 2.30 (s, 3H).

### Example 233. N-(2-(Dimethylamino)ethyl)-2-(naphthalen-2-yl)-5-(2-nitrophenyl)oxazole-4-carboxamide (259)

This compound was obtained in 55% yield with a typical procedure of hydrolysis and amide coupling reaction, following the same procedure described for the synthesis of compound **114.** ¹H NMR (600 MHz, CDCL₃) *δ* 8.55 (s, 1H), 8.15-8.13 (m, 2H), 7.99 (dd, *J=* 1.2 Hz and *J* = 7.8 Hz, 1H), 7.97-7.95 (m, 2H), 7.90 (d, *J* = 7.8 Hz, 1H), 7.75 (td, *J* = 7.8 Hz and *J* = 1.2 Hz, 1H), 7.65 (td, *J=* 7.8 Hz and *J =* 1.2 Hz, 1H), 7.60-7.55 (m, 3H), 3.53 (q, *J=* 6.0 Hz, 2H), 2.55 (t, *J* = 6.0 Hz, 2H), 2.33 (s, 6H).

### Example 234. N-(2-(4-Methylpiperazin-1-yl)ethyl)-2-(naphthalen-2-yl)-5-(2-nitrophenyl)oxazole-4-carboxamide (260)

This compound was obtained in 29% yield with a typical procedure of hydrolysis and amide coupling reaction, following the same procedure described for the synthesis of compound **114.** ¹H NMR (600 MHz, CDCl₃) *δ* 8.54 (s, 1H), 8.15-8.13 (m, 2H), 8.00 (dd, *J* = 1.2 Hz and *J* = 7.8 Hz, 1H), 7.98-7.95 (m, 2H), 7.91-7.90 (m, 1H), 7.74 (td, *J=* 7.8 Hz and *J* = 1.2 Hz, 1H), 7.69 (t, *J* = 4.8 Hz, 1H), 7.66 (td, *J* = 7.8 Hz and *J* = 1.2 Hz, 1H), 7.60-7.55 (m, 2H), 3.54 (q, *J* = 6.0 Hz, 2H), 2.64 (t, *J=* 6.0 Hz, 2H), 2.63-2.45 (brs, 8H), 2.35 (s, 3H).

### Example 235. N-(2-(Dimethylamino)ethyl)-2-(4-(dimethylamino)phenyl)-5-phenyloxazole-4-carboxamide (261)

This compound was obtained in 59% yield with a typical procedure of hydrolysis and amide coupling reaction, following the same procedure described for the synthesis of compound **114.** ¹H NMR (600 MHz, MeOD) *δ* 8.26 (d, *J=* 7.2 Hz, 2H), 7.95 (d, *J* = 9.0 Hz, 2H), 7.49 (t, *J=* 7.2 Hz, 2H), 7.44 (d, *J=* 7.2 Hz, 1H), 6.83 (d, *J=* 9.0 Hz, 2H), 3.57 (t, *J=* 6.6 Hz, 2H), 3.05 (s, 6H), 2.63 (t, *J=* 6.6 Hz, 2H), 2.36 (s, 6H).

### Example 236. 2-(4-(Dimethylamino)phenyl)-N-(2-(4-methylpiperazin-1-yl)ethyl)-5-phenyloxazole-4-carboxamide (262)

This compound was obtained in 37% yield with a typical procedure of hydrolysis and amide coupling reaction, following the same procedure described for the synthesis of compound **114.** ¹H NMR (600 MHz, MeOD) *δ* 8.25 (d, *J=* 7.2 Hz, 2H), 7.91 (d, *J* = 9.0 Hz, 2H), 7.48 (t, *J=* 7.2 Hz, 2H), 7.43 (d, *J=* 7.2 Hz, 1H), 6.79 (d, *J* = 9.0 Hz, 2H), 3.55 (t, *J=* 6.6 Hz, 2H), 3.03 (s, 6H), 2.65 (t, *J=* 6.6 Hz, 2H), 2.76-2.47 (m, 8H), 2.30 (s, 3H).

### Example 237. N-(2-(Dimethylamino)ethyl)-2-(4-(dimethylamino)phenyl)-5-(2-nitrophenyl)oxazole-4-carboxamide (263)

This compound was obtained in 72% yield with a typical procedure of hydrolysis and amide coupling reaction, following the same procedure described for the synthesis of compound **114.** ¹H NMR (600 MHz, MeOD) *δ* 8.13 (dd, *J* = 1.2 Hz and *J* = 8.4 Hz, 1H), 7.92-7.89 (m, 3H), 7.80 *(td, J=* 7.2 Hz and *J* = 1.2 Hz, 1H), 7.72-7.69 (m, 1H), 6.83 (d, *J* = 9.0 Hz, 2H), 3.48 (t, *J=* 6.6 Hz, 2H), 3.06 (s, 6H), 2.57 (t, *J=* 6.6 Hz, 2H), 2.32 (s, 6H).

### Example 238. 2-(4-(Dimethylamino)phenyl)-N-(2-(4-methylpiperazin-1-yl)ethyl)-5-(2-nitrophenyl)oxazole-4-carboxamide (264)

This compound was obtained in 67% yield with a typical procedure of hydrolysis and amide coupling reaction, following the same procedure described for the synthesis of compound **114.** ¹H NMR (600 MHz, MeOD) *δ* 8.13 (dd, *J* = 1.2 Hz and *J* = 7.8 Hz, 1H), 7.91-7,90 (m, 3H), 7.81 (td, *J=* 7.8 Hz and *J =* 1.2 Hz, 1H), 7.72-7.69 (m, 1H), 6.84 (d, *J=* 9.0 Hz, 2H), 3.48 (t, *J=* 6.6 Hz, 2H), 3,07 (s, 6H), 2,62 (t, *J=* 6.6 Hz, 2H), 2.55 (brs, 8H), 2.31 (s, 3H).

### Example 239. 2-(4-(tert-Butyl)phenyl)-N-(2-(dimethylamino)ethyl)-5-phenyloxazole-4-carboxamide (265)

This compound was obtained in 49% yield with a typical procedure of hydrolysis and amide coupling reaction, following the same procedure described for the synthesis of compound **114.** ¹H NMR (600 MHz, MeOD) *δ* 8.25 (d, *J* = 8.4 Hz, 2H), 8.03 (d, *J* = 9.0 Hz, 2H), 7.56 (t, *J=* 8.4 Hz, 2H), 7.48 (t, *J=* 7.2 Hz, 2H), 7.45 (d, *J=* 7.2 Hz, 1H), 3.55 (t, *J=* 6.6 Hz, 2H), 2.60 (t, *J* = 6.6 Hz, 2H), 2.34 (s, 6H), 1.36 (s, 9H).

### Example 240. 2-(4-(tert-butyl)phenyl)-N-(2-(4-methylpiperazin-1-yl)ethyl)-5-phenyloxazole-4-carboxamide (266)

This compound was obtained in quantitative yield with a typical procedure of hydrolysis and amide coupling reaction, following the same procedure described for the synthesis of compound **114.** ¹H NMR (600 MHz, MeOD) *δ* 8.29 (d, *J=* 8.4 Hz, 2H), 8.07 (d, *J* = 9.0 Hz, 2H), 7.65-7.57 (m, 2H), 7.55-7.43 (m, 3H), 3.60 (t, *J=* 6.6 Hz, 2H), 2.66 (t, *J=* 6.6 Hz, 2H), 2.93-2.39 (m, 8H), 2.31 (s, 3H), 1.39 (s, 9H).

### Example 241. 2-(4-(tert-butyl)phenyl)-N-(2-(diethylamino)ethyl)-5-phenyloxazole-4-carboxamide (267)

This compound was obtained in 60% yield with a typical procedure of hydrolysis and amide coupling reaction, following the same procedure described for the synthesis of compound **114.** ¹H NMR (600 MHz, MeOD) *δ* 8.27 (d, *J=* 8.4 Hz, 2H), 7.98 (d, *J* = 6.4 Hz, 2H), 7.53 (d, *J=* 8.4 Hz, 2H), 7.47 (t, *J* = 8.4 Hz, 2H), 7.46-7.41 (m, 1H), 3.51 (t, *J=* 6.6 Hz, 2H), 2.75 (t, *J* = 6.6 Hz, 2H), 2.67 (dd, *J* = 7.8 Hz and *J =* 14.1 Hz, 4H), 1.35 (s, 9H), 1.11 (t, *J=* 7.2 Hz, 6H).

### Example 242. 2-(4-(tert-Butyl)phenyl)-N-(2-(dimethylamino)ethyl)-5-(2-nitrophenyl)oxazole-4-carboxamide (268)

This compound was obtained in 76% yield with a typical procedure of hydrolysis and amide coupling reaction, following the same procedure described for the synthesis of compound **114.** ¹H NMR (600 MHz, MeOD) *δ* 8.18 (dd, *J* = 1.2 Hz and *J* = 7.8 Hz, 1H), 8.03 (d, *J* = 8.4 Hz, 2H), 7.75 (t, *J=* 7.8 Hz, 1H), 7.92 (dd, *J* = 1.8 Hz and J= 8.4 Hz, 1H), 7.84 (td, *J=* 8.4 Hz and *J* = 1.8 Hz, 1H), 7.77 (td, *J* = 8.4 Hz and *J* = 1.8 Hz, 1H), 7.62 (t, *J* = 8.4 Hz, 2H), 3.49 (t, *J=* 6.6 Hz, 2H), 2.58 (t, *J=* 6.6 Hz, 2H), 2.33 (s, 6H).

### Example 243. 2-(4-(tert-Butyl)phenyl)-N-(2-(4-methylpiperazin-1-yl)ethyl)-5-(2-nitrophenyl)oxazole-4-carboxamide (269)

This compound was obtained in 86% yield with a typical procedure of hydrolysis and amide coupling reaction, following the same procedure described for the synthesis of compound **114.** ¹H NMR (600 MHz, MeOD) *δ* 8.15 (d, *J=* 8.4 Hz, 4H), 8.01 (d, *J* = 8.4 Hz, 2H), 7.90 (t, *J=* 7.8 Hz, 1H), 7.82 (d, *J=* 7.8 Hz, 1H), 7.74 (t, *J=* 8.4 Hz, 1H), 7.59 (d, *J=* 8.4 Hz, 2H), 3.48 (t, *J=* 6.6 Hz, 2H), 2.61 (t, *J=* 6.6 Hz, 2H), 2.30 (s, 3H), 1.38 (s, 9H).

### Example 244. 2-(4-(tert-Butyl)phenyl)-N-(2-(diethylamino)ethyl)-5-(2-nitrophenyl)oxazole-4-carboxamide (270)

This compound was obtained in 70% yield with a typical procedure of hydrolysis and amide coupling reaction, following the same procedure described for the synthesis of compound **114.** ¹H NMR (600 MHz, MeOD) *δ* 8.18 (dd, *J* = 1.2 Hz and *J* = 7.8 Hz, 1H), 8.04 (d, *J=* 8.4 Hz, 2H), 7.93 (dd, *J=* 1.2 Hz and *J* =7.8 Hz, 1H), 7.84 (td, *J =* 7.8 Hz and *J* = 1.2 Hz, 1H), 7.76-7.74 (m, 1H), 7.62 (d, *J=* 8.4 Hz, 2H), 3.46 (t, *J* = 6.6 Hz, 2H), 2.74 (t, *J=* 6.6 Hz, 2H), 2.68 (q, *J=* 7.2 Hz, 4H), 1.40 (s, 9H), 1.12 (t, *J=* 7.2 Hz, 6H).

### Example 245. N-(2-(Dimethylamino)ethyl)-2-(4-(methylthio)phenyl)-5-phenyloxazole-4-carboxamide (271)

This compound was obtained in 58% yield with a typical procedure of hydrolysis and amide coupling reaction, following the same procedure described for the synthesis of compound **114.** ¹H NMR (600 MHz, MeOD) *δ* 8.28 (d, *J* = 8.4 Hz, 2H), 8.04 (d, *J* = 9.0 Hz, 2H), 7.54-7.49 (m, 3H), 7.39 (d, *J=* 8.4 Hz, 2H), 3.57 (t, *J=* 6.6 Hz, 2H), 2.63 (t, *J=* 6.6 Hz, 2H), 2.56 (s, 3H), 2.36 (s, 6H).

### Example 246. N-(2-(4-Methylpiperazin-1-yl)ethyl)-2-(4-(methylthio)phenyl)-5-phenyloxazole-4-carboxamide (272)

This compound was obtained in 69% yield with a typical procedure of hydrolysis and amide coupling reaction, following the same procedure described for the synthesis of compound **114.** ¹H NMR (600 MHz, MeOD) *δ* 8.23 (d, *J* = 8.4 Hz, 2H), 7.90 (d, *J* = 9.0 Hz, 2H), 7.49-7.40 (m, 3H), 7.29 (d, *J* = 9.0 Hz, 2H), 3.51 (t, *J=* 6.6 Hz, 2H), 2.61 (t, *J* = 6.6 Hz, 2H), 2.49 (s, 3H), 2.29 (s, 3H).

### Example 247. N-(2-(Dimethylamino)ethyl)-2-(4-(methylthio)phenyl)-5-(2-nitrophenyl)oxazole-4-carboxamide (273)

This compound was obtained in 35% yield with a typical procedure of hydrolysis and amide coupling reaction, following the same procedure described for the synthesis of compound **114.** ¹H NMR (600 MHz, MeOD) *δ* 8.16 (dd, *J=* 1.2 Hz and *J =* 7.8 Hz, 4H), 7.98 (d, *J* = 9.0 Hz, 2H), 7.90 (dd, *J* = 1.2 Hz and *J* = 9.0 Hz, 1H), 7.83 (td, *J* = 7.2 Hz and *J* = 1.2 Hz, 1H), 7.75 (td, *J* = 7.8 Hz and *J =* 1.2 Hz, 1H), 7.39 (d, *J=* 9.0 Hz, 2H), 3.48 (t, *J=* 6.6 Hz, 2H), 2.57 (t, *J* = 6.6 Hz, 2H), 2.56 (s, 3H), 2.32 (s, 6H).

### Example 248. N-(2-(4-Methylpiperazin-1-yl)ethyl)-2-(4-(methylthio)phenyl)-5-(2-nitrophenyl)oxazole-4-carboxamide (274)

This compound was obtained in 76% yield with a typical procedure of hydrolysis and amide coupling reaction, following the same procedure described for the synthesis of compound **114.** ¹H NMR (600 MHz, MeOD) *δ* 8.13 (dd, *J=* 1.2 Hz and *J =* 7.8 Hz, 1H), 7.95 (d, *J* = 9.0 Hz, 2H), 7.88 (dd, *J* = 1.2 Hz and *J* = 7.8 Hz, 1H), 7.80 (td, *J* = 7.8 Hz and *J =* 1.2 Hz, 1H), 7.73 (td, *J=* 7.8 Hz and *J =* 1.2 Hz, 1H), 7.36 (d, *J* = 8.4 Hz, 2H), 3.47 (t, *J* = 6.6 Hz, 2H), 2.59 (t, *J=* 6.6 Hz, 2H), 2.53 (s, 3H), 2.74-2.32 (m, 8H), 2.29 (s, 3H).

### Example 249. N-(2-(Dimethylamino)ethyl)-2-(4-nitrophenyl)-5-phenyloxazole-4-carboxamide (275)

This compound was obtained in quantitative yield with a typical procedure of hydrolysis and amide coupling reaction, following the same procedure described for the synthesis of compound **114.** ¹H NMR (600 MHz, DMSO-d₆) *δ* 8.37 (d, *J* = 7.8 Hz, 2H), 8.33 (d, *J* = 7.8 Hz, 2H), 8.23 (d, *J=* 7.2 Hz, 2H), 7.51-7.44 (m, 3H), 3.39 (t, *J=* 6.6 Hz, 2H), 2.45 (t, *J* = 6.6 Hz, 2H), 2.17 (s, 6H).

### Example 250. N-(2-(4-Methylpiperazin-1-yl)ethyl)-2-(4-nitrophenyl)-5-phenyloxazole-4-carboxamide (276)

This compound was obtained in quantitative yield with a typical procedure of hydrolysis and amide coupling reaction, following the same procedure described for the synthesis of compound **114.** ¹H NMR (600 MHz, MeOD) *δ* 8.31 (d, *J* = 9.0 Hz, 2H), 8.25 (dd, *J* = 1.8 Hz and J=7.8 Hz, 2H), 8.22 (d, *J* = 9.0 Hz, 2H), 7.48-7.44 (m, 3H), 3.52 (t, *J* = 6.6 Hz, 2H), 2,63 (t, *J* = 6.6 Hz, 2H), 2.60 (brs, 8H), 2.29 (s, 3H).

### Example 251. N-(2-(Dimethylamino)ethyl)-5-(2-nitrophenyl)-2-(4-nitrophenyl)oxazole-4-carboxamide (277)

This compound was obtained in quantitative yield with a typical procedure of hydrolysis and amide coupling reaction, following the same procedure described for the synthesis of compound **114.** ¹H NMR (600 MHz, DMSO-d₆) *δ* 8.41 (d, *J* = 8.4 Hz, 2H), 8.28 (d, *J=* 8.4 Hz, 2H), 8.18 (dd, *J* = 1.2 Hz and *J* = 8.4 Hz, 1H), 7.91 (td, *J* = 7.8 Hz and *J* = 1.2 Hz, 1H), 7.87 (dd, *J* = 1.2 Hz and *J* = 7.8 Hz, 1H), 7.80 (td, *J* = 7.8 Hz and *J* = 1.8 Hz, 1H), 3.27 (t, *J=* 6.6 Hz, 2H), 2.36 (t, *J=* 6.6 Hz, 2H), 2.13 (s, 6H).

### Example 252. N-(2-(4-Methylpiperazin-1-yl)ethyl)-5-(2-nitrophenyl)-2-(4-nitrophenyl)oxazole-4-carboxamide (278)

This compound was obtained in quantitative yield with a typical procedure of hydrolysis and amide coupling reaction, following the same procedure described for the synthesis of compound **114.** ¹H NMR (600 MHz, DMSO-d₆) *δ* 8.45 (d, *J=* 8.4 Hz, 2H), 8.29 (d, *J* = 8.4 Hz, 2H), 8.20 (dd, *J* = 1.2 Hz and *J* = 8.4 Hz, 1H), 7.96 (dd, *J* = 1.2 Hz and *J* =7.8 Hz, 1H), 7.90 (td, *J=* 7.8 Hz and *J =* 1.2 Hz, 1H), 7.82 (td, *J=* 7.8 Hz and *J* = 1.2 Hz, 1H), 3.29 (t, *J=* 6.6 Hz, 2H), 2.41 (t, *J=* 6.6 Hz, 2H), 2.39 (brs, 8H), 2.12 (s, 6H).

### Example 253. 2-(4-Cyclopropylphenyl)-N-(2-(dimethylamino)ethyl)-5-phenyloxazole-4-carboxamide (279)

This compound was obtained in quantitative yield with a typical procedure of hydrolysis and amide coupling reaction, following the same procedure described for the synthesis of compound **114.** ¹H NMR (600 MHz, MeOD) *δ* 8.23 (dd, *J* = 1.2 Hz and *J* = 8.4 Hz, 2H), 7.89 (d, *J = 7.8,* Hz, 2H), 7.45-7.39 (m, 3H), 7.14 (d, *J=* 7.8, Hz, 2H), 3.50 (t, *J=* 6.6 Hz, 2H), 2.60 (t, *J* = 6.6 Hz, 2H), 2.50 (brs, 8H), 2.26 (s, 3H), 1.93-1.89 (m, 1H), 1.03-1.00 (m, 2H), 0.74-0.71 (m, 2H).

### Example 254. 2-(4-Cyclopropylphenyl)-N-(2-(4-Methylpiperazin-1-yl)ethyl)-5-phenyloxazole-4-carboxamide (280)

This compound was obtained in quantitative yield with a typical procedure of hydrolysis and amide coupling reaction, following the same procedure described for the synthesis of compound **114.** ¹H NMR (600 MHz, MeOD) *δ* 8.24 (dd, *J=* 1.8 Hz and *J =* 9.0 Hz, 2H), 7.94 (d, *J=* 7.8 Hz, 2H), 7.47-7.42 (m, 3H), 7.17 (d, *J=* 7.8 Hz, 2H), 3.52 (t, *J=* 6.6 Hz, 2H), 2.58 (t, *J=* 6.6 Hz, 2H), 2.32 (s, 6H), 1.97-1.92 (m, 1H), 1.05-1.02 (m, 2H), 0.76-0.74 (m, 2H).

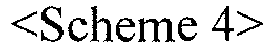

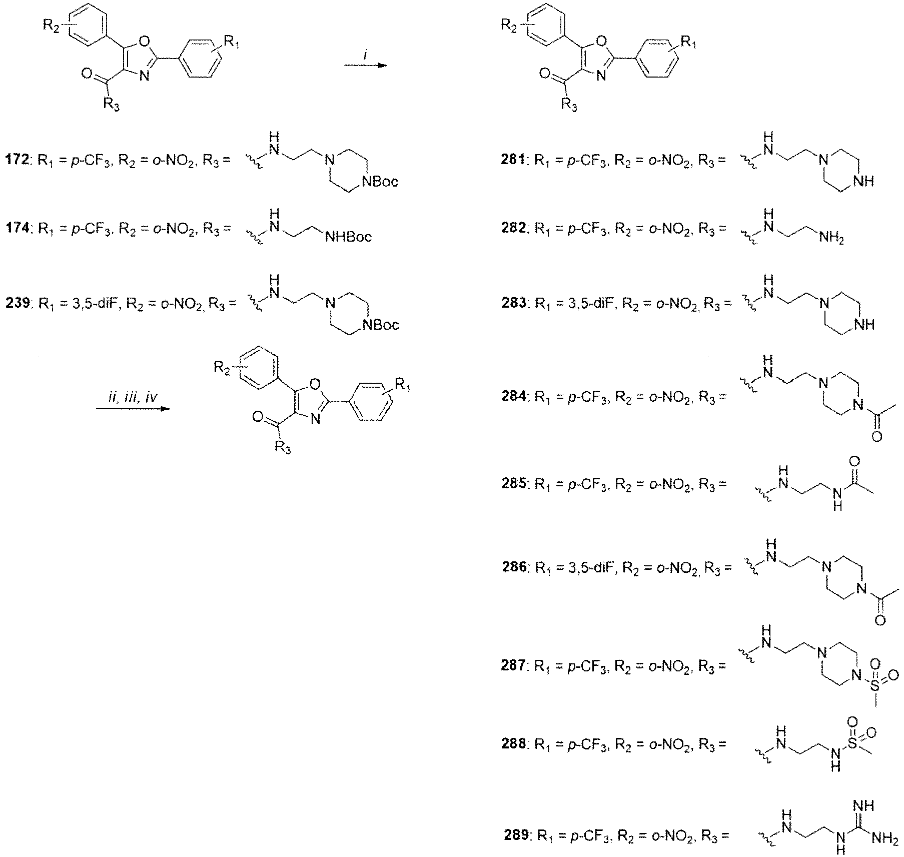

[*Reagents and conditions: i*) 25% TFA in anhydrous CH₂Cl₂, 0 °C to rt, 1 h-4 h, quantitative yield for compound **281,** 45% yield for compound **282,** quantitative yield for compound **283;** ii) acetyl chloride, DIPEA, anhydrous CH₂Cl₂, 0 °C to rt, 12 h, 53% yield for compound **284,** 49% yield for compound **285,** 41% yield for compound **286;** *iii*) methanesulfonyl chloride, DIPEA, anhydrous CH₂Cl₂, 0 °C to rt, 1 h, 45% yield for compound **287, 62%** yield for compound **288;** iv) 1-[*N,N*'-(Di-Boc)amidino]pyrazole, Et₃N, MeOH, rt, 20 h, 50% yield for compound **289.]**

### Example 255. Typical procedure of deprotection of Boc for the synthesis: 5-(2-nitrophenyl)-N-(2-(piperazin-1-yl)ethyl)-2-(4-(trifluoromethyl)phenyl)oxazole-4-carboxamide (281)

To a solution of *N-*Boc protected amine (217 mg, 0.37 mmol) in anhydrous CH₂Cl₂ (6 mL) was added trifluoroacetic acid (2 mL) at 0 °C. Then the reaction mixture was stirred at 25 °C for 4 h. The volatile components were evaporated and replaced by anhydrous toluene which was then evaporated to azeotrope excess trifluoroacetic acid. This operation was repeated three times to yield an oil which was dried *in vacuo.* The residue was purified by column chromatography on a silica gel (eluting with CH₂Cl₂ : MeOH = 20:1 to 10:1, v/v) to afford compound **281** as a yellow solid (230 mg, quantitative yield). ¹H NMR (600 MHz, CDCl₃) *δ* 8.15 (d, *J=* 8.2 Hz, 1H), 8.12 (d, *J=* 7.9 Hz, 1H), 7.88-7.87 (m, 1H), 7.76 (d, *J* = 8.8 Hz, 3H), 7.68-7.65 (m, 1H), 7.57 *(t, J=* 5.4 Hz, 1H), 3.58 (q*, J* = 5.6 Hz, 2H), 3.31 (s, 4H), 2.95 (s, 4H), 2.80 (s, 2H).

Compound **282** and **283** were prepared using a similar method as described for compound **281.**

### Example 256. N-(2-Aminoethyl)-5-(2-nitrophenyl)-2-(4-(trifluoromethyl)phenyl)oxazole-4-carboxamide (282)

This compound was obtained in 45% yield, following the same procedure described for the synthesis of compound **281** which is typical procedure deprotection of Boc reaction. ¹H NMR (600 MHz, CDCl₃) *δ* 8.18 (brs, 2H), 8.08 (d, *J=* 7.8 Hz, 1H), 8.06 (d, *J=* 8.4 Hz, 2H), 7.90 (s, 1H), 7.79 (d, *J* = 7.8 Hz, 1H), 7.72 (t, *J=* 7.8 Hz, 1H), 7.67 (d, *J=* 7.8 Hz, 2H), 7.63 (t, *J* = 7.8 Hz, 1H), 3.55 (brs, 2H), 3.08 (brs, 2H).

### Example 257. 2-(3,5-Difluorophenyl)-5-(2-nitrophenyl)-N-(2-(piperazin-1-yl)ethyl)oxazole-4-carboxamide (283)

This compound was obtained in quantitative yield, following the same procedure described for the synthesis of compound **281** which is typical procedure deprotection of Boc reaction. ¹H NMR (600 MHz, CDCl₃) *δ* 8.14 (d, *J=* 8.4 Hz, 1H), 7.83 (d, *J=* 7.8 Hz, 1H), 7.75 (t, *J* = 7.8 Hz, 1H), 7.67 (t, *J* = 7.2 Hz, 1H), 7.59-7.49 (m, 3H), 6.96 (t, *J* = 9.0 Hz, 1H), 3.57 (brs, 2H), 3.32 (brs, 3H), 3.01 (brs, 3H), 2.85 (brs, 2H).

### Example 258. N-(2-(4-acetylpiperazin-1-yl)ethyl)-5-(2-nitrophenyl)-2-(4-(trifluoromethyl)phenyl)oxazole-4-carboxamide (284)

To a solution of compound **281** (70 mg, 0.14 mmol) in anhydrous CH₂Cl₂ (5.0 mL) was added DIPEA (50 µL, 0.29 mmol) under an argon atmosphere. After acetyl chloride (20 µL, 0.29 mmol) was added slowly dropwise to the mixture at 0 °C, the reaction mixture was stirred at room temperature for 12 h. The volatile components were evaporated and the residue was purified by column chromatography in a silica gel (eluting with CH₂Cl₂ : MeOH = 20:1 to 10:1, v/v) to afford compound **284** as a yellow solid (41 mg, 53%). ¹H NMR (600 MHz, CDCL₃) *δ* 8.19-8.15 (m, 3H), 7.95 (d, *J=* 8.4 Hz, 1H), 7.80-7.76 (m, 3H), 7.70-7.68 (m, 1H), 7.57 (brs, 1H), 3.70 (brs, 2H), 3.57-3.53 (m, 4H), 2.65 (t, *J=* 6.0 Hz, 2H), 2.56-2.53 (m, 4H), 2.13 (s, 3H).

Compound **285** and **286** were prepared using a similar method as described for compound **284.**

### Example 259. N-(2-acetamidoethyl)-5-(2-nitrophenyl)-2-(4-(trifluoromethyl)phenyl)oxazole-4-carboxamide (285)

This compound was obtained in 49% yield, following the same procedure described for the synthesis of compound **284** which is typical procedure acetylation reaction. ¹H NMR (600 MHz, CDCL₃) *δ* 8.32 (d, *J* = 8.4 Hz, 2H), 8.21 (dd, *J* = 0.6 Hz and *J* = 8.4 Hz, 1H), 7.93 (dd, *J=* 1.2 Hz and *J =* 7.8 Hz, 1H), 7.89 (d, *J=* 8.4 Hz, 2H), 7.88-7.85 (m, 1H), 7.81-7.78 (m, 1H), 3.46 (t, *J=* 5.4 Hz, 2H), 3.39 (t, *J=* 6.0 Hz, 2H), 1.97(s, 3H).

### Example 260. N-(2-(4-Acetylpiperazin-1-yl)ethyl)-2-(3,5-difluorophenyl)-5-(2-nitrophenyl)oxazole-4-carboxamide (286)

This compound was obtained in 41% yield, following the same procedure described for the synthesis of compound **284** which is typical procedure acetylation reaction. ¹H NMR (600 MHz, CDCl₃) *δ* 8.18 (dd, *J=* 0.6 Hz and *J =* 7.8 Hz, 1H), 7.89 (dd, *J* = 1.2 Hz and *J* = 7.8 Hz, 1H), 7.76 (td, *J* = 7.2 Hz and *J =* 1.2 Hz, 1H), 7.68 (td, *J* = 8.4 Hz and *J* = 1.2 Hz, 1H), 7.61-7.54 (m, 2H), 7.51 (t, *J* = 5.4 Hz, 1H), 7.02-6.94 (m, 1H), 3.68 (t, *J* = 4.8 Hz, 2H), 3.57-3.49 (m, 4H), 2.63 (t, *J=* 6.0 Hz, 2H), 2.54 (t, *J* = 4.8 Hz, 2H), 2.50 (t, *J=* 4.8 Hz, 2H), 2.12 (s, 3H).

### Example 261. Typical procedure of sulfonylation for the synthesis: N-(2-(4-(methylsulfonyl)piperazin-1-yl)ethyl)-5-(2-nitrophenyl)-2-(4-(trifluoromethyl)phenyl)oxazole-4-carboxamide (287)

To a solution of compound **281** (70 mg, 0.14 mmol) in anhydrous CH₂Cl₂ (5.0 mL) was added DIPEA (50 µL, 0.29 mmol) and the solution was cooled to 0 °C. Methanesulfonyl chloride (22 µL, 0.29 mmol) was added dropwise. The reaction mixture was stirred for 5 min and then at room temperature for 1 h. The volatile components were evaporated and the residue was purified by column chromatography on a silica gel (eluting with CH₂Cl₂: MeOH = 20:1 to 10:1, v/v) to afford compound **287** as a yellow solid (37 mg, 45%). ¹H NMR (600 MHz, CDCl₃) *δ* 8.19-8.15 (m, 3H), 7.94-7.92 (m, 1H), 7.79-7.76 (m, 3H), 7.71-7.68 (m, 1H), 7.43 (t, *J=* 5.4 Hz, 1H), 3.55 (q, *J* = 6.0 Hz, 2H), 3.32-3.30 (m, 4H), 2.83 (s, 3H), 2.68-2.65 (m, 6H).

Compound **288** was prepared using a similar method as described for compound **287.**

### Example 262. N-(2-(Methylsulfonamido)ethyl)-5-(2-nitrophenyl)-2-(4-(trifluoromethyl)phenyl)oxazole-4-carboxamide (288)

This compound was obtained in 62% yield as a fluffy white solid, following the same procedure described for the synthesis of compound **287** which is typical procedure sulfonylation reaction. ¹H NMR (600 MHz, CDCl₃) *δ* 8.31 (d, *J* = 7.8 Hz, 2H), 8.21 (dd, *J=* 1.2 Hz and *J =* 8.4 Hz, 1H), 7.94 (dd, *J* = 1.2 Hz and *J* = 7.8 Hz, 1H), 7.90-7.85 (m, 3H), 7.81-7.79 (m, 1H), 3.50 (t, *J=* 6.0 Hz, 2H), 3.37 (s, 1H), 3.29 (t, *J* = 6.0 Hz, 2H).

### Example 263. N-(2-Guanidinoethyl)-5-(2-nitrophenyl)-2-(4-(trifluoromethyl)phenyl)oxazole-4-carboxamide (289)

To a solution of compound **282** (200 mg, 0.48 mmol) in anhydrous MeOH (5.0mL) were added *N,N'*-Bis(tert-butoxycarbonyl)-1*H*-pyrazole-1-carboxamidine (243 mg, 0.78 mmol) and Et₃N (0.3 mL, 1.6 mmol), and stirred at room temperature for 20 h under an argon atmosphere. The volatile components were evaporated. The reaction mixture was dissolved in anhydrous CH₂Cl₂ (3 mL). Then trifluoroacetic acid (1 mL) was added in the solution and cooled to 0 °C. The reaction mixture was stirred at room temperature for 6 h. CH₂Cl₂ was evaporated and replaced by anhydrous toluene which was then evaporated to azeotrope excess trifluoroacetic acid. This operation was repeated three times. The residue was purified by column chromatography on silica gel (eluting with CH₂Cl₂ : MeOH = 20:1 to 10:1, v/v) to afford compound **289** (111 mg, 50%). ¹H NMR (600 MHz, DMSO-d₆) *δ* 10.8 (s, 1H), 8.66 (s, 1H), 8.41 (s, 1H), 8.27 (d, *J* = 6.0 Hz, 1H), 8.21 (dd, *J* = 0.9 Hz and *J* = 8.2 Hz, 1H), 8.02 (d, *J* = 8.4 Hz, 2H), 7.99 (dd, *J=* 1.3 Hz and *J* = 7.8 Hz, 1H), 7.93-7.90 (m, 1H), 7.85-7.83 (m, 1H), 3.46 (t, *J=* 6.0 Hz, 2H), 3.43 (t, *J* = 5.4 Hz, 2H).

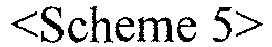

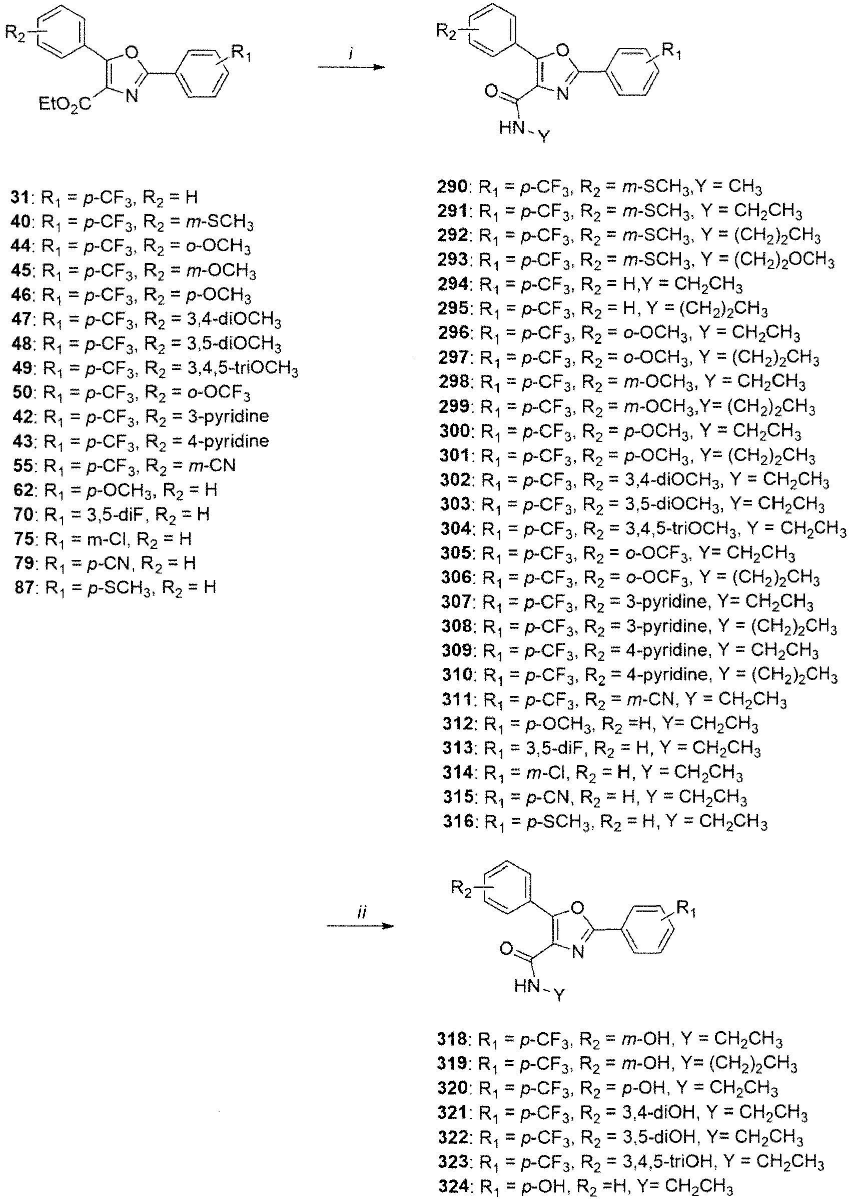

[*Reagents and conditions: i*) CH₃NH₂·HCl, CH₃CH₂NH₂·HCl or CH₃(CH₂)₂NH₂·HBr, EDC·HCl, HOBt, DIPEA, DMF, rt, 15 h, 61% yield for compound **290,** 74% yield for compound **291,** 79% yield for compound **292,** 38% yield for compound **293,** 67% yield for compound **294,** 70% yield for compound **295,** 85% yield for compound **296,** 85% yield for compound **297,** 71% yield for compound **298,** 78% yield for compound **299,** 80% yield for compound **300,** 72% yield for compound **301,** quantitative yield for compound **302,** 82% yield for compound **303,** 40% yield for compound **304,** 73% yield for compound **305,** 77% yield for compound **306,** 35% yield for compound **307,** 18% yield for compound **308,** 39% yield for compound **309,** 12% yield for compound **310,** 61% yield for compound **311,** 100% yield for compound **312,** 85% yield for compound **313,** 85% yield for compound **314,** 68% yield for compound **315,** 65% yield for compound **316,** 69% yield for compound **317;** ii) BBr₃ 1 M solution in DCM, DCM, 0 °C to rt, 15 h, 58% yield for compound **318, 85%** yield for compound **319,** 87% yield for compound **320,** 43% yield for compound **321,** 84% yield for compound **322,** 62% yield for compound **323,** 54% yield for compound **324.]**

### Example 264. Typical procedure of hydrolysis and amide coupling reaction for the synthesis: N-Methyl-5-(3-(methylthio)phenyl)-2-(4-(trifluoromethyl)phenyl)oxazole-4-carboxamide (290)

To a solution of ethyl 5-(3-(methylthio)phenyl)-2-(4-(trifluoromethyl)phenyl)oxazole-4-carboxylate **40** (900 mg, 2.21 mmol) in a mixture of THF and EtOH (20 mL, 1:1) was added 3N NaOH (3.70 mL, 11.1 mmol), and stirred at room temperature for 1 h. The reaction mixture was evaporated under reduced pressure and acidified by 3 N HCl, extracted with EtOAc three times. The organic layer was washed with brine, dried over MgSO₄ and concentrated *in vacuo.* The obtained carboxylic acid compound was used for the next step without further purification.

The carboxylic acid compound (190 mg, 0.50 mmol), methylamine hydrochloride (66 mg, 0.75 mmol), EDC·HCl (144 mg, 0.75 mmol), HOBt (101 mg, 0.75 mmol), DIPEA (435 µL, 2.50 mmol) was dissolved in DMF (5 mL). The reaction mixture stirred for 15 h at room temperature. The reaction mixture was evaporated under reduced pressure, diluted with water and extracted with EtOAc three times. The organic layer was washed with brine, dried over MgSO₄ and concentrated *in vacuo.* The residue was purified by column chromatography on silica gel (eluting with hexane : Et₂OAc = 4:1, v/v) to afford pure compound **290** (119 mg, 61%). ¹H NMR (600 MHz, CDCL₃) *δ* 8.39 (s, 1H), 8.22 (d, *J=* 7.8 Hz, 2H), 8.17 (d, *J* = 7.8 Hz, 1H), 7.78 (d, *J* = 7.8 Hz, 1H), 7.42 (t, *J* = 7.8 Hz, 2H), 7.41 (brs, 1H), 7.35 (d, *J* = 7.8 Hz, 1H), 3.06 (d, *J=* 4.8 Hz, 3H), 2.60 (s, 3H).

Compound **291-317** were prepared using a similar method as described for compound **290.**

### Example 265. N-Ethyl-5-(3-(methylthio)phenyl)-2-(4-(trifluoromethyl)phenyl)oxazole-4-carboxamide (291)

This compound was obtained in 74% yield as a white solid with a reaction of compound **40** following the same procedure described for the synthesis of compound **290** which is typical procedure hydrolysis and amide coupling reaction. ¹H NMR (600 MHz, CDCL₃) *δ* 8.36 (s, 1H), 8.24 (d, *J=* 7.8 Hz, 2H), 8.18 (d, *J* = 7.8 Hz, 1H), 7.78 (d, *J =* 8.4 Hz, 2H), 7.42 (t, *J* = 7.2 Hz, 1H), 7.39 (brs, 1H), 7.35 (d, *J* = 8.4 Hz, 1H), 3.57-3.49 (m, 2H), 2.59 (s, 3H), 1.31 (t, *J* = 7.2 Hz, 3H).

### Example 266. 5-(3-(Methylthio)phenyl)-N-propyl-2-(4-(trifluoromethyl)phenyl)oxazole-4-carboxamide (292)

This compound was obtained in 79% yield as a white solid with a reaction of compound **40** following the same procedure described for the synthesis of compound **290** which is typical procedure hydrolysis and amide coupling reaction. ¹H NMR (600 MHz, CDCL₃) *δ* 8.35 (s, 1H), 8.24 (d, *J=* 7.8 Hz, 2H), 8.18 (d, *J=* 7.8 Hz, 1H), 7.78 (d, *J=* 8.4 Hz, 2H), 7.43 (brs, 1H), 7.42 (t, *J=* 7.2 Hz, 1H), 7.35 (d, *J=* 8.4 Hz, 1H), 3.47 (q, *J* = 7.2 Hz, 2H), 2.59 (s, 3H), 1.75-1.59 (m, 2H), 1.03 (t, *J=* 7.2 Hz, 3H).

### Example 267. N-(2-Methoxyethyl)-5-(3-(methylthio)phenyl)-2-(4-(trifluoromethyl)phenyl)oxazole-4-carboxamide (293)

This compound was obtained in 38% yield as a white solid with a reaction of compound **40** following the same procedure described for the synthesis of compound **293** which is typical procedure hydrolysis and amide coupling reaction. ¹H NMR (600 MHz, CDCL₃) *δ* 8.36 (s, 1H), 8.24 (d, *J=* 7.8 Hz, 2H), 8.17 (d, *J=* 7.8 Hz, 1H), 7.19 (d, *J=* 8.4 Hz, 2H), 7.70 (brs, 1H), 7.42 (t, *J* = 7.2 Hz, 1H), 7.35 (d, *J* = 8.4 Hz, 1H), 3.70 (q, *J=* 4.8 Hz, 2H), 3.62 (t, *J* = 5.4 Hz, 2H), 3.44 (s, 3H), 2.59 (s, 3H).

### Example 268. N-Ethyl-5-phenyl-2-(4-(trifluoromethyl)phenyl)oxazole-4-carboxamide (294)

This compound was obtained in 67% yield as a white solid with a reaction of compound **31** following the same procedure described for the synthesis of compound **290** which is typical procedure of hydrolysis and amide coupling reaction. ¹H NMR (600 MHz, CDCl₃) *δ* 8.41 (d, *J* = 7.8 Hz, 2H), 8.25 (d, *J* = 7.8 Hz, 2H), 7.79 (d, *J=* 8.4 Hz, 2H), 7.52 (t, *J* = 7.2 Hz, 2H), 7.47 (t, *J=* 7.2 Hz, 1H), 7.40 (brs, 1H), 3.56 (q, *J=* 6.6 Hz, 2H), 1.33 (t, *J=* 7.8 Hz, 3H).

### Example 269. 5-Phenyl-N-propyl-2-(4-(trifluoromethyl)phenyl)oxazole-4-carboxamide (295)

This compound was obtained in 70% yield as a white solid with a reaction of compound **31** following the same procedure described for the synthesis of compound **290** which is typical procedure of hydrolysis and amide coupling reaction. ¹H NMR (600 MHz, CDCl₃) *δ* 8.41 (d, *J* = 7.8 Hz, 2H), 8.25 (d, *J=* 7.8 Hz, 2H), 7.79 (d, *J=* 8.4 Hz, 2H), 7.52 (t, *J* = 7.2 Hz, 2H), 7.47 (t, *J* = 7.2 Hz, 1H), 7.40 (brs, 1H), 3.56 (q, *J* = 6.6 Hz, 2H), 1.78-1.68 (m, 2H), 1.05 (t, *J =* 7.8 Hz, 3H).

### Example 270. N-Ethyl-5-(2-methoxyphenyl)-2-(4-(trifluoromethyl)phenyl)oxazole-4-carboxamide (296)

This compound was obtained in 85% yield as a white solid with a reaction of compound **44** following the same procedure described for the synthesis of compound **290** which is typical procedure of hydrolysis and amide coupling reaction. ¹H NMR (600 MHz, CDCl₃) *δ* 8.20 (d, *J* = 8.4 Hz, 2H), 7.75 (d, *J=* 7.8 Hz, 2H), 7.73 (d, *J=* 7.8 Hz, 1H), 7.47 (t, *J=* 8.4 Hz, 1H), 7.14 (brs, 1H), 7.08 (t, *J* = 7.8 Hz, 1H), 7.02 (d, *J* = 8.4 Hz, 1H), 3.88 (s, 3H), 3.51-3.41 (m, 2H), 1.27 (t, *J*= 7.2 Hz, 3H).

### Example 271. 5-(2-Methoxyphenyl)-N-propyl-2-(4-(trifluoromethyl)phenyl)oxazole-4-carboxamide (297)

This compound was obtained in 85% yield as a white solid with a reaction of compound **44** following the same procedure described for the synthesis of compound **290** which is typical procedure of hydrolysis and amide coupling reaction. ¹H NMR (600 MHz, CDCl₃) *δ* 8.20 (d, *J* = 8.4 Hz, 2H), 7.75 (d, *J=* 7.8 Hz, 2H), 7.73 (d, *J=* 7.8 Hz, 1H), 7.47 (t, *J=* 8.4 Hz, 1H), 7.18 (brs, 1H), 7.08 (t, *J=* 7.8 Hz, 1H), 7.02 (d, *J=* 8.4 Hz, 1H), 3.88 (s, 3H), 3.40 (q, *J=* 6.6 Hz, 2H), 1.71-1.59 (m, 2H), 1.00 (t, *J*= 7.2 Hz, 3H).

### Example 272. N-Ethyl-5-(3-methoxyphenyl)-2-(4-(trifluoromethyl)phenyl)oxazole-4-carboxamide (298)

This compound was obtained in 71% yield as a white solid with a reaction of compound **45** following the same procedure described for the synthesis of compound **290** which is typical procedure of hydrolysis and amide coupling reaction. ¹H NMR (600 MHz, CDCl₃) *δ* 8.23 (d, *J* = 7.8 Hz, 2H), 8.17 (d, *J* = 1.2 Hz, 1H), 7.94 (d, *J=* 7.8 Hz, 1H), 7.78 (d, *J* = 8.4 Hz, 2H), 7.41 (t, *J=* 7.8 Hz, 1H), 7.40 (brs, 1H), 7.01 (dd, *J=* 2.4 Hz and *J =* 6.0 Hz, 1H), 3.97 (s, 3H), 3.59-3.50 (m, 2H), 1.32 (t, *J* = 7.2 Hz, 3H).

### Example 273. 5-(3-Methoxyphenyl)-N-propyl-2-(4-(Trifluoromethyl)phenyl)oxazole-4-carboxamide (299)

This compound was obtained in 78% yield as a white solid with a reaction of compound **45** following the same procedure described for the synthesis of compound **290** which is typical procedure of hydrolysis and amide coupling reaction. ¹H NMR (600 MHz, CDCl₃) *δ* 8.23 (d, *J* = 7.8 Hz, 2H), 8.17 (d, *J* = 1.2 Hz, 1H), 7.94 (d, *J=* 7.8 Hz, 1H), 7.78 (d, *J=* 8.4 Hz, 2H), 7.44 (brs, 1H), 7.41 (t, *J=* 7.8 Hz, 1H), 7.01 (dd, *J=* 2.4 Hz and *J* = 6.0 Hz, 1H), 3.98 (s, 3H), 3.47 (q, *J=* 7.2 Hz, 2H), 1.75-1.64 (m, 2H), 1.04 (t, *J=* 7.2 Hz, 3H).

### Example 274. N-Ethyl-5-(4-methoxyphenyl)-2-(4-(trifluoromethyl)phenyl)oxazole-4-carboxamide (300)

This compound was obtained in 80% yield as a white solid with a reaction of compound **46** following the same procedure described for the synthesis of compound **290** which is typical procedure of hydrolysis and amide coupling reaction. ¹H NMR (600 MHz, CDCl₃) *δ* 8.38 (d, *J* = 9.0 Hz, 2H), 8.22 (d, *J=* 8.4 Hz, 2H), 7.77 (d, *J* = 8.4 Hz, 2H), 7.63 (brs, 1H), 7.02 (d, *J* = 9.0 Hz, 2H), 3.89 (s, 3H), 3.58-3.49 (m, 2H), 1.31 (t, *J=* 7.2 Hz, 3H).

### Example 275. 5-(4-Methoxyphenyl)-N-propyl-2-(4-(Trifluoromethyl)phenyl)oxazole-4-carboxamide (301)

This compound was obtained in 72% yield as a white solid with a reaction of compound **46** following the same procedure described for the synthesis of compound **290** which is typical procedure of hydrolysis and amide coupling reaction. ¹H NMR (600 MHz, CDCl₃) *δ* 8.38 (d, *J* = 9.0 Hz, 2H), 8.22 (d, *J=* 8.4 Hz, 2H), 7.77 (d, *J* = 8.4 Hz, 2H), 7.41 (brs, 1H), 7.02 (d, *J* = 9.0 Hz, 2H), 3.89 (s, 3H), 3.45 (q, *J=* 6.6 Hz, 2H), 1.75-1.65 (m, 2H), 1.03 (t, *J=* 7.2 Hz, 3H).

### Example 276. 5-(3,4-dimethoxyphenyl)-N-ethyl-2-(4-(Trifluoromethyl)phenyl)oxazole-4-carboxamide (302)

This compound was obtained in 79% yield as a white solid with a reaction of compound **47** following the same procedure described for the synthesis of compound **290** which is typical procedure of hydrolysis and amide coupling reaction. ¹H NMR (600 MHz, CDCl₃) *δ* 8.37 (d, *J* = 1.8 Hz, 1H), 8.22 (d, *J* = 8.4 Hz, 2H), 7.91 (dd, *J* = 2.4 Hz and *J =* 8.4 Hz, 1H), 7.78 (d, *J* = 7.8 Hz, 2H), 7.40 (brs, 1H), 6.98 (d, *J=* 8.4 Hz, 1H), 4.04 (s, 3H), 3.97 (s, 3H), 3.58-3.51 (m, 2H), 1.32 (t, *J* = 7.5 Hz, 3H).

### Example 277. 5-(3,5-Dimethoxyphenyl)-N-ethyl-2-(4-(trifluoromethyl)phenyl)oxazole-4-carboxamide (303)

This compound was obtained in 82% yield as a white solid with a reaction of compound **48** following the same procedure described for the synthesis of compound **290** which is typical procedure of hydrolysis and amide coupling reaction. ¹H NMR (600 MHz, CDCl₃) *δ* 8.23 (d, *J* = 8.4 Hz, 2H), 7.78 (d, *J=* 7.8 Hz, 2H), 7.73 (d, *J=* 2.4 Hz, 2H), 7.42 (brs, 1H), 6.58 (t, *J=* 2.4 Hz, 1H), 3.91 (s, 6H), 3.57-3.51 (m, 2H), 1.31 (t, *J=* 7.2 Hz, 3H).

### Example 278. N-Ethyl-2-(4-(trifluoromethyl)phenyl)-5-(3,4,5-trimethoxyphenyl)oxazole-4-carboxamide (304)

This compound was obtained in 40% yield as a white solid with a reaction of compound **49** following the same procedure described for the synthesis of compound **290** which is typical procedure of hydrolysis and amide coupling reaction. ¹H NMR (600 MHz, CDCl₃) *δ* 8.23 (d, *J* = 8.4 Hz, 2H), 7.84 (s, 2H), 7.79 (d, *J=* 8.4 Hz, 2H), 7.42 (brt, *J* = 5.4 Hz, 1H), 4.00 (s, 6H), 3.96 (s, 3H), 3.57-3.51 (m, 2H), 1.32 (t, *J=* 7.2 Hz, 3H).

### Example 279. N-Ethyl-5-(2-(trifluoromethoxy)phenyl)-2-(4-(trifluoromethyl)phenyl)oxazole-4-carboxamide (305)

This compound was obtained in 73% yield as a white solid with a reaction of compound **50** following the same procedure described for the synthesis of compound **290** which is typical procedure of hydrolysis and amide coupling reaction. ¹H NMR (600 MHz, CDCl₃) *δ* 8.22 (d, *J* = 8.4 Hz, 2H), 8.07 (dd, *J=* 1.8 Hz and *J =* 8.4 Hz, 1H), 7.78 (d, *J=* 8.4 Hz, 2H), 7.54 (td, *J=* 1.2 Hz and *J* = 7.2 Hz, 1H), 7.44 (td, *J=* 1.2 Hz and *J* = 7.2 Hz, 1H), 7.41 (d, *J* = 8.4 Hz, 1H), 7.25 (brs, 1H), 3.52-3.47 (m, 2H), 1.29 (t, *J=* 7.2 Hz, 3H).

### Example 280. N-Propyl-5-(2-(trifluoromethoxy)phenyl)-2-(4-(trifluoromethyl)phenyl)oxazole-4-carboxamide (306)

This compound was obtained in 77% yield as a white solid with a reaction of compound **50** following the same procedure described for the synthesis of compound **290** which is typical procedure of hydrolysis and amide coupling reaction. ¹H NMR (600 MHz, CDCl₃) *δ* 8.22 (d, *J* = 7.8 Hz, 2H), 8.07 (dd, *J* = 1.8 Hz and *J=* 7.2 Hz, 1H), 7.78 (d, *J=* 7.8 Hz, *J=* 2H), 7.54 (td, *J* = 1.8 Hz and *J* = 7.8 Hz, 1H), 7.44 (td, *J=* 1.2 Hz and *J* = 7.8 Hz, 1H), 7.41 (d, *J* = 7.2 Hz, 1H), 7.29 (br s, 1H), 3.42 (q, *J=* 7.2 Hz, 2H), 1.71-1.65 (m, 2H), 1.01 (t, *J* = 7.2 Hz, 3H).

### Example 281. N-Ethyl-5-(pyridin-3-yl)-2-(4-(trifluoromethyl)phenyl)oxazole-4-carboxamide (307)

This compound was obtained in 76% yield as a white solid with a reaction of compound **42** following the same procedure described for the synthesis of compound **290** which is typical procedure of hydrolysis and amide coupling reaction. ¹H NMR (600 MHz, CDCl₃) *δ* 9.35 (d, *J* = 1.8 Hz, 1H), 9.00 (td, *J=* 1.8 Hz and *J =* 8.4 Hz, 1H), 8.67 (dd, *J* = 1.2 Hz and *J* = 5.1 Hz, 1H), 8.25 (d, *J=* 8.4 Hz, 2H), 7.80 (d, *J* = 8.4 Hz, 2H), 7.44 (q, *J=* 4.8 Hz, 1H), 7.40 (brs, 1H), 3.59-3.50 (m, 2H), 1.33 (t, *J=* 7.2 Hz, 3H).

### Example 282. N-Propyl-5-(pyridin-3-yl)-2-(4-(trifluoromethyl)phenyl)oxazole-4-carboxamide (308)

This compound was obtained in 18% yield as a white solid with a reaction of compound **42** following the same procedure described for the synthesis of compound **290** which is typical procedure of hydrolysis and amide coupling reaction. ¹H NMR (600 MHz, CDCl₃) *δ* 9.35 (d, *J* = 1.8 Hz, 1H), 9.00 (td, *J* = 1.8 Hz and *J* = 8.4 Hz, 1H), 8.67 (dd, *J* = 1.2 Hz and *J* = 5.1 Hz, 1H), 8.25 (d, *J=* 8.4 Hz, 2H), 7.80 (d, *J* = 8.4 Hz, 2H), 7.44 (q, *J* = 4.8 Hz, 1H), 3.47 (q, J = 6.6 Hz, 2H), 1.75-1.66 (m, 2H), 1.04 (t, *J*= 7.2 Hz, 3H).

### Example 283. N-Ethyl-5-(pyridin-4-yl)-2-(4-(trifluoromethyl)phenyl)oxazole-4-carboxamide (309)

This compound was obtained in 39% yield as a white solid with a reaction of compound **43** following the same procedure described for the synthesis of compound **290** which is typical procedure of hydrolysis and amide coupling reaction. ¹H NMR (600 MHz, CDCl₃) *δ* 8.78 (d, *J* = 6.0 Hz, 2H), 8.35 (d, *J=* 6.0 Hz, 2H), 8.26 (d, *J=* 7.8 Hz, 2H), 7.80 (d, *J=* 9.0 Hz, 2H), 7.44 (brs, 1H), 3.60-3.51 (m, 2H), 1.33 (t, *J=* 7.2 Hz, 3H).

### Example 284. N-Propyl-5-(pyridin-4-yl)-2-(4-(trifluoromethyl)phenyl)oxazole-4-carboxamide (310)

This compound was obtained in 12% yield with a reaction of compound **43** following the same procedure described for the synthesis of which is compound **290** typical procedure of hydrolysis and amide coupling reaction. ¹H NMR (600 MHz, CDCl₃) *δ* 8.78 (dd, *J =* 1.8 Hz and *J* = 6.6 Hz, 2H), 8.36 (dd*, J=* 1.8 Hz and *J* = 6.0 Hz, 2H), 8.27 (d, *J=* 8.4 Hz, 2H), 7.81 (d, *J* = 8.4 Hz, 2H), 7.81 (d, *J* = 8.4 Hz, 2H), 7.84 (brs, 1H), 3.48 (q, *J* = 6.6 Hz, 2H), 1.76-1.69 (m, 2H), 1.05 (t, *J* = 7.2 Hz, 3H).

### Example 285. 5-(3-Cyanophenyl)-N-ethyl-2-(4-(Trifluoromethyl)phenyl)oxazole-4-carboxamide (311)

This compound was obtained in 61% yield as a white solid with a reaction of compound **55** following the same procedure described for the synthesis of compound **290** which is typical procedure of hydrolysis and amide coupling reaction. ¹H NMR (600 MHz, CDCl₃) *δ* 8.82-8.80 (m, 1H), 8.68 (t, *J=* 1.8 Hz, 1H), 8.26 (d, *J* = 8.4 Hz, 2H), 7.81 (d, *J=* 8.4 Hz, 2H), 7.73 (dt, *J* = 1.8 Hz and *J* = 7.8 Hz, 1H), 7.63 (t, *J* = 7.8 Hz, 1H), 7.42 (br s, 1H), 3.57-3.53 (m, 2H), 1.33 (t, *J=* 7.2 Hz, 3H).

### Example 286. N-Ethyl-2-(4-methoxyphenyl)-5-phenyloxazole-4-carboxamide (312)

This compound was obtained in 73% yield as a white solid with a reaction of compound **62** following the same procedure described for the synthesis of compound **290** which is typical procedure of hydrolysis and amide coupling reaction. ¹H NMR (600 MHz, CDCl₃) *δ* 8.39 (d, *J* = 7.2 Hz, 2H), 8.06 (d, *J=* 9.0 Hz, 2H), 7.49 (t, *J* = 7.8 Hz, 2H), 7.42 (td, *J=* 1.2 Hz and *J =* 7.5 Hz, 2H), 7.02 (d, *J=* 9.0 Hz, 2H), 3.90 (s, 3H), 3.57-3.49 (m, 2H), 1.30 (t, *J* = 7.2 Hz, 3H).

### Example 287. 2-(3,5-Difluorophenyl)-N-ethyl-5-phenyloxazole-4-carboxamide (313)

This compound was obtained in 85% yield as a white solid with a reaction of compound **70** following the same procedure described for the synthesis of compound **290** which is typical procedure of hydrolysis and amide coupling reaction. ¹H NMR (600 MHz, CDCl₃) *δ* 8.40-8.36 (m, 2H), 7.66-7.62 (m, 2H), 7.53-7.49 (m, 2H), 7.48-7.44 (m, 1H), 7.35 (brs, 1H), 6.79 (tt, *J=* 2.4 Hz and *J* = 8.7 Hz, 1H), 3.57-3.50 (m, 2H), 1.31 (t, *J* = 6.9 Hz, 3H).

### Example 288. 2-(3-Chlorophenyl)-N-ethyl-5-phenyloxazole-4-carboxamide (314)

This compound was obtained in 85% yield as a white solid with a reaction of compound **75** following the same procedure described for the synthesis of compound **290** which is typical procedure of hydrolysis and amide coupling reaction. ¹H NMR (600 MHz, CDCl₃) *δ* 8.41-8.38 (m, 2H), 8.11 (t, *J* = 1.8 H, 1H), 8.00 (td, *J* = 1.5 Hz and *J* = 7.2 Hz, 1H), 7.52-7.48 (m, 3H), 7.47-7.43 (m, 2H), 7.39 (brs, 1H), 3.56-3.50 (m, 2H), 1.31 (t, *J* = 7.2 Hz, 3H).

### Example 289. 2-(4-cyanophenyl)-N-ethyl-5-phenyloxazole-4-carboxamide (315)

This compound was obtained in 68% yield as a white solid with a reaction of compound **79** following the same procedure described for the synthesis of compound **290** which is typical procedure of hydrolysis and amide coupling reaction. ¹H NMR (600 MHz, CDCl₃) *δ* 8.42-8.37 (m, 2H), 8.23 (d, *J* = 9.0 Hz, 2H), 7.82 (d, *J=* 8.4 Hz, 2H), 7.53-7.49 (m, 2H), 7.48-7.44 (m, 1H), 7.36 (brs, 1H), 3.57-3.50 (m, 2H), 1.31 (t, *J=* 7.2 H, 3H).

### Example 290. N-Ethyl-2-(4-(methylthio)phenyl)-5-phenyloxazole-4-carboxamide (316)

This compound was obtained in 65% yield as a white solid with a reaction of compound **87** following the same procedure described for the synthesis of compound **290** which is typical procedure of hydrolysis and amide coupling reaction. ¹H NMR (600 MHz, CDCl₃) *δ* 8.40 (d, *J* = 7.2 Hz, 2H), 8.03 (d, *J* = 8.4 Hz, 2H), 7.51 (t, *J=* 7.2 Hz, 2H), 7.44 (t, *J=* 7.2 Hz, 1H), 7.43 (brs, 1H), 7.36 (d, *J=* 8.4 Hz, 2H), 3.58-3.49 (m, 2H), 1.32 (t, *J=* 6.6 Hz, 3H).

### Example 291. 2-(4-(Methylthio)phenyl)-5-phenyl-N-propyloxazole-4-carboxamide (317)

This compound was obtained in 69% yield as a white solid with a reaction of compound **87** following the same procedure described for the synthesis of compound **290** which is typical procedure of hydrolysis and amide coupling reaction. ¹H NMR (600 MHz, CDCl₃) *δ* 8.40 (d, *J* = 7.2 Hz, 2H), 8.03 (d, *J=* 9.0 Hz, 2H), 7.50 (t, *J=* 7.8 Hz, 2H), 7.51-7.41 (m, 2H), 7.36 (d, *J* = 8.4 Hz, 2H), 3.46 (q,*J* = 6.6 Hz, 2H), 1.76-1.67 (m, 2H), 1.04 (t, *J* = 7.2 Hz, 3H).

### Example 292. Typical procedure of deprotection of methoxy for the synthesis: N-Ethyl-5-(3-hydroxyphenyl)-2-(4-(trifluoromethyl)phenyl)oxazole-4-carboxamide (318)

To a solution of compound **298** (310 mg, 0.79 mmol) in anhydrous CH₂Cl₂ (10 mL) was added boron tribromide solution (2.37 mL, 1.0 M in CH₂Cl₂) at 0 °C, and stirred at room temperature for 15 h. The reaction mixture was added to water and extracted with CH₂Cl₂ three times. The organic layer was dried over Na₂SO₄, filtered and evaporated under reduced pressure. The residue was purified by column chromatography on silica gel (eluting with hexane : Et₂OAc = 3:1, v/v) to afford compound **318** as a white solid (172 mg, 58%). ¹H NMR (600 MHz, DMSO-d₆) *δ* 9.74 (brs, 1H), 8.53. (t, *J* = 5.7 Hz, 1H), 8.33 (d, *J=* 8.4 Hz, 2H), 7.99 (d, *J* = 8.4 Hz, 2H), 7.83 (t, *J* = 1.8 Hz, 1H), 7.79-7.76 (m, 1H), 7.33 (t, *J* = 5.4 Hz, 1H), 6.90 (ddd, *J* = 0.6 Hz, *J=* 2.4 Hz and *J =* 8.4 Hz, 1H), 1.16 (t, *J=* 7.2 Hz, 3H).

Compound **319-324** were prepared using a similar method as described for compound **318.**

### Example 293. 5-(3-Hydroxyphenyl)-N-propyl-2-(4-(trifluoromethyl)phenyl)oxazole-4-carboxamide (319)

This compound was obtained in 85% yield as a white solid following the same procedure described for the synthesis of compound **318** which is typical procedure of deprotection reaction of methoxy. ¹H NMR (600 MHz, CDCl₃) *δ* 8.23 (d, *J=* 8.4 Hz, 2H), 8.15 (t*, J* = 1.8 Hz, 1H), 7.81-7.79 (m, 1H), 7.78 (d*, J* = 8.4 Hz, 2H), 7.49 (brs, 1H), 7.36 (t, *J* = 8.4 Hz, 1H), 6.96 (ddd, *J =* 0.6 Hz, *J* = 2.4 Hz and *J* = 8.4Hz, 1H), 3.47-3.44 (m, 2H), 1.73-1.67 (m, 2H), 1.02 (t, *J=* 7.2 Hz, 2H).

### Example 294. N-Ethyl-5-(4-hydroxyphenyl)-2-(4-(trifluoromethyl)phenyl)oxazole-4-carboxamide (320)

This compound was obtained in 87% yield as a white solid following the same procedure described for the synthesis of compound **318** which is typical procedure of deprotection reaction of methoxy. ¹H NMR (600 MHz, MeOD) *δ* 8.31 (d, *J=* 8.4 Hz, 2H), 8.18 (d, *J=* 8.4 Hz, 2H), 7.85 (d, *J=* 8.4 Hz, 2H), 6.90 (d, *J=* 8.4 Hz, 2H), 3.44 (q, *J=* 7.2 Hz, 2H), 1.26 (t, *J=* 7.2 Hz, 3H).

### Example 295. 5-(3,4-Dihydroxyphenyl)-N-ethyl-2-(4-(trifluoromethyl)phenyl)oxazole-4-carboxamide (321)

This compound was obtained in 43% yield as a white solid following the same procedure described for the synthesis of compound **318** which is typical procedure of deprotection reaction of methoxy. ¹H NMR (600 MHz, MeOD) *δ* 8.30 (d, *J=* 8.4 Hz, 2H), 7.85 (d, *J=* 8.4 Hz, 2H), 7.79 (d, *J=* 2.4 Hz, 1H), 7.72 (dd, *J=* 2.4 Hz and *J =* 8.4 Hz, 1H), 6.88 (d, *J* = 8.4 Hz, 1H), 3.45 (q, *J* = *7.2* Hz, 2H), 1.26 (t, *J=* 7.2 Hz, 3H).

### Example 296. 5-(3,5-Dihydroxyphenyl)-N-ethyl-2-(4-(trifluoromethyl)phenyl)oxazole-4-carboxamide (322)

This compound was obtained in 84% yield as a white solid following the same procedure described for the synthesis of compound **318** which is typical procedure of deprotection reaction of methoxy. ¹H NMR (600 MHz, MeOD) *δ* 8.31 (d, *J=* 8.4 Hz, 2H), 7.86 (d, *J=* 8.4 Hz, 2H), 7.22 (d, *J=* 1.8 Hz, 2H), 6.39 (t, *J=* 1.8 Hz, 1H), 3.45 (q, *J=* 7.2 Hz, 2H), 1.26 (t, *J* = 7.2 Hz, 3H).

### Example 297. N-Ethyl-2-(4-(trifluoromethyl)phenyl)-5-(3,4,5-trihydroxyphenyl)oxazole-4-carboxamide (323)

This compound was obtained in 62% yield as a white solid following the same procedure described for the synthesis of compound **318** which is typical procedure of deprotection reaction of methoxy. ¹H NMR (600 MHz, MeOD)*δ* 8.29 (d, *J* = 8.4 Hz, 2H), 7.85 (d, *J=* 8.4 Hz, 2H), 7.38 (s, 2H), 3.45 (q, *J=* 7.2 Hz, 2H), 1.26 (t, *J=* 7.2 Hz, 3H).

### Example 298. N-Ethyl-2-(4-hydroxyphenyl)-5-phenyloxazole-4-carboxamide (324)

This compound was obtained in 54% yield following the same procedure described for the synthesis of compound **318** which is typical procedure of deprotection reaction of methoxy. ¹H NMR (600 MHz, MeOD) *δ* 8.23 (d, *J=* 7.8 Hz, 2H), 7.99 (d, *J=* 9.0 Hz, 2H), 7.48 (t, *J* = 7.5 HZ, 2H), 7.46-7.43 (m, 1H), 6.93 (d, *J=* 9.0 Hz, 2H), 3.44 (q, *J=* 7.4 Hz, 2H), 1.26 (t, *J=* 7.2 Hz, 3H).

### Experimental example 1. ELISA assay

### 1.1. Coating

100 nM of Flag tagged IL-33 human recombinant protein was prepared in 1X coating buffer (Biolegend) and placed in a 96 well microplate (Corning) using a Multichannel pipette (Gilson) and coated for at least 12 hours at 4°C.

### 1.2. Blocking

After washing three times with PBST (1X PBS (Welgene), 1% Tween 20 (sigma)), it was reacted with 1% BSA (Bovogen) blocking buffer for 1 hour at room temperature.

### 1.3. Binding

Samples containing 200 nM of His-tagged ST2 human recombinant protein and 60 µM or 200 µM Example synthetic compounds were prepared, added in equal volumes, and reacted for at least 2 hours at room temperature.

### 1.4. Detection

After washing three times with PBST (1X PBS (Welgene), 1% Tween 20 (sigma)), anti-His HRP (BioLegend) antibody was diluted 5000:1 and reacted for at least 1 hour at room temperature.

### 1.5. Reaction

After washing three times with PBST (1X PBS (Welgene), 1% Tween 20 (sigma)), TMB substrate solution (Thermo) was added and reacted for more than 5 minutes. The reaction was stopped by adding 1 N HCl (Samchun) and the absorbance value was obtained at 450 nm after orbital shaking for 5 s with a Microplate reader (Tecan, Spark^{®}).

### 1.6. Analysis

The results derived from the program linked to the microplate reader were analyzed using an Excel program. IL33-ST2 binding rate (binding %) was calculated by averaging the three values obtained in triplicate, subtracting the positive control value, and converting the negative control value to 100%. The inhibition % value of each compound was obtained by subtracting the IL33-ST2 binding % from 100%, and the error range of triplicate was also obtained and reflected in the inhibition graph of the compound.

### 1.7. Results

Inhibition % results of IL33 and ST2 binding are shown in Table 1 to Table 8 below.

**Table 1**

| Example | Compound No. | ELISA (Inhibition %) | | ELISAIC₅₀(µM) |
|---|---|---|---|---|
| | | 30 µM | 100 µM | |
| Synthesis Example 5 | **6** | - | 110 | - |
| Synthesis Example 21 | **22** | 1 | 45 | - |
| 1 | **27** | 34 | 84 | - |
| 2 | **28** | 30 | 53 | - |
| 3 | **29** | 19 | 33 | - |
| 4 | **30** | 42 | 102 | - |
| 5 | **31** | 110 | 109 | 0.78 |
| 6 | **32** | 107 | 108 | - |
| 7 | **33** | 107 | 108 | 1.71 |
| 8 | **34** | 108 | 108 | 0.91 |
| 14 | **40** | 108 | 115 | - |
| 18 | **44** | 100 | - | 1.26 |
| 21 | **47** | -57 | - | - |
| 22 | **48** | 87 | - | - |
| 23 | **49** | -65 | - | - |
| 26 | **52** | 115 | 115 | 7.99 |
| 29 | **55** | 115 | 116 | - |
| 30 | **56** | 40 | 93 | - |
| 32 | **58** | 44 | 105 | - |
| 34 | **60** | 27 | 68 | - |
| 36 | **62** | 33 | 65 | - |
| 37 | **63** | 26 | 57 | - |

**Table 2**

| Example | Compound No. | ELISA (Inhibition %) | | ELISAIC₅₀(µM) |
|---|---|---|---|---|
| | | 30 µM | 100 µM | |
| 38 | **64** | 13 | 35 | - |
| 39 | **65** | 35 | 32 | - |
| 40 | **66** | 49 | 71 | - |
| 41 | **67** | 9 | 15 | - |
| 42 | **68** | 55 | 105 | - |
| 43 | **69** | 32 | 48 | - |
| 44 | **70** | 18 | 41 | - |
| 45 | **71** | 29 | 49 | - |
| 47 | **73** | 12 | 22 | - |
| 49 | **75** | 93 | 108 | 11.73 |
| 51 | **77** | 95 | 110 | - |
| 52 | **78** | 23 | 68 | - |
| 53 | **79** | 41 | 107 | - |
| 54 | **80** | 37 | 89 | - |
| 55 | **81** | 59 | 109 | - |
| 57 | **83** | 22 | 42 | - |
| 58 | **84** | 20 | 54 | - |
| 64 | **90** | 108 | 107 | 0.28 |
| 72 | **98** | 110 | 110 | 5.21 |
| 74 | **100** | 8 | 5 | - |
| 75 | **101** | 1 | -24 | - |

**Table 3**

| Example | Compound No. | ELISA (Inhibition %) | | ELISAIC₅₀(µM) |
|---|---|---|---|---|
| | | 30 µM | 100 µM | |
| 80 | **106** | -20 | -2 | - |
| 81 | **107** | -15 | -9 | - |
| 82 | **108** | -14 | -21 | - |
| 83 | **109** | 19 | -2 | - |
| 84 | **110** | 19 | 26 | - |
| 85 | **111** | 10 | 12 | - |
| 88 | **114** | 10 | 9 | - |
| 89 | **115** | -22 | -17 | - |
| 90 | **116** | 13 | 26 | - |
| 91 | **117** | 4 | 10 | - |
| 92 | **118** | 24 | 36 | - |
| 93 | **119** | -6 | -14 | - |
| 94 | **120** | 7 | 40 | - |
| 95 | **121** | -20 | -18 | - |
| 96 | **122** | -18 | 0 | - |
| 97 | **123** | -22 | 39 | - |
| 98 | **124** | 50 | 31 | - |
| 99 | **125** | 20 | 24 | - |
| 100 | **126** | -14 | 6 | - |
| 101 | **127** | -24 | -5 | - |
| 102 | **128** | -28 | | - |
| 103 | **129** | 45 | 79 | - |
| 104 | **130** | 11 | 37 | - |
| 105 | **131** | 91 | 92 | - |
| 112 | **138** | 6 | 11 | - |
| 113 | **139** | 4 | 13 | - |
| 114 | **140** | 10 | 15 | - |
| 115 | **141** | -11 | -3 | - |
| 116 | **142** | 6 | 8 | - |
| 117 | **143** | 9 | 8 | - |
| 118 | **144** | -19 | -34 | - |

**Table 4**

| Example | Compound No. | ELISA (Inhibition %) | | ELISAIC₅₀(µM) |
|---|---|---|---|---|
| | | 30 µM | 100 µM | |
| 119 | **145** | 3 | 11 | - |
| 120 | **146** | -35 | -57 | - |
| 121 | **147** | 1 | 4 | - |
| 122 | **148** | 12 | 19 | - |
| 123 | **149** | 20 | 19 | - |
| 124 | **150** | -18 | 7 | - |
| 125 | **151** | -33 | -16 | - |
| 126 | **152** | 36 | 48 | - |
| 127 | **153** | 92 | 95 | - |
| 128 | **154** | 0 | -25 | - |
| 129 | **155** | -31 | -18 | - |
| 130 | **156** | -1 | 41 | - |
| 131 | **157** | 22 | 15 | - |
| 132 | **158** | 31 | 26 | - |
| 133 | **159** | 2 | -1 | - |
| 134 | **160** | 18 | 29 | - |
| 135 | **161** | -16 | -11 | - |
| 136 | **162** | 3 | 5 | - |
| 137 | **163** | -30 | -19 | - |
| 138 | **164** | 9 | 4 | - |
| 139 | **165** | 10 | 13 | - |
| 140 | **166** | 14 | 5 | - |
| 141 | **167** | -24 | -9 | - |
| 142 | **168** | 12 | -12 | - |
| 143 | **169** | -4 | 7 | - |
| 144 | **170** | 3 | 2 | - |
| 146 | **172** | 51 | 50 | - |
| 147 | **173** | 54 | 60 | - |
| 149 | **175** | 27 | 53 | - |
| 150 | **176** | 27 | 36 | - |

**Table 5**

| Example | Compound No. | ELISA (Inhibition %) | | ELISAIC₅₀(µM) |
|---|---|---|---|---|
| | | 30 µM | 100 µM | |
| 168 | **194** | 41 | - | - |
| 171 | **197** | 99 | | - |
| 172 | **198** | 5 | | - |
| 173 | **199** | 7 | 2 | - |
| 174 | **200** | 5 | 0 | - |
| 175 | **201** | -7 | -5 | - |
| 176 | **202** | 7 | 32 | - |
| 177 | **203** | -9 | 14 | - |
| 178 | **204** | 1 | 36 | - |
| 179 | **205** | -11 | 4 | - |
| 180 | **206** | 23 | 49 | - |
| 182 | **208** | 19 | 55 | - |
| 184 | **210** | -11 | -19 | - |
| 185 | **211** | 13 | 20 | - |
| 186 | **212** | -26 | -22 | - |
| 187 | **213** | -34 | -9 | - |
| 188 | **214** | -28 | -49 | - |
| 189 | **215** | -12 | 33 | - |
| 190 | **216** | 46 | 84 | - |
| 191 | **217** | -38 | -51 | - |
| 192 | **218** | 32 | 39 | - |
| 193 | **219** | 11 | 44 | - |
| 194 | **220** | -2 | -14 | - |
| 195 | **221** | -34 | -21 | - |
| 196 | **222** | -28 | -16 | - |
| 197 | **223** | 29 | 38 | - |
| 198 | **224** | -13 | -18 | - |
| 199 | **225** | -6 | 7 | - |

**Table 6**

| Example | Compound No. | ELISA (Inhibition %) | | ELISAIC₅₀(µM) |
|---|---|---|---|---|
| | | 30 µM | 100 µM | |
| 200 | **226** | -25 | -8 | - |
| 201 | **227** | -22 | -28 | - |
| 202 | **228** | 24 | 43 | - |
| 203 | **229** | -54 | -98 | - |
| 204 | **230** | 15 | 0 | - |
| 205 | **231** | -8 | 5 | - |
| 206 | **232** | -9 | 3 | - |
| 207 | **233** | -27 | -27 | - |
| 208 | **234** | 13 | 19 | - |
| 209 | **235** | 3 | 36 | - |
| 210 | **236** | -8 | -7 | - |
| 211 | **237** | -34 | -16 | - |
| 212 | **238** | 40 | 103 | - |
| 214 | **240** | 7 | 70 | - |
| 215 | **241** | 31 | 38 | - |
| 216 | **242** | -14 | -12 | - |
| 217 | **243** | 14 | -6 | - |
| 218 | **244** | 0 | 38 | - |
| 219 | **245** | 15 | 26 | - |
| 220 | **246** | 110 | 112 | 4.78 |
| 221 | **247** | 8 | 32 | - |
| 222 | **248** | 33 | 63 | - |
| 223 | **249** | 81 | 97 | 6.46 |
| 224 | **250** | 14 | 9 | - |
| 225 | **251** | -6 | -21 | - |
| 226 | **252** | 28 | 19 | - |
| 227 | **253** | 11 | 60 | - |
| 228 | **254** | -20 | 11 | - |
| 229 | **255** | -10 | 1 | - |
| 230 | **256** | 3 | 49 | - |
| 231 | **257** | 6 | 1 | - |
| 232 | **258** | 35 | 49 | - |
| 233 | **259** | 6 | 1 | - |
| 234 | **260** | 9 | 23 | - |
| 235 | **261** | 3 | 8 | - |
| 236 | **262** | 14 | 33 | - |
| 237 | **263** | -35 | -10 | - |
| 238 | **264** | 29 | 35 | - |
| 239 | **265** | 5 | 14 | - |
| 240 | **266** | -29 | 10 | - |

**Table 7**

| Example | Compound No. | ELISA (Inhibition %) | | ELISAIC₅₀(µM) |
|---|---|---|---|---|
| | | 30 µM | 100 µM | |
| 241 | **267** | 23 | 29 | - |
| 242 | **268** | -15 | -6 | - |
| 243 | **269** | 33 | 44 | - |
| 244 | **270** | 20 | 37 | - |
| 245 | **271** | 31 | 105 | - |
| 246 | **272** | 97 | 97 | 4.17 |
| 247 | **273** | -25 | -18 | - |
| 248 | **274** | 18 | 20 | - |
| 249 | **275** | -16 | -28 | - |
| 250 | **276** | 2 | -23 | - |
| 251 | **277** | 60 | 111 | 14.4 |
| 252 | **278** | 46 | 47 | - |
| 253 | **279** | 16 | 4 | - |
| 254 | **280** | 2 | 0 | - |
| 255 | **281** | 73 | 71 | - |
| 257 | **283** | 31 | 49 | - |
| 258 | **284** | 101 | 104 | 3.15 |
| 259 | **285** | 73 | 72 | - |
| 260 | **286** | 18 | 33 | - |
| 261 | **287** | 41 | 46 | - |
| 262 | **288** | 43 | 50 | - |
| 264 | **290** | 112 | 112 | 4.51 |
| 265 | **291** | 112 | 111 | 0.64 |
| 266 | **292** | 110 | 112 | 1.13 |
| 267 | **293** | 40 | 107 | - |
| 268 | **294** | 95 | - | - |
| 272 | **298** | 87 | - | - |
| 279 | **305** | -17 | - | - |
| 280 | **306** | 38 | - | - |
| 281 | **307** | 106 | - | - |

**Table 8**

| Example | Compound No. | ELISA (Inhibition %) | | ELISAIC₅₀(µM) |
|---|---|---|---|---|
| | | 30 µM | 100 µM | |
| 285 | **311** | -5 | - | - |
| 286 | **312** | -47 | - | - |
| 287 | **313** | -29 | - | - |
| 288 | **314** | -18 | - | - |
| 289 | **315** | -4 | - | - |
| 290 | **316** | 47 | - | - |

### Experimental example 2. IL-6 production inhibition assay method

### 2.1. Cell Culture

Human mast cell line HMC 1.1 cells were cultured in medium (Iscove's Modified Dulbecco's Medium, IMDM, HyClone) supplemented with 10% fetal bovine serum (FBS, HyClone) and 1% antibiotics and maintained at 37°C with 5% COz.

### 2.2. Treatment of Example compounds

HMC 1.1 cells, which are non-adherent cells, were cultured in 0.5 mL of culture medium containing 200 nM of IL-33 wild type human recombinant protein after centrifugation of cells in culture flasks filled to 80% capacity and incubated for 24 hours with 5 µM, 10 µM, 25 µM, 30 µM or 50 µM of Example compound.

### 2.3. Cell Culture Recovery

Medium was harvested by centrifugation (2,000 xg, 5 min) and stored at -80°C until ELISA assay.

### 2.4. IL-6 ELISA Assay

Experiments were performed according to the protocol using the Human IL-6 ELISA kit (abCam).

### 2.5. Analysis Method

The results from the program linked to the microplate reader were analyzed using an Excel program. The average of the three values obtained in triplicate was calculated and the amount of IL-6 secretion was measured using a standard curve. The measured IL-6 secretion was converted to 100% based on the vehicle treatment sample value to obtain the vehicle %. The inhibition % value of each compound was obtained by subtracting the vehicle % from 100%, and the error range of triplicates was also obtained and reflected when deriving the graph of compound inhibition rate.

### 2.6. Results

The results of the inhibition % of IL-6 secretion in the human mast cell line HMC 1.1 are shown in Table 9 to Table 11 below.

**Table 9**

| Example | Compound No. | Inhibition of hIL-6 production (%) | | | EC₅₀(µM) |
|---|---|---|---|---|---|
| | | 0.1 µM | 1 µM | 10 µM | |
| 5 | **31** | 46 | 56 | 69 | 0.23 |
| 7 | **33** | - | 30 | 32 | - |
| 18 | **44** | - | 38 | 77 | 2.56 |
| 21 | **47** | - | 36 | 26 | - |
| 22 | **48** | - | 22 | 39 | - |
| 23 | **49** | - | -2 | 16 | - |
| 26 | **52** | - | 22 | 48 | 2.26 |
| 29 | **55** | - | 45 | 52 | 0.09 |
| 49 | **75** | - | 5 | 26 | - |
| 64 | **90** | 33 | 54 | 55 | 0.49 |
| 72 | **98** | - | -12 | 30 | - |
| 102 | **128** | - | 66 | 80 | - |
| 168 | **194** | - | 44 | 68 | - |
| 171 | **197** | - | 62 | 70 | - |
| 172 | **198** | - | 62 | 77 | - |

**Table 10**

| Example | Compound No. | Inhibition of hIL-6 production (%) | | | EC₅₀(µM) |
|---|---|---|---|---|---|
| | | 0.1 µM | 1 µM | 10 µM | |
| 212 | **238** | - | 14 | 45 | 6.3 |
| 214 | **240** | - | 6 | 44 | 3.7 |
| 220 | **246** | - | 20 | 45 | 6.15 |
| 223 | **249** | - | 8 | 35 | - |
| 245 | **271** | - | 14 | 69 | 0.86 |
| 246 | **272** | - | 52 | 79 | 0.42 |
| 251 | **277** | - | 27 | 31 | - |
| 258 | **284** | - | 27 | 39 | - |
| 264 | **290** | 28 | 49 | 59 | |
| 265 | **291** | 25 | 40 | 66 | 0.49 |
| 266 | **292** | 29 | 39 | 42 | 0.09 |
| 268 | **294** | 50 | 88 | 94 | 0.13 |
| 269 | **295** | - | 53 | 57 | - |
| 270 | **296** | - | 0 | 32 | - |
| 271 | **297** | - | 3 | 34 | - |
| 272 | **298** | 12 | 70 | 75 | 0.64 |
| 273 | **299** | - | 40 | 57 | - |
| 274 | **300** | - | 37 | 47 | - |
| 275 | **301** | - | 21 | 31 | - |
| 276 | **302** | - | -34 | -29 | - |
| 277 | **303** | - | 26 | 26 | - |

**Table 11**

| Example | Compound No. | Inhibition of hIL-6 production (%) | | | EC₅₀(µM) |
|---|---|---|---|---|---|
| | | 0.1 µM | 0.1 µM | 10 µM | |
| 278 | **304** | - | -47 | -74 | - |
| 279 | **305** | - | -4 | 26 | - |
| 280 | **306** | - | -38 | 45 | - |
| 281 | **307** | 61 | 69 | 79 | 0.11 |
| 282 | **308** | - | 56 | 62 | - |
| 283 | **309** | - | 56 | 51 | - |
| 284 | **310** | - | 49 | 58 | - |
| 285 | **311** | - | 64 | 80 | - |
| 286 | **312** | - | 66 | 81 | - |
| 287 | **313** | - | -7 | 27 | - |
| 288 | **314** | - | 10 | 81 | - |
| 289 | **315** | - | 47 | 61 | - |
| 290 | **316** | 25 | 69 | 77 | 0.35 |
| 291 | **317** | - | 44 | 43 | - |
| 292 | **318** | - | 70 | 79 | - |
| 293 | **319** | - | 75 | 80 | - |
| 295 | **321** | - | 50 | 72 | - |
| 296 | **322** | - | 86 | 73 | - |
| 297 | **323** | - | 45 | 84 | - |

### Experimental example 3. Determining Cellular Viability

### 3.1. Cell Culture

Human liver cancer cell line HepG2 cells were cultured in a medium (high glucose Dulbecco's Modified Eagle Medium, DMEM, HyClone) supplemented with 10% fetal bovine serum (FBS, HyClone) and 1% antibiotic in a thermostat maintained at 37°C with 5% CO₂.

### 3.2. Example compound treatment

HepG2 cells, which are adherent cells, were cultured in 100 µM of culture medium with 1 × 10⁴ for 18 hours and treated with 25 µM, 30 µM, or 50 µM of Example compound for 24 hours in the absence or presence of human liver microsomes (Gibco) and NADPH regenerating system.

### 3.3. Cell Culture Media Exchange

Cell cultures were exchanged using 100 µM of medium (high glucose Dulbecco's Modified Eagle Medium, DMEM, HyClone) and incubated for 24 hours.

### 3.4. WST-8 Assay

After adding 10 µL of WST-8 (abCam), the absorbance was obtained at 460 nm after 5 seconds of orbital shaking with a microplate reader (Tecan, Spark^{®}) 2 hours later.

### 3.5. Analysis

The results derived from the program linked to the microplate reader were analyzed using an Excel program. The mean of the three values obtained in triplicate was calculated and expressed as % cellular viability compared to vehicle treatment.

### 3.6 Results

The stability results for human and mouse liver S9 fractions are shown in Table 12 below.

**Table 12**

| Example | Compou nd No. | Cellular viability (%) | | CC₅₀(µM) | |
|---|---|---|---|---|---|
| | | (-) microsome | (+) microsome | (-) microsome | (+) microsome |
| Synthesis Example 5 | **6** | 66 at 45 µM | 99 at 45 µM | | |
| 5 | **31** | 83 at 135 µM | 66 at 135 µM | - | - |
| 6 | **32** | 81 at 25 µM | 95 at 25 µM | - | - |
| 7 | **33** | 73 at 100 µM | 68 at 100 µM | 67 | 43.5 |
| 8 | **34** | 87 at 100 µM | 85 at 100 µM | - | - |
| 18 | **44** | 68 at 100 µM | 99 at 100 µM | - | - |
| 26 | **52** | 117 at 100 µM | 97 at 100 µM | - | - |
| 29 | **55** | 90 at 100 µM | 91 at 100 µM | - | - |
| 48 | **74** | 101 at 45 µM | 96 at 45 µM | - | - |
| 49 | **75** | 106 at 100 µM | 100 at 100 µM | - | - |
| 64 | **90** | 53 at 135 µM | 74 at 135 µM | 14 | 40.8 |
| 72 | **98** | 83 at 100 µM | 90 at 100 µM | - | - |
| 73 | **99** | 79 at 25 µM | 96 at 25 µM | - | - |
| 127 | **153** | 19 at 50 µM | 36 at 50 µM | - | - |
| 132 | **158** | 17 at 50 µM | 17 at 50 µM | - | - |
| 177 | **203** | 19 at 50 µM | 30 at 50 µM | - | - |
| 191 | **217** | 19 at 100 µM | 34 at 100 µM | - | - |
| 205 | **231** | 17 at 50 µM | 29 at 50 µM | - | - |
| 212 | **238** | 49 at 100 µM | 53 at 100 µM | 19 | 213 |
| 214 | **240** | 45 at 100 µM | 50 at 100 µM | 5.46 | 6.36 |
| 220 | **246** | 47 at 100 µM | 52 at 100 µM | 11.8 | 16 |
| 222 | **248** | 19 at 50 µM | 31 at 50 µM | - | - |
| 223 | **249** | 43 at 100 µM | 50 at 100 µM | 19 | 29.1 |
| 245 | **271** | 45 at 100 µM | 52 at 100 µM | 3.74 | 6.12 |
| 246 | **272** | 46 at 100 µM | 49 at 100 µM | 4.73 | 5.6 |
| 251 | **277** | 46 at 100 µM | 49 at 100 µM | 25.4 | 46.2 |
| 258 | **284** | 40 at 100 µM | 49 at 100 µM | 48.6 | 46.5 |
| 259 | **285** | 54 at 30 µM | 59 at 30 µM | - | - |
| 264 | **290** | 47 at 135 µM | 60 at 135 µM | - | - |
| 265 | **291** | 106 at 160 µM | 102 at 160 µM | - | - |
| 266 | **292** | 55 at 135 µM | 58 at 135 µM | - | - |
| 268 | **294** | 95 at 160 µM | 90 at 160 µM | - | - |
| 281 | **307** | 105 at 160 µM | 108 at 160 µM | - | - |
| 282 | **308** | 27 at 160 µM | 29 at 160 µM | | |
| 290 | **316** | 84 at 160 µM | 100 at 160 µM | - | - |
| 291 | **317** | 47 at 160 µM | 89 at 160 µM | - | - |
| 296 | **322** | 63 at 160 µM | 90 at 160 µM | - | - |
| 297 | **323** | 63 at 160 µM | 77 at 160 µM | - | - |

### Experimental example 4. Verifing the efficacy in HDM- and ovalbumin-induced asthma animal models

### 4.1. Experimental Animals

Animals were 6-week-old specific pathogen-free (SPF) BALB/C female mice purchased from Orientbio Inc. (Seongnam, Korea).

### 4.2. Test Substances

### 4.2.1. Name

① MITE, DUST D. FARINAE (XPB81D3A25, GREER)
② MITE, DUST D. PTERONYSSINUS (XPB82D3A25, GREER)
③ Albumin from chicken egg white (A5503, SIGMA)
④ phosphate buffer saline (SH30256.01, CYTIVA)

### 4.2.2. Storage conditions: frozen

### 4. 3. Overview of Experimental Methods

Before administration of HDM and ovalbumin, 1×10⁶ of DO11.10 CD4 T cells were injected into the tail vein. The naive group consisted of 5 to 6 mice and the HDM-OVA acute asthma mouse model group consisted of 20 to 24 mice. HDM (*D. farinae* 50 µg/mouse, *D. pteronyssinus* 50 µg/mouse) and OVA (100 µg/mouse) were dissolved in PBS, and 40 µl per mouse was administered by intranasal injection for 3 days. Example compound (10 mg/kg in vehicle (DMSO : labrafil = 10 : 90 (vol%)) was administered orally at 5 mL/kg. Naive group was administered with 40 µL of normal saline instead of HDM-OVA. Wherever possible, test substances and HDM should be administered at the same time. Care was taken to ensure that aseptic conditions were maintained for both intratracheally instillation and SC administration. Mice were sacrificed on day 7 after the first sensitization to obtain alveolar lavage fluid and lungs were harvested.

### 4. 4. Analysis of eosinophils in bronchial lavage fluid

Mice were sacrificed by cervical dislocation, and the lungs were lavaged with 0.8 ml of PBS in the trachea (BAL fluids), which was repeated three times. Total cell counts were counted with a hemocytometer, and BAL fluids cell smears were prepared with cytospin (TXT3, Korea). For cell differentiation, eosinophils were counted after staining with Diff-Quik solution (Dade diagnostics of P.R. Inc. Aguada, Puerto Rico).

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each of the components described in a single form may also be implemented in a distributed manner, and similarly, components described as distributed may also be implemented in a combined form.

The scope of the invention is indicated by the following patent claims. The meaning and scope of the patent claims and all modifications or variations derived from their equivalents are considered to be falling within the scope of the invention.

## Claims

1. An oxazole derivative compound represented by the following Formula I, a hydrate thereof, a solvate thereof or a pharmaceutically acceptable salt thereof, wherein: 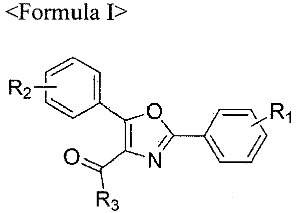
R₁ is hydrogen, C₁-C₆ straight or branched alkyl, C₃-C₆ cycloalkyl, C₁-C₄ alkoxy, halogen, C₁-C₄ haloalkyl, C₁-C₄ haloalkoxy, C₁-C₄ alkylthio, hydroxy, cyano, nitro, NRₐR_{b}, or C₅-C₁₂ aryl of 1-2 ring(s),
R₁ is singular or plural substituent, each of which is independent when existing plurally,
R₂ is hydrogen, C₁-C₄ alkoxy, C₁-C₄ haloalkyl, C₁-C₄ haloalkoxy, C₁-C₄ alkylthio, C₁-C₄ alkylsulfinyl, C₁-C₄ alkylsulfonyl, acetyl, hydroxy, cyano, nitro, NRₐR_{b}, or 4- to 7-membered heteroaryl having 1 to 3 of N,
R₂ is singular or plural substituent, each of which is independent when existing plurally,
R₃ is X substituted with Y or not,
X is C₁-C₄ alkoxy, amino(-NH-), C₅-C₇ aryl, or 5- to 7-membered non-aromatic heterocycle having 1 to 2 heteroatom(s) selected from the group consisting of N and O,
Y is hydrogen, C₁-C₆ straight or branched alkyl, C₃-C₆ cycloalkyl, hydroxy, COO, COO-alkyl, C₅-C₇ aryl, alkylaryl, or 5- to 7-membered aromatic or non-aromatic heterocycle having 1 to 2 of N,
Y is substituted with Z or not,
Z is C₁-C₆ straight or branched alkyl, C₁-C₄ alkoxy, hydroxy, C₁-C₄ haloalkyl, acetyl, guanidino, NRₐR_{b}, C₅-C₇ aryl, alkoxyaryl, 5- to 7-membered aromatic or non-aromatic heterocycle having 1 to 2 heteroatom(s) selected from the group consisting of N and O,
wherein Rₐ and R_{b} are independently hydrogen, C₁-C₄ straight or branched alkyl, acetyl, C₁-C₄ alkylsulfonyl or C₁-C₆ alkoxycarbonyl,
Z is substituted with Q or not,
Q is C₁-C₆ straight or branched alkyl, hydroxy, halogen, acetyl, nitro, C₁-C₄ alkylsulfonyl, or C₁-C₆ alkoxycarbonyl.

2. The oxazole derivative compound, a hydrate thereof, a solvate thereof or a pharmaceutically acceptable salt thereof of Claim 1, **characterized in that** R₁ is hydrogen, butyl, cyclopropyl, methoxy, F, Cl, trifluoromethyl, trifluoromethoxy, methylthio, hydroxy, cyano, nitro, NRₐR_{b}, phenyl, or naphthyl.

3. The oxazole derivative compound, a hydrate thereof, a solvate thereof or a pharmaceutically acceptable salt thereof of Claim 2, **characterized in that** Rₐ and R_{b} 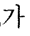 independently hydrogen, methyl, acetyl, or butoxycarbonyl.

4. The oxazole derivative compound, a hydrate thereof, a solvate thereof or a pharmaceutically acceptable salt thereof of Claim 1, **characterized in that** R₂ is hydrogen, methoxy, trifluoromethyl, trifluoromethoxy, methylthio, methylsulfinyl, methylsulfonyl, acetyl, hydroxy, cyano, nitro, NRₐR_{b}, or pyridinyl.

5. The oxazole derivative compound, a hydrate thereof, a solvate thereof or a pharmaceutically acceptable salt thereof of Claim 4, **characterized in that** Rₐ and R_{b} are independently hydrogen, acetyl, or methylsulfonyl.

6. The oxazole derivative compound, a hydrate thereof, a solvate thereof or a pharmaceutically acceptable salt thereof of Claim 1, X is ethoxy, amino(-NH-), phenyl, pyrrolidinyl, piperidinyl, piperazinyl, or morpholino.

7. The oxazole derivative compound, a hydrate thereof, a solvate thereof or a pharmaceutically acceptable salt thereof of Claim 1, **characterized in that** Y is hydrogen, methyl, ethyl, propyl, cyclopropyl, hydroxy, COO, COO-ethyl, phenyl, benzyl, piperidinyl, or pyridinyl.

8. The oxazole derivative compound, a hydrate thereof, a solvate thereof or a pharmaceutically acceptable salt thereof of Claim 1, **characterized in that** Z is ethyl, methoxy, hydroxy, trifluoromethyl, acetyl, guanidino, NRₐR_{b}, phenyl, benzyloxy, pyrrolidinyl, tetrahydrofuranyl, piperidinyl, piperazinyl, morpholino, imidazolyl, or pyridinyl.

9. The oxazole derivative compound, a hydrate thereof, a solvate thereof or a pharmaceutically acceptable salt thereof of Claim 8, **characterized in that** Rₐ and R_{b} are independently hydrogen, methyl, ethyl, propyl, acetyl, methylsulfonyl or butoxycarbonyl.

10. The oxazole derivative compound, a hydrate thereof, a solvate thereof or a pharmaceutically acceptable salt thereof of Claim 1, **characterized in that** the oxazole derivative compound represented by the following Formula I is any one selected from the group consisting of:
ethyl 5-phenyl-2-(2-(trifluoromethyl)phenyl)oxazole-4-carboxylate (27),
ethyl 5-(2-nitrophenyl)-2-(2-(trifluoromethyl)phenyl)oxazole-4-carboxylate (28),
ethyl 5-phenyl-2-(3-(trifluoromethyl)phenyl)oxazole-4-carboxylate (29),
ethyl 5-(2-nitrophenyl)-2-(3-(trifluoromethyl)phenyl)oxazole-4-carboxylate (30),
ethyl 5-phenyl-2-(4-(trifluoromethyl)phenyl)oxazole-4-carboxylate (31),
ethyl 5-(2-nitrophenyl)-2-(4-(trifluoromethyl)phenyl)oxazole-4-carboxylate (32),
ethyl 5-(3-nitrophenyl)-2-(4-(trifluoromethyl)phenyl)oxazole-4-carboxylate (33),
ethyl 5-(4-nitrophenyl)-2-(4-(trifluoromethyl)phenyl)oxazole-4-carboxylate (34),
ethyl 5-(2-acetamidophenyl)-2-(4-(trifluoromethyl)phenyl)oxazole-4-carboxylate (35),
ethyl 5-(3-(methylsulfonyl)phenyl)-2-(4-(trifluoromethyl)phenyl)oxazole-4-carboxylate (36),
ethyl 5-(4-(methylsulfonyl)phenyl)-2-(4-(trifluoromethyl)phenyl)oxazole-4-carboxylate (37),
ethyl 5-(3-acetylphenyl)-2-(4-(trifluoromethyl)phenyl)oxazole-4-carboxylate (38),
ethyl 5-(4-acetylphenyl)-2-(4-(trifluoromethyl)phenyl)oxazole-4-carboxylate (39),
ethyl 5-(3-(methylthio)phenyl)-2-(4-(trifluoromethyl)phenyl)oxazole-4-carboxylate (40),
ethyl 5-(4-(methylthio)phenyl)-2-(4-(trifluoromethyl)phenyl)oxazole-4-carboxylate (41),
ethyl 5-(pyridin-3-yl)-2-(4-(trifluoromethyl)phenyl)oxazole-4-carboxylate (42),
ethyl 5-(pyridin-4-yl)-2-(4-(trifluoromethyl)phenyl)oxazole-4-carboxylate (43),
ethyl 5-(2-methoxyphenyl)-2-(4-(trifluoromethyl)phenyl)oxazole-4-carboxylate (44),
ethyl 5-(3-methoxyphenyl)-2-(4-(trifluoromethyl)phenyl)oxazole-4-carboxylate (45),
ethyl 5-(4-methoxyphenyl)-2-(4-(trifluoromethyl)phenyl)oxazole-4-carboxylate (46),
ethyl 5-(3,4-dimethoxyphenyl)-2-(4-trifluoromethyl)phenyl)oxazole-4-carboxylate (47),
ethyl 5-(3,5-dimethoxyphenyl)-2-(4-trifluoromethyl)phenyl)oxazole-4-carboxylate (48),
ethyl 2-(4-trifluoromethyl)phenyl)-5-(3,4,5-trimethoxyphenyl)oxazole-4-carboxylate (49),
ethyl 5-(2-(trifluoromethyl)phenyl)-2-(4-(trifluoromethyl)phenyl)oxazole-4-carboxylate (50),
ethyl 5-(3-(trifluoromethyl)phenyl)-2-(4-(trifluoromethyl)phenyl)oxazole-4-carboxylate (51),
ethyl 5-(2-(trifluoromethoxy)phenyl)-2-(4-(trifluoromethyl)phenyl)oxazole-4-carboxylate (52),
ethyl 5-(3-(trifluoromethoxy)phenyl)-2-(4-(trifluoromethyl)phenyl)oxazole-4-carboxylate (53),
ethyl 5-(2-cyanophenyl)-2-(4-(trifluoromethyl)phenyl)oxazole-4-carboxylate (54),
ethyl 5-(3-cyanophenyl)-2-(4-(trifluoromethyl)phenyl)oxazole-4-carboxylate (55),
ethyl 5-phenyl-2-(3-(trifluoromethoxy)phenyl)oxazole-4-carboxylate (56),
ethyl 5-(2-nitrophenyl)-2-(3-(trifluoromethoxy)phenyl)oxazole-4-carboxylate (57),
ethyl 5-phenyl-2-(4-(trifluoromethoxy)phenyl)oxazole-4-carboxylate (58),
ethyl 5-(2-nitrophenyl)-2-(4-(trifluoromethoxy)phenyl)oxazole-4-carboxylate (59),
ethyl 2-(3-methoxyphenyl)-5-phenyloxazole-4-carboxylate (60),
ethyl 2-(3-methoxyphenyl)-5-(2-nitrophenyl)oxazole-4-carboxylate (61),
ethyl 2-(4-methoxyphenyl)-5-phenyloxazole-4-carboxylate (62),
ethyl 2-(4-methoxyphenyl)-5-(2-nitrophenyl)oxazole-4-carboxylate (63),
ethyl 2-(4-methoxyphenyl)-5-(4-nitrophenyl)oxazole-4-carboxylate (64),
ethyl 2-(3-fluorophenyl)-5-phenyloxazole-4-carboxylate (65),
ethyl 2-(3-fluorophenyl)-5-(2-nitrophenyl)oxazole-4-carboxylate (66),
ethyl 2-(4-fluorophenyl)-5-phenyloxazole-4-carboxylate (67),
ethyl 2-(4-fluorophenyl)-5-(2-nitrophenyl)oxazole-4-carboxylate (68),
ethyl 2-(4-fluorophenyl)-5-(4-nitrophenyl)oxazole-4-carboxylate (69),
ethyl 2-(3,4-difluorophenyl)-5-phenyloxazole-4-carboxylate (70),
ethyl 2-(3,4-difluorophenyl)-5-(2-nitrophenyl)oxazole-4-carboxylate (71),
ethyl 2-(3,4-difluorophenyl)-5-(4-(methylthio)phenyl)oxazole-4-carboxylate (72),
ethyl 2-(3,5-difluorophenyl)-5-phenyloxazole-4-carboxylate (73),
ethyl 2-(3,5-difluorophenyl)-5-(2-nitrophenyl)oxazole-4-carboxylate (74),
ethyl 2-(3-chlorophenyl)-5-phenyloxazole-4-carboxylate (75),
ethyl 2-(3-chlorophenyl)-5-(2-nitrophenyl)oxazole-4-carboxylate (76),
ethyl 2-(4-chlorophenyl)-5-phenyloxazole-4-carboxylate (77),
ethyl 2-(4-chlorophenyl)-5-(2-nitrophenyl)oxazole-4-carboxylate (78),
ethyl 2-(4-cyanophenyl)-5-phenyloxazole-4-carboxylate (79),
ethyl 2-(4-cyanophenyl)-5-(2-nitrophenyl)oxazole-4-carboxylate (80),
ethyl 2-([1,1'-biphenyl]-4-yl)-5-phenyloxazole-4-carboxylate (81),
ethyl 2-([1,1'-biphenyl]-4-yl)-5-(2-nitrophenyl)oxazole-4-carboxylate (82),
ethyl 2-(naphthalen-2-yl)-5-phenyloxazole-4-carboxylate (83),
ethyl 2-(naphthalen-2-yl)-5-(2-nitrophenyl)oxazole-4-carboxylate (84),
ethyl 2-(4-(dimethylamino)phenyl)-5-phenyloxazole-4-carboxylate (85),
ethyl 2-(4-(dimethylamino)phenyl)-5-(2-nitrophenyl)oxazole-4-carboxylate (86),
ethyl 2-(4-(*tert*-butyl)phenyl)-5-phenyloxazole-4-carboxylate (87),
ethyl 2-(4-(*tert*-butyl)phenyl)-5-(2-nitrophenyl)oxazole-4-carboxylate (88),
ethyl 2-(4-(*tert*-butyl)phenyl)-5-(4-nitrophenyl)oxazole-4-carboxylate (89),
ethyl 2-(4-(methylthio)phenyl)-5-phenyloxazole-4-carboxylate (90),
ethyl 2-(4-(methylthio)phenyl)-5-(2-nitrophenyl)oxazole-4-carboxylate (91),
ethyl 5-(3-methoxyphenyl)-2-(4-(methylthio)phenyl)oxazole-4-carboxylate (92),
ethyl 5-(4-methoxyphenyl)-2-(4-(methylthio)phenyl)oxazole-4-carboxylate (93),
ethyl 2-(4-nitrophenyl)-5-phenyloxazole-4-carboxylate (94),
ethyl 5-(2-nitrophenyl)-2-(4-nitrophenyl)oxazole-4-carboxylate (95),
ethyl 2-(4-((*tert*-butoxycarbonyl)amino)phenyl)-5-(3-(methylthio)phenyl)oxazole-4-carboxylate (96),
ethyl 2-(4-cyclopropylphenyl)-5-phenyloxazole-4-carboxylate (97),
ethyl 2-(4-chloro-3-(trifluoromethyl)phenyl)-5-phenyloxazole-4-carboxylate (98),
ethyl 5-(2-aminophenyl)-2-(4-(trifluoromethyl)phenyl)oxazole-4-carboxylate (99),
ethyl 5-(3-aminophenyl)-2-(4-(trifluoromethyl)phenyl)oxazole-4-carboxylate (100),
ethyl 5-(4-aminophenyl)-2-(4-(trifluoromethyl)phenyl)oxazole-4-carboxylate (101),
ethyl 5-(4-aminophenyl)-2-(4-(*tert*-butyl)phenyl)oxazole-4-carboxylate (102),
ethyl 5-(3-(methylsulfinyl)phenyl)-2-(4-(trifluoromethyl)phenyl)oxazole-4-carboxylate (103),
ethyl 5-(4-(methylsulfinyl)phenyl)-2-(4-(trifluoromethyl)phenyl)oxazole-4-carboxylate (104),
ethyl 2-(4-aminophenyl)-5-(3-(methylthio)phenyl)oxazole-4-carboxylate (105),
ethyl 5-(3-acetamidophenyl)-2-(4-(trifluoromethyl)phenyl)oxazole-4-carboxylate (106),
ethyl 5-(4-acetamidophenyl)-2-(4-(trifluoromethyl)phenyl)oxazole-4-carboxylate (107),
ethyl 5-(3-(N-acetylacetamido)phenyl)-2-(4-(trifluoromethyl)phenyl)oxazole-4-carboxylate (108),
ethyl 5-(4-(N-acetylacetamido)phenyl)-2-(4-(trifluoromethyl)phenyl)oxazole-4-carboxylate (109),
ethyl 5-(4-(N-acetylacetamido)phenyl)-2-(4-(*tert*-butyl)phenyl)oxazole-4-carboxylate (110),
ethyl 5-(4-acetamidophenyl)-2-(4-(*tert*-butyl)phenyl)oxazole-4-carboxylate (111),
ethyl 2-(4-acetamidophenyl)-5-(3-(methylthio)phenyl)oxazole-4-carboxylate(112),
ethyl 5-(4-(methylsulfonamido)phenyl)-2-(4-(trifluoromethyl)phenyl)oxazole-4-carboxylate (113),
N-(2-dimethylamino)ethyl-2-(4-(trifluoromethyl)phenyl)oxazole-4-carboxamide (114),
N-(2-(4-methylpiperazin-1-yl)ethyl)-2-(4-(trifluoromethyl)phenyl)oxazole-4-carboxamide (115),
(4-(2-(dimethylamino)ethyl)piperazin-1-yl)(2-(4-(trifluoromethyl)phenyl)oxazol-4-yl)methanone (116),
N-(2-(dimethylamino)ethyl)-5-phenyl-2-(2-(trifluoromethyl)phenyl)oxazole-4-carboxamide (117),
N-(2-(4-methylpiperazin-1-yl)ethyl)-5-phenyl-2-(2-(trifluoromethyl)phenyl)oxazole-4-carboxamide (118),
N-(2-(dimethylamino)ethyl)-5-(2-nitrophenyl)-2-(2-(trifluoromethyl)phenyl)oxazole-4-carboxamide (119),
N-(2-(4-methylpiperazin-1-yl)ethyl)-5-(2-nitrophenyl)-2-(2-(trifluoromethyl)phenyl)oxazole-4-carboxamide (120),
N-(2-(dimethylamino)ethyl)-5-phenyl-2-(3-(trifluoromethyl)phenyl)oxazole-4-carboxamide (121),
N-(2-(4-methylpiperazin-1 -yl)ethyl)-5-phenyl-2-(3 -(trifluoromethyl)phenyl)oxazole-4-carboxamide (122),
N-(2-(dimethylamino)ethyl)-5-(2-nitrophenyl)-2-(3-(trifluoromethyl)phenyl)oxazole-4-carboxamide (123),
N-(2-(4-methylpiperazin-1-yl)ethyl)-5-(2-nitrophenyl)-2-(3-(trifluoromethyl)phenyl)oxazole-4-carboxamide (124),
(4-(2-(dimethylamino)ethyl)piperazin-1-yl)(5-(2-nitrophenyl)-2-(3-(trifluoromethyl)phenyl)oxazol-4-yl)methanone (125),
N-(2-(dimethylamino)ethyl)-5-phenyl-2-(4-(trifluoromethyl)phenyl)oxazole-4-carboxamide (126),
N-(2-(4-methylpiperazin-1-yl)ethyl)-5-phenyl-2-(4-(trifluoromethyl)phenyl)oxazole-4-carboxamide (127),
N-(2-(4-acetylpiperazin-1-yl)ethyl)-5-phenyl-2-(2-(trifluoromethyl)phenyl)oxazole-4-carboxamide (128),
(4-(2-(dimethylamino)ethyl)piperazin-1-yl)-5-phenyl-2-(4-(trifluoromethyl)phenyl)oxazol-4-yl)methanone (129),
N-(4-Hydroxyphenethyl)-5-phenyl-2-(4-(trifluoromethyl)phenyl)oxazole-4-carboxamide (130),
N-(2-(dimethylamino)ethyl)-5-(2-nitrophenyl)-2-(4-(trifluoromethyl)phenyl)oxazole-4-carboxamide (131),
(5-(2-nitrophenyl)-2-(4-(trifluoromethyl)phenyl)oxazol-4-yl)(piperazin-1-yl)methanone (132),
(4-(2-(dimethylamino)ethyl)piperazin-1-yl)(5-(2-nitrophenyl)-2-(4-(trifluoromethyl)phenyl)oxazol-4-yl)methanone (133),
(4-(2-methoxyphenyl)piperazin-1-yl)(5-(2-nitrophenyl)-2-(4-(trifluoromethyl)phenyl)oxazol-4-yl)methanone (134),
ethyl 4-(5-(2-nitrophenyl)-2-(4-(trifluoromethyl)phenyl)oxazole-4-carbonyl)piperazin-1-carboxylate (135),
4-nitrophenyl4-(5-(2-nitrophenyl)-2-(4-(trifluoromethyl)phenyl)oxazole-4-carbonyl)piperazin-1-carboxylate (136),
2-(dimethylamino)ethyl 4-(5-(2-nitrophenyl)-2-(4-(trifluoromethyl)phenyl)oxazole-4-carbonyl) piperazin-1-carboxylate (137),
(5-(2-nitrophenyl)-2-(4-(trifluoromethyl)phenyl)oxazol-4-yl)(pyrrolidin-1-yl)methanone (138),
5-(2-nitrophenyl)-N-(2-(pyrrolidin-1-yl)ethyl)-2-(4-(trifluoromethyl)phenyl)oxazole-4-carboxamide (139),
5-(2-nitrophenyl)-N-(3-(pyrrolidin-1-yl)propyl)-2-(4-(trifluoromethyl)phenyl)oxazole-4-carboxamide (140),
5-(2-nitrophenyl)-N-(pyridin-4-ylmethyl)-2-(4-(trifluoromethyl)phenyl)oxazole-4-carboxamide (141),
5-(2-nitrophenyl)-N-(pyridin-3-ylmethyl)-2-(4-(trifluoromethyl)phenyl)oxazole-4-carboxamide (142),
5-(2-nitrophenyl)-N-(pyridin-2-yl)-2-(4-(trifluoromethyl)phenyl)oxazole-4-carboxamide (143),
(5-(2-nitrophenyl)-2-(4-(trifluoromethyl)phenyl)oxazol-4-yl)(piperidin-1-yl)methanone (144),
(5-(2-nitrophenyl)-2-(4-(trifluoromethyl)phenyl)oxazol-4-yl)(4-phenylpiperidin-1-yl)methanone (145),
(4-cyclopropylpiperazin-1-yl)(5-(2-nitrophenyl)-2-(4-(trifluoromethyl)phenyl)oxazol-4-yl)methanone (146),
N-(4-acetylphenyl)-5-(2-nitrophenyl)-2-(4-(trifluoromethyl)phenyl)oxazole-4-carboxamide (147),
5-(2-nitrophenyl)-N-(4-(trifluoromethyl)benzyl)-2-(4-(trifluoromethyl)phenyl)oxazole-4-carboxamide (148),
N-(4-fluorophenethyl)-5-(2-nitrophenyl)-2-(4-(trifluoromethyl)phenyl)oxazole-4-carboxamide (149),
(4-(3-(dimethylamino)propyl)piperazin-1-yl)(5-(2-nitrophenyl)-2-(4-(trifluoromethyl)phenyl)oxazol-4-yl)methanone (150),
(5-(2-nitrophenyl)-2-(4-(trifluoromethyl)phenyl)oxazol-4-yl)(4-(2-(piperidin-1-yl)ethyl)piperazin-1-yl)methanone (151),
(4-(2-morpholinoethyl)piperazin-1-yl)(5-(2-nitrophenyl)-2-(4-(trifluoromethyl)phenyl)oxazol-4-yl)methanone (152),
N-(2-(4-methylpiperazin-1-yl)ethyl)-5-(2-nitrophenyl)-2-(4-(trifluoromethyl)phenyl)oxazole-4-carboxamide (153),
(4-(2-(diethylamino)ethyl)piperazin-1-yl)(5-(2-nitrophenyl)-2-(4-(trifluoromethyl)phenyl)oxazol-4-yl)methanone (154),
(4-methylpiperazin-1-yl)(5-(2-nitrophenyl)-2-(4-(trifluoromethyl)phenyl)oxazol-4-yl)methanone (155),
morpholino(5-(2-nitrophenyl)-2-(4-(trifluoromethyl)phenyl)oxazol-4-yl)methanone (156),
N-(3-morpholinopropyl)-5-(2-nitrophenyl)-2-(4-(trifluoromethyl)phenyl)oxazole-4-carboxamide (157),
N-(3-(1H-imidazol-1-yl)propyl)-5-(2-nitrophenyl)-2-(4-(trifluoromethyl)phenyl)oxazole-4-carboxamide (158),
5-(2-nitrophenyl)-N-((tetrahydrofuran-2-yl)methyl)-2-(4-(trifluoromethyl)phenyl)oxazole-4-carboxamide (159),
N-(2-methoxyethyl)-5-(2-nitrophenyl)-2-(4-(trifluoromethyl)phenyl)oxazole-4-carboxamide (160),
(4-hydroxypiperidin-1-yl)(5-(2-nitrophenyl)-2-(4-(trifluoromethyl)phenyl)oxazol-4-yl)methanone (161),
(5-(2-nitrophenyl)-2-(4-(trifluoromethyl)phenyl)oxazol-4-yl)(4-(piperidin-1-yl)phenyl)methanone (162),
ethyl 4-(5-(2-nitrophenyl)-2-(4-(trifluoromethyl)phenyl)oxazole-4-carbonyl)piperazin-1-carboxylate (163),
N-benzyl-5-(2-nitrophenyl)-2-(4-(trifluoromethyl)phenyl)oxazole-4-carboxamide (164),
N-(4-(benzyloxy)phenyl)-5-(2-nitrophenyl)-2-(4-(trifluoromethyl)phenyl)oxazole-4-carboxamide (165),
N-(4-methoxybenzyl)-5-(2-nitrophenyl)-2-(4-(trifluoromethyl)phenyl)oxazole-4-carboxamide (166),
(4-(2-(diisopropylamino)ethyl)piperazin-1-yl)(5-(2-nitrophenyl)-2-(4-(trifluoromethyl)phenyl)oxazol-4-yl)methanone (167),
(4-isopropylpiperazin-1-yl)(5-(2-nitrophenyl)-2-(4-(trifluoromethyl)phenyl)oxazol-4-yl)methanone (168),
(4-(2-hydroxyethyl)piperazin-1-yl)(5-(2-nitrophenyl)-2-(4-(trifluoromethyl)phenyl)oxazol-4-yl)methanone (169),
(5-(2-nitrophenyl)-2-(4-(trifluoromethyl)phenyl)oxazol-4-yl)(4-(2-(pyrrolidin-1-yl)ethyl)piperazin-1-yl)methanone (170),
N-(3-(4-methylpiperazin-1-yl)propyl)-5-(2-nitrophenyl)-2-(4-(trifluoromethyl)phenyl)oxazole-4-carboxamide (171),
*tert*-butyl-4-(2-(5-(2-nitrophenyl)-2-(4-(trifluoromethyl)phenyl)oxazole-4-carboxamido)ethyl)piperazin-1-carboxylate (172),
5-(2-nitrophenyl)-N-(2-(piperidin-1-yl)ethyl)-2-(4-(trifluoromethyl)phenyl)oxazole-4-carboxamide (173),
*tert*-butyl (2-(5-(2-nitrophenyl)-2-(4-(trifluoromethyl)phenyl)oxazole-4-carboxamido)ethyl)carbamate (174),
N-(4-acetamidophenyl)-5-(2-nitrophenyl)-2-(4-(trifluoromethyl)phenyl)oxazole-4-carboxamide (175),
N-(4-Hydroxyphenethyl)-5-(2-nitrophenyl)-2-(4-(trifluoromethyl)phenyl)oxazole-4-carboxamide (176),
N-(2-(dimethylamino)ethyl)-5-(3-nitrophenyl)-2-(4-(trifluoromethyl)phenyl)oxazole-4-carboxamide (177),
(5-(3-nitrophenyl)-2-(4-(trifluoromethyl)phenyl)oxazol-4-yl)(piperazin-1-yl)methanone (178),
(4-(2-(dimethylamino)ethyl)piperazin-1-yl)(5-(3-nitrophenyl)-2-(4-(trifluoromethyl)phenyl)oxazol-4-yl)methanone (179),
N-(2-(dimethylamino)ethyl)-5-(4-nitrophenyl)-2-(4-(trifluoromethyl)phenyl)oxazole-4-carboxamide (180),
(5-(4-nitrophenyl)-2-(4-(trifluoromethyl)phenyl)oxazol-4-yl)(piperazin-1 - yl)methanone (181),
(4-(2-(dimethylamino)ethyl)piperazin-1-yl)(5-(4-nitrophenyl)-2-(4-(trifluoromethyl)phenyl)oxazol-4-yl)methanone (182),
5-(2-aminophenyl)-N-(2-(dimethylamino)ethyl)-2-(4-(trifluoromethyl)phenyl)oxazole-4-carboxamide (183),
(5-(2-aminophenyl)-2-(4-(trifluoromethyl)phenyl)oxazol-4-yl)(4-(2-(dimethylamino)ethyl)piperazin-1-yl)methanone (184),
5-(3-aminophenyl)-N-(2-(dimethylamino)ethyl)-2-(4-(trifluoromethyl)phenyl)oxazole-4-carboxamide (185),
5-(4-aminophenyl)-N-(2-(dimethylamino)ethyl)-2-(4-(trifluoromethyl)phenyl)oxazole-4-carboxamide (186),
5-(2-acetamidophenyl)-N-(2-(dimethylamino)ethyl)-2-(4-(trifluoromethyl)phenyl)oxazole-4-carboxamide (187),
N-(2-(4-(4-(2-(dimethylamino)ethyl)piperazin-1-carbonyl)-2-(4-(trifluoromethyl)phenyl)oxazol-5-yl)phenyl)acetamide (188),
5-(3-acetamidophenyl)-N-(2-(dimethylamino)ethyl)-2-(4-(trifluoromethyl)phenyl)oxazole-4-carboxamide (189),
N-(3-(4-(4-(2-(dimethylamino)ethyl)piperazin-1-carbonyl)-2-(4-(trifluoromethyl)phenyl)oxazol-5-yl)phenyl)acetamide (190),
5-(4-acetamidophenyl)-N-(2-(dimethylamino)ethyl)-2-(4-(trifluoromethyl)phenyl)oxazole-4-carboxamide (191),
N-(4-(4-(4-(2-(dimethylamino)ethyl)piperazin-1-carbonyl)-2-(4-(trifluoromethyl)phenyl)oxazol-5-yl)phenyl)acetamide (192),
5-(4-(methylsulfonyl)phenyl)-N-(pyridin-3-ylmethyl)-2-(4-(trifluoromethyl)phenyl)oxazole-4-carboxamide (193),
N-(2-(4-acetylpiperazin-1-yl)ethyl)-5-(4-(methylthio)phenyl)-2-(4-trifluoromethyl)phenyl)oxazole-4-carboxamide (194),
5-(4-(methylthio)phenyl)-N-(pyridin-3-ylmethyl)-2-(4-(trifluoromethyl)phenyl)oxazole-4-carboxamide (195),
N-(2-(dimethylamino)ethyl)-5-(pyridin-3-yl)-2-(4-(trifluoromethyl)phenyl)oxazole-4-carboxamide (196),
N-(2-(4-acetylpiperazin-1-yl)ethyl)-5-(pyridin-3-yl)-2-(4-(trifluoromethyl)phenyl)oxazole-4-carboxamide (197),
N-(2-(4-acetylpiperazin-1-yl)ethyl)-5-(3-methoxyphenyl)-2-(4-(trifluoromethyl)phenyl)oxazole-4-carboxamide (198),
N-(2-(dimethylamino)ethyl)-5-phenyl-2-(3-(trifluoromethoxy)phenyl)oxazole-4-carboxamide (199),
N-(2-(4-methylpiperazin-1-yl)ethyl)-5-phenyl-2-(3-(trifluoromethoxy)phenyl)oxazole-4-carboxamide (200),
N-(2-(dimethylamino)ethyl)-5-(2-nitrophenyl)-2-(3-(trifluoromethoxy)phenyl)oxazole-4-carboxamide (201),
N-(2-(4-methylpiperazin-1-yl)ethyl)-5-(2-nitrophenyl)-2-(3-(trifluoromethoxy)phenyl)oxazole-4-carboxamide (202),
N-(2-(dimethylamino)ethyl)-5-phenyl-2-(4-(trifluoromethoxy)phenyl)oxazole-4-carboxamide (203),
N-(2-(4-methylpiperazin-1-yl)ethyl)-5-phenyl-2-(4-(trifluoromethoxy)phenyl)oxazole-4-carboxamide (204),
N-(2-(dimethylamino)ethyl)-5-(2-nitrophenyl)-2-(4-(trifluoromethoxy)phenyl)oxazole-4-carboxamide (205),
N-(2-(4-methylpiperazin-1-yl)ethyl)-5-(2-nitrophenyl)-2-(4-(trifluoromethoxy)phenyl)oxazole-4-carboxamide (206),
(4-(2-(dimethylamino)ethyl)piperazin-1-yl)(5-(2-nitrophenyl)-2-(4-(trifluoromethoxy)phenyl)oxazol-4-yl)methanone (207),
N-(2-(dimethylamino)ethyl)-2-(3-methoxyphenyl)-5-phenyloxazole-4-carboxamide (208),
2-(3-methoxyphenyl)-N-(2-(4-methylpiperazin-1-yl)ethyl)-5-phenyloxazole-4-carboxamide (209),
N-(2-(dimethylamino)ethyl)-2-(3-methoxyphenyl)-5-(2-nitrophenyl)oxazole-4-carboxamide (210),
2-(3-methoxyphenyl)-N-(2-(4-methylpiperazin-1-yl)ethyl)-5-(2-nitrophenyl)oxazole-4-carboxamide (211),
N-(2-(dimethylamino)ethyl)-2-(4-methoxyphenyl)-5-phenyloxazole-4-carboxamide (212),
2-(4-methoxyphenyl)-N-(2-(4-methylpiperazin-1-yl)ethyl)-5-phenyloxazole-4-carboxamide (213),
N-(2-(dimethylamino)ethyl)-2-(4-methoxyphenyl)-5-(2-nitrophenyl)oxazole-4-carboxamide (214),
2-(4-methoxyphenyl)-N-(2-(4-methylpiperazin-1-yl)ethyl)-5-(2-nitrophenyl)oxazole-4-carboxamide (215),
(4-(2-(dimethylamino)ethyl)piperazin-1-yl)(2-(4-methoxyphenyl)-5-(2-nitrophenyl)oxazol-4-yl)methanone (216),
N-(2-(dimethylamino)ethyl)-2-(4-methoxyphenyl)-5-(4-nitrophenyl)oxazole-4-carboxamide (217),
(4-(2-(dimethylamino)ethyl)piperazin-1-yl)(2-(4-methoxyphenyl)-5-(4-nitrophenyl)oxazol-4-yl)methanone (218),
2-(4-methoxyphenyl)-N-(2-(4-methylpiperazin-1-yl)ethyl)-5-(4-nitrophenyl)oxazole-4-carboxamide (219),
N-(2-(dimethylamino)ethyl)-2-(3-fluorophenyl)-5-phenyloxazole-4-carboxamide (220),
2-(3-fluorophenyl)N-(2-(4-methylpiperazin-1-yl)ethyl)-5-phenyloxazole-4-carboxamide (221),
N-(2-(dimethylamino)ethyl)-2-(3-fluorophenyl)-5-(2-nitrophenyl)oxazole-4-carboxamide (222),
2-(3-fluorophenyl)-N-(2-(4-methylpiperazin-1-yl)ethyl)-5-(2-nitrophenyl)oxazole-4-carboxamide (223),
N-(2-(dimethylamino)ethyl)-2-(4-fluorophenyl)-5-phenyloxazole-4-carboxamide (224),
2-(4-fluorophenyl)-N-(2-(4-methylpiperazin-1-yl)ethyl)-5-phenyloxazole-4-carboxamide (225),
N-(2-(dimethylamino)ethyl)-2-(4-fluorophenyl)-5-(2-nitrophenyl)oxazole-4-carboxamide (226),
2-(4-fluorophenyl)-N-(2-(4-methylpiperazin-1-yl)ethyl)-5-(2-nitrophenyl)oxazole-4-carboxamide (227),
(4-(2-(dimethylamino)ethyl)piperazin-1-yl)(2-(4-fluorophenyl)-5-(2-nitrophenyl)oxazol-4-yl)methanone (228),
N-(2-(dimethylamino)ethyl)-2-(4-fluorophenyl)-5-(4-nitrophenyl)oxazole-4-carboxamide (229),
2-(4-fluorophenyl)-N-(2-(4-methylpiperazin-1-yl)ethyl)-5-(4-nitrophenyl)oxazole-4-carboxamide (230),
2-(3,4-difluorophenyl)-N-(2-(dimethylamino)ethyl)-5-phenyloxazole-4-carboxamide (231),
2-(3,4-difluorophenyl)-N-(2-(4-methylpiperazin-1-yl)ethyl)-5-phenyloxazole-4-carboxamide (232),
2-(3,4-difluorophenyl)-N-(2-(dimethylamino)ethyl)-5-(2-nitrophenyl)oxazole-4-carboxamide (233),
2-(3,4-difluorophenyl)-N-(2-(4-methylpiperazin-1-yl)ethyl)-5-(2-nitrophenyl)oxazole-4-carboxamide (234),
2-(3,5-difluorophenyl)-N-(2-(dimethylamino)ethyl)-5-phenyloxazole-4-carboxamide (235),
2-(3,5-difluorophenyl)-N-(2-(4-methylpiperazin-1-yl)ethyl)-5-phenyloxazole-4-carboxamide (236),
2-(3,5-difluorophenyl)-N-(2-(dimethylamino)ethyl)-5-(2-nitrophenyl)oxazole-4-carboxamide (237),
2-(3,5-difluorophenyl)-N-(2-(4-methylpiperazin-1-yl)ethyl)-5-(2-nitrophenyl)oxazole-4-carboxamide (238),
tert-butyl 4-(2-(2-(3,5-difluorophenyl)-5-(2-nitrophenyl)oxazole-4-carboxamido)ethyl)piperazin-1-carboxylate (239),
2-(3-chlorophenyl)-N-(2-(dimethylamino)ethyl)-5-phenyloxazole-4-carboxamide (240),
2-(3-chlorophenyl)-N-(2-(4-methylpiperazin-1-yl)ethyl)-5-phenyloxazole-4-carboxamide (241),
2-(3-chlorophenyl)-N-(2-(dimethylamino)ethyl)-5-(2-nitrophenyl)oxazole-4-carboxamide (242),
2-(3-chlorophenyl)-N-(2-(4-methylpiperazin-1-yl)ethyl)-5-(2-nitrophenyl)oxazole-4-carboxamide (243),
2-(4-chlorophenyl)-N-(2-(dimethylamino)ethyl)-5-phenyloxazole-4-carboxamide (244),
2-(4-chlorophenyl)-N-(2-(4-methylpiperazin-1-yl)ethyl)-5-phenyloxazole-4-carboxamide (245),
2-(4-chlorophenyl)-N-(2-(dimethylamino)ethyl)-5-(2-nitrophenyl)oxazole-4-carboxamide (246),
2-(4-(chlorophenyl)-N-(2-(4-methylpiperazin-1-yl)ethyl)-5-(2-nitrophenyl)oxazole-4-carboxamide (247),
(2-(4-chlorophenyl)-5-(2-nitrophenyl)oxazol-4-yl)(4-(2-(dimethylamino)ethyl)piperazin-1-yl)methanone (248),
2-(4-cyanophenyl)-N-(2-(dimethylamino)ethyl)-5-phenyloxazole-4-carboxamide (249),
2-(4-cyanophenyl)-N-(2-(4-methylpiperazin-1-yl)ethyl)-5-phenyloxazole-4-carboxamide (250),
2-(4-cyanophenyl)-N-(2-(dimethylamino)ethyl)-5-(2-nitrophenyl)oxazole-4-carboxamide (251),
2-(4-cyanophenyl)-N-(2-(4-methylpiperazin-1-yl)ethyl)-5-(2-nitrophenyl)oxazole-4-carboxamide (252),
2-([1,1'-biphenyl]-4-yl)-N-(2-(dimethylamino)ethyl)-5-phenyloxazole-4-carboxamide (253),
2-([1,1'-biphenyl]-4-yl)-N-2-(4-methylpiperazin-1-yl)ethyl)-5-phenyloxazole-4-carboxamide (254),
2-([1,1'-biphenyl]-4-yl)-N-(2-(dimethylamino)ethyl)-5-(2-nitrophenyl)oxazole-4-carboxamide (255),
2-([1,1'-biphenyl]-4-yl)-N-(2-(4-methylpiperazin-1-yl)ethyl)-5-(2-nitrophenyl)oxazole-4-carboxamide (256),
N-(2-(dimethylamino)ethyl)-2-(naphthalen-2-yl)-5-phenyloxazole-4-carboxamide (257),
N-(2-(4-methylpiperazin-1-yl)ethyl)-2-(naphthalen-2-yl)-5-phenyloxazole-4-carboxamide (258),
N-(2-(dimethylamino)ethyl)-2-(naphthalen-2-yl)-5-(2-nitrophenyl)oxazole-4-carboxamide (259),
N-(2-(4-methylpiperazin-1-yl)ethyl)-2-(naphthalen-2-yl)-5-(2-nitrophenyl)oxazole-4-carboxamide (260),
N-(2-(dimethylamino)ethyl)-2-(4-(dimethylamino)phenyl)-5-phenyloxazole-4-carboxamide (261),
2-(4-(dimethylamino)phenyl)-N-(2-(4-methylpiperazin-1-yl)ethyl)-5-phenyloxazole-4-carboxamide (262),
N-(2-(dimethylamino)ethyl)-2-(4-(dimethylamino)phenyl)-5-(2-nitrophenyl)oxazole-4-carboxamide (263),
2-(4-(dimethylamino)phenyl)-N-(2-(4-methylpiperazin-1-yl)ethyl)-5-(2-nitrophenyl)oxazole-4-carboxamide (264),
2-(4-(*tert-*butyl)phenyl)-N-(2-(dimethylamino)ethyl)-5-phenyloxazole-4-carboxamide (265),
2-(4-(*tert*-butyl)phenyl)-N-(2-(4-methylpiperazin-1-yl)ethyl)-5-phenyloxazole-4-carboxamide (266),
2-(4-(*tert*-butyl)phenyl)-N-(2-(diethylamino)ethyl)-5-phenyloxazole-4-carboxamide (267),
2-(4-(*tert*-butyl)phenyl)-N-(2-(dimethylamino)ethyl)-5-(2-nitrophenyl)oxazole-4-carboxamide (268),
2-(4-(*tert*-butyl)phenyl)-N-(2-(4-methylpiperazin-1-yl)ethyl)-5-(2-nitrophenyl)oxazole-4-carboxamide (269),
2-(4-(*tert*-butyl)phenyl)-N-(2-(diethylamino)ethyl)-5-(2-nitrophenyl)oxazole-4-carboxamide (270),
N-(2-(dimethylamino)ethyl)-2-(4-(methylthio)phenyl)-5-phenyloxazole-4-carboxamide (271),
N-(2-(4-methylpiperazin-1-yl)ethyl)-2-(4-(methylthio)phenyl)-5-phenyloxazole-4-carboxamide (272),
N-(2-(dimethylamino)ethyl)-2-(4-(methylthio)phenyl)-5-(2-nitrophenyl)oxazole-4-carboxamide (273),
N-(2-(4-methylpiperazin-1-yl)ethyl)-2-(4-(methylthio)phenyl)-5-(2-nitrophenyl)oxazole-4-carboxamide (274),
N-(2-(dimethylamino)ethyl)-2-(4-nitrophenyl)-5-phenyloxazole-4-carboxamide (275),
N-(2-(4-methylpiperazin-1-yl)ethyl)-2-(4-nitrophenyl)-5-phenyloxazole-4-carboxamide (276),
N-(2-(dimethylamino)ethyl)-5-(2-nitrophenyl)-2-(4-nitrophenyl)oxazole-4-carboxamide (277),
N-(2-(4-methylpiperazin-1-yl)ethyl)-5-(2-nitrophenyl)-2-(4-nitrophenyl)oxazole-4-carboxamide (278),
2-(4-cyclopropylphenyl)-N-(2-(dimethylamino)ethyl)-5-phenyloxazole-4-carboxamide (279),
2-(4-cyclopropylphenyl)-N-(2-(4-methylpiperazin-1-yl)ethyl)-5-phenyloxazole-4-carboxamide (280),
5-(2-nitrophenyl)-N-(2-(piperazin-1-yl)ethyl)-2-(4-(trifluoromethyl)phenyl)oxazole-4-carboxamide (281),
N-(2-aminoethyl)-5-(2-nitrophenyl)-2-(4-(trifluoromethyl)phenyl)oxazole-4-carboxamide (282),
2-(3,5-difluorophenyl)-5-(2-nitrophenyl)-N-(2-(piperazin-1-yl)ethyl)oxazole-4-carboxamide (283),
N-(2-(4-acetylpiperazin-1-yl)ethyl)-5-(2-nitrophenyl)-2-(4-(trifluoromethyl)phenyl)oxazole-4-carboxamide (284),
N-(2-acetamidoethyl)-5-(2-nitrophenyl)-2-(4-(trifluoromethyl)phenyl)oxazole-4-carboxamide (285),
N-(2-(4-acetylpiperazin-1-yl)ethyl)-2-(3,5-difluorophenyl)-5-(2-nitrophenyl)oxazole-4-carboxamide (286),
N-(2-(4-(methylsulfonyl)piperazin-1-yl)ethyl)-5-(2-nitrophenyl)-2-(4-(trifluoromethyl)phenyl)oxazole-4-carboxamide (287),
N-(2-(methylsulfonamido)ethyl)-5-(2-nitrophenyl)-2-(4-(trifluoromethyl)phenyl)oxazole-4-carboxamide (288),
N-(2-guanidinoethyl)-5-(2-nitrophenyl)-2-(4-(trifluoromethyl)phenyl)oxazole-4-carboxamide (289),
N-methyl-5-(3-(methylthio)phenyl)-2-(4-(trifluoromethyl)phenyl)oxazole-4-carboxamide (290),
N-ethyl-5-(3-(methylthio)phenyl)-2-(4-(trifluoromethyl)phenyl)oxazole-4-carboxamide (291),
5-(3-(methylthio)phenyl)-N-propyl-2-(4-(trifluoromethyl)phenyl)oxazole-4-carboxamide (292),
N-(2-methoxyethyl)-5-(3-(methylthio)phenyl)-2-(4-(trifluoromethyl)phenyl)oxazole-4-carboxamide (293),
N-ethyl-5-phenyl-2-(4-(trifluoromethyl)phenyl)oxazole-4-carboxamide (294),
5-phenyl-N-propyl-2-(4-(trifluoromethyl)phenyl)oxazole-4-carboxamide (295),
N-ethyl-5-(2-methoxyphenyl)-2-(4-(trifluoromethyl)phenyl)oxazole-4-carboxamide (296),
5-(2-methoxyphenyl)-N-propyl-2-(4-(trifluoromethyl)phenyl)oxazole-4-carboxamide (297),
N-ethyl-5-(3-methoxyphenyl)-2-(4-(trifluoromethyl)phenyl)oxazole-4-carboxamide (298),
5-(3-methoxyphenyl)-N-propyl-2-(4-(trifluoromethyl)phenyl)oxazole-4-carboxamide (299),
N-ethyl-5-(4-methoxyphenyl)-2-(4-(trifluoromethyl)phenyl)oxazole-4-carboxamide (300),
5-(4-methoxyphenyl)-N-propyl-2-(4-(trifluoromethyl)phenyl)oxazole-4-carboxamide (301),
5-(3,4-dimethoxyphenyl)-N-ethyl-2-(4-(trifluoromethyl)phenyl)oxazole-4-carboxamide (302),
5-(3,5-dimethoxyphenyl)-N-ethyl-2-(4-(trifluoromethyl)phenyl)oxazole-4-carboxamide (303),
N-ethyl-2-(4-(trifluoromethyl)phenyl)-5-(3,4,5-trimethoxyphenyl)oxazole-4-carboxamide (304),
N-ethyl-5-(2-(trifluoromethoxy)phenyl)-2-(4-(trifluoromethyl)phenyl)oxazole-4-carboxamide (305),
N-propyl-5-(2-(trifluoromethoxy)phenyl)-2-(4-(trifluoromethyl)phenyl)oxazole-4-carboxamide (306),
N-ethyl-5-(pyridin-3-yl)-2-(4-(trifluoromethyl)phenyl)oxazole-4-carboxamide (307),
N-propyl-5-(pyridin-3-yl)-2-(4-(trifluoromethyl)phenyl)oxazole-4-carboxamide (308),
N-ethyl-5-(pyridin-4-yl)-2-(4-(trifluoromethyl)phenyl)oxazole-4-carboxamide (309),
N-propyl-5-(pyridin-4-yl)-2-(4-(trifluoromethyl)phenyl)oxazole-4-carboxamide (310),
5-(3-cyanophenyl)-N-ethyl-2-(4-(trifluoromethyl)phenyl)oxazole-4-carboxamide (311),
N-ethyl-2-(4-methoxyphenyl)-5-phenyloxazole-4-carboxamide (312),
2-(3,5-difluorophenyl)-N-ethyl-5-phenyloxazole-4-carboxamide (313),
2-(3-chlorophenyl)-N-ethyl-5-phenyloxazole-4-carboxamide (314),
2-(4-cyanophenyl)-N-ethyl-5-phenyloxazole-4-carboxamide (315),
N-ethyl-2-(4-(methylthio)phenyl)-5-phenyloxazole-4-carboxamide (316),
2-(4-(methylthio)phenyl)-5-phenyl-N-propyloxazole-4-carboxamide (317),
N-ethyl-5-(3-hydroxyphenyl)-2-(4-(trifluoromethyl)phenyl)oxazole-4-carboxamide (318),
5-(3-hydroxyphenyl)-N-propyl-2-(4-(trifluoromethyl)phenyl)oxazole-4-carboxamide (319),
N-ethyl-5-(4-hydroxyphenyl)-2-(4-(trifluoromethyl)phenyl)oxazole-4-carboxamide (320),
5-(3,4-dihydroxyphenyl)-N-ethyl-2-(4-(trifluoromethyl)phenyl)oxazole-4-carboxamide (321),
5-(3,5-dihydroxyphenyl)-N-ethyl-2-(4-(trifluoromethyl)phenyl)oxazole-4-carboxamide (322),
N-ethyl-2-(4-(trifluoromethyl)phenyl)-5-(3,4,5-trihydroxyphenyl)oxazole-4-carboxamide (323), and
N-ethyl-2-(4-hydroxyphenyl)-5-phenyloxazole-4-carboxamide (324).

11. A pharmaceutical composition for preventing or treating a disease associated with IL-33, comprising the oxazole derivative compound, a hydrate thereof, a solvate thereof or a pharmaceutically acceptable salt thereof of any one of Claims 1-10 as an active ingredient.

12. A pharmaceutical composition for preventing or treating an allergic disease, comprising the oxazole derivative compound, a hydrate thereof, a solvate thereof or a pharmaceutically acceptable salt thereof of any one of Claims 1-10 as an active ingredient.

13. A pharmaceutical composition for preventing or treating one of more diseases selected from the group consisting of one or more allergic diseases selected from asthma, allergic rhinitis, chronic sinusitis, allergic contact dermatitis, atopic dermatitis, chronic spontaneous urticaria and anaphylaxis; one or more autoimmune diseases selected from Graves' disease, Sjögren's syndrome, immune thrombocytopenia, autoimmune hemolytic anemia, inflammatory bowel disease and primary biliary cholangitis; and chronic obstructive pulmonary disease, comprising the oxazole derivative compound, a hydrate thereof, a solvate thereof or a pharmaceutically acceptable salt thereof of any one of Claims 1-10 as an active ingredient.

14. A pharmaceutical composition comprising the oxazole derivative compound, a hydrate thereof, a solvate thereof or a pharmaceutically acceptable salt thereof of any one of Claims 1-10 and a pharmaceutically acceptable additive.

15. A food composition for improving symptoms of allergic diseases, comprising the oxazole derivative compound, a hydrate thereof, a solvate thereof or a pharmaceutically acceptable salt thereof of any one of Claims 1-10.
